(19) Europäisches Patentamt / European Patent Office / Office européen des brevets

(11) **EP 2 813 493 A1**

(12) **EUROPEAN PATENT APPLICATION**
published in accordance with Art. 153(4) EPC


(43) Date of publication:
**17.12.2014 Bulletin 2014/51**

(21) Application number: **13746514.2**

(22) Date of filing: **05.02.2013**

(51) Int Cl.:
***C07D 307/14*** $^{(2006.01)}$ ***A01N 43/16*** $^{(2006.01)}$
***A01N 43/32*** $^{(2006.01)}$ ***A01P 7/04*** $^{(2006.01)}$
***C07D 309/04*** $^{(2006.01)}$ ***C07D 319/06*** $^{(2006.01)}$
***C07D 319/12*** $^{(2006.01)}$ ***C07D 335/02*** $^{(2006.01)}$

(86) International application number:
**PCT/JP2013/053167**

(87) International publication number:
**WO 2013/118907 (15.08.2013 Gazette 2013/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.02.2012 JP 2012027002**



(71) Applicant: **Sumitomo Chemical Company Limited**
**Tokyo 104-8260 (JP)**

(72) Inventors:
- **IHARA, Hideki**
**Osaka-shi**
**Osaka 555-0021 (JP)**
- **AWANO, Tomotsugu**
**Takarazuka-shi**
**Hyogo 665-8555 (JP)**
- **OHSHITA, Jun**
**Takarazuka-shi**
**Hyogo 665-8555 (JP)**
- **MATSUO, Noritada**
**Takarazuka-shi**
**Hyogo 665-8555 (JP)**

(74) Representative: **Nieuwenhuys, William Francis**
**Marks & Clerk LLP**
**90 Long Acre**
**London**
**WC2E 9RA (GB)**


(54) **AMIDE COMPOUND AND USE THEREOF FOR PEST CONTROL**


(57) An amide compound represented by formula (I) has an excellent pest control effect. (In the formula, Y represents a 3-7 membered saturated heterocyclic ring which contains, as ring-forming component(s), one or more atoms or groups that are selected from the group consisting of an oxygen atom and -S(O)$_t$-, the saturated heterocyclic ring may have one to three atoms or groups selected from group D and t represents 0 or the like; X represents a $C_1$-$C_{10}$ chain hydrocarbon group which may have one or more atoms or groups that are selected from group A, or the like; W represents -$CR^8$- or the like; r represents 1 or the like; $R^1$, $R^2$, $R^3$, $R^4$, $R^5$, $R^6$, $R^7$ and $R^8$ may be the same or different and each represents a hydrogen atom or the like; and n represents 1 or the like.)


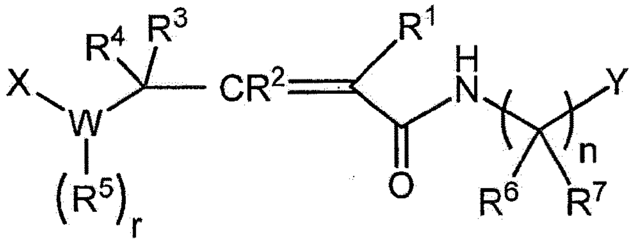

(I)

EP 2 813 493 A1

## Description

TECHNICAL FIELD

The present invention relates to an amide compound and a use thereof for pest control.

BACKGROUND ART

Conventionally, many pest control agents have been developed for controlling pests, and put to practical use. In addition, a certain type of amide compound is described in The Journal of Organic Chemistry, 1945, 10, pages 236 to 242.

SUMMARY OF THE INVENTION

PROBLEMS TO BE SOLVED BY THE INVENTION

MEANS FOR SOLVING THE PROBLEMS

The present invention provides a novel compound having an excellent control effect on pests.
More specifically, the present invention is as described below.

[1] An amide compound represented by formula (I)

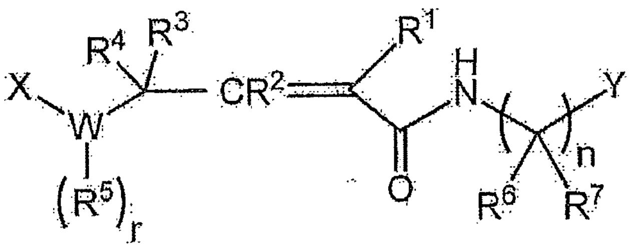

(I)

wherein

Y represents a 3 to 7-membered saturated heterocyclic ring which contains, as ring-forming component(s), one or more atoms or groups that are selected from the group consisting of an oxygen atom and $-S(O)_t-$, and the saturated heterocyclic ring may have one to three atoms or groups selected from group D,
t represents 0, 1 or 2,
X represents a C1 to C10 chain hydrocarbon group optionally having one or more atoms or groups that are selected from group A or a cyclopropyl group optionally having one or more groups selected from group B,
W represents $-CR^8-$, an oxygen atom or $-S(O)_u-$,
when W is $-CR^8-$, r represents 1,
when W is an oxygen atom or $-S(O)_u-$, r represents 0,
u represents 0, 1 or 2,
$R^8$ represents a C1 to C4 alkyl group optionally having one or more halogen atoms, a halogen atom or a hydrogen atom,
$R^1$ and $R^2$ are the same or different and each represents a C1 to C4 alkyl group optionally having one or more halogen atoms, a C1 to C4 alkoxy group optionally having one or more halogen atoms, a halogen atom or a hydrogen atom,
$R^4$ represents a C1 to C4 alkyl group optionally having one or more halogen atoms, a halogen atom or a hydrogen atom,
$R^3$ and $R^5$ are the same or different and each represents a C1 to C4 alkyl group optionally having one or more halogen atoms, a halogen atom or a hydrogen atom, or $R^3$ and $R^5$ are taken together to represent $-(CR^9R^{10})_v-$, $-NR^{11}-$, an oxygen atom or a sulfur atom,
$R^9$ and $R^{10}$ are the same or different and each represents a C1 to C4 alkyl group optionally having one or more halogen atoms, a halogen atom or a hydrogen atom,
$R^{11}$ represents a C1 to C4 alkyl group optionally having one or more halogen atoms or a hydrogen atom,
v represents 1, 2, 3 or 4,

$R^6$ and $R^7$ are the same or different and each represents a C1 to C4 alkyl group optionally having one or more halogen atoms or a hydrogen atom,
n represents 0, 1 or 2,
Group D: A group consisting of C1 to C4 alkyl groups optionally having one or more halogen atoms, C1 to C4 alkoxy groups optionally having one or more halogen atoms and halogen atoms.
Group A: A group consisting of C1 to C4 alkoxy groups optionally having one or more halogen atoms, C1 to C4 alkylthio groups optionally having one or more halogen atoms, C1 to C4 alkylsulfinyl groups optionally having one or more halogen atoms, C1 to C4 alkylsulfonyl groups optionally having one or more halogen atoms, C1 to C4 alkoxycarbonyl groups optionally having one or more halogen atoms, C1 to C4 alkoxyimino groups optionally having one or more halogen atoms, $-CONR^{12}R^{13}$ group, a carboxyl group and halogen atoms.
$R^{12}$ and $R^{13}$ are the same or different and are a C1 to C4 alkyl group optionally having one or more halogen atoms or a hydrogen atom.
Group B: A group consisting of C1 to C8 chain hydrocarbon groups optionally having one or more atoms or groups selected from group A.
(hereinafter, described as the compound of the present invention).

[2] The amide compound according to [1], wherein Y is represented by the following formula (II-a) or (II-b)

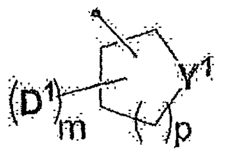 (II-a) 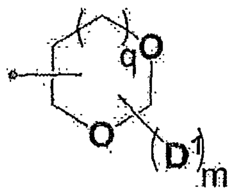 (II-b)

wherein $Y^1$ represents an oxygen atom or a sulfur atom, $D^1$ represents an atom or a group selected from group D, m represents 0 or 1, p represents 0, 1 or 2, and q is 0 or 1.
[3] The amide compound according to [2], wherein p is 1, or q is 0.
[4] The amide compound according to [2], wherein p is 2, or q is 1.
[5] The amide compound according to any of [2] to [4], wherein $Y^1$ is an oxygen atom.
[6] The amide compound according to [2], wherein Y is a group represented by the formula (II-a), and $Y^1$ is an oxygen atom.
[7] The amide compound according to [2], wherein Y is a group represented by the formula (II-b).
[8] The amide compound according to any of [1] to [7], wherein
W is $-CR^8-$, r is 1, $R^1$ is a halogen atom or a hydrogen atom,
$R^2$ is a C1 to C4 alkyl group optionally having one or more halogen atoms or a hydrogen atom,
$R^9$ is a C1 to C4 alkyl group optionally having one or more halogen atoms, a halogen atom or a hydrogen atom,
$R^3$ and $R^5$ are the same or different and are a C1 to C4 alkyl group optionally having one or more halogen atoms, a halogen atom or a hydrogen atom, or $R^3$ and $R^5$ are taken together to be $-(CR^9R^{10})_v-$, v is 1, $R^9$ and $R^{10}$ are the same or different and are a C1 to C4 alkyl group optionally having one or more halogen atoms, a halogen atom or a hydrogen atom,
$R^8$ is a hydrogen atom, and
$R^6$ and $R^7$ are the same or different and are a C1 to C4 alkyl group optionally having one or more halogen atoms or a hydrogen atom.
[9] The amide compound according to any of [1] to [7], wherein
W is $-CR^8-$, r is 1, $R^7$ is a halogen atom or a hydrogen atom,
$R^2$ is a C1 to C4 alkyl group optionally having one or more halogen atoms or a hydrogen atom,
$R^4$ is a C1 to C4 alkyl group optionally having one or more halogen atoms, a halogen atom or a hydrogen atom,
$R^3$ and $R^5$ are the same or different and are a C1 to C4 alkyl group optionally having one or more halogen atoms, a halogen atom or a hydrogen atom,
$R^8$ is a hydrogen atom, and
$R^6$ and $R^7$ are the same or different and are a C1 to C4 alkyl group optionally having one or more halogen atoms or a hydrogen atom.
[10] The amide compound according to any of [1] to [7], wherein
W is $-CR^8-$, r is 1, $R^1$ is a halogen atom or a hydrogen atom,
$R^2$ is a C1 to C4 alkyl group optionally having one or more halogen atoms or a hydrogen atom,
$R^4$ is a C1 to C4 alkyl group optionally having one or more halogen atoms, a halogen atom or a hydrogen atom,

$R^3$ and $R^5$ are taken together to be $-(CR^9R^{10})_v-$, v is 1, $R^9$ and $R^{10}$ are the same or different and are a C1 to C4 alkyl group optionally having one or more halogen atoms, a halogen atom or a hydrogen atom,
$R^8$ is a hydrogen atom, and
$R^6$ and $R^7$ are the same or different and are a C1 to C4 alkyl group optionally having one or more halogen atoms or a hydrogen atom.
[11] The amide compound according to any of [1] to [10], wherein
X is a C1 to C5 alkyl group optionally having one or more atoms or groups selected from group A, a C2 to C5 alkenyl group optionally having one or more atoms or groups selected from group A, or a C2 to C5 alkynyl group optionally having one or more atoms or groups selected from group A.
[12] The amide compound according to any of [1] to [10], wherein X is a C1 to C5 alkyl group, a C2 to C5 alkenyl group or a C2 to C5 alkynyl group.
[13] A pest control agent containing the amide compound as defined in any of [1] to [12] and an inert carrier.
[14] A method for controlling pests including the step of applying an effective amount of the amide compound as defined in any of [1] to [12] to a pest or a place where a pest inhabits.

MODE FOR CARRYING OUT THE INVENTION

The compound of the present invention may include an isomer derived from an asymmetric carbon atom, and an isomer derived from a double bond present at position 2 of the carbonyl group or the like, and the present invention includes each isomer having a pest control activity and an isomeric mixture at an arbitrary ratio.

In the compound of the present invention, "position 2 of the carbonyl group" means a position of the carbon atom represented by "2" in the following formula.

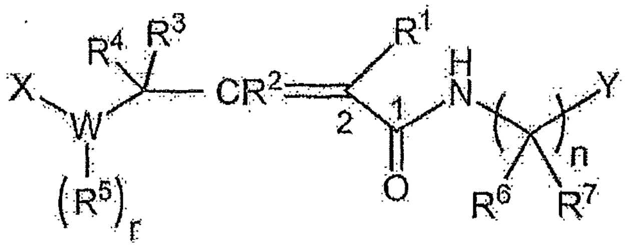

In the present invention, examples of the "a 3 to 7-membered saturated heterocyclic ring which contains, as ring-forming component (s), one or more atoms or groups selected from the group consisting of an oxygen atom and $-S(O)_t-$ (herein, the saturated heterocyclic ring may have one to three atoms or groups selected from group D.)" include 3 to 7-membered saturated heterocyclic rings which contain, as ring-forming component(s), one or more oxygen atoms and optionally having one to three atoms or groups selected from group D, such as an oxetan-2-yl group, an oxetan-3-yl group, a tetrahydrofuran-2-yl group, a tetrahydrofuran-3-yl group, a 3-methyltetrahydrofuran-3-yl group, a 1,3-dioxolan-2-yl group, an 2-ethyl-1,3-dioxolan-2-yl group, a 1,3-dioxolan-4-yl group, a tetrahydropyran-2-yl group, a tetrahydropyran-3-yl group, a tetrahydropyran-4-yl group, a 1,3-dioxan-4-yl group, a 1,3-dioxan-5-yl group, a 1, 4-dioxan-2-yl group, an oxepan-2-yl group, an oxepan-3-yl group, an oxepan-4-yl group, a 1,3-dioxepan-4-yl group, a 1,3-dioxepan-5-yl group, a 1,4-dioxepan-2-yl group, a 1,4-dioxepan-5-yl group and a 1,4-dioxepan-6-yl group; 3 to 7-membered saturated heterocyclic rings which contain, as ring-forming component(s), one or more sulfur atoms and optionally having one to three atoms or groups selected from group D, such as a thietan-2-yl group, a thietan-3-yl group, a tetrahydrothiophen-2-yl group, a tetrahydrothiophen-3-yl group, a 1,3-dithiolan-4-yl group, a tetrahydrothiopyran-2-yl group, a tetrahydrothiopyran-3-yl group, a tetrahydrothiopyran-4-yl group, a 1,3-dithian-4-yl group, a 1,3-dithian-5-yl group, a 1,4-dithian-2-yl group, a thiepan-2-yl group, a thiepan-3-yl group, a thiepan-4-yl group, a 1,3-dithiepan-4-yl group, a 1,3-dithiepan-5-yl group, a 1,4-dithiepan-2-yl group, a 1,4-dithiepan-5-yl group and a 1,4-dithiepan-6-yl group; 3 to 7-membered saturated heterocyclic rings which contains, as ring-forming component(s), one or more -SO- and optionally having one to three atoms or groups selected from group D, such as an 1-oxo-thietan-2-yl group, an 1-oxo-thietan-3-yl group, an 1-oxo-tetrahydrothiophen-2-yl group, an 1-oxo-tetrahydrothiophen-3-yl group, an 1-oxo-tetrahydrothiopyran-2-yl group, an 1-oxo-tetrahydrothiopyran-3-yl group, an 1-oxo-tetrahydrothiopyran-4-yl group, an 1-oxo-thiepan-2-yl group, an 1-oxo-thiepan-3-yl group and an 1-oxo-thiepan-4-yl group; 3 to 7-membered saturated heterocyclic rings which contains, as ring-forming component(s), one or more $-SO_2-$ and optionally having one to three atoms or groups selected from group D, such as a 1,1-dioxo-thietan-2-yl group, a 1,1-dioxo-thietan-3-yl group, a 1,1-dioxo-tetrahydrothiophen-2-yl group, a 1,1-dioxo-tetrahydrothiophen-3-yl group, a 1,1-dioxo-tetrahydrothiopyran-2-yl group, a 1,1-dioxo-tetrahydrothiopyran-3-yl group, a 1,1-dioxo-tetrahydrothiopyran-4-yl group, a 1,1-dioxo-thiepan-2-yl group, a 1,1-dioxe-thiepan-3-yl group and a 1,1-dioxo-thiepan-4-yl group; and 3 to 7-membered saturated heterocyclic rings which contains, as ring-forming component(s), one or more oxygen atoms and one or more -$S(O)_t$- and optionally having one to three atoms or groups selected from group D, such as an 1,3-oxathietan-2-yl group, an 1,3-oxathiolan-2-yl group, an 1,3-oxathiolan-4-yl group, an 1,3-oxathiolan-5-yl group, an 1,3-oxathian-2-yl group, an 1,3-oxathiolan-4-yl group, an 1,3-oxathiolan-5-yl group, an 1,4-oxathian-2-yl group and an 1,4-oxathian-3-yl group.

Examples of the term "halogen atom" in the present invention include a fluorine atom, a chlorine atom, a bromine atom, and an iodine atom.

Examples of the phrase "a C1 to C4 alkyl group optionally having one or more halogen atoms" in the present invention include a methyl group, an ethyl group, a propyl group, an isopropyl group, a butyl group, an isobutyl group, a sec-butyl group, a tert-butyl group, a chloromethyl group, a difluoromethyl group, a trifluoromethyl group, a trichloromethyl group, a 2,2,2-trifluoroethyl group, a 1,1,2,2-tetrafluoroethyl group and a 1,1,2,2,2-pentafluoroethyl group.

Examples of the phrase "a C1 to C4 alkoxy groups optionally having one or more halogen atoms" in the present invention include a methoxy group, an ethoxy group, a propoxy group, an isopropoxy group, a butoxy group, a difluoromethoxy group, a trifluoromethoxy group, a trichloromethoxy group, a 2,2,2-trifluoroethoxy group, a 1,1,2,2-tetrafluoroethoxy group and a 1,1,2,2,2-pentafluoroethoxy group.

Examples of the phrase "a C1 to C4 alkylthio group optionally having one or more halogen atoms" in the present invention include a methylthio group, an ethylthio group, a propylthio group, an isopropylthio group, a butylthio group, a difluoromethylthio group, a trifluoromethylthio group, a trichloromethylthio group, a 2,2,2-trifluoroethylthio group, a 1,1,2,2-tetrafluoroethylthio group and a 1,1,2,2,2-pentafluoroethylthio group.

Examples of the phrase "a C1 to C4 alkylsulfinyl group optionally having one or more halogen atoms" in the present invention include a methylsulfinyl group, an ethylsulfinyl group, a propylsulfinyl group, an isopropylsulfinyl group, a butylsulfinyl group, a difluoromethylsulfinyl group, a trifluoromethylsulfinyl group, a trichloromethylsulfinyl group, a 2,2,2-trifluoroethylsulfinyl group, a 1,1,2,2-tetrafluoroethylsulfinyl group and a 1,1,2,2,2-pentafluoroethylsulfinyl group.

Examples of the phrase "a C1 to C4 alkylsulfonyl group optionally having one or more halogen atoms" in the present invention include a methylsulfonyl group, an ethylsulfonyl group, a propylsulfonyl group, an isopropylsulfonyl group, a butylsulfonyl group, a difluoromethylsulfonyl group, a trifluoromethylsulfonyl group, a trichloromethylsulfonyl group, a 2,2,2-trifluoroethylsulfonyl group, a 1,1,2,2-tetrafluoroethylsulfonyl group and a 1,1,2,2,2-pentafluoroethylsulfonyl group.

Examples of the phrase "a C1 to C4 alkoxycarbonyl group optionally having one or more halogen atoms" in the present invention include a methoxycarbonyl group, an ethoxycarbonyl group, a propoxycarbonyl group, an isopropoxycarbonyl group, a butoxycarbonyl group, a tert-butoxycarbonyl group, a difluoromethoxycarbonyl group, a trifluoromethoxycarbonyl group, a trichloromethoxycarbonyl group, a 2,2,2-trifluoroethoxycarbonyl group, a 1,1,2,2-tetrafluoroethoxycarbonyl group and a 1,1,2,2,2-pentafluoroethoxycarbonyl group.

Examples of the phrase "a C1 to C4 alkoxyimino group optionally having one or more halogen atoms" in the present invention include a methoxyimino group, an ethoxyimino group, a propoxyimino group, an isopropoxyimino group, a butoxyimino group, a tert-butoxyimino group, a difluoromethoxyimino group, a trifluoromethoxyimino group, a trichloromethoxyimino group, a 2,2,2-trifluoroethoxyimino group, a 1,1,2,2-tetrafluoroethoxyimino group and a 1,1,2,2,2-pentafluoroethoxyimino group.

Examples of the phrase "C1 to C8 chain hydrocarbon groups optionally having one or more atoms or groups selected from group A" represented by group B in the present invention include:

"C1 to C8 alkyl groups optionally having one or more atoms or groups selected from group A" such as a methyl group, an ethyl group, an isopropyl group, a tert-butyl group, a propyl group, a sec-butyl group, an isobutyl group, a 1,1-dimethylpropyl group, a 2,2-dimethylpropyl group, a 1, 2-dimethylpropyl group, an 1-ethylpropyl group, a butyl group, a 1-methylbutyl group, a 2-methylbutyl group, a 3-methylbutyl group, a 1,1-dimethylbutyl group, a 2,2-dimethylbutyl group, a 3,3-dimethylbutyl group, a 1,2,3-trimethylbutyl group, an 1-ethylbutyl group, an 2-ethylbutyl group, a pentyl group, a 1-methylpentyl group, a 2-methylpentyl group, a 3-methylpentyl group, a 4-methylpentyl group, a 1,1-dimethylpentyl group, a 2,2-dimethylpentyl group, a 3,3-dimethylpentyl group, a 4,4-dimethylpentyl group, an 1-ethylpentyl group, an 2-ethylpentyl group, an 3-ethylpentyl group, a 1-propylpentyl group, a 2-propylpentyl group, a hexyl group, a 1-methylhexyl group, a 2-methylhexyl group, a 3-methylhexyl group, a 4-methylhexyl group, a 5-methylhexyl group, a 1,1-dimethylhexyl group, a 2,2-dimethylhexyl group, a 3,3-dimethylhexyl group, a 4,4-dimethylhexyl group, a 5,5-dimethylhexyl group, an 1-ethylhexyl group, an 2-ethylhexyl group, an 3-ethylhexyl group, an 4-ethylhexyl group, a heptyl group, a 1-methylheptyl group, a 2-methylheptyl group, a 3-methylheptyl group, a 4-methylheptyl group, a 5-methylheptyl group, a 6-methylheptyl group, an octyl group, a trifluoromethyl group, a 2,2,2-trifluoroethyl group, a 3,3,3-trifluoropropyl group, a 4,4,4-trifluorobutyl group, a 5,5,5-trifluoropentyl group, a 6,6,6-trifluorohexyl group, a 7,7,7-trifluoroheptyl group, a 8,8,8-trifluorooctyl group, a methoxymethyl group, a 2-methoxyethyl group, a 3-methoxypropyl group, a 4-methoxybutyl group, a 5-methoxypentyl group, a 6-methoxyhexyl group, a 7-methoxyheptyl group, a 8-methoxyoctyl group, a trifluoromethoxymethyl group, a 2-trifluoromethoxyethyl group, a 3-trifluoromethoxypropyl group, a 4-trifluoromethoxybutyl group, a 5-trifluoromethoxypentyl group, a 6-trifluoromethoxyhexyl group, a 7-trifluoromethoxyheptyl group, a 8-trifluoromethoxyoctyl group, a 2,2,2-trifluoroethoxymethyl group, a 2-(2,2,2-trifluoroethoxy)ethyl group, a 3-(2,2,2-trifluoroethoxy)propyl group, a 4-(2,2,2-trifluoroethoxy)butyl group, a 5-(2,2,2-trifluoroethoxy)pentyl group, a 6-(2,2,2-trifluoroethoxy)hexyl group, a 7-(2,2,2-trifluoroethoxy)heptyl group, a 8-(2,2,2-trifluoroethoxy)octyl group, a methylthiomethyl group, a 2-methylthioethyl group, a 3-methylthiopropyl group, a 4-methylthiobutyl group, a 5-methylthiopentyl group, a 6-methylthiohexyl group, a 7-methylthioheptyl group, a 8-methylthiooctyl group, a methylsulfinylmethyl group, a 2-methylsulfinylethyl group, a 3-methylsulfinylpropyl group, a 4-methylsulfinylbutyl group, a 5-methylsulfinylpentyl group, a 6-methylsulfinylhexyl group, a 7-methylsulfinylheptyl group, a 8-methylsulfinyloctyl group, a methylsulfonylmethyl group, a 2-methylsulfonylethyl group, a 3-methylsulfonylpropyl group, a 4-methylsulfonylbutyl group, a 5-methylsulfonylpentyl group, a 6-methylsulfonylhexyl group, a 7-methylsulfonylheptyl group, a 8-methylsulfonyloctyl group, a methylcarbonylmethyl group, a 2-methylcarbonylethyl group, a 3-methylcarbonylpropyl group, a 4-methylcarbonylbutyl group, a 5-methylcarbonylpentyl group, a 6-methylcarbonylhexyl group, a 7-methylcarbonylheptyl group, a 8-methylcarbonyloctyl group, an N-methylcarboamidemethyl group, a 2-(N-methylcarboamide)ethyl group, a 3-(N-methylcarboamide)propyl group, a 4-(N-methylcarboamide)butyl group, a 5-(N-methylcarboamide)pentyl group, a 6-(N-methylcarboamide)hexyl group, a 7-(N-methylcarboamide)heptyl group, a 8-(N-methylcarboamide)octyl group, an N,N-dimethylcarboamidemethyl group, a 2-(N,N-dimethylcarboamide)ethyl group, a 3-(N,N-dimethylcarboamide)propyl group, a 4-(N,N-dimethylcarboamide)butyl group, a 5-(N,N-dimethylcarboamide)pentyl group, a 6-(N,N-dimethylcarboamide)hexyl group, a 7-(N,N-dimethylcarboamide)heptyl group, a 8-(N,N-dimethylcarboamide)octyl group, a methoxyiminomethyl group, a 2-methoxyiminoethyl group, a 3-methoxyiminopropyl group, a 4-methoxyiminobutyl group, a 5-methoxyiminopentyl group, a 6-methoxyiminohexyl group, a 7-methoxyiminoheptyl group, a 8-methoxyiminooctyl group, a carboxylmethyl group, a 2-carboxylethyl group, a 3-carboxylpropyl group, a 4-carboxylbutyl group, a 5-carboxylpentyl group, a 6-carboxylhexyl group, a 7-carboxylheptyl group and a 8-carboxyloctyl group;

"C2 to C8 alkenyl groups optionally having one or more atoms or groups selected from group A" such as a vinyl group, a 1-propenyl group, a 2-propenyl group, a 1-methyl-1-propenyl group, a 1-methyl-2-propenyl group, a 1,2-dimethyl-1-propenyl group, a 1,1-dimethyl-2-propenyl group, an 1-ethyl-1-propenyl group, an 1-ethyl-2-propenyl group, a 1-butenyl group, a 2-butenyl group, a 3-butenyl group, a 1-methyl-1-butenyl group, a 2-methyl-1-butenyl group, a 2-methyl-2-butenyl group, a 2-methyl-3-butenyl group, a 3-methyl-3-butenyl group, a 1,1-dimethyl-3-butenyl group, a 1,1-dimethyl-2-butenyl group, an 1-ethyl-1-butenyl group, an 1-ethyl-2-butenyl group, an 1-ethyl-3-butenyl group, a 1-pentenyl group, a 2-pentenyl group, a 3-pentenyl group, a 4-pentenyl group, a 1-methyl-1-pentenyl group, a 1-methyl-2-pentenyl group, a 1-methyl-3-pentenyl group, a 1-methyl-4-pentenyl group, a 2-methyl-1-pentenyl group, a 2-methyl-2-pentenyl group, a 3-methyl-2-pentenyl group, a 3-methyl-3-pentenyl group, a 4-methyl-3-pentenyl group, a 4-methyl-4-pentenyl group, a 1,1-dimethyl-2-pentenyl group, a 1,1-dimethyl-3-pentenyl group, a 1,1-dimethyl-4-pentenyl group, an 1-ethyl-1-pentenyl group, an 1-ethyl-2-pentenyl group, an 1-ethyl-3-pentenyl group, an 1-ethyl-4-pentenyl group, a 1-hexenyl group, a 2-hexenyl group, a 3-hexenyl group, a 4-hexenyl group, a 5-hexenyl group, a 1-methyl-1-hexenyl group, a 1-methyl-2-hexenyl group, a 1-methyl-3-hexenyl group, a 1-methyl-4-hexenyl group, a 1-methyl-5-hexenyl group, a 2-methyl-1-hexenyl group, a 2-methyl-2-hexenyl group, a 3-methyl-2-hexenyl group, a 3-methyl-3-hexenyl group, a 4-methyl-3-hexenyl group, a 4-methyl-4-hexenyl group, a 5-methyl-4-hexenyl group, a 5-methyl-5-hexenyl group, a 1,1-dimethyl-2-hexenyl group, a 1,1-dimethyl-3-hexenyl group, a 1,1-dimethyl-4-hexenyl group, a 1,1-dimethyl-5-hexenyl group, an 1-ethyl-1-hexenyl group, an 1-ethyl-2-hexenyl group, an 1-ethyl-3-hexenyl group, an 1-ethyl-4-hexenyl group, an 1-ethyl-5-hexenyl group, a 1-heptenyl group, a 2-heptenyl group, a 3-heptenyl group, a 4-heptenyl group, a 5-heptenyl group, a 6-heptenyl group, a 1-methyl-1-heptenyl group, a 1-methyl-2-heptenyl group, a 1-methyl-3-heptenyl group, a 1-methyl-4-heptenyl group, a 1-methyl-5-heptenyl group, a 1-methyl-6-heptenyl group, a 2-methyl-1-heptenyl group, a 2-methyl-2-heptenyl group, a 3-methyl-2-heptenyl group, a 3-methyl-3-heptenyl group, a 4-methyl-3-heptenyl group, a 4-methyl-4-heptenyl group, a 5-methyl-4-heptenyl group, a 5-methyl-5-heptenyl group, a 6-methyl-5-heptenyl group, a 6-methyl-6-heptenyl group, an 1-octenyl group, an 2-octenyl group, an 3-octenyl group, an 4-octenyl group, an 5-octenyl group, an 6-octenyl group, an 7-octenyl group, a propen-2-yl group, a 1-buten-2-yl group, a 1-buten-3-yl group, a 1-penten-2-yl group, a 1-penten-3-yl group, a 1-hexen-2-yl group, a 1-hexen-3-yl group, a 1-hepten-2-yl group, a 1-hepten-3-yl group, an 1-octen-2-yl group, an 1-octen-3-yl group, a propen-2-ylmethyl group, a 1-buten-2-ylmethyl group, a 1-buten-3-ylmethyl group, a 1-penten-2-ylmethyl group, a 1-penten-3-ylmethyl group, a 1-hexen-2-ylmethyl group, a 1-hexen-3-ylmethyl group, a 1-hepten-2-ylmethyl group, a 1-hepten-3-ylmethyl group, a propen-2-ylethyl group, a 1-buten-2-ylethyl group, a 1-buten-3-ylethyl group, a 1-penten-2-ylethyl group, a 1-penten-3-ylethyl group, a 1-hexen-2-ylethyl group, a 1-hexen-3-ylethyl group, a propen-2-ylpropyl group, a 1-buten-2-ylpropyl group, a 1-buten-3-ylpropyl group, a 1-penten-2-ylpropyl group, a 1-penten-3-ylpropyl group, a propen-2-ylbutyl group, a 1-buten-2-ylbutyl group, a 1-penten-2-ylbutyl group, a 1-penten-3-ylbutyl group, a propen-2-ylpentyl group, a 1-buten-2-ylpentyl group, a 2,2-difluorovinyl group, a 3,3-difluoro-2-propenyl group, a 4,4-difluoro-3-butenyl group, a 5,5-difluoro-4-pentenyl group, a 6,6-difluoro-5-hexenyl group, a 7,7-difluoro-6-heptenyl group, a 8,8-difluoro-7-octenyl group, a 2-methoxyvinyl group, a 3-methoxy-1-propenyl group, a 3-methoxy-2-propenyl group, a 4-methoxy-1-butenyl group,

a 4-methoxy-2-butenyl group, a 4-methoxy-3-butenyl group, a 5-methoxy-1-pentenyl group, a 5-methoxy-2-pentenyl group, a 5-methoxy-3-pentenyl group, a 5-methoxy-4-pentenyl group, a 6-methoxy-1-hexenyl group, a 6-methoxy-2-hexenyl group, a 6-methoxy-3-hexenyl group, a 6-methoxy-4-hexenyl group, a 6-methoxy-5-hexenyl group, a 7-methoxy-1-heptenyl group, a 7-methoxy-2-heptenyl group, a 7-methoxy-3-heptenyl group, a 7-methoxy-4-heptenyl group, a 7-methoxy-5-heptenyl group, a 7-methoxy-6-heptenyl group, a 8-methoxy-1-octenyl group, a 8-methoxy-2-octenyl group, a 8-methoxy-3-octenyl group, a 8-methoxy-4-octenyl group, a 8-methoxy-5-octenyl group, a 8-methoxy-6-octenyl group, a 8-methoxy-7-octenyl group, a 2-methylthiovinyl group, a 3-methylthio-1-propenyl group, a 3-methylthio-2-propenyl group, a 4-methylthio-1-butenyl group, a 4-methylthio-2-butenyl group, a 4-methylthio-3-butenyl group, a 5-methylthio-1-pentenyl group, a 5-methylthio-2-pentenyl group, a 5-methylthio-3-pentenyl group, a 5-methylthio-4-pentenyl group, a 6-methylthio-1-hexenyl group, a 6-methylthio-2-hexenyl group, a 6-methylthio-3-hexenyl group, a 6-methylthio-4-hexenyl group, a 6-methylthio-5-hexenyl group, a 7-methylthio-1-heptenyl group, a 7-methylthio-2-heptenyl group, a 7-methylthio-3-heptenyl group, a 7-methylthio-4-heptenyl group, a 7-methylthio-5-heptenyl group, a 7-methylthio-6-heptenyl group, a 8-methylthio-1-octenyl group, a 8-methylthio-2-octenyl group, a 8-methylthio-3-octenyl group, a 8-methylthio-4-octenyl group, a 8-methylthio-5-octenyl group, a 8-methylthio-6-octenyl group, a 8-methylthio-7-octenyl group, a 2-methoxycarbonylvinyl group, a 3-methoxycarbonyl-1-propenyl group, a 3-methoxycarbonyl-2-propenyl group, a 4-methoxycarbonyl-1-butenyl group, a 4-methoxycarbonyl-2-butenyl group, a 4-methoxycarbonyl-3-butenyl group, a 5-methoxycarbonyl-1-pentenyl group, a 5-methoxycarbonyl-2-pentenyl group, a 5-methoxycarbonyl-3-pentenyl group, a 5-methoxycarbonyl-4-pentenyl group, a 6-methoxycarbonyl-1-hexenyl group, a 6-methoxycarbonyl-2-hexenyl group, a 6-methoxycarbonyl-3-hexenyl group, a 6-methoxycarbonyl-4-hexenyl group, a 6-methoxycarbonyl-5-hexenyl group, a 7-methoxycarbonyl-1-heptenyl group, a 7-methoxycarbonyl-2-heptenyl group, a 7-methoxycarbonyl-3-heptenyl group, a 7-methoxycarbonyl-4-heptenyl group, a 7-methoxycarbonyl-5-heptenyl group, a 7-methoxycarbonyl-6-heptenyl group, a 8-methoxycarbonyl-1-octenyl group, a 8-methoxycarbonyl-2-octenyl group, a 8-methoxycarbonyl-3-octenyl group, a 8-methoxycarbonyl-4-octenyl group, a 8-methoxycarbonyl-5-octenyl group, a 8-rnethoxycarbonyl-6-octenyl group, a 8-methoxycarbonyl-7-octenyl group, a 2-(N-methylcarboamide)vinyl group, a 3-(N-methylcarboamide)-1-propenyl group, a 3-(N-methylcarboamide)-2-propenyl group, a 4-(N-methylcarboamide)-1-butenyl group, a 4-(N-methylcarboamide)-2-butenyl group, a 4-(N-methylcarboamide)-3-butenyl group, a 5-(N-methylcarboamide)-1-pentenyl group, a 5-(N-methylcarboamide)-2-pentenyl group, a 5-(N-methylcarboamide)-3-pentenyl group, a 5-(N-methylcarboamide)-4-pentenyl group, a 6-(N-methylcarboamide)-1-hexenyl group, a 6-(N-methylcarboamide)-2-hexenyl group, a 6-(N-methylcarboamide)-3-hexenyl group, a 6-(N-methylcarboamide)-4-hexenyl group, a 6-(N-methylcarboamide)-5-hexenyl group, a 7-(N-methylcarboamide)-1-heptenyl group, a 7-(N-methylcarboamide)-2-heptenyl group, a 7-(N-methylcarboamide)-3-heptenyl group, a 7-(N-methylcarboamide)-4-heptenyl group, a 7-(N-methylcarboamide)-5-heptenyl group, a 7-(N-methylcarboamide)-6-heptenyl group, a 8-(N-methylcarboamide)-1-octenyl group, a 8-(N-methylcarboamide)-2-octenyl group, a 8-(N-methylcarboamide)-3-octenyl group, a 8-(N-methylcarboamide)-4-octenyl group, a 8-(N-methylcarboamide)-5-octenyl group, a 8-(N-methylcarboamide)-6-octenyl group, a 8-(N-methylcarboamide)-7-octenyl group, a 2-(N,N-dimethylcarboamide)vinyl group, a 3-(N,N-dimethylcarboamide)-1-propenyl group, a 3-(N,N-dimethylcarboamide)-2-propenyl group, a 4-(N,N-dimethylcarboamide)-1-butenyl group, a 4-(N,N-dimethylcarboamide)-2-butenyl group, a 4-(N,N-dimethylcarboamide)-3-butenyl group, a 5-(N,N-dimethylcarboamide)-1-pentenyl group, a 5-(N,N-dimethylcarboamide)-2-pentenyl group, a 5-(N,N-dimethylcarboamide)-3-pentenyl group, a 5-(N,N-dimethylcarboamide)-4-pentenyl group, a 6-(N,N-dimethylcarboamide)-1-hexenyl group, a 6-(N,N-dimethylcarboamide)-2-hexenyl group, a 6-(N,N-dimethylcarboamide)-3-hexenyl group, a 6-(N,N-dimethylcarboamide)-4-hexenyl group, a 6-(N,N-dimethylcarboamide)-5-hexenyl group, a 7-(N,N-dimethylcarboamide)-1-heptenyl group, a 7-(N,N-dimethylcarboamide)-2-heptenyl group, a 7-(N,N-dimethylcarboamide)-3-heptenyl group, a 7-(N,N-dimethylcarboamide)-4-heptenyl group, a 7-(N,N-dimethylcarboamide)-5-heptenyl group, a 7-(N,N-dimethylcarboamide)-6-heptenyl group, a 8-(N,N-dimethylcarboamide)-1-octenyl group, a 8-(N,N-dimethylcarboamide)-2-octenyl group, a 8-(N,N-dimethylcarboamide)-3-octenyl group, a 8-(N,N-dimethylcarboamide)-4-octenyl group, a 8-(N,N-dimethylcarboamide)-5-octenyl group, a 8-(N,N-dimethylcarboamide)-6-octenyl group, a 8-(N,N-dimethylcarboamide)-7-octenyl group, a 3-methoxyimino-1-propenyl group, a 4-methoxyimino-1-butenyl group, a 4-methoxyimino-2-butenyl group, a 5-methoxyimino-1-pentenyl group, a 5-methoxyimino-2-pentenyl group, a 5-methoxyimino-3-pentenyl group, a 6-methoxyimino-1-hexenyl group, a 6-methoxyimino-2-hexenyl group, a 6-methoxyimino-3-hexenyl group, a 6-methoxyimino-4-hexenyl group, a 7-methoxyimino-1-heptenyl group, a 7-methoxyimino-2-heptenyl group, a 7-methoxyimino-3-heptenyl group, a 7-methoxyimino-4-heptenyl group, a 7-methoxyimino-5-heptenyl group, a 8-methoxyimino-1-octenyl group, a 8-methoxyimino-2-octenyl group, a 8-methoxyimino-3-octenyl group, a 8-methoxyimino-4-octenyl group, a 8-methoxyimino-5-octenyl group, a 8-methoxyimino-6-octenyl group, a 2-carboxylvinyl group, a 3-carboxyl-1-propenyl group, a 3-carboxyl-2-propenyl group, a 4-carboxyl-1-butenyl group, a 4-carboxyl-2-butenyl group, a 4-carboxyl-3-butenyl group, a 5-carboxyl-1-pentenyl group, a 5-carboxyl-2-pentenyl group, a 5-carboxyl-3-pentenyl group, a 5-carboxyl-4-pentenyl group, a 6-carboxyl-1-hexenyl group, a 6-carboxyl-2-hexenyl group, a 6-carboxyl-3-hexenyl group, a 6-carboxyl-4-hexenyl group, a 6-carboxyl-5-hexenyl group, a 7-carboxyl-1-heptenyl

group, a 7-carboxyl-2-heptenyl group, a 7-carboxyl-3-heptenyl group, a 7-carboxyl-4-heptenyl group, a 7-carboxyl-5-heptenyl group, a 7-carboxyl-6-heptenyl group, a 8-carboxyl-1-octenyl group, a 8-carboxyl-2-octenyl group, a 8-carboxyl-3-octenyl group, a 8-carboxyl-4-octenyl group, a 8-carboxyl-5-octenyl group, a 8-carboxyl-6-octenyl group, a 8-carboxyl-7-octenyl group, a 1,3-butadienyl group, a 2,4-pentadienyl group, a 3,5-hexadienyl group, a 4,6-heptadienyl group, an 5,7-octadienyl group, a 4,6-heptadienyl group, an 5,7-octadienyl group, a 4,4-difluoro-1,3-butadienyl group, a 5,5-difluoro-2,4-pentadienyl group, a 6,6-difluoro-3,5-hexadienyl group, a 7,7-difluoro-4,6-heptadienyl group, a 8,8-difluoro-5,7-octadienyl group, a 1,1-difluoro-1-hepten-3-yl group, a 1,1-difluoro-1-hexen-3-ylmethyl group, a 5,5-difluoro-3-ethyl-4-pentenyl group, a 6,6-difluoro-4-methyl-5-hexenyl group, a 1,1-difluoro-1-octen-3-yl group, a 1,1-difluoro-1-hepten-3-ylethyl group, a 1,1-difluoro-1-hexen-3-ylethyl group, a 6,6-difluoro-4-ethyl-5-hexenyl group and a 7,7-difluoro-5-methyl-6-heptenyl group;

"C2 to C8 alkynyl groups optionally having one or more atoms or groups selected from group A" such as an ethynyl group, a 1-propynyl group, a 2-propynyl group, a 1-methyl-2-propynyl group, a 1,1-dimethyl-2-propynyl group, a 1-ethyl-2-propynyl group, a 1-butynyl group, a 2-butynyl group, a 3-butynyl group, a 1-methyl-2-butynyl group, a 1-methyl-3-butynyl group, a 1,1-dimethyl-2-butynyl group, a 1,1-dimethyl-3-butynyl group, an 1-ethyl-2-butynyl group, an 1-ethyl-3-butynyl group, a 1-pentynyl group, a 2-pentynyl group, a 3-pentynyl group, a 4-pentynyl group, a 1-methyl-2-pentynyl group, a 1-methyl-3-pentynyl group, a 1-methyl-4-pentynyl group, a 1,1-dimethyl-2-pentynyl group, a 1,1-dimethyl-3-pentynyl group, a 1,1-dimethyl-4-pentynyl group, an 1-ethyl-2-pentynyl group, an 1-ethyl-3-pentynyl group, an 1-ethyl-4-pentynyl group, a 1-hexynyl group, a 2-hexynyl group, a 3-hexynyl group, a 4-hexynyl group, a 5-hexynyl group, a 1-methyl-2-hexynyl group, a 1-methyl-3-hexynyl group, a 1-methyl-4-hexynyl group, a 1-methyl-5-hexynyl group, a 1,1-dimethyl-2-hexynyl group, a 1,1-dimethyl-3-hexynyl group, a 1,1-dimethyl-4-hexynyl group, a 1,1-dimethyl-5-hexynyl group, an 1-ethyl-2-hexynyl group, an 1-ethyl-3-hexynyl group, an 1-ethyl-4-hexynyl group, an 1-ethyl-5-hexynyl group, a 1-heptynyl group, a 2-heptynyl group, a 3-heptynyl group, a 4-heptynyl group, a 5-heptynyl group, a 6-heptynyl group, a 1-methyl-2-heptynyl group, a 1-methyl-3-heptynyl group, a 1-methyl-4-heptynyl group, a 1-methyl-5-heptynyl group, a 1-methyl-6-heptynyl group, an 1-octynyl group, an 2-octynyl group, an 3-octynyl group, an 4-octynyl group, an 5-octynyl group, an 6-octynyl group, an 7-octynyl group, a 1-butyn-3-yl group, a 1-pentyn-3-yl group, a 1-hexyn-3-yl group, a 1-heptyn-3-yl group, an 1-octyn-3-yl group, a 1-butyn-3-ylmethyl group, a 1-pentyn-3-ylmethyl group, a 1-hexyn-3-ylmethyl group, a 1-heptyn-3-ylmethyl group, a 1-butyn-3-ylpropyl group, a 1-pentyn-3-ylpropyl group, a 1-butyn-3-ylbutyl group, a 1-pentyn-3-ylbutyl group, a 2-fluoroethynyl group, a 3-fluoro-2-propynyl group, a 4-fluoro-3-butynyl group, a 5-fluoro-4-pentynyl group, a 6-fluoro-5-hexynyl group, a 7-fluoro-6-heptynyl group, a 8-fluoro-3-octynyl group, a 2-methoxyethynyl group, a 3-methoxy-1-propynyl group, a 3-methoxy-2-propynyl group, a 4-methoxy-1-butynyl group, a 4-methoxy-2-butynyl group, a 4-methoxy-3-butynyl group, a 5-methoxy-1-pentynyl group, a 5-methoxy-2-pentynyl group, a 5-methoxy-3-pentynyl group, a 5-methoxy-4-pentynyl group, a 6-methoxy-1-hexynyl group, a 6-methoxy-2-hexynyl group, a 6-methoxy-3-hexynyl group, a 6-methoxy-4-hexynyl group, a 6-methoxy-5-hexynyl group, a 7-methoxy-1-heptynyl group, a 7-methoxy-2-heptynyl group, a 7-methoxy-3-heptynyl group, a 7-methoxy-4-heptynyl group, a 7-methoxy-5-heptynyl group, a 7-methoxy-6-heptynyl group, a 8-methoxy-1-octynyl group, a 8-methoxy-2-octynyl group, a 8-methoxy-3-octynyl group, a 8-methoxy-4-octynyl group, a 8-methoxy-5-octynyl group, a 8-methoxy-6-octynyl group, a 8-methoxy-7-octynyl group, a 2-methylthioethynyl group, a 3-methylthio-1-propynyl group, a 3-methylthio-2-propynyl group, a 4-methylthio-1-butynyl group, a 4-methylthio-2-butynyl group, a 4-methylthio-3-butynyl group, a 5-methylthio-1-pentynyl group, a 5-methylthio-2-pentynyl group, a 5-methylthio-3-pentynyl group, a 5-methylthio-4-pentynyl group, a 6-methylthio-1-hexynyl group, a 6-methylthio-2-hexynyl group, a 6-methylthio-3-hexynyl group, a 6-methylthio-4-hexynyl group, a 6-methylthio-5-hexynyl group, a 7-methylthio-1-heptynyl group, a 7-methylthio-2-heptynyl group, a 7-methylthio-3-heptynyl group, a 7-methylthio-4-heptynyl group, a 7-methylthio-5-heptynyl group, a 7-methylthio-6-heptynyl group, a 8-methylthio-1-octynyl group, a 8-methylthio-2-octynyl group, a 8-methylthio-3-octynylgroup, a 8-methylthio-4-octynyl group, a 8-methylthio-5-octynyl group, a 8-methylthio-6-octynyl group, a 8-methylthio-7-octynyl group, a 2-methoxycarbonylethynyl group, a 3-methoxycarbonyl-1-propynyl group, a 3-methoxycarbonyl-2-propynyl group, a 4-methoxycarbonyl-1-butynyl group, a 4-methoxycarbonyl-2-butynyl group, a 4-methoxycarbonyl-3-butynyl group, a 5-methoxycarbonyl-1-pentynyl group, a 5-methoxycarbonyl-2-pentynyl group, a 5-methoxycarbonyl-3-pentynyl group, a 5-methoxycarbonyl-4-pentynyl group, a 6-methoxycarbonyl-1-hexynyl group, a 6-methoxycarbonyl-2-hexynyl group, a 6-methoxycarbonyl-3-hexynyl group, a 6-methoxycarbonyl-4-hexynyl group, a 6-methoxycarbonyl-5-hexynyl group, a 7-methoxycarbonyl-1-heptynyl group, a 7-methoxycarbonyl-2-heptynyl group, a 7-methoxycarbonyl-3-heptynyl group, a 7-methoxycarbonyl-4-heptynyl group, a 7-methoxycarbonyl-5-heptynyl group, a 7-methoxycarbonyl-6-heptynyl group, a 8-methoxycarbonyl-1-octynyl group, a 8-methoxycarbonyl-2-octynyl group, a 8-methoxycarbonyl-3-octynyl group, a 8-methoxycarbonyl-4-octynyl group, a 8-methoxycarbonyl-5-octynyl group, a 8-methoxycarbonyl-6-octynyl group, a 8-methoxycarbonyl-7-octynyl group, a 2-(N-methylcarboamide)ethynyl group, a 3-(N-methylcarboamide)-1-propynyl group, a 3-(N-methylcarboamide)-2-propynyl group, a 4-(N-methylcarboamide)-1-butynyl group, a 4-(N-methylcarboamide)-2-butynyl group, a 4-(N-methylcarboamide)-3-butynyl group, a 5-(N-methylcarboamide)-1-pentynyl group, a 5-(N-methylcarboamide)-2-pentynyl group, a 5-(N-methylcarboamide)-3-pentynyl group, a 5-(N-methylcarboamide)-4-pentynyl group, a 6-(N-methylcarboamide)-1-hexynyl group, a 6-(N-methylcarboamide)-2-hexynyl group, a 6-(N-methylcarboamide)-3-hexynyl group, a 6-(N-methylcarboamide)-4-hexynyl group, a 6-(N-methylcarboamide)-5-hexynyl group, a 7-(N-methylcarboamide)-1-heptynyl group, a 7-(N-methylcarboamide)-2-heptynyl group, a 7-(N-methylcarboamide)-3-heptynyl group, a 7-(N-methylcarboamide)-4-heptynyl group, a 7-(N-methylcarboamide)-5-heptynyl group, a 7-(N-methylcarboamide)-6-heptynyl group, a 8-(N-methylcarboamide)-1-octynyl group, a 8-(N-methylcarboamide)-2-octynyl group, a 8-(N-methylcarboamide)-3-octynyl group, a 8-(N-methylcarboamide)-4-octynyl group, a 8-(N-methylcarboamide)-5-octynyl group, a 8-(N-methylcarboamide)-6-octynyl group, a 8-(N-methylcarboamide)-7-octynyl group, a 2-(N,N-dimethylcarboamide)ethynyl group, a 3-(N,N-dimethylcarboamide)-1-propynyl group, a 3-(N,N-dimethylcarboamide)-2-propynyl group, a 4-(N,N-dimethylcarboamide)-1-butynyl group, a 4-(N,N-dimethylcarboamide)-2-butynyl group, a 4-(N,N-dimethylcarboamide)-3-butynyl group, a 5-(N,N-dimethylcarboamide)-1-pentynyl group, a 5-(N,N-dimethylcarboamide)-2-pentynyl group, a 5-(N,N-dimethylcarboamide)-3-pentynyl group, a 5-(N,N-dimethylcarboamide)-4-pentynyl group, a 6-(N,N-dimethylcarboamide)-1-hexynyl group, a 6-(N,N-dimethylcarboamide)-2-hexynyl group, a 6-(N,N-dimethylcarboamide)-3-hexynyl group, a 6-(N,N-dimethylcarboamide)-4-hexynyl group, a 6-(N,N-dimethylcarboamide)-5-hexynyl group, a 7-(N,N-dimethylcarboamide)-1-heptynyl group, a 7-(N,N-dimethylcarboamide)-2-heptynyl group, a 7-(N,N-dimethylcarboamide)-3-heptynyl group, a 7-(N,N-dimethylcarboamide)-4-heptynyl group, a 7-(N,N-dimethylcarboamide)-5-heptynyl group, a 7-(N,N-dimethylcarboamide)-6-heptynyl group, a 8-(N,N-dimethylcarboamide)-1-octynyl group, a 8-(N,N-dimethylcarboamide)-2-octynyl group, a 8-(N,N-dimethylcarboamide)-3-octynyl group, a 8-(N,N-dimethylcarboamide)-4-octynyl group, a 8-(N,N-dimethylcarboamide)-5-octynyl group, a 8-(N,N-dimethylcarboamide)-6-octynyl group, a 8-(N,N-dimethylcarboamide)-7-octynyl group, a 3-(methoxyimino)-1-propynyl group, a 4-(methoxyimino)-1-butynyl group, a 4-(methoxyimino)-2-butynyl group, a 5-(methoxyimino)-1-pentynyl group, a 5-(methoxyimino)-2-pentynyl group, a 5-(methoxyimino)-3-pentynyl group, a 6-(methoxyimino)-1-hexynyl group, a 6-(methoxyimino)-2-hexynyl group, a 6-(methoxyimino)-3-hexynyl group, a 6-(methoxyimino)-4-hexynyl group, a 7-(methoxyirnino)-1-heptynyl group, a 7-(methoxyimino)-2-heptynyl group, a 7-(methoxyimino)-3-heptynyl group, a 7-(methoxyimino)-4-heptynyl group, a 7-(methoxyimino)-5-heptynyl group, a 8-(methoxyimino)-1-octynyl group, a 8-(methoxyimino)-2-octynyl group, a 8-(methoxyimino)-3-octynyl group, a 8-(methoxyimino)-4-octynyl group, a 8-(methoxyimino)-5-octynyl group, a 8-(methoxyimino)-6-octynyl group, a 2-carboxylethynyl group, a 3-carboxyl-1-propynyl group, a 3-carboxyl-1-propynyl group, a 3-carboxyl-2-propynyl group, a 4-carboxyl-1-butynyl group, a 4-carboxyl-2-butynyl group, a 4-carboxyl-3-butynyl group, a 5-carboxyl-1-pentynyl group, a 5-carboxyl-2-pentynyl group, a 5-carboxyl-3-pentynyl group, a 5-carboxyl-4-pentynyl group, a 6-carboxyl-1-hexynyl group, a 6-carboxyl-2-hexynyl group, a 6-carboxyl-3-hexynyl group, a 6-carboxyl-4-hexynyl group, a 6-carboxyl-5-hexynyl group, a 7-carboxyl-1-heptynyl group, a 7-carboxyl-2-heptynyl group, a 7-carboxyl-3-heptynyl group, a 7-carboxyl-4-heptynyl group, a 7-carboxyl-5-heptynyl group, a 7-carboxyl-6-heptynyl group, a 8-carboxyl-1-octynyl group, a 8-carboxyl-2-octynyl group, a 8-carboxyl-3-octynyl group, a 8-carboxyl-4-octynyl group, a 8-carboxyl-5-octynyl group, a 8-carboxyl-6-octynyl group, a 8-carboxyl-7-octynyl group, a but-3-en-1-ynyl group, a pent-4-en-2-ynyl group, a hex-5-en-3-ynyl group, a hept-6-en-4-ynyl group, an oct-7-en-5-ynyl group, a hept-6-en-4-ynyl group, an oct-7-en-5-ynyl group, a but-1-en-3-ynyl group, a pent-2-en-4-ynyl group, a hex-3-en-5-ynyl group, a hept-4-en-6-ynyl group, an oct-5-en-7-ynyl group, a 4-fluorobut-1-en-3-ynyl group, a 5-fluoropent-2-en-4-ynyl group, a 6-fluorohex-3-en-5-ynyl group, a 7-fluorohept-4-en-6-ynyl group, a 8-fluorooct-5-en-7-ynyl group, a 4,4-diflorobut-3-en-1-ynyl group, a 5,5-difluoropent-4-en-2-ynyl group, a 6,6-difluorohex-5-en-3-ynyl group, a 7,7-difluorohept-6-en-4-ynyl group, a 8,8-difluorooct-7-en-5-ynyl group, a 1,3-butadiynyl group, a 2,4-pentadiynyl group, a 3,5-hexadiynyl group, a 4,6-heptadiynyl group, a 5,7-octadiynyl group, a 4-fluoro-1,3-butadiynyl group, a 5-fluoro-2,4-pentadiynyl group, a 6-fluoro-3,5-hexadiynyl group, a 7-fluoro-4,6-heptadiynyl group and a 8-fluoro-5,7-octadiynyl group.

Examples of "C1 to C10 chain hydrocarbon groups optionally having one or more atoms or groups selected from group A" represented by X in the present invention include: "C1 to C10 alkyl groups optionally having one or more atoms or groups selected from group A" such as a methyl group, an ethyl group, an isopropyl group, a tert-butyl group, a propyl group, a sec-butyl group, an isobutyl group, a 1,1-dimethylpropyl group, a 2, 2-dimethylpropyl group, a 1, 2-dimethylpropyl group, an 1-ethylpropyl group, a butyl group, a 1-methylbutyl group, a 2-methylbutyl group, a 3-methylbutyl group, a 1,1-dimethylbutyl group, a 2,2-dimethylbutyl group, a 3,3-dimethylbutyl group, a 1,2,3-trimethylbutyl group, an 1-ethylbutyl group, an 2-ethylbutyl group, a pentyl group, a 1-methylpentyl group, a 2-methylpentyl group, a 3-methylpentyl group, a 4-methylpentyl group, a 1,1-dimethylpentyl group, a 2,2-dimethylpentyl group, a 3,3-dimethylpentyl group, a 4,4-dimethylpentyl group, an 1-ethylpentyl group, an 2-ethylpentyl group, an 3-ethylpentyl group, a 1-propylpentyl group, a 2-propylpentyl group, a hexyl group, a 1-methylhexyl group, a 2-methylhexyl group, a 3-methylhexyl group, a 4-methylhexyl group, a 5-methylhexyl group, a 1,1-dimethylhexyl group, a 2,2-dimethylhexyl group, a 3,3-dimethylhexyl group, a 4,4-dimethylhexyl group, a 5,5-dimethylhexyl group, an 1-ethylhexyl group, an 2-ethylhexyl group, an 3-ethylhexyl group, an 4-ethylhexyl group, a heptyl group, a 1-methylheptyl group, a 2-methylheptyl group, a 3-methylheptyl group, a 4-methylheptyl group, a 5-methylheptyl group, a 6-methylheptyl group, a 1,1-dimethylheptyl group, a 2,2-dimethylheptyl group, a 3,3-dimethylheptyl group, a 4,4-dimethylheptyl group, a 5,5-dimethylheptyl group, a 6,6-dimethylheptyl group, an 1-ethylheptyl group, an 2-ethylheptyl group, an 3-ethylheptyl group, an 4-ethylheptyl group, an 5-ethylheptyl group, an octyl group, a 1-methyloctyl group, a 2-methyloctyl group, a 3-methyloctyl group, a 4-methyloctyl group, a 5-methyloctyl group, a 6-methyloctyl group, a 7-methyloctyl group, a 1,1-dimethyloctyl group, a 2,2-dimethyloctyl group, a 3,3-dimethyloctyl group, a 4,4-dimethyloctyl group, a 5,5-dimethyloctyl group, a 6,6-dimethyloctyl group, a 7,7-dimethyloctyl group, an 1-ethyloctyl group, an 2-ethyloctyl group, an 3-ethyloctyl group, an 4-ethyloctyl group, an 5-ethyloctyl group, an 6-ethyloctyl group, a nonyl group, a 1-methylnonyl group, a 2-methylnonyl group, a 3-methylnonyl group, a 4-methylnonyl group, a 5-methylnonyl group, a 6-methylnonyl group, a 7-methylnonyl group, a 8-methylnonyl group, a decanyl group, a trifluoromethyl group, a 2-chloroethyl group, a 2,2,2-trifluoroethyl group, a 3,3,3-trifluoropropyl group, a 4,4,4-trifluorobutyl group, a 5,5,5-trifluoropentyl group, a 6,6,6-trifluorohexyl group, a 7,7,7-trifluoroheptyl group, a 8,8,8-trifluorooctyl group, a 9,9,9-trifluorononyl group, a 10,10,10-trifluorodecanyl group, a methoxymethyl group, a 2-methoxyethyl group, a 3-methoxypropyl group, a 4-methoxybutyl group, a 5-methoxypentyl group, a 6-methoxyhexyl group, a 7-methoxyheptyl group, a 8-methoxyoctyl group, a 9-methoxynonyl group, a 10-methoxyndecanyl group, a trifluoromethoxymethyl group, a 2-trifluoromethoxyethyl group, a 3-trifluoromethoxypropyl group, a 4-trifluoromethoxybutyl group, a 5-trifluoromethoxypentyl group, a 6-trifluoromethoxyhexyl group, a 7-trifluoromethoxyheptyl group, a 8-trifluoromethoxyoctyl group, a 9-trifluoromethoxynonyl group, a 10-trifluoromethoxyndecanyl group, a 2,2,2-trifluoroethoxymethyl group, a 2-(2,2,2-trifluoroethoxy)ethyl group, a 3-(2,2,2-trifluoroethoxy)propyl group, a 4-(2,2,2-trifluoroethoxy)butyl group, a 5-(2,2,2-trifluoroethoxy)pentyl group, a 6-(2,2,2-trifluoroethoxy)hexyl group, a 7-(2,2,2-trifluoroethoxy)heptyl group, a 8-(2,2,2-trifluoroethoxy)octyl group, a 9-(2,2,2-trifluoroethoxy)nonyl group, a 10-(2,2,2-trifluoroethoxy)decanyl group, a methylthiomethyl group, a 2-methylthioethyl group, a 3-methylthiopropyl group, a 4-methylthiobutyl group, a 5-methylthiopentyl group, a 6-methylthiohexyl group, a 7-methylthioheptyl group, a 8-methylthiooctyl group, a 9-methylthiononyl group, a 10-methylthiodecanyl group, a methylsulfinylmethyl group, a 2-methylsulfinylethyl group, a 3-methylsulfinylpropyl group, a 4-methylsulfinylbutyl group, a 5-methylsulfinylpentyl group, a 6-methylsulfinylhexyl group, a 7-methylsulfinylheptyl group, a 8-methylsulfinyloctyl group, a 9-methylsulfinylnonyl group, a 10-methylsulfinyldecanyl group, a methylsulfonylmethyl group, a 2-methylsulfonylethyl group, a 3-methylsulfonylpropyl group, a 4-methylsulfonylbutyl group, a 5-methylsulfonylpentyl group, a 6-methylsulfonylhexyl group, a 7-methylsulfonylheptyl group, a 8-methylsulfonyloctyl group, a 9-methylsulfonylnonyl group, a 10-methylsulfonyldecanyl group, a methoxycarbonylmethyl group, a 2-methoxycarbonylethyl group, a 3-methoxycarbonylpropyl group, a 4-methoxycarbonylbutyl group, a 5-methoxycarbonylpentyl group, a 6-methoxycarbonylhexyl group, a 7-methoxycarbonylheptyl group, a 8-methoxycarbonyloctyl group, a 9-methoxycarbonylnonyl group, a 10-methoxycarbonyldecanyl group, an N-methylcarboamidemethyl group, a 2-(N-methylcarboamide)ethyl group, a 3-(N-methylcarboamide)propyl group, a 4-(N-methylcarboamide)butyl group, a 5-(N-methylcarboamide)pentyl group, a 6-(N-methylcarboamide)hexyl group, a 7-(N-methylcarboamide)heptyl group, a 8-(N-methylcarboamide)octyl group, a 9-(N-methylcarboamide)nonyl group, a 10-(N-methylcarboamide)decanyl group, an N,N-dimethylcarboamidemethyl group, a 2-(N,N-dimethylcarboamide)ethyl group, a 3-(N,N-dimethylcarboamide)propyl group, a 4-(N,N-dimethylcarboamide)butyl group, a 5-(N,N-dimethylcarboamide)pentyl group, a 6-(N,N-dimethylcarboamide)hexyl group, a 7-(N,N-dimethylcarboamide)heptyl group, a 8-(N,N-dimethylcarboamide)octyl group, a 9-(N,N-dimethylcarboamide)nonyl group, a 10-(N,N-dimethylcarboamide)decanyl group, a methoxyiminomethyl group, a 2-methoxyiminoethyl group, a 3-methoxyiminopropyl group, a 4-methoxyiminobutyl group, a 5-methoxyiminopentyl group, a 6-methoxyiminohexyl group, a 7-methoxyiminoheptyl group, a 8-methoxyiminooctyl group, a carboxylmethyl group, a 2-carboxylethyl group, a 3-carboxylpropyl group, a 4-carboxylbutyl group, a 5-carboxylpentyl group, a 6-carboxylhexyl group, a 7-carboxylheptyl group, a 8-carboxyloctyl group, a 9-carboxylnonyl group and a 10-carboxyldecanyl group; "C2 to C10 alkenyl groups optionally having one or more atoms or groups selected from group A" such as a vinyl group, a 1-propenyl group, a 2-propenyl group, a 1-methyl-1-propenyl group, a 2-methyl-1-propenyl group, a 1-methyl-2-propenyl group, a 1,2-dimethyl-1-propenyl group, a 1,1-dimethyl-2-propenyl group, an 1-ethyl-1-propenyl group, an 1-ethyl-2-propenyl group, a 1-butenyl group, a 2-butenyl group, a 3-butenyl group, a 1-methyl-1-butenyl group, a 2-methyl-1-butenyl group, a 2-methyl-2-butenyl group, a 2-methyl-3-butenyl group, a 3-methyl-3-butenyl group, a 1,1-dimethyl-3-butenyl group, a 1,1-dimethyl-2-butenyl group, an 1-ethyl-1-butenyl group, an 1-ethyl-2-butenyl group, an 1-ethyl-3-butenyl group, a 1-pentenyl group, a 2-pentenyl group, a 3-pentenyl group, a 4-pentenyl group, a 1-methyl-1-pentenyl group, a 1-methyl-2-pentenyl group, a 1-methyl-3-pentenyl group, a 1-methyl-4-pentenyl group, a 2-methyl-1-pentenyl group, a 2-methyl-2-pentenyl group, a 3-methyl-2-pentenyl group, a 3-methyl-3-pentenyl group, a 4-methyl-3-pentenyl group, a 4-methyl-4-pentenyl group, a 1,1-dimethyl-2-pentenyl group, a 1,1-dimethyl-3-pentenyl group, a 1,1-dimethyl-4-pentenyl group, an 1-ethyl-1-pentenyl group, an 1-ethyl-2-pentenyl group, an 1-ethyl-3-pentenyl group, an 1-ethyl-4-pentenyl group, a 1-hexenyl group, a 2-hexenyl group, a 3-hexenyl group, a 4-hexenyl group, a 5-hexenyl group, a 1-methyl-1-hexenyl group, a 1-methyl-2-hexenyl group, a 1-methyl-3-hexenyl group, a 1-methyl-4-hexenyl group, a 1-methyl-5-hexenyl group, a 2-methyl-1-hexenyl group, a 2-methyl-2-hexenyl group, a 3-methyl-2-hexenyl group, a 3-methyl-3-hexenyl group, a 4-methyl-3-hexenyl group, a 4-methyl-4-hexenyl group, a 5-methyl-4-hexenyl group, a 5-methyl-5-hexenyl group, a 1,1-dimethyl-2-hexenyl group, a 1,1-dimethyl-3-hexenyl group, a 1,1-dimethyl-4-hexenyl group, a 1,1-dimethyl-5-hexenyl group, an 1-ethyl-1-hexenyl group, an 1-ethyl-2-hexenyl group,

an 1-ethyl-3-hexenyl group, an 1-ethyl-4-hexenyl group, an 1-ethyl-5-hexenyl group, a 1-heptenyl group, a 2-heptenyl group, a 3-heptenyl group, a 4-heptenyl group, a 5-heptenyl group, a 6-heptenyl group, a 1-methyl-1-heptenyl group, a 1-methyl-2-heptenyl group, a 1-methyl-3-heptenyl group, a 1-methyl-4-heptenyl group, a 1-methyl-5-heptenyl group, a 1-methyl-6-heptenyl group, a 2-methyl-1-heptenyl group, a 2-methyl-2-heptenyl group, a 3-methyl-2-heptenyl group, a 3-methyl-3-heptenyl group, a 4-methyl-3-heptenyl group, a 4-methyl-4-heptenyl group, a 5-methyl-4-heptenyl group, a 5-methyl-5-heptenyl group, a 6-methyl-5-heptenyl group, a 6-methyl-6-heptenyl group, an 1-ethyl-1-heptenyl group, an 1-ethyl-2-heptenyl group, an 1-ethyl-3-heptenyl group, an 1-ethyl-4-heptenyl group, an 1-ethyl-5-heptenyl group, an 1-ethyl-6-heptenyl group, an 2-ethyl-1-heptenyl group, an 2-ethyl-2-heptenyl group, an 3-ethyl-2-heptenyl group, an 3-ethyl-3-heptenyl group, an 4-ethyl-3-heptenyl group, an 4-ethyl-4-heptenyl group, an 5-ethyl-4-heptenyl group, an 5-ethyl-5-heptenyl group, an 6-ethyl-5-heptenyl group, an 6-ethyl-6-heptenyl group, an 1-octenyl group, an 2-octenyl group, an 3-octenyl group, an 4-octenyl group, an 5-octenyl group, an 6-octenyl group, an 7-octenyl group, a 1-methyl-1-octenyl group, a 1-methyl-2-octenyl group, a 1-methyl-3-octenyl group, a 1-methyl-4-octenyl group, a 1-methyl-5-octenyl group, a 1-methyl-6-octenyl group, a 1-methyl-7-octenyl group, a 2-methyl-1-octenyl group, a 2-methyl-2-octenyl group, a 3-methyl-2-octenyl group, a 3-methyl-3-octenyl group, a 4-methyl-3-octenyl group, a 4-methyl-4-octenyl group, a 5-methyl-4-octenyl group, a 5-methyl-5-octenyl group, a 6-methyl-5-octenyl group, a 6-methyl-6-octenyl group, a 7-methyl-6-octenyl group, a 7-methyl-7-octenyl group, an 1-ethyl-1-octenyl group, an 1-ethyl-2-octenyl group, an 1-ethyl-3-octenyl group, an 1-ethyl-4-octenyl group, an 1-ethyl-5-actenyl group, an 1-ethyl-6-octenyl group, an 1-ethyl-7-octenyl group, an 2-ethyl-1-octenyl group, an 2-ethyl-2-octenyl group, an 3-ethyl-2-octenyl group, an 3-ethyl-3-octenyl group, an 4-ethyl-3-octenyl group, an 4-ethyl-4-octenyl group, an 5-ethyl-4-octenyl group, an 5-ethyl-5-octenyl group, an 6-ethyl-5-octenyl group, an 6-ethyl-6-octenyl group, an 6-ethyl-7-octenyl group, a 1-nonenyl group, a 2-nonenyl group, a 3-nonenyl group, a 4-nonenyl group, a 5-nonenyl group, a 6-nonenyl group, a 7-nonenyl group, a 8-nonenyl group, a 1-methyl-1-nonenyl group, a 1-methyl-2-nonenyl group, a 1-methyl-3-nonenyl group, a 1-methyl-4-nonenyl group, a 1-methyl-5-nonenyl group, a 1-methyl-6-nonenyl group, a 1-methyl-7-nonenyl group, a 2-methyl-1-nonenyl group, a 2-methyl-2-nonenyl group, a 3-methyl-2-nonenyl group, a 3-methyl-3-nonenyl group, a 4-methyl-3-nonenyl group, a 4-methyl-4-nonenyl group, a 5-methyl-4-nonenyl group, a 5-methyl-5-nonenyl group, a 6-methyl-5-nonenyl group, a 6-methyl-6-nonenyl group, a 7-methyl-6-nonenyl group, a 7-methyl-7-nonenyl group, a 8-methyl-7-nonenyl group, a 8-methyl-8-nonenyl group, a 1-decenyl group, a 2-decenyl group, a 3-decenyl group, a 4-decenyl group, a 5-decenyl group, a 6-decenyl group, a 7-decenyl group, a 8-decenyl group, a 9-decenyl group, a propen-2-yl group, a 1-buten-2-yl group, a 1-buten-3-yl group, a 1-penten-2-yl group, a 1-penten-3-yl group, a 1-hexen-2-yl group, a 1-hexen-3-yl group, a 1-hepten-2-yl group, a 1-hepten-3-yl group, an 1-octen-2-yl group, an 1-octen-3-yl group, a 1-nonen-2-yl group, a 1-nonen-3-yl group, a 1-decen-2-yl group, a 1-decen-3-yl group, a propen-2-ylmethyl group, a 1-buten-2-ylmethyl group, a 1-buten-3-ylmethyl group, a 1-penten-2-ylmethyl group, a 1-penten-3-ylmethyl group, a 1-hexen-2-ylmethyl group, a 1-hexen-3-ylmethyl group, a 1-hepten-2-ylmethyl group, a 1-hepten-3-ylmethyl group, an 1-octen-2-ylmethyl group, an 1-octen-3-ylmethyl group, a 1-nonen-2-ylmethyl group, a 1-nonen-3-ylmethyl group, a propen-2-ylethyl group, a 1-buten-2-ylethyl group, a 1-buten-3-ylethyl group, a 1-penten-2-ylethyl group, a 1-penten-3-ylethyl group, a 1-hexen-2-ylethyl group, a 1-hexen-3-ylethyl group, a 1-hepten-2-ylethyl group, a 1-hepten-3-ylethyl group, an 1-octen-2-ylethyl group, an 1-octen-3-ylethyl group, a propen-2-ylpropyl group, a 1-buten-2-ylpropyl group, a 1-buten-3-ylpropyl group, a 1-penten-2-ylpropyl group, a 1-penten-3-ylpropyl group, a 1-hexen-2-ylpropyl group, a 1-hexen-3-ylpropyl group, a 1-hepten-2-ylpropyl group, a 1-hepten-3-ylpropyl group, a propen-2-ylbutyl group, a 1-buten-2-ylbutyl group, a 1-buten-3-ylbutyl group, a 1-penten-2-ylbutyl group, a 1-penten-3-ylbutyl group, a 1-hexen-2-ylbutyl group, a 1-hexen-3-ylbutyl group, a propen-2-ylpentyl group, a 1-buten-2-ylpentyl group, a 1-buten-3-ylpentyl group, a 1-penten-2-ylpentyl group, a 1-penten-3-ylpentyl group, a 2,2-difluorovinyl group, a 3,3-difluoro-2-propenyl group, a 4,4-difluoro-3-butenyl group, a 5,5-difluoro-4-pentenyl group, a 6,6-difluoro-5-hexenyl group, a 7,7-difluoro-6-heptenyl group, a 8,8-difluoro-7-octenyl group, a 9,9-difluoro-8-nonenyl group, a 10,10-difluoro-9-decenyl group, a 2-methoxyvinyl group, a 3-methoxy-1-propenyl group, a 3-methoxy-2-propenyl group, a 4-methoxy-1-butenyl group, a 4-methoxy-2-butenyl group, a 4-methoxy-3-butenyl group, a 5-methoxy-1-pentenyl group, a 5-methoxy-2-pentenyl group, a 5-methoxy-3-pentenyl group, a 5-methoxy-4-pentenyl group, a 6-methoxy-1-hexenyl group, a 6-methoxy-2-hexenyl group, a 6-methoxy-3-hexenyl group, a 6-methoxy-4-hexenyl group, a 6-methoxy-5-hexenyl group, a 7-methoxy-1-heptenyl group, a 7-methoxy-2-heptenyl group, a 7-methoxy-3-heptenyl group, a 7-methoxy-4-heptenyl group, a 7-methoxy-5-heptenyl group, a 7-methoxy-6-heptenyl group, a 8-methoxy-1-octenyl group, a 8-methoxy-2-octenyl group, a 8-methoxy-3-octenyl group, a 8-methoxy-4-octenyl group, a 8-methoxy-5-octenyl group, a 8-methoxy-6-octenyl group, a 8-methoxy-7-octenyl group, a 9-methoxy-8-nonenyl group, a 10-methoxy-9-decenyl group, a 2-methylthiovinyl group, a 3-methylthio-1-propenyl group, a 3-methylthio-2-propenyl group, a 4-methylthio-1-butenyl group, a 4-methylthio-2-butenyl group, a 4-methylthio-3-butenyl group, a 5-methylthio-1-pentenyl group, a 5-methylthio-2-pentenyl group, a 5-methylthio-3-pentenyl group, a 5-methylthio-4-pentenyl group, a 6-methylthio-1-hexenyl group, a 6-methylthio-2-hexenyl group, a 6-methylthio-3-hexenyl group, a 6-methylthio-4-hexenyl group, a 6-methylthio-5-hexenyl group, a 7-methylthio-1-heptenyl group, a 7-methylthio-2-heptenyl group, a 7-methylthio-3-heptenyl group, a 7-methylthio-4-heptenyl group, a 7-methylthio-5-heptenyl group, a 7-methylthio-6-heptenyl group, a 8-methylthio-1-octenyl group, a 8-methylthio-2-octenyl group, a 8-methylthio-3-octenyl group, a 8-methylthio-4-octenyl group, a

8-methylthio-5-octenyl group, a 8-methylthio-6-octenyl group, a 8-methylthio-7-octenyl group, a 9-methylthio-8-nonenyl group, a 10-methylthio-9-decenyl group, a 2-methoxycarbonylvinyl group, a 3-methoxycarbonyl-1-propenyl group, a 3-methoxycarbonyl-2-propenyl group, a 4-methoxycarbonyl-1-butenyl group, a 4-methoxycarbonyl-2-butenyl group, a 4-methoxycarbonyl-3-butenyl group, a 5-methoxycarbonyl-1-pentenyl group, a 5-methoxycarbonyl-2-pentenyl group, a 5-methoxycarbonyl-3-pentenyl group, a 5-methoxycarbonyl-4-pentenyl group, a 6-methoxycarbonyl-1-hexenyl group, a 6-methoxycarbonyl-2-hexenyl group, a 6-methoxycarbonyl-3-hexenyl group, a 6-methoxycarbonyl-4-hexenyl group, a 6-methoxycarbonyl-5-hexenyl group, a 7-methoxycarbonyl-1-heptenyl group, a 7-methoxycarbonyl-2-heptenyl group, a 7-methoxycarbonyl-3-heptenyl group, a 7-methoxycarbonyl-4-heptenyl group, a 7-methoxycarbonyl-5-heptenyl group, a 7-methoxycarbonyl-6-heptenyl group, a 8-methoxycarbonyl-1-octenyl group, a 8-methoxycarbonyl-2-octenyl group, a 8-methoxycarbonyl-3-octenyl group, a 8-methoxycarbonyl-4-octenyl group, a 8-methoxycarbonyl-5-octenyl group, a 8-methoxycarbonyl-6-octenyl group, a 8-methoxycarbonyl-7-octenyl group, a 9-methoxycarbonyl-8-nonenyl group, a 10-methoxycarbonyl-9-decenyl group, a 2-(N-methylcarboamide)vinyl group, a 3-(N-methylcarboamide)-1-propenyl group, a 3-(N-methylcarboamide)-2-propenyl group, a 4-(N-methylcarboamide)-1-butenyl group, a 4-(N-methylcarboamide)-2-butenyl group, a 4-(N-methylcarboamide)-3-butenyl group, a 5-(N-methylcarboamide)-1-pentenyl group, a 5-(N-methylcarboamide)-2-pentenyl group, a 5-(N-methylcarboamide)-3-pentenyl group, a 5-(N-methylcarboamide)-4-pentenyl group, a 6-(N-methylcarboamide)-1-hexenyl group, a 6-(N-methylcarboamide)-2-hexenyl group, a 6-(N-methylcarboamide)-3-hexenyl group, a 6-(N-methylcarboamide)-4-hexenyl group, a 6-(N-methylcarboamide)-5-hexenyl group, a 7-(N-methylcarboamide)-1-heptenyl group, a 7-(N-methylcarboamide)-2-heptenyl group, a 7-(N-methylcarboamide)-3-heptenyl group, a 7-(N-methylcarboamide)-4-heptenyl group, a 7-(N-methylcarboamide)-5-heptenyl group, a 7-(N-methylcarboamide)-6-heptenyl group, a 8-(N-methylcarboamide)-1-octenyl group, a 8-(N-methylcarboamide)-2-octenyl group, a 8-(N-methylcarboamide)-3-octenyl group, a 8-(N-methylcarboamide)-4-octenyl group, a 8-(N-methylcarboamide)-5-octenyl group, a 8-(N-methylcarboamide)-6-octenyl group, a 8-(N-methylcarboamide)-7-octenyl group, a 9-(N-methylcarboamide)-8-nonenyl group, a 10-(N-methylcarboamide)-9-decenyl group, a 2-(N,N-dimethylcarboamide)vinyl group, a 3-(N,N-dimethylcarboamide)-1-propenyl group, a 3-(N,N-dimethylcarboamide)-2-propenyl group, a 4-(N,N-dimethylcarboamide)-1-butenyl group, a 4-(N,N-dimethylcarboamide)-2-butenyl group, a 4-(N,N-dimethylcarboamide)-3-butenyl group, a 5-(N,N-dimethylcarboamide)-1-pentenyl group, a 5-(N,N-dimethylcarboamide)-2-pentenyl group, a 5-(N,N-dimethylcarboamide)-3-pentenyl group, a 5-(N,N-dimethylcarboamide)-4-pentenyl group, a 6-(N,N-dimethylcarboamide)-1-hexenyl group, a 6-(N,N-dimethylcarboamide)-2-hexenyl group, a 6-(N,N-dimethylcarboamide)-3-hexenyl group, a 6-(N,N-dimethylcarboamide)-4-hexenyl group, a 6-(N,N-dimethylcarboamide)-5-hexenyl group, a 7-(N,N-dimethylcarboamide)-1-heptenyl group, a 7-(N,N-dimethylcarboamide)-2-heptenyl group, a 7-(N,N-dimethylcarboamide)-3-heptenyl group, a 7-(N,N-dimethylcarboamide)-4-heptenyl group, a 7-(N,N-dimethylcarboamide)-5-heptenyl group, a 7-(N,N-dimethylcarboamide)-6-heptenyl group, a 8-(N,N-dimethylcarboamide)-1-octenyl group, a 8-(N,N-dimethylcarboamide)-2-octenyl group, a 8-(N,N-dimethylcarboamide)-3-octenyl group, a 8-(N,N-dimethylcarboamide)-4-octenyl group, a 8-(N,N-dimethylcarboamide)-5-octenyl group, a 8-(N,N-dimethylcarboamide)-6-octenyl group, a 8-(N,N-dimethylcarboamide)-7-octenyl group, a 9-(N,N-dimethylcarboamide)-8-nonenyl group, a 10-(N,N-dimethylcarboamide)-9-decenyl group, a 3-methoxyimino-1-propenyl group, a 4-methoxyimino-1-butenyl group, a 4-methoxyimino-2-butenyl group, a 5-methoxyimino-1-pentenyl group, a 5-methoxyimino-2-pentenyl group, a 5-methoxyimino-3-pentenyl group, a 6-methoxyimino-1-hexenyl group, a 6-methoxyimino-2-hexenyl group, a 6-methoxyimino-3-hexenyl group, a 6-methoxyimino-4-hexenyl group, a 7-methoxyimino-1-heptenyl group, a 7-methoxyimino-2-heptenyl group, a 7-methoxyimino-3-heptenyl group, a 7-methoxyimino-4-heptenyl group, a 7-methoxyimino-5-heptenyl group, a 8-methoxyimino-1-octenyl group, a 8-methoxyimino-2-octenyl group, a 8-methoxyimino-3-octenyl group, a 8-methoxyimino-4-octenyl group, a 8-methoxyimino-5-octenyl group, a 8-methoxyimino-6-octenyl group, a 2-carboxylvinyl group, a 3-carboxyl-1-propenyl group, a 3-carboxyl-2-propenyl group, a 4-carboxyl-1-butenyl group, a 4-carboxyl-2-butenyl group, a 4-carboxyl-3-butenyl group, a 5-carboxyl-1-pentenyl group, a 5-carboxyl-2-pentenyl group, a 5-carboxyl-3-pentenyl group, a 5-carboxyl-4-pentenyl group, a 6-carboxyl-1-hexenyl group, a 6-carboxyl-2-hexenyl group, a 6-carboxyl-3-hexenyl group, a 6-carboxyl-4-hexenyl group, a 6-carboxyl-5-hexenyl group, a 7-carboxyl-1-heptenyl group, a 7-carboxyl-2-heptenyl group, a 7-carboxyl-3-heptenyl group, a 7-carboxyl-4-heptenyl group, a 7-carboxyl-5-heptenyl group, a 7-carboxyl-6-heptenyl group, a 8-carboxyl-1-octenyl group, a 8-carboxyl-2-octenyl group, a 8-carboxyl-3-octenyl group, a 8-carboxyl-4-octenyl group, a 8-carboxyl-5-octenyl group, a 8-carboxyl-6-octenyl group, a 8-carboxyl-7-octenyl group, a 9-carboxyl-8-nonenyl group, a 10-carboxyl-9-decenyl group, a 1,3-butadienyl group, a 2,4-pentadienyl group, a 3,5-hexadienyl group, a 4,6-heptadienyl group, an 5,7-octadienyl group, a 6,8-nonadienyl group, a 7,9-decadienyl group, a 4,4-difluoro-1,3-butadienyl group, a 5,5-difluoro-2,4-pentadienyl group, a 6,6-difluoro-3,5-hexadienyl group, a 7,7-difluoro-4,6-heptadienyl group, a 8,8-difluoro-5,7-octadienyl group, a 9,9-difluoro-6,8-nonadienyl group and a 10,10-difluoro-7,9-decadienyl group;

"C2 to C10 alkynyl groups optionally having one or more atoms or groups selected from group A" such as an ethynyl group, a 1-propynyl group, a 2-propynyl group, a 1-methyl-2-propynyl group, a 1,1-dimethyl-2-propynyl group, a 1-ethyl-2-propynyl group, a 1-butynyl group, a 2-butynyl group, a 3-butynyl group, a 1-methyl-2-butynyl group, a 1-methyl-3-butynyl group, a 1,1-dimethyl-2-butynyl group, a 1,1-dimethyl-3-butynyl group, an 1-ethyl-2-butynyl group, an 1-ethyl-3-butynyl group, a 1-pentynyl group, a 2-pentynyl group, a 3-pentynyl group, a 4-pentynyl group, a 1-methyl-2-pentynyl

group, a 1-methyl-3-pentynyl group, a 1-methyl-4-pentynyl group, a 1,1-dimethyl-2-pentynyl group, a 1,1-dimethyl-3-pentynyl group, a 1,1-dimethyl-4-pentynyl group, an 1-ethyl-2-pentynyl group, an 1-ethyl-3-pentynyl group, an 1-ethyl-4-pentynyl group, a 1-hexynyl group, a 2-hexynyl group, a 3-hexynyl group, a 4-hexynyl group, a 5-hexynyl group, a 1-methyl-2-hexynyl group, a 1-methyl-3-hexynyl group, a 1-methyl-4-hexynyl group, a 1-methyl-5-hexynyl group, a 1,1-dimethyl-2-hexynyl group, a 1,1-dimethyl-3-hexynyl group, a 1,1-dimethyl-4-hexynyl group, a 1,1-dimethyl-5-hexynyl group, an 1-ethyl-2-hexynyl group, an 1-ethyl-3-hexynyl group, an 1-ethyl-4-hexynyl group, an 1-ethyl-5-hexynyl group, a 1-heptynyl group, a 2-heptynyl group, a 3-heptynyl group, a 4-heptynyl group, a 5-heptynyl group, a 6-heptynyl group, a 1-methyl-2-heptynyl group, a 1-methyl-3-heptynyl group, a 1-methyl-4-heptynyl group, a 1-methyl-5-heptynyl group, a 1-methyl-6-heptynyl group, an 1-octynyl group, an 2-octynyl group, an 3-octynyl group, an 4-octynyl group, an 5-octynyl group, an 6-octynyl group, an 7-octynyl group, a 1-methyl-2-octynyl group, a 1-methyl-3-octynyl group, a 1-methyl-4-octynyl group, a 1-methyl-5-octynyl group, a 1-methyl-6-octynyl group, a 1-methyl-7-octynyl group, a 1,1-dimethyl-2-octynyl group, a 1,1-dimethyl-3-octynyl group, a 1,1-dimethyl-4-octynyl group, a 1,1-dimethyl-5-octynyl group, a 1,1-dimethyl-6-octynyl group, a 1,1-dimethyl-7-octynyl group, a 1-nonynyl group, a 2-nonynyl group, a 3-nonynyl group, a 4-nonynyl group, a 5-nonynyl group, a 6-nonynyl group, a 7-nonynyl group, a 8-nonynyl group, a 1-methyl-2-nonynyl group, a 1-methyl-3-nonynyl group, a 1-methyl-4-nonynyl group, a 1-methyl-5-nonynyl group, a 1-methyl-6-nonynyl group, a 1-methyl-7-nonynyl group, a 1-methyl-8-nonynyl group, a 1-decynyl group, a 2-decynyl group, a 3-decynyl group, a 4-decynyl group, a 5-decynyl group, a 6-decynyl group, a 7-decynyl group, a 8-decynyl group, a 9-decynyl group, a 1-butyn-3-yl group, a 1-pentyn-3-yl group, a 1-hexyn-3-yl group, a 1-heptyn-3-yl group, an 1-octyn-3-yl group, a 1-nonyn-3-yl group, a 1-decyn-3-yl group, a 1-butyn-3-ylmethyl group, a 1-pentyn-3-ylmethyl group, a 1-hexyn-3-ylmethyl group, a 1-heptyn-3-ylmethyl group, an 1-octyn-3-ylrnethyl group, a 1-nonyn-3-ylmethyl group, a 1-butyn-3-ylethyl group, a 1-pentyn-3-ylethyl group, a 1-hexyn-3-ylethyl group, a 1-heptyn-3-ylethyl group, an 1-octyn-3-ylethyl group, a 1-butyn-3-ylpropyl group, a 1-pentyn-3-ylpropyl group, a 1-hexyn-3-ylpropyl group, a 1-heptyn-3-ylpropyl group, a 1-butyn-3-ylbutyl group, a 1-pentyn-3-ylbutyl group, a 1-hexyn-3-ylbutyl group, a 1-butyn-3-ylpenyl group, a 1-pentyn-3-ylpenyl group, a 2-fluoroethynyl group, a 3-fluoro-2-propynyl group, a 4-fluoro-3-butynyl group, a 5-fluoro-4-pentynyl group, a 6-fluoro-5-hexynyl group, a 7-fluoro-6-heptynyl group, a 8-fluoro-7-octynyl group, a 9-fluoro-8-nonynyl group, a 10-fluoro-9-decynyl group, a 2-methoxyethynyl group, a 3-methoxy-1-propynyl group, a 3-methoxy-2-propynyl group, a 4-methoxy-1-butynyl group, a 4-methoxy-2-butynyl group, a 4-methoxy-3-butynyl group, a 5-methoxy-1-pentynyl group, a 5-methoxy-2-pentynyl group, a 5-methoxy-3-pentynyl group, a 5-methoxy-4-pentynyl group, a 6-methoxy-1-hexynyl group, a 6-methoxy-2-hexynyl group, a 6-methoxy-3-hexynyl group, a 6-methoxy-4-hexynyl group, a 6-methoxy-5-hexynyl group, a 7-methoxy-1-heptynyl group, a 7-methoxy-2-heptynyl group, a 7-methoxy-3-heptynyl group, a 7-methoxy-4-heptynyl group, a 7-methoxy-5-heptynyl group, a 7-methoxy-6-heptynyl group, a 8-methoxy-1-octynyl group, a 8-methoxy-2-octynyl group, a 8-methoxy-3-octynyl group, a 8-methoxy-4-octynyl group, a 8-methoxy-5-octynyl group, a 8-methoxy-6-octynyl group, a 8-methoxy-7-octynyl group, a 9-methoxy-1-nonynyl group, a 9-methoxy-2-nonynyl group, a 9-methoxy-3-nonynyl group, a 9-methoxy-4-nonynyl group, a 9-methoxy-5-nonynyl group, a 9-methoxy-6-nonynyl group, a 9-methoxy-7-nonynyl group, a 9-methoxy-8-nonynyl group, a 10-methoxy-1-decynyl group, a 10-methoxy-2-decynyl group, a 10-methoxy-3-decynyl group, a 10-methoxy-4-decynyl group, a 10-methoxy-5-decynyl group, a 10-methoxy-6-decynyl group, a 10-methoxy-7-decynyl group, a 10-methoxy-8-decynyl group, a 10-methoxy-9-decynyl group, a 2-methylthioethynyl group, a 3-methylthio-1-propynyl group, a 3-methylthio-2-propynyl group, a 4-methylthio-1-butynyl group, a 4-methylthio-2-butynyl group, a 4-methylthio-3-butynyl group, a 5-methylthio-1-pentynyl group, a 5-methylthio-2-pentynyl group, a 5-methylthio-3-pentynyl group, a 5-methylthio-4-pentynyl group, a 6-methylthio-1-hexynyl group, a 6-methylthio-2-hexynyl group, a 6-methylthio-3-hexynyl group, a 6-methylthio-4-hexynyl group, a 6-methylthio-5-hexynyl group, a 7-methylthio-1-heptynyl group, a 7-methylthio-2-heptynyl group, a 7-methylthio-3-heptynyl group, a 7-methylthio-4-heptynyl group, a 7-methylthio-5-heptynyl group, a 7-methylthio-6-heptynyl group, a 8-methylthio-1-octynyl group, a 8-methylthio-2-octynyl group, a 8-methylthio-3-octynyl group, a 8-methylthio-4-octynyl group, a 8-methylthio-5-octynyl group, a 8-methylthio-6-octynyl group, a 8-methylthio-7-octynyl group, a 9-methylthio-1-nonynyl group, a 9-methylthio-2-nonynyl group, a 9-methylthio-3-nonynyl group, a 9-methylthio-4-nonynyl group, a 9-methylthio-5-nonynyl group, a 9-methylthio-6-nonynyl group, a 9-methylthio-7-nonynyl group, a 9-methylthio-8-nonynyl group, a 10-methylthio-1-decynyl group, a 10-methylthio-2-decynyl group, a 10-methylthio-3-decynyl group, a 10-methylthio-4-decynyl group, a 10-methylthio-5-decynyl group, a 10-methylthio-6-decynyl group, a 10-methylthio-7-decynyl group, a 10-methylthio-8-decynyl group, a 10-methylthio-9-decynyl group, a 2-methoxycarbonylethynyl group, a 3-methoxycarbonyl-1-propynyl group, a 3-methoxycarbonyl-2-propynyl group, a 4-methoxycarbonyl-1-butynyl group, a 4-methoxycarbonyl-2-butynyl group, a 4-methoxycarbonyl-3-butynyl group, a 5-methoxycarbonyl-1-pentynyl group, a 5-methoxycarbonyl-2-pentynyl group, a 5-methoxycarbonyl-3-pentynyl group, a 5-methoxycarbonyl-4-pentynyl group, a 6-methoxycarbonyl-1-hexynyl group, a 6-methoxycarbonyl-2-hexynyl group, a 6-methoxycarbonyl-3-hexynyl group, a 6-methoxycarbonyl-4-hexynyl group, a 6-methoxycarbonyl-5-hexynyl group, a 7-methoxycarbonyl-1-heptynyl group, a 7-methoxycarbonyl-2-heptynyl group, a 7-methoxycarbonyl-3-heptynyl group, a 7-methoxycarbonyl-4-heptynyl group, a 7-methoxycarbonyl-5-heptynyl group, a 7-methoxycarbonyl-6-heptynyl group, a 8-methoxycarbonyl-1-octynyl group, a 8-methoxycarbonyl-2-octynyl group, a 8-methoxycarbonyl-3-octynyl group, a 8-methoxycarbonyl-4-octynyl group, a 8-methoxycarbonyl-5-octynyl group, a 8-methoxycarbonyl-6-octynyl group, a 8-methoxycarbonyl-7-octynyl group, a 9-methoxycarbonyl-1-nonynyl group, a 9-methoxycarbonyl-2-nonynyl group, a 9-methoxycarbonyl-3-nonynyl group, a 9-methoxycarbonyl-4-nonynyl group, a 9-methoxycarbonyl-5-nonynyl group, a 9-methoxycarbonyl-6-nonynyl group, a 9-methoxycarbonyl-7-nonynyl group, a 9-methoxycarbonyl-8-nonynyl group, a 10-methoxycarbonyl-1-decynyl group, a 10-methoxycarbonyl-2-decynyl group, a 10-methoxycarbonyl-3-decynyl group, a 10-methoxycarbonyl-4-decynyl group, a 10-methoxycarbonyl-5-decynyl group, a 10-methoxycarbonyl-6-decynyl group, a 10-methoxycarbonyl-7-decynyl group, a 10-methoxycarbonyl-8-decynyl group, a 10-methoxycarbonyl-9-decynyl group, a 2-(N-methylcarboamide)ethynyl group, a 3-(N-methylcarboamide)-1-propynyl group, a 3-(N-methylcarboamide)-2-propynyl group, a 4-(N-methylcarboamide)-1-butynyl group, a 4-(N-methylcarboamide)-2-butynyl group, a 4-(N-methylcarboamide)-3-butynyl group, a 5-(N-methylcarboamide)-1-pentynyl group, a 5-(N-methylcarboamide)-2-pentynyl group, a 5-(N-methylcarboamide)-3-pentynyl group, a 5-(N-methylcarboamide)-4-pentynyl group, a 6-(N-methylcarboamide)-1-hexynyl group, a 6-(N-methylcarboamide)-2-hexynyl group, a 6-(N-methylcarboamide)-3-hexynyl group, a 6-(N-methylcarboamide)-4-hexynyl group, a 6-(N-methylcarboamide)-5-hexynyl group, a 7-(N-methylcarboamide)-1-heptynyl group, a 7-(N-methylcarboamide)-2-heptynyl group, a 7-(N-methylcarboamide)-3-heptynyl group, a 7-(N-methylcarboamide)-4-heptynyl group, a 7-(N-methylcarboamide)-5-heptynyl group, a 7-(N-methylcarboamide)-6-heptynyl group, a 8-(N-methylcarboamide)-1-octynyl group, a 8-(N-methylcarboamide)-2-octynyl group, a 8-(N-methylcarboamide)-3-octynyl group, a 8-(N-methylcarboamide)-4-octynyl group, a 8-(N-methylcarboamide)-5-octynyl group, a 8-(N-methylcarboamide)-6-octynyl group, a 8-(N-methylcarboamide)-7-octynyl group, a 9-(N-methylcarboamide)-1-nonynyl group, a 9-(N-methylcarboamide)-2-nonynyl group, a 9-(N-methylcarboamide)-3-nonynyl group, a 9-(N-methylcarboamide)-4-nonynyl group, a 9-(N-methylcarboamide)-5-nonynyl group, a 9-(N-methylcarboamide)-6-nonynyl group, a 9-(N-methylcarboamide)-7-nonynyl group, a 9-(N-methylcarboamide)-8-nonynyl group, a 10-(N-methylcarboamide)-1-decynyl group, a 10-(N-methylcarboamide)-2-decynyl group, a 10-(N-methylcarboamide)-3-decynyl group, a 10-(N-methylcarboamide)-4-decynyl group, a 10-(N-methylcarboamide)-5-decynyl group, a 10-(N-methylcarboamide)-6-decynyl group, a 10-(N-methylcarboamide)-7-decynyl group, a 10-(N-methylcarboamide)-8-decynyl group, a 10-(N-methylcarboamide)-9-decynyl group, a 2-(N,N-dimethylcarboamide)ethynyl group, a 3-(N,N-dimethylcarboamide)-1-propynyl group, a 3-(N,N-dimethylcarboamide)-2-propynyl group, a 4-(N,N-dimethylcarboamide)-1-butynyl group, a 4-(N,N-dimethylcarboamide)-2-butynyl group, a 4-(N,N-dimethylcarboamide)-3-butynyl group, a 5-(N,N-dimethylcarboamide)-1-pentynyl group, a 5-(N,N-dimethylcarboamide)-2-pentynyl group, a 5-(N,N-dimethylcarboamide)-3-pentynyl group, a 5-(N,N-dimethylcarboamide)-4-pentynyl group, a 6-(N,N-dimethylcarboamide)-1-hexynyl group, a 6-(N,N-dimethylcarboamide)-2-hexynyl group, a 6-(N,N-dimethylcarboamide)-3-hexynyl group, a 6-(N,N-dimethylcarboamide)-4-hexynyl group, a 6-(N,N-dimethylcarboamide)-5-hexynyl group, a 7-(N,N-dimethylcarboamide)-1-heptynyl group, a 7-(N,N-dimethylcarboamide)-2-heptynyl group, a 7-(N,N-dimethylcarboamide)-3-heptynyl group, a 7-(N,N-dimethylcarboamide)-4-heptynyl group, a 7-(N,N-dimethylcarboamide)-5-heptynyl group, a 7-(N,N-dimethylcarboamide)-6-heptynyl group, a 8-(N,N-dimethylcarboamide)-1-octynyl group, a 8-(N,N-dimethylcarboamide)-2-octynyl group, a 8-(N,N-dimethylcarboamide)-3-octynyl group, a 8-(N,N-dimethylcarboamide)-4-octynyl group, a 8-(N,N-dimethylcarboamide)-5-octynyl group, a 8-(N,N-dimethylcarboamide)-6-octynyl group, a 8-(N,N-dimethylcarboamide)-7-octynyl group, a 9-(N,N-dimethylcarboamide)-1-nonynyl group, a 9-(N,N-dimethylcarboamide)-2-nonynyl group, a 9-(N,N-dimethylcarboamide)-3-nonynyl group, a 9-(N,N-dimethylcarboamide)-4-nonynyl group, a 9-(N,N-dimethylcarboamide)-5-nonynyl group, a 9-(N,N-dimethylcarboamide)-6-nonynyl group, a 9-(N,N-dimethylcarboamide)-7-nonynyl group, a 9-(N,N-dimethylcarboamide)-8-nonynyl group, a 10-(N,N-dimethylcarboamide)-1-decynyl group, a 10-(N,N-dimethylcarboamide)-2-decynyl group, a 10-(N,N-dimethylcarboamide)-3-decynyl group, a 10-(N,N-dimethylcarboamide)-4-decynyl group, a 10-(N,N-dimethylcarboamide)-5-decynyl group, a 10-(N,N-dimethylcarboamide)-6-decynyl group, a 10-(N,N-dimethylcarboamide)-7-decynyl group, a 10-(N,N-dimethylcarboamide)-8-decynyl group, a 10-(N,N-dimethylcarboamide)-9-decynyl group, a 3-(methoxyimino)-1-propynyl group, a 4-(methoxyimino)-1-butynyl group, a 4-(methoxyimino)-2-butynyl group, a 5-(methoxyimino)-1-pentynyl group, a 5-(methoxyimino)-2-pentynyl group, a 5-(methoxyimino)-3-pentynyl group, a 6-(methoxyimino)-1-hexynyl group, a 6-(methoxyimino)-2-hexynyl group, a 6-(methoxyimino)-3-hexynyl group, a 6-(methoxyimino)-4-hexynyl group, a 7-(methoxyimino)-1-heptynyl group, a 7-(methoxyimino)-2-heptynyl group, a 7-(methoxyimino)-3-heptynyl group, a 7-(methoxyimino)-4-heptynyl group, a 7-(methoxyimino)-5-heptynyl group, a 8-(methoxyimino)-1-octynyl group, a 8-(methoxyimino)-2-octynyl group, a 8-(methoxyimino)-3-octynyl group, a 8-(methoxyimino)-4-octynyl group, a 8-(methoxyimino)-5-octynyl group, a 8-(methoxyimino)-6-octynyl group, a 9-(methoxyimino)-1-nonynyl group, a 9-(methoxyimino)-2-nonynyl group, a 9-(methoxyimino)-3-nonynyl group, a 9-(methoxyimino)-4-nonynyl group, a 9-(methoxyimino)-5-nonynyl group, a 9-(methoxyimino)-6-nonynyl group, a 9-(methoxyimino)-7-nonynyl group, a 10-(methoxyimino)-1-decynyl group, a 10-(methoxyimino)-2-decynyl group, a 10-(methoxyimino)-3-decynyl group, a 10-(methoxyimino)-4-decynyl group, a 10-(methoxyimino)-5-decynyl group, a 10-(methoxyimino)-6-decynyl group, a 10-(methoxyimino)-7-decynyl group, a 10-(methoxyimino)-8-decynyl group, a 2-carboxylethynyl group, a 3-carboxyl-1-propynyl group, a 3-carboxyl-2-propynyl group, a 4-carboxyl-1-butynyl group, a 4-carboxyl-2-butynyl group, a 4-carboxyl-3-butynyl group, a 5-carboxyl-1-pentynyl group, a 5-carboxyl-2-pentynyl group, a 5-carboxyl-3-pentynyl group, a 5-carboxyl-4-pentynyl group, a 6-carboxyl-1-hexynyl group, a 6-carboxyl-2-hexynyl group, a 6-carboxyl-3-hexynyl group, a 6-carboxyl-4-hexynyl group, a 6-carboxyl-5-hexynyl group, a 7-carboxyl-1-heptynyl group, a 7-carboxyl-2-heptynyl group, a 7-carboxyl-3-heptynyl group, a 7-carboxyl-4-heptynyl group, a 7-carboxyl-5-heptynyl group, a 7-carboxyl-6-heptynyl group, a 8-carboxyl-1-octynyl group, a 8-carboxyl-2-octynyl group, a 8-carboxyl-3-octynyl group, a 8-carboxyl-4-octynyl group, a 8-carboxyl-5-octynyl group, a 8-carboxyl-6-octynyl group, a 8-carboxyl-7-octynyl group, a 9-carboxyl-1-nonynyl group, a 9-carboxyl-2-nonynyl group, a 9-carboxyl-3-nonynyl group, a 9-carboxyl-4-nonynyl group, a 9-carboxyl-5-nonynyl group, a 9-carboxyl-6-nonynyl group, a 9-carboxyl-7-nonynyl group, a 9-carboxyl-8-nonynyl group, a 10-carboxyl-1-decynyl group, a 10-carboxyl-2-decynyl group, a 10-carboxyl-3-decynyl group, a 10-carboxyl-4-decynyl group, a 10-carboxyl-5-decynyl group, a 10-carboxyl-6-decynyl group, a 10-carboxyl-7-decynyl group, a 10-carboxyl-8-decynyl group, a 10-carboxyl-9-decynyl group, a but-3-en-1-ynyl group, a pent-4-en-2-ynyl group, a hex-5-en-3-ynyl group, a hept-6-en-4-ynyl group, an oct-7-en-5-ynyl group, a non-8-en-6-ynyl group, a dec-9-en-7-ynyl group, a 4,4-diflorobut-3-en-1-ynyl group, a 5,5-difluoropent-4-en-2-ynyl group, a 6,6-difluorohex-5-en-3-ynyl group, a 7,7-difluorohept-6-en-4-ynyl group, a 8,8-difluorooct-7-en-5-ynyl group, a 9,9-difluoronon-8-en-6-ynyl group, a 10,10-difluorodec-9-en-7-ynyl group, a but-1-en-3-ynyl group, a pent-2-en-4-ynyl group, a hex-3-en-5-ynyl group, a hept-4-en-6-ynyl group, an oct-5-en-7-ynyl group, a non-6-en-8-ynyl group, a dec-7-en-9-ynyl group, a 1,3-butadiynyl group, a 2,4-pentadiynyl group, a 3,5-hexadiynyl group, a 4,6-heptadiynyl group, an 5, 7-octadiynyl group, a 6, 8-nonadiynyl group, a 7,9-decadiynyl group, a 4-fluoro-1,3-butadiynyl group, a 5-fluoro-2,4-pentadiynyl group, a 6-fluoro-3,5-hexadiynyl group, a 7-fluoro-4,6-heptadiynyl group, a 8-fluoro-5,7-octadiynyl group, a 9-fluoro-6,8-nonadiynyl group, a 10-fluoro-7,9-decadiynyl group, a 4-fluorobut-1-en-3-ynyl group, a 5-fluoropent-2-en-4-ynyl group, a 6-fluorohex-3-en-5-ynyl group, a 7-fluorohept-4-en-6-ynyl group, a 8-fluorooct-5-en-7-ynyl group, a 9-fluoronon-6-en-8-ynyl group and a 10-fluorodec-7-en-9-ynyl group.

Examples of "cyclopropyl groups optionally having one or more atoms or groups selected from group B" represented by X in the present invention include:

a cyclopropyl group, a 2-methylcyclopropyl group, a 2,2-dimethylcyclopropyl group, a 2,3-dimethylcyclopropyl group, a 2,2,3,3-tetramethylcyclopropyl group, an 2-ethylcyclopropyl group, an 2-(isopropyl)cyclopropyl group, a 2-(tert-butyl)cyclopropyl group, a 2-propylcyclopropyl group, a 2-(sec-butyl)cyclopropyl group, an 2-(isobutyl)cyclopropyl group, a 2-(1',1'-dimethylpropyl)cyclopropyl group, a 2-(2',2'-dimethylpropyl)cyclopropyl group, a 2-(1',2'-dimethylpropyl)cyclopropyl group, an 2-(1'-ethylpropyl)cyclopropyl group, a 2-butylcyclopropyl group, a 2-(1'-methylbutyl)cyclopropyl group, a 2-(2'-methylbutyl)cyclopropyl group, a 2-(3'-methylbutyl)cyclopropyl group, a (1',1'-dimethylbutyl)cyclopropyl group, a 2-(2',2'-dimethylbutyl)cyclopropyl group, a 2-(3',3'-dimethylbutyl)cyclopropyl group, an 2-(1'-ethylbutyl)cyclopropyl group, an 2-(2'-ethylbutyl)cyclopropyl group, a 2-pentylcyclopropyl group, a 2-(1'-methylpentyl)cyclopropyl group, a 2-(2'-methylpentyl)cyclopropyl group, a 2-(3'-methylpentyl)cyclopropyl group, a 2-(4'-methylpentyl)cyclopropyl group, a 2-hexylcyclopropyl group, an 2-ethyl-3,3-dimethylcyclopropyl group, a 3,3-dimethyl-2-(isopropyl)cyclopropyl group, a 2-(tert-butyl)-3,3-dimethylcyclopropyl group, a 3,3-dimethyl-2-propylcyclopropyl group, a 2-(sec-butyl)-3,3-dimethylcyclopropyl group, an 2-(isobutyl)-3,3-dimethylcyclopropyl group, a 3,3-dimethyl-2-(1',1'-dimethylpropyl)cyclopropyl group, a 3,3-dimethyl-2-(2',2'-dimethylpropyl)cyclopropyl group, a 3,3-dimethyl-2-(1',2'-dimethylpropyl)cyclopropyl group, a 3,3-dimethyl-2-(1'-ethylpropyl)cyclopropyl group, a 2-butyl-3,3-dimethylcyclopropyl group, a 3,3-dimethyl-2-(1'-methylbutyl)cyclopropyl group, a 3,3-dimethyl-2-(2'-methylbutyl)cyclopropyl group, a 3,3-dimethyl-2-(3'-methylbutyl)cyclopropyl group, a 3,3-dimethyl-(1',1'-dimethylbutyl)cyclopropyl group, a 3,3-dimethyl-2-(2',2'-dimethylbutyl)cyclopropyl group, a 3,3-dimethyl-2-(3',3'-dimethylbutyl)cyclopropyl group, a 3,3-dimethyl-2-(1'-ethylbutyl)cyclopropyl group, a 3,3-dimethyl-2-(2'-ethylbutyl)cyclopropyl group, a 3,3-dimethyl-2-pentylcyclopropyl group, a 3,3-dimethyl-2-(1'-methylpenyl)cyclopropyl group, a 3,3-dimethyl-2-(2'-methylpenyl)cyclopropyl group, a 3,3-dimethyl-2-(3'-methylpenyl)cyclopropyl group, a 3,3-dimethyl-2-(4'-methylpenyl)cyclopropyl group, a 3,3-dimethyl-2-hexylcyclopropyl group, a 2-trifluoromethylcyclopropyl group, a 2-(2',2',2'-trifluoroethyl)cyclopropyl group, a 2-(3',3',3'-trifluoroproyl)cyclopropyl group, a 2-(4',4',4'-trifluorobutyl)cyclopropyl group, a 2-(5',5',5'-trifluoropentyl)cyclopropyl group, a 2-(6',6',6'-trifluorohexyl)cyclopropyl group, a 2-methoxymethylcyclopropyl group, a 2-(2'-methoxyethyl)cyclopropyl group, a 2-(3'-methoxypropyl)cyclopropyl group, a 2-(4'-methoxybutyl)cyclopropyl group, a 2-(5'-methoxypentyl)cyclopropyl group, a 2-(6'-methoxyhexyl)cyclopropyl group, a 2-trifluoromethoxymethylcyclopropyl group, a 2-(2'-trifluoromethoxyethyl)cyclopropyl group, a 2-(3'-trifluoromethoxypropyl)cyclopropyl group, a 2-(4'-trifluoromethoxybutyl)cyclopropyl group, a 2-(5'-trifluoromethoxypentyl)cyclopropyl group, a 2-(6'-trifluoromethoxyhexyl)cyclopropyl group, a 2-methylthiomethylcyclopropyl group, a 2-(2'-methylthioethyl)cyclopropyl group, a 2-(3'-methylthiopropyl)cyclopropyl group, a 2-(4'-methylthiobutyl)cyclopropyl group, a 2-(5'-methylthiopentyl)cyclopropyl group, a 2-(6'-methylthiohexyl)cyclopropyl group, a 2-methylsulfinylmethylcyclopropyl group, a 2-(2'-methylsulfinylethyl)cyclopropyl group, a 2-(3'-methylsulfinylpropyl)cyclopropyl group, a 2-(4'-methylsulfinylbutyl)cyclopropyl group, a 2-(5'-methylsulfinylpentyl)cyclopropyl group, a 2-(6'-methylsulfinylhexyl)cyclopropyl group, a 2-methylsulfonylmethylcyclopropyl group, a 2-(2'-methylsulfonylethyl)cyclopropyl group, a 2-(3'-methylsulfonylpropyl)cyclopropyl group, a 2-(4'-methylsulfonylbutyl)cyclopropyl group, a 2-(5'-methylsulfonylpentyl)cyclopropyl group, a 2-(6'-methylsulfonylhexyl)cyclopropyl group, a 2-methoxycarbonylmethylcyclopropyl group, a 2-(2'-methoxycarbonylethyl)cyclopropyl group, a 2-(3'-methoxycarbonylpropyl)cyclopropyl group, a 2-(4'-methoxycarbonylbutyl)cyclopropyl group, a 2-(5'-methoxycarbonylpentyl)cyclopropyl group, a 2-(6'-methoxycarbonylhexyl)cyclopropyl group, a 2-(N-methylcarboamidemethyl)cyclopropyl group, a 2-(2'-(N-methylcarboamide)ethyl)cyclopropyl group, a 2-(3'-(N-methylcarboamide)propyl)cyclopropyl group, a 2-(4'-(N-methylcarboamide)butyl)cyclopropyl group, a 2-(5'-(N-methylcarboamide)pentyl)cyclopropyl group, a 2-(6'-(N-methylcarboamide)hexyl)cyclopropyl group, a 2-(N,N-dimethylcarboamidemethyl)cyclopropyl group, a 2-(2'-(N,N-dimethylcarboamide)ethyl)cyclopropyl group, a 2-(3'-(N,N-dimethylcarboamide)propyl)cyclopropyl group, a 2-(4'-(N,N-dimethylcarboamide)butyl)cyclopropyl group, a 2-(5'-(N,N-dimethylcarboamide)pentyl)cyclopropyl group, a 2-(6'-(N,N-dimethylcarboamide)hexyl)cyclopropyl group, a 2-methoxyiminomethylcyclopropyl group, a 2-(2'-methoxyiminoethyl)cyclopropyl group, a 2-(3'-methoxyiminopropyl)cyclopropyl group, a 2-(4'-methoxyiminobutyl)cyclopropyl group, a 2-(5'-methoxyiminopentyl)cyclopropyl group, a 2-(6'-methoxyiminohexyl)cyclopropyl group, a 2-carboxylmethylcyclopropyl group, a 2-(2'-carboxylethyl)cyclopropyl group, a 2-(3'-carboxylpropyl)cyclopropyl group, a 2-(4'-carboxylbutyl)cyclopropyl group, a 2-(5'-carboxylpentyl)cyclopropyl group, a 2-(6'-carboxylhexyl)cyclopropyl group, a 2-(1'-hepten-3'-yl)cyclopropyl group, a 2-(1'-hexen-3'-ylmethyl)cyclopropyl group, an 2-(3'-ethyl-4'-pentenyl)cyclopropyl group, a 2-(4'-methyl-5'-hexenyl)cyclopropyl group, a 2-(6'-heptenyl)cyclopropyl group, an 2-(1'-octen-3'-yl)cyclopropyl group, a 2-(1'-hepten-3'-ylmethyl)cyclopropyl group, a 2-(1'-hexen-3'-ylethyl)cyclopropyl group, an 2-(4'-ethyl-5'-hexenyl)cyclopropyl group, a 2-(5'-methyl-6'-heptenyl)cyclopropyl group, an 2-(7'-octenyl)cyclopropyl group, a 2-(1',1'-difluoro-1'-hepten-3'-yl)cyclopropyl group, a 2-(1',1'-difluoro-1'-hexen-3'-ylmethyl)cyclopropyl group, a 2-(5',5'-difluoro-3'-ethyl-4'-pentenyl)cyclopropyl group, a 2-(6',6'-difluoro-4'-methyl-5'-hexenyl)cyclopropyl group, a 2-(7',7'-difluoro-6'-heptenyl)cyclopropyl group, a 2-(1',1'-difluoro-1'-octen-3'-yl)cyclopropyl group, a 2-(1',1'-difluoro-1'-hepten-3'-ylmethyl)cyclopropyl group, a 2-(1',1'-difluoro-1'-hexen-3'-ylethyl)cyclopropyl group, a 2-(6',6'-difluoro-4'-ethyl-5'-hexenyl)cyclopropyl group, a 2-(7',7'-difluoro-5'-methyl-6'-heptenyl)cyclopropyl group, a 2-(8',8'-difluoro-7'-octenyl)cyclopropyl group, a 2-vinylcyclopropyl group, a 2-(1'-propenyl)cyclopropyl group, a 2-(2'-propenyl)cyclopropyl group, a 2-(1'-methyl-1'-propenyl)cyclopropyl group, a 2-(1'-methyl-2'-propenyl)cyclopropyl group, a 2-(1',2'-dimethyl-1'-propenyl)cyclopropyl group, a 2-(1',1'-dimethyl-2'-propenyl)cyclopropyl group, an 2-(1'-ethyl-1'-propenyl)cyclopropyl group, an 2-(1'-ethyl-2'-propenyl)cyclopropyl group, a 2-(1'-butenyl)cyclopropyl group, a 2-(2'-butenyl)cyclopropyl group, a 2-(3'-butenyl)cyclopropyl group, a 2-(1'-methyl-1'-butenyl)cyclopropyl group, a 2-(2'-methyl-1'-butenyl)cyclopropyl group, a 2-(2'-methyl-2'-butenyl)cyclopropyl group, a 2-(2'-methyl-3'-butenyl)cyclopropyl group, a 2-(3'-methyl-3'-butenyl)cyclopropyl group, a 2-(1',1'-dimethyl-3'-butenyl)cyclopropyl group, a 2-(1',1'-dimethyl-2'-butenyl)cyclopropyl group, an 2-(1'-ethyl-1'-butenyl)cyclopropyl group, an 2-(1'-ethyl-2'-butenyl)cyclopropyl group, an 2-(1'-ethyl-3'-butenyl)cyclopropyl group, a 2-(1'-pentenyl)cyclopropyl group, a 2-(2'-pentenyl)cyclopropyl group, a 2-(3'-pentenyl)cyclopropyl group, a 2-(4'-pentenyl)cyclopropyl group, a 2-(1'-methyl-1'-pentenyl)cyclopropyl group, a 2-(1'-methyl-2'-pentenyl)cyclopropyl group, a 2-(1'-methyl-3'-pentenyl)cyclopropyl group, a 2-(1'-methyl-4'-pentenyl)cyclopropyl group, a 2-(2'-methyl-1'-pentenyl)cyclopropyl group, a 2-(2'-methyl-2'-pentenyl)cyclopropyl group, a 2-(3'-methyl-2'-pentenyl)cyclopropyl group, a 2-(3'-methyl-3'-pentenyl)cyclopropyl group, a 2-(4'-methyl-3'-pentenyl)cyclopropyl group, a 2-(4'-methyl-4'-pentenyl)cyclopropyl group, a 2-(1'-hexenyl)cyclopropyl group, a 2-(2'-hexenyl)cyclopropyl group, a 2-(3'-hexenyl)cyclopropyl group, a 2-(4'-hexenyl)cyclopropyl group, a 2-(5'-hexenyl)cyclopropyl group, a 2-(2',2'-difluorovinyl) group, a 2-(3',3'-difluoro-2'-propenyl) group, a 2-(4',4'-difluoro-3'-butenyl) group, a 2-(5',5'-difluoro-4'-pentenyl) group, a 2-(6',6'-difluoro-5'-hexenyl) group, a 2-(4'-methoxy-1'-butenyl)cyclopropyl group, a 2-(4'-methoxy-2'-butenyl)cyclopropyl group, a 2-(4'-methoxy-3'-butenyl)cyclopropyl group, a 2-(5'-methoxy-1'-pentenyl)cyclopropyl group, a 2-(5'-methoxy-2'-pentenyl)cyclopropyl group, a 2-(5'-methoxy-3'-pentenyl)cyclopropyl group, a 2-(5'-methoxy-4'-pentenyl)cyclopropyl group, a 2-(6'-methoxy-1'-hexenyl)cyclopropyl group, a 2-(6'-methoxy-2'-hexenyl)cyclopropyl group, a 2-(6'-methoxy-3'-hexenyl)cyclopropyl group, a 2-(6'-methoxy-4'-hexenyl)cyclopropyl group, a 2-(6'-methoxy-5'-hexenyl)cyclopropyl group, a 2-(4'-methylthio-1'-butenyl)cyclopropyl group, a 2-(4'-methylthio-2'-butenyl)cyclopropyl group, a 2-(4'-methylthio-3'-butenyl)cyclopropyl group, a 2-(5'-methylthio-1'-pentenyl)cyclopropyl group, a 2-(5'-methylthio-2'-pentenyl)cyclopropyl group, a 2-(5'-methylthio-3'-pentenyl)cyclopropyl group, a 2-(5'-methylthio-4'-pentenyl)cyclopropyl group, a 2-(6'-methylthio-1'-hexenyl)cyclopropyl group, a 2-(6'-methylthio-2'-hexenyl)cyclopropyl group, a 2-(6'-methylthio-3'-hexenyl)cyclopropyl group, a 2-(6'-methylthio-4'-hexenyl)cyclopropyl group, a 2-(6'-methylthio-5'-hexenyl)cyclopropyl group, a 2-(4'-methoxycarbonyl-1'-butenyl)cyclopropyl group, a 2-(4'-methoxycarbonyl-2'-butenyl)cyclopropyl group, a 2-(4'-methoxycarbonyl-3'-butenyl)cyclopropyl group, a 2-(5'-methoxycarbonyl-1'-pentenyl)cyclopropyl group, a 2-(5'-methoxycarbonyl-2'-pentenyl)cyclopropyl group, a 2-(5'-methoxycarbonyl-3'-pentenyl)cyclopropyl group, a 2-(5'-methoxycarbonyl-4'-pentenyl)cyclopropyl group, a 2-(6'-methoxycarbonyl-1'-hexenyl)cyclopropyl group, a 2-(6'-methoxycarbonyl-2'-hexenyl)cyclopropyl group, a 2-(6'-methoxycarbonyl-3'-hexenyl)cyclopropyl group, a 2-(6'-methoxycarbonyl-4'-hexenyl)cyclopropyl group, a 2-(6'-methoxycarbonyl-5'-hexenyl)cyclopropyl group, a 2-(4'-(N-methylcarboamide)-1'-butenyl)cyclopropyl group, a 2-(4'-(N-methylcarboamide)-2'-butenyl)cyclopropyl group, a 2-(4'-(N-methylcarboamide)-3'-butenyl)cyclopropyl group, a 2-(5'-(N-methylcarboamide)-1'-pentenyl)cyclopropyl group, a 2-(5'-(N-methylcarboamide)-2'-pentenyl)cyclopropyl group, a 2-(5'-(N-methylcarboamide)-3'-pentenyl)cyclopropyl group, a 2-(5'-(N-methylcarboamide)-4'-pentenyl)cyclopropyl group, a 2-(6'-(N-methylcarboamide)-1'-hexenyl)cyclopropyl group, a 2-(6'-(N-methylcarboamide)-2'-hexenyl)cyclopropyl group, a 2-(6'-(N-methylcarboamide)-3'-hexenyl)cyclopropyl group, a 2-(6'-(N-methylcarboamide)-4'-hexenyl)cyclopropyl group, a 2-(6'-(N-methylcarboamide)-5'-hexenyl)cyclopropyl group, a 2-(4'-(N,N-dimethylcarboamide)-1'-butenyl)cyclopropyl group, a 2-(4'-(N,N-dimethylcarboamide)-2'-butenyl)cyclopropyl group, a 2-(4'-(N,N-dimethylcarboamide)-3'-butenyl)cyclopropyl group, a 2-(5'-(N,N-dimethylcarboamide)-1'-pentenyl)cyclopropyl group, a 2-(5'-(N,N-dimethylcarboamide)-2'-pentenyl)cyclopropyl group, a 2-(5'-(N,N-dimethylcarboamide)-3'-pentenyl)cyclopropyl group, a 2-(5'-(N,N-dimethylcarboamide)-4'-pentenyl)cyclopropyl group, a 2-(6'-(N,N-dimethylcarboamide)-1'-hexenyl)cyclopropyl group, a 2-(6'-(N,N-dimethylcarboamide)-2'-hexenyl)cyclopropyl group, a 2-(6'-(N,N-dimethylcarboamide)-3'-hexenyl)cyclopropyl group, a 2-(6'-(N,N-dimethylcarboamide)-4'-hexenyl)cyclopropyl group, a 2-(6'-(N,N-dimethylcarboamide)-5'-hexenyl)cyclopropyl group, a 2-(4'-methoxyimino-1'-butenyl)cyclopropyl group, a 2-(4'-methoxyimino-2'-butenyl)cyclopropyl group, a 2-(4'-methoxyimino-3'-butenyl)cyclopropyl group, a 2-(5'-methoxyimino-1'-pentenyl)cyclopropyl group, a 2-(5'-methoxyimino-2'-pentenyl)cyclopropyl group, a 2-(5'-methoxyimino-3'-pentenyl)cyclopropyl group, a 2-(5'-methoxyimino-4'-pentenyl)cyclopropyl group, a 2-(6'-methoxyimino-1'-hexenyl)cyclopropyl group, a 2-(6'-methoxyimino-2'-hexenyl)cyclopropyl group, a 2-(6'-methoxyimino-3'-hexenyl)cyclopropyl group, a 2-(6'-methoxyimino-4'-hexenyl)cyclopropyl group, a 2-(6'-methoxyimino-5'-hexenyl)cyclopropyl group, a 2-(4'-carboxyl-1'-butenyl)cyclopropyl group, a 2-(4'-carboxyl-2'-butenyl)cyclopropyl group, a 2-(4'-carboxyl-3'-butenyl)cyclopropyl group, a 2-(5'-carboxyl-1'-pentenyl)cyclopropyl group, a 2-(5'-carboxyl-2'-pentenyl)cyclopropyl group, a 2-(5'-carboxyl-3'-pentenyl)cyclopropyl group, a 2-(5'-carboxyl-4'-pentenyl)cyclopropyl group, a 2-(6'-carboxyl-1'-hexenyl)cyclopropyl group, a 2-(6'-carboxyl-2'-hexenyl)cyclopropyl group, a 2-(6'-carboxyl-3'-hexenyl)cyclopropyl group, a 2-(6'-carboxyl-4'-hexenyl)cyclopropyl group, a 2-(6'-carboxyl-5'-hexenyl)cyclopropyl group, a 2-(1',3'-butadienyl)cyclopropyl group, a 2-(2',4'-pentadienyl)cyclopropyl group, a 2-(3',5'-hexadienyl)cyclopropyl group, a 2-(4',6'-heptadienyl)cyclopropyl group, an 2-(5',7'-octadienyl)cyclopropyl group, a 2-(4',4'-difluoro-1',3'-butadienyl)cyclopropyl group, a 2-(5',5'-difluoro-2',4'-pentadienyl)cyclopropyl group, a 2-(6',6'-difluoro-3',5'-hexadienyl)cyclopropyl group, a 2-(7',7'-difluoro-4',6'-heptadienyl)cyclopropyl group, a 2-(8',8'-difluoro-5',7'-octadienyl)cyclopropyl group, a 2-(9',9'-difluoro-6',8'-octadienyl)cyclopropyl group, a 2-(10',10'-difluoro-7',9'-octadienyl)cyclopropyl group, a 2-(but-1'-en-3'-ynyl)cyclopropyl group, a 2-(pent-2'-en-4'-ynyl)cyclopropyl group, a 2-(hex-3'-en-5'-ynyl)cyclopropyl group, a 2-(hept-4'-en-6'-ynyl)cyclopropyl group, an 2-(oct-5'-en-7'-ynyl)cyclopropyl group, a 2-(4'-fluorobut-1'-en-3'-ynyl)cyclopropyl group, a 2-(5'-fluoropent-2'-en-4'-ynyl)cyclopropyl group, a 2-(6'-fluorohex-3'-en-5'-ynyl)cyclopropyl group, a 2-(7'-fluorohept-4'-en-6'-ynyl)cyclopropyl group, a 2-(8'-fluorooct-5'-en-7'-ynyl)cyclopropyl group, an 2-ethynylcyclopropyl group, a 2-(1'-propynyl)cyclopropyl group, a 2-(2'-propynyl)cyclopropyl group, a 2-(1'-methyl-2'-propynyl)cyclopropyl group, a 2-(1',1'-dimethyl-2'-propynyl)cyclopropyl group, an 2-(1'-ethyl-2'-propynyl)cyclopropyl group, a 2-(1'-butynyl)cyclopropyl group, a 2-(2'-butynyl)cyclopropyl group, a 2-(3'-butynyl)cyclopropyl group, a 2-(1'-methyl-2'-butynyl)cyclopropyl group, a 2-(1'-methyl-3'-butynyl)cyclopropyl group, a 2-(1',1'-dimethyl-2'-butynyl)cyclopropyl group, a 2-(1',1'-dimethyl-3'-butynyl)cyclopropyl group, an 2-(1'-ethyl-2'-butynyl)cyclopropyl group, an 2-(1'-ethyl-3'-butynyl)cyclopropyl group, a 2-(1'-pentynyl)cyclopropyl group, a 2-(2'-pentynyl)cyclopropyl group, a 2-(3'-pentynyl)cyclopropyl group, a 2-(4'-pentynyl)cyclopropyl group, a 2-(1'-methyl-2'-pentynyl)cyclopropyl group, a 2-(1'-methyl-3'-pentynyl)cyclopropyl group, a 2-(1'-methyl-4'-pentynyl)cyclopropyl group, a 2-(1'-hexynyl)cyclopropyl group, a 2-(2'-hexynyl)cyclopropyl group, a 2-(3'-hexynyl)cyclopropyl group, a 2-(4'-hexynyl)cyclopropyl group, a 2-(5'-hexynyl)cyclopropyl group, a 2-(2'-fluoroethynyl)cyclopropyl group, a 2-(3'-fluoro-2'-propynyl)cyclopropyl group, a 2-(4'-fluoro-3'-butynyl)cyclopropyl group, a 2-(5'-fluoro-4'-pentynyl)cyclopropyl group, a 2-(6'-fluoro-4'-hexynyl)cyclopropyl group, a 2-(4'-methoxy-1'-butynyl)cyclopropyl group, a 2-(4'-methoxy-2'-butynyl)cyclopropyl group, a 2-(4'-methoxy-3'-butynyl)cyclopropyl group, a 2-(5'-methoxy-1'-pentynyl)cyclopropyl group, a 2-(5'-methoxy-2'-penynyl)cyclopropyl group, a 2-(5'-methoxy-3'-pentynyl)cyclopropyl group, a 2-(5'-methoxy-4'-pentynyl)cyclopropyl group, a 2-(6'-methoxy-1'-hexynyl)cyclopropyl group, a 2-(6'-methoxy-2'-hexynyl)cyclopropyl group, a 2-(6'-methoxy-3'-hexynyl)cyclopropyl group, a 2-(6'-methoxy-4'-hexynyl)cyclopropyl group, a 2-(6'-methoxy-5'-hexynyl)cyclopropyl group, a 2-(4'-methylthio-1'-butynyl)cyclopropyl group, a 2-(4'-methylthio-2'-butynyl)cyclopropyl group, a 2-(4'-methylthio-3'-butynyl)cyclopropyl group, a 2-(5'-methylthio-1'-pentynyl)cyclopropyl group, a 2-(5'-methylthio-2'-penynyl)cyclopropyl group, a 2-(5'-methylthio-3'-pentynyl)cyclopropyl group, a 2-(5'-methylthio-4'-pentynyl)cyclopropyl group, a 2-(6'-methylthio-1'-hexynyl)cyclopropyl group, a 2-(6'-methylthio-2'-hexynyl)cyclopropyl group, a 2-(6'-methylthio-3'-hexynyl)cyclopropyl group, a 2-(6'-methylthio-4'-hexynyl)cyclopropyl group, a 2-(6'-methylthio-5'-hexynyl)cyclopropyl group, a 2-(4'-methoxycarbonyl-1'-butynyl)cyclopropyl group, a 2-(4'-methoxycarbonyl-2'-butynyl)cyclopropyl group, a 2-(4'-methoxycarbonyl-3'-butynyl)cyclopropyl group, a 2-(5'-methoxycarbonyl-1'-pentynyl)cyclopropyl group, a 2-(5'-methoxycarbonyl-2'-penynyl)cyclopropyl group, a 2-(5'-methoxycarbonyl-3'-pentynyl)cyclopropyl group, a 2-(5'-methoxycarbonyl-4'-pentynyl)cyclopropyl group, a 2-(6'-methoxycarbonyl-1'-hexynyl)cyclopropyl group, a 2-(6'-methoxycarbonyl-2'-hexynyl)cyclopropyl group, a 2-(6'-methoxycarbonyl-3'-hexynyl)cyclopropyl group, a 2-(6'-methoxycarbonyl-4'-hexynyl)cyclopropyl group, a 2-(6'-methoxycarbonyl-5'-hexynyl)cyclopropyl group, a 2-(4'-(N-methylcarboamide)-1'-butynyl)cyclopropyl group, a 2-(4'-(N-methylcarboamide)-2'-butynyl)cyclopropyl group, a 2-(4'-(N-methylcarboamide)-3'-butynyl)cyclopropyl group, a 2-(5'-(N-methylcarboamide)-1'-pentynyl)cyclopropyl group, a 2-(5'-(N-methylcarboamide)-2'-penynyl)cyclopropyl group, a 2-(5'-(N-methylcarboamide)-3'-pentynyl)cyclopropyl group, a 2-(5'-(N-methylcarboamide)-4'-pentynyl)cyclopropyl group, a 2-(6'-(N-methylcarboamide)-1'-hexynyl)cyclopropyl group, a 2-(6'-(N-methylcarboamide)-2'-hexynyl)cyclopropyl group, a 2-(6'-(N-methylcarboamide)-3'-hexynyl)cyclopropyl group, a 2-(6'-(N-methylcarboamide)-4'-hexynyl)cyclopropyl group, a 2-(6'-(N-methylcarboamide)-5'-hexynyl)cyclopropyl group, a 2-(4'-(N,N-dimethylcarboamide)-1'-butynyl)cyclopropyl group, a 2-(4'-(N,N-dimethylcarboamide)-2'-butynyl)cyclopropyl group, a 2-(4'-(N,N-dimethylcarboamide)-3'-butynyl)cyclopropyl group, a 2-(5'-(N,N-dimethylcarboamide)-1'-pentynyl)cyclopropyl group, a 2-(5'-(N,N-dimethylcarboamide)-2'-penynyl)cyclopropyl group, a 2-(5'-(N,N-dimethylcarboamide)-3'-pentynyl)cyclopropyl group, a 2-(5'-(N,N-dimethylcarboamide)-4'-pentynyl)cyclopropyl group, a 2-(6'-(N,N-dimethylcarboamide)-1'-hexynyl)cyclopropyl group, a 2-(6'-(N,N-dimethylcarboamide)-2'-hexynyl)cyclopropyl group, a 2-(6'-(N,N-dimethylcarboamide)-3'-hexynyl)cyclopropyl group, a 2-(6'-(N,N-dimethylcarboamide)-4'-hexynyl)cyclopropyl group, a 2-(6'-(N,N-dimethylcarboamide)-5'-hexynyl)cyclopropyl group, a 2-(4'-methoxyimino-1'-butynyl)cyclopropyl group, a 2-(4'-methoxyimino-2'-butynyl)cyclopropyl group, a 2-(4'-methoxyimino-3'-butynyl)cyclopropyl group, a 2-(5'-methoxyimino-1'-pentynyl)cyclopropyl group, a 2-(5'-methoxyimino-2'-penynyl)cyclopropyl group, a 2-(5'-methoxyimino-3'-pentynyl)cyclopropyl group, a 2-(5'-methoxyimino-4'-pentynyl)cyclopropyl group, a 2-(6'-methoxyimino-1'-hexynyl)cyclopropyl group, a 2-(6'-methoxyimino-2'-hexynyl)cyclopropyl group, a 2-(6'-methoxyimino-3'-hexynyl)cyclopropyl group, a 2-(6'-methoxyimino-4'-hexynyl)cyclopropyl group, a 2-(6'-methoxyimino-5'-hexynyl)cyclopropyl group, a 2-(4'-carboxyl-1'-butynyl)cyclopropyl group, a 2-(4'-carboxyl-2'-butynyl)cyclopropyl group, a 2-(4'-carboxyl-3'-butynyl)cyclopropyl group, a 2-(5'-carboxyl-1'-pentynyl)cyclopropyl group, a 2-(5'-carboxyl-2'-penynyl)cyclopropyl group, a 2-(5'-carboxyl-3'-pentynyl)cyclopropyl group, a 2-(5'-carboxyl-4'-pentynyl)cyclopropyl group, a 2-(6'-carboxyl-1'-hexynyl)cyclopropyl group, a 2-(6'-carboxyl-2'-hexynyl)cyclopropyl group, a 2-(6'-carboxyl-3'-hexynyl)cyclopropyl group, a 2-(6'-carboxyl-4'-hexynyl)cyclopropyl group, a 2-(6'-carboxyl-5'-hexynyl)cyclopropyl group, a 2-(but-3'-en-1'-ynyl)cyclopropyl group, a 2-(pent-4'-en-2'-ynyl)cyclopropyl group, a 2-(hex-5'-en-3'-ynyl)cyclopropyl group, a 2-(hept-6'-en-4'-ynyl)cyclopropyl group, an 2-(oct-7'-en-5'-ynyl)cyclopropyl group, a 2-(4',4'-diflorobut-3'-en-1'-ynyl)cyclopropyl group, a 2-(5',5'-difluoropent-4'-en-2'-ynyl)cyclopropyl group, a 2-(6',6'-difluorohex-5'-en-3'-ynyl)cyclopropyl group, a 2-(7',7'-difluorohept-6'-en-4'-ynyl)cyclopropyl group, a 2-(8',8'-difluorooct-7'-en-5'-ynyl)cyclopropyl group, a 2-(1',3'-butadiynyl)cyclopropyl group, a 2-(2',4'-pentadiynyl)cyclopropyl group, a 2-(3',5'-hexadiynyl)cyclopropyl group, a 2-(4',6'-heptadiynyl)cyclopropyl group, an 2-(5',7'-octadiynyl)cyclopropyl group, a 2-(4'-fluoro-1',3'-butadiynyl)cyclopropyl group, a 2-(5'-fluoro-2',4'-pentadiynyl)cyclopropyl group, a 2-(6'-fluoro-3',5'-hexadiynyl)cyclopropyl group, a 2-(7'-fluoro-4',6'-heptadiynyl)cyclopropyl group, a 2-(8'-fluoro-5',7'-octadiynyl)cyclopropyl group and the like.

Examples of the "C1 to C5 alkyl groups optionally having one or more atoms or groups selected from group A" in the present invention include:

a methyl group, an ethyl group, an isopropyl group, a tert-butyl group, a propyl group, a sec-butyl group, an isobutyl group, a 1,1-dimethylpropyl group, a 2,2-dimethylpropyl group, a 1, 2-dimethylpropyl group, an 1-ethylpropyl group, a butyl group, a 1-methylbutyl group, a 2-methylbutyl group, a 3-methylbutyl group, a pentyl group, a trifluoromethyl group, a 2-chloroethyl group, a 2,2,2-trifluroethyl group, a 3,3,3-trifluropropyl group, a 4,4,4-triflurobutyl group, a 5,5,5-trifluropentyl group, a methoxymethyl group, a 2-methoxyethyl group, a 3-methoxypropyl group, a 4-methoxybutyl group, a 5-methoxypentyl group, a trifluoromethoxymethyl group, a 2-trifluoromethoxyethyl group, a 3-trifluoromethoxypropyl group, a 4-trifluoromethoxybutyl group, a 5-trifluoromethoxypentyl group, a 2,2,2-trifluroethoxymethyl group, a 2-(2,2,2-trifluroethoxy)ethyl group, a 3-(2,2,2-trifluroethoxy)propyl group, a 4-(2,2,2-trifluroethoxy)butyl group, a 5-(2,2,2-trifluroethoxy)pentyl group, a methylthiomethyl group, a 2-methylthioethyl group, a 3-methylthiopropyl group, a 4-methylthiobutyl group, a 5-methylthiopentyl group, a methylsulfinylmethyl group, a 2-methylsulfinylethyl group, a 3-methylsulfinylpropyl group, a 4-methylsulfinylbutyl group, a 5-methylsulfinylpentyl group, a methylsulfonylmethyl group, a 2-methylsulfonylethyl group, a 3-methylsulfonylpropyl group, a 4-methylsulfonylbutyl group, a 5-methylsulfonylpentyl group, a methoxycarbonylmethyl group, a 2-methoxycarbonylethyl group, a 3-methoxycarbonylpropyl group, a 4-methoxycarbonylbutyl group, a 5-methoxycarbonylpentyl group, an N-methylcarboamidemethyl group, a 2-(N-methylcarboamide)ethyl group, a 3-(N-methylcarboamide)propyl group, a 4-(N-methylcarboamide)butyl group, a 5-(N-methylcarboamide)pentyl group, an N,N-dimethylcarboamidemethyl group, a 2-(N,N-dimethylcarboamide)ethyl group, a 3-(N,N-dimethylcarboamide)propyl group, a 4-(N,N-dimethylcarboamide)butyl group, a 5-(N,N-dimethylcarboamide)pentyl group, a methoxyiminomethyl group, a 2-methoxyiminoethyl group, a 3-methoxyiminopropyl group, a 4-methoxyiminobutyl group, a carboxymethyl group, a 2-carboxyethyl group, a 3-carboxypropyl group, a 4-carboxybutyl group, a 5-carboxypentyl group and the like.

Examples of the "C2 to C5 alkenyl groups optionally having one or more atoms or groups selected from group A" in the present invention include:

a vinyl group, a 1-propenyl group, a 2-propenyl group, a 1-methyl-1-propenyl group, a 2-methyl-1-propenyl group, a 1-methyl-2-propenyl group, a 1,2-dimethyl-1-propenyl group, a 1,1-dimethyl-2-propenyl group, an 1-ethyl-1-propenyl group, an 1-ethyl-2-propenyl group, a 1-butenyl group, a 2-butenyl group, a 3-butenyl group, a 1-methyl-1-butenyl group, a 2-methyl-1-butenyl group, a 2-methyl-2-butenyl group, a 2-methyl-3-butenyl group, a 3-methyl-3-butenyl group, a 1-pentenyl group, a 2-pentenyl group, a 3-pentenyl group, a 4-pentenyl group, a propen-2-yl group, a 1-buten-2-yl group, a 1-buten-3-yl group, a 1-penten-2-yl group, a 1-penten-3-yl group, a propen-2-ylmethyl group, a 1-buten-2-ylmethyl group, a 1-buten-3-ylmethyl group, a propen-2-ylethyl group, a 2,2-difluorovinyl group, a 3,3-difluoro-2-propenyl group, a 4,4-difluoro-3-butenyl group, a 5,5-difluoro-4-pentenyl group, a 2-methoxyvinyl group, a 3-methoxy-1-propenyl group, a 3-methoxy-2-propenyl group, a 4-methoxy-1-butenyl group, a 4-methoxy-2-butenyl group, a 4-methoxy-3-butenyl group, a 5-methoxy-1-pentenyl group, a 5-methoxy-2-pentenyl group, a 5-methoxy-3-pentenyl group, a 5-methoxy-4-pentenyl group, a 2-methylthiovinyl group, a 3-methylthio-1-propenyl group, a 3-methylthio-2-propenyl group, a 4-methylthio-1-butenyl group, a 4-methylthio-2-butenyl group, a 4-methylthio-3-butenyl group, a 5-methylthio-1-pentenyl group, a 5-methylthio-2-pentenyl group, a 5-methylthio-3-pentenyl group, a 5-methylthio-4-pentenyl group, a 2-methoxycarbonylvinyl group, a 3-methoxycarbonyl-1-propenyl group, a 3-methoxycarbonyl-2-propenyl group, a 4-methoxycarbonyl-1-butenyl group, a 4-methoxycarbonyl-2-butenyl group, a 4-methoxycarbonyl-3-butenyl group, a 5-methoxycarbonyl-1-pentenyl group, a 5-methoxycarbonyl-2-pentenyl group, a 5-methoxycarbonyl-3-pentenyl group, a 5-methoxycarbonyl-4-pentenyl group, a 2-(N-methylcarboamide)vinyl group, a 3-(N-methylcarboamide)-1-propenyl group, a 3-(N-methylcarboamide)-2-propenyl group, a 4-(N-methylcarboamide)-1-butenyl group, a 4-(N-methylcarboamide)-2-butenyl group, a 4-(N-methylcarboamide)-3-butenyl group, a 5-(N-methylcarboamide)-1-pentenyl group, a 5-(N-methylcarboamide)-2-pentenyl group, a 5-(N-methylcarboamide)-3-pentenyl group, a 5-(N-methylcarboamide)-4-pentenyl group, a 2-(N,N-dimethylcarboamide)vinyl group, a 3-(N,N-dimethylcarboamide)-1-propenyl group, a 3-(N,N-dimethylcarboamide)-2-propenyl group, a 4-(N,N-dimethylcarboamide)-1-butenyl group, a 4-(N,N-dimethylcarboamide)-2-butenyl group, a 4-(N,N-dimethylcarboamide)-3-butenyl group, a 5-(N,N-dimethylcarboamide)-1-pentenyl group, a 5-(N,N-dimethylcarboamide)-2-pentenyl group, a 5-(N,N-dimethylcarboamide)-3-pentenyl group, a 5-(N,N-dimethylcarboamide)-4-pentenyl group, a 3-methoxyimino-1-propenyl group, a 4-methoxyimino-1-butenyl group, a 4-methoxyimino-2-butenyl group, a 2-carboxylvinyl group, a 3-carboxyl-1-propenyl group, a 3-carboxyl-2-propenyl group, a 4-carboxyl-1-butenyl group, a 4-carboxyl-2-butenyl group, a 4-carboxyl-3-butenyl group, a 5-carboxyl-1-pentenyl group, a 5-carboxyl-2-pentenyl group, a 5-carboxyl-3-pentenyl group, a 5-carboxyl-4-pentenyl group and the like.

Examples of the "C2 to C5 alkynyl groups optionally having one or more atoms or groups selected from group A" in the present invention include:

an ethynyl group, a 1-propynyl group, a 2-propynyl group, a 1-methyl-2-propynyl group, a 1,1-dimethyl-2-propynyl group, an 1-ethyl-2-propynyl group, a 1-butynyl group, a 2-butynyl group, a 3-butynyl group, a 1-methyl-2-butynyl group, a 1-methyl-3-butynyl group, a 1-pentynyl group, a 2-pentynyl group, a 3-pentynyl group, a 4-pentynyl group, a 1-butyn-3-yl group, a 1-pentyn-3-yl group, a 1-butyn-3-ylmethyl group, a 2-methoxyethynyl group, a 3-methoxy-1-propynyl group, a 3-methoxy-2-propynyl group, a 4-methoxy-1-butynyl group, a 4-methoxy-2-butynyl group, a 4-methoxy-3-butynyl group, a 5-methoxy-1-pentynyl group, a 5-methoxy-2-pentynyl group, a 5-methoxy-3-pentynyl group, a 5-methoxy-4-pentynyl group, a 2-methylthioethynyl group, a 3-methylthio-1-propynyl group, a 3-methylthio-2-propynyl group, a 4-methylthio-1-butynyl group, a 4-methylthio-2-butynyl group, a 4-methylthio-3-butynyl group, a 5-methylthio-1-pentynyl group, a 5-methylthio-2-pentynyl group, a 5-methylthio-3-pentynyl group, a 5-methylthio-4-pentynyl group, a 2-methoxycarbonylethynyl group, a 3-methoxycarbonyl-1-propynyl group, a 3-methoxycarbonyl-2-propynyl group, a 4-methoxycarbonyl-1-butynyl group, a 4-methoxycarbonyl-2-butynyl group, a 4-methoxycarbonyl-3-butynyl group, a 5-methoxycarbonyl-1-pentynyl group, a 5-methoxycarbonyl-2-pentynyl group, a 5-methoxycarbonyl-3-pentynyl group, a 5-methoxycarbonyl-4-pentynyl group, a 2-(N-methylcarboamide)ethynyl group, a 3-(N-methylcarboamide)-1-propynyl group, a 3-(N-methylcarboamide)-2-propynyl group, a 4-(N-methylcarboamide)-1-butynyl group, a 4-(N-methylcarboamide)-2-butynyl group, a 4-(N-methylcarboamide)-3-butynyl group, a 5-(N-methylcarboamide)-1-pentynyl group, a 5-(N-methylcarboamide)-2-pentynyl group, a 5-(N-methylcarboamide)-3-pentynyl group, a 5-(N-methylcarboamide)-4-pentynyl group, a 2-(N,N-dimethylcarboamide)ethynyl group, a 3-(N,N-dimethylcarboamide)-1-propynyl group, a 3-(N,N-dimethylcarboamide)-2-propynyl group, a 4-(N,N-dimethylcarboamide)-1-butynyl group, a 4-(N,N-dimethylcarboamide)-2-butynyl group, a 4-(N,N-dimethylcarboamide)-3-butynyl group, a 5-(N,N-dimethylcarboamide)-1-pentynyl group, a 5-(N,N-dimethylcarboamide)-2-pentynyl group, a 5-(N,N-dimethylcarboamide)-3-pentynyl group, a 5-(N,N-dimethylcarboamide)-4-pentynyl group, a 3-(methoxyimino)-1-propynyl group, a 4-(methoxyimino)-1-butynyl group, a 4-(methoxyimino)-2-butynyl group, a 2-carboxylethynyl group, a 3-carboxyl-1-propynyl group, a 3-carboxyl-2-propynyl group, a 4-carboxyl-1-butynyl group, a 4-carboxyl-2-butynyl group, a 4-carboxyl-3-butynyl group, a 5-carboxyl-1-pentynyl group, a 5-carboxyl-2-pentynyl group, a 5-carboxyl-3-pentynyl group, a 5-carboxyl-4-pentynyl group and the like.

Examples of the "C1 to C5 alkyl groups" in the present invention include a methyl group, an ethyl group, an isopropyl group, a tert-butyl group, a propyl group, a sec-butyl group, an isobutyl group, a 1,1-dimethylpropyl group, a 2,2-dimethylpropyl group, a 1,2-dimethylpropyl group, a 1-ethylpropyl group, a butyl group, a 1-methylbutyl group, a 2-methylbutyl group, a 3-methylbutyl group and a pentyl group.

Examples of the "C2 to C5 alkenyl groups" in the present invention include a vinyl group, a 1-propenyl group, a 2-propenyl group, a 1-methyl-1-propenyl group, a 2-methyl-1-propenyl group, a 1-methyl-2-propenyl group, a 1,2-dimethyl-1-propenyl group, a 1,1-dimethyl-2-propenyl group, an 1-ethyl-1-propenyl group, an 1-ethyl-2-propenyl group, a 1-butenyl group, a 2-butenyl group, a 3-butenyl group, a 1-methyl-1-butenyl group, a 2-methyl-1-butenyl group, a 2-methyl-2-butenyl group, a 2-methyl-3-butenyl group, a 3-methyl-3-butenyl group, a 1-pentenyl group, a 2-pentenyl group, a 3-pentenyl group, a 4-pentenyl group, a propen-2-yl group, a 1-buten-2-yl group, a 1-buten-3-yl group, a 1-penten-2-yl group, a 1-penten-3-yl group, a propen-2-ylmethyl group, a 1-buten-2-ylmethyl group, a 1-buten-3-ylmethyl group, a propen-2-ylethyl group and the like.

Examples of the "C2 to C5 alkenyl groups" in the present invention include:

an ethynyl group, a 1-propynyl group, a 2-propynyl group, a 1-methyl-2-propynyl group, a 1,1-dimethyl-2-propynyl group, a 1-ethyl-2-propynyl group, a 1-butynyl group, a 2-butynyl group, a 3-butynyl group, a 1-methyl-2-butynyl group, a 1-methyl-3-butynyl group, a 1-pentynyl group, a 2-pentynyl group, a 3-pentynyl group, a 4-pentynyl group, a 1-butyn-3-yl group, a 1-pentyn-3-yl group, a 1-butyn-3-ylmethyl group and the like.

Examples of the compound of the present invention include the following compounds.
In the formula (I), compounds wherein X is a C1 to C5 chain hydrocarbon group;
In the formula (I), compounds wherein X is a C1 to C6 chain hydrocarbon group;
In the formula (I), compounds wherein X is a methyl group;
In the formula (I), compounds wherein X is an ethyl group;
In the formula (I), compounds wherein X is a propyl group;
In the formula (I), compounds wherein X is a butyl group;
In the formula (I), compounds wherein X is a pentyl group;
In the formula (I), compounds wherein X is a hexyl group;
In the formula (I), compounds wherein X is a C1 to C5 alkyl group;
In the formula (I), compounds wherein X is a C1 to C6 alkyl group;
In the formula (I), compounds wherein X is a trifluoromethyl group;
In the formula (I), compounds wherein X is a 2,2,2-trifluoroethyl group;
In the formula (I), compounds wherein X is a 3,3,3-trifluoropropyl group;
In the formula (I), compounds wherein X is a 4,4,4-trifluorobutyl group;
In the formula (I), compounds wherein X is a 5,5,5-trifluoropentyl group;
In the formula (I), compounds wherein X is a 2-chloroethyl group;
In the formula (I), compounds wherein X is a 2-methoxymethyl group;
In the formula (I), compounds wherein X is a 2-methoxyethyl group;
In the formula (I), compounds wherein X is a 3-methoxypropyl group;
In the formula (I), compounds wherein X is a 4-methoxybutyl group;
In the formula (I), compounds wherein X is a 5-methoxypentyl group;
In the formula (I), compounds wherein X is a C1 to C5 alkyl group optionally having a fluorine atom or a methoxy group;
In the formula (I), compounds wherein X is a vinyl group;
In the formula (I), compounds wherein X is a 1-propenyl group;
In the formula (I), compounds wherein X is a 2-methyl-1-propenyl group;
In the formula (I), compounds wherein X is a 2-propenyl group;
In the formula (I), compounds wherein X is a 1-butenyl group;
In the formula (I), compounds wherein X is a 2-butenyl group;
In the formula (I), compounds wherein X is a 3-butenyl group;
In the formula (I), compounds wherein X is a 1-pentenyl group;
In the formula (I), compounds wherein X is a 2-pentenyl group;
In the formula (I), compounds wherein X is a 3-pentenyl group;
In the formula (I), compounds wherein X is a 4-pentenyl group;
In the formula (I), compounds wherein X is a C2 to C5 alkenyl group;
In the formula (I), compounds wherein X is a 2,2-difluorovinyl group;
In the formula (I), compounds wherein X is a 3,3-difluoro-2-propenyl group;
In the formula (I), compounds wherein X is a 4,4-difluoro-3-butenyl group;
In the formula (I), compounds wherein X is a 5,5-difluoro-4-pentenyl group;

In the formula (I), compounds wherein X is a 4-methoxy-1-butenyl group;
In the formula (I), compounds wherein X is a 4-methoxy-2-butenyl group;
In the formula (I), compounds wherein X is a 4-methoxy-3-butenyl group;
In the formula (I), compounds wherein X is a 5-methoxy-1-pentenyl group;
In the formula (I), compounds wherein X is a 5-methoxy-2-pentenyl group;
In the formula (I), compounds wherein X is a 5-methoxy-3-pentenyl group;
In the formula (I), compounds wherein X is a 5-methoxy-4-pentenyl group;
In the formula (I), compounds wherein X is a C2 to C5 alkenyl group optionally having a fluorine atom or a methoxy group;
In the formula (I), compounds wherein X is an ethynyl group;
In the formula (I), compounds wherein X is a 1-propynyl group;
In the formula (I), compounds wherein X is a 2-propynyl group;
In the formula (I), compounds wherein X is a 1-butynyl group;
In the formula (I), compounds wherein X is a 2-butynyl group;
In the formula (I), compounds wherein X is a 3-butynyl group;
In the formula (I), compounds wherein X is a 1-pentynyl group;
In the formula (I), compounds wherein X is a 2-pentynyl group;
In the formula (I), compounds wherein X is a 3-pentynyl group;
In the formula (I), compounds wherein X is a 4-pentynyl group;
In the formula (I), compounds wherein X is a C2 to C5 alkynyl group;
In the formula (I), compounds wherein X is a 2-methyl-cyclopropyl group;
In the formula (I), compounds wherein X is an 2-ethyl-cyclopropyl group;
In the formula (I), compounds wherein the relative configuration of $R^1$ and $R^2$ is a trans configuration, more specifically, compounds represented by formula (III);

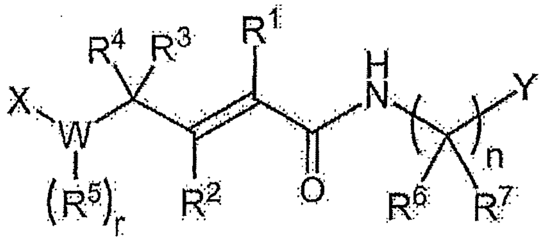 (III)

In the formula (I), compounds that are rich in compounds wherein the relative configuration of $R^1$ and $R^2$ is a trans configuration; In the formula (I), compounds that contain 80% or more of compounds wherein the relative configuration of $R^1$ and $R^2$ is a trans configuration;
In the formula (I), compounds that contain 90% or more of compounds wherein the relative configuration of $R^1$ and $R^2$ is a trans configuration;
In the formula (I), compounds wherein $R^1$ is a hydrogen atom or a halogen atom, and $R^2$ is a C1 to C4 alkyl group optionally having one or more halogen atoms or a hydrogen atom;
In the formula (I), compounds wherein $R^1$ is a hydrogen atom or a fluorine atom, and $R^2$ is a methyl group or a hydrogen atom; In the formula (I), compounds wherein W is $-CR^8-$, and r is 1, more specifically, compounds represented by formula (IV);

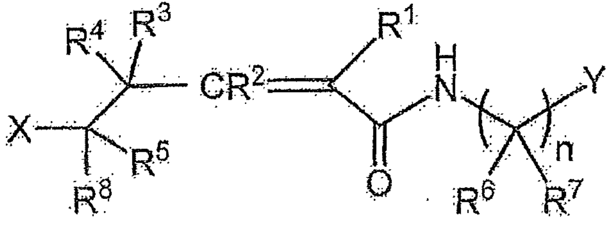 (IV)

In the formula (IV), compounds wherein $R^8$ is a C1 to C4 alkyl group optionally having one or more halogen atoms, a hydrogen atom or a halogen atom, $R^5$ is a C1 to C4 alkyl group optionally having one or more halogen atoms, a hydrogen atom or a halogen atom, and $R^3$ and $R^4$ are the same or different and are a C1 to C4 alkyl group optionally having one or more halogen atoms, a hydrogen atom or a halogen atom;
In the formula (IV), compounds wherein $R^8$ is a methyl group optionally having one or more halogen atoms, a hydrogen

atom or a halogen atom, $R^5$ is a methyl group optionally having one or more halogen atoms, a hydrogen atom or a halogen atom, and $R^3$ and $R^4$ are the same or different and are a methyl group optionally having one or more halogen atoms, a hydrogen atom or a halogen atom;

In the formula (IV), compounds wherein $R^8$ is a methyl group or a hydrogen atom, $R^5$ is a methyl group or a hydrogen atom, and $R^3$ and $R^9$ are a hydrogen atom;

In the formula (IV), compounds wherein $R^8$ is a hydrogen atom, $R^5$ is a hydrogen atom, and $R^3$ and $R^4$ are a hydrogen atom;

In the formula (IV), compounds wherein $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, and $R^2$ is a methyl group or a hydrogen atom (hereinafter represented as compound (IV-1));

In the formula (IV), compounds wherein $R^8$ is a hydrogen atom, $R^5$ is a hydrogen atom, $R^3$ and $R^4$ are a hydrogen atom, $R^1$ is a hydrogen atom or a fluorine atom, and $R^2$ is a methyl group or a hydrogen atom (hereinafter represented as compound (IV-2));

In the formula (IV-1), compounds wherein the relative configuration of $R^1$ and $R^2$ is a trans configuration;

In the formula (IV-1), compounds that are rich in compounds wherein the relative configuration of $R^1$ and $R^2$ is a trans configuration;

In the formula (IV-1), compounds that contain 80% or more of compounds wherein the relative configuration of $R^1$ and $R^2$ is a trans configuration;

In the formula (IV-1), compounds that contain 90% or more of compounds wherein the relative configuration of $R^1$ and $R^2$ is a trans configuration;

In the formula (IV-2), compounds wherein the relative configuration of $R^1$ and $R^2$ is a trans configuration;

In the formula (IV-2), compounds that are rich in compounds wherein the relative configuration of $R^1$ and $R^2$ is a trans configuration;

In the formula (IV-2), compounds that contain 80% or more of compounds wherein the relative configuration of $R^1$ and $R^2$ is a trans configuration;

In the formula (IV-2), compounds that contain 90% or more of compounds wherein the relative configuration of $R^1$ and $R^2$ is a trans configuration;

In the formula (I), compounds wherein W is $-CR^8-$, r is 1, and $R^3$ and $R^5$ are taken together to be $-(CR^9R^{10})_v-$, more specifically, compounds represented by formula (V);

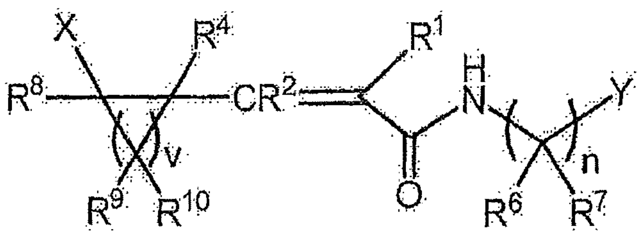

(V)

In the formula (V), compounds wherein $R^4$ is a C1 to C4 alkyl group optionally having one or more halogen atoms, a hydrogen atom or a halogen atom, $R^8$ is a C1 to C4 alkyl group optionally having one or more halogen atoms, a hydrogen atom or a halogen atom, and $R^9$ and $R^{10}$ are the same or different and are a C1 to C4 alkyl group optionally having one or more halogen atoms, a hydrogen atom or a halogen atom;

In the formula (V), compounds wherein v is 1, $R^4$ is a C1 to C4 alkyl group optionally having one or more halogen atoms, a hydrogen atom or a halogen atom, $R^8$ is a C1 to C4 alkyl group optionally having one or more halogen atoms, a hydrogen atom or a halogen atom, and $R^9$ and $R^{10}$ are the same or different and are a C1 to C4 alkyl group optionally having one or more halogen atoms, a hydrogen atom or a halogen atom;

In the formula (V), compounds wherein v is 1, $R^4$ is a methyl group optionally having one or more halogen atoms, a hydrogen atom or a halogen atom, $R^8$ is a methyl group optionally having one or more halogen atoms, a hydrogen atom or a halogen atom, and $R^9$ and $R^{10}$ are the same or different and are a methyl group optionally having one or more halogen atoms, a hydrogen atom or a halogen atom;

In the formula (V), compounds wherein v is 1, $R^4$ is a methyl group, a hydrogen atom or a fluorine atom, $R^8$ is a methyl group or a hydrogen atom, and $R^9$ and $R^{10}$ are the same or different and are a methyl group or a hydrogen atom;

In the formula (V), compounds wherein v is 1, $R^4$ is a hydrogen atom, $R^8$ is a hydrogen atom, and $R^9$ and $R^{10}$ are a hydrogen atom;

In the formula (V), compounds wherein v is 1, $R^4$ is a hydrogen atom, $R^8$ is a hydrogen atom, and $R^9$ and $R^{10}$ are a methyl group;

In the formula (V), compounds wherein v is 1, the relative configuration of $R^4$ and $R^8$ is trans, $R^4$ is a methyl group, a hydrogen atom or a fluorine group, $R^8$ is a methyl group or a hydrogen atom, and $R^9$ and $R^{10}$ are the same or different

and are a methyl group or a hydrogen atom;
In the formula (V), compounds wherein v is 1, the relative configuration of $R^4$ and $R^8$ is trans, $R^4$ is a hydrogen atom, $R^8$ is a hydrogen atom, and $R^9$ and $R^{10}$ are a hydrogen atom;
In the formula (V), compounds wherein v is 1, the relative configuration of $R^4$ and $R^8$ is trans, $R^4$ is a methyl group, a hydrogen atom or a fluorine atom, $R^8$ is a methyl group or a hydrogen atom, $R^9$ and $R^{10}$ are the same or different and are a methyl group or a hydrogen atom, $R^1$ is a hydrogen atom or a fluorine atom, and $R^2$ is a methyl group or a hydrogen atom (hereinafter represented as compound (V-1));
In the formula (V), compounds wherein v is 1, the relative configuration of $R^4$ and $R^8$ is trans, $R^4$ is a hydrogen atom, $R^8$ is a hydrogen atom, $R^9$ and $R^{10}$ are a hydrogen atom, $R^1$ is a hydrogen atom or a fluorine atom, and $R^2$ is a methyl group or a hydrogen atom (hereinafter represented as compound (V-2));
In the formula (V), compounds wherein v is 1, the relative configuration of $R^4$ and $R^8$ is trans, $R^4$ is a hydrogen atom, $R^8$ is a hydrogen atom, $R^9$ and $R^{10}$ are a methyl group, $R^1$ is a hydrogen atom or a fluorine atom, and $R^2$ is a methyl group or a hydrogen atom (hereinafter represented as compound (V-3));
In the formula (V-1), compounds wherein the relative configuration of $R^1$ and $R^2$ is a trans configuration;
In the formula (V-1), compounds that are rich in compounds wherein the relative configuration of $R^1$ and $R^2$ is a trans configuration;
In the formula (V-1), compounds that contain 80% or more of compounds wherein the relative configuration of $R^1$ and $R^2$ is a trans configuration;
In the formula (V-1), compounds that contain 90% or more of compounds wherein the relative configuration of $R^1$ and $R^2$ is a trans configuration;
In the formula (V-2), compounds wherein the relative configuration of $R^1$ and $R^2$ is a trans configuration;
In the formula (V-2), compounds that are rich in compounds wherein the relative configuration of $R^1$ and $R^2$ is a trans configuration;
In the formula (V-2), compounds that contain 80% or more of compounds wherein the relative configuration of $R^1$ and $R^2$ is a trans configuration;
In the formula (V-2), compounds that contain 90% or more of compounds wherein the relative configuration of $R^1$ and $R^2$ is a trans configuration;
In the formula (V-3), compounds wherein the relative configuration of $R^1$ and $R^2$ is a trans configuration;
In the formula (V-3), compounds that are rich in compounds wherein the relative configuration of $R^1$ and $R^2$ is a trans configuration;
In the formula (V-3), compounds that contain 80% or more of compounds wherein the relative configuration of $R^1$ and $R^2$ is a trans configuration;
In the formula (V-3), compounds that contain 90% or more of compounds wherein the relative configuration of $R^1$ and $R^2$ is a trans configuration;
In the formula (I), compounds wherein $R^6$ and $R^7$ are the same or different and are a C1 to C4 alkyl group optionally having one or more halogen atoms or a hydrogen atom;
In the formula (I), compounds wherein $R^6$ and $R^7$ are the same or different and are a methyl group optionally having one or more halogen atoms or a hydrogen atom;
In the formula (I), compounds wherein $R^6$ and $R^7$ are the same or different and are a methyl group or a hydrogen atom;
In the formula (I), compounds wherein Y is a 4 to 6-membered saturated heterocyclic ring which contains, as ring-forming component(s), one or two oxygen atoms;
In the formula (I), compounds wherein Y is a 4 to 6-membered saturated heterocyclic ring which contains, as ring-forming component(s), one oxygen atom;
In the formula (I), compounds wherein Y is a 4 to 6-membered saturated heterocyclic ring which contains, as ring-forming component(s), two oxygen atoms;
In the formula (I), compounds wherein Y is a 4 to 6-membered saturated heterocyclic ring which contains, as ring-forming component(s), one sulfur atom;
In the formula (I), compounds wherein Y is a saturated 4 to 6-membered ring which contains, as ring-forming component(s), one oxygen atom or one $-S(O)_t-$;
In the formula (I), compounds wherein Y is a 4 to 6-membered saturated heterocyclic ring which contains, as ring-forming component(s), one oxygen atom or one $-S(O)_t-$, and t is 0;
In the formula (I), compounds wherein Y is a group represented by formula (II-a),

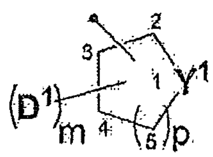

(II-a)

$Y^1$ is an oxygen atom or a sulfur atom, m is 0 or 1, and p is 0, 1 or 2, more specifically, compounds represented by formula (I-A);

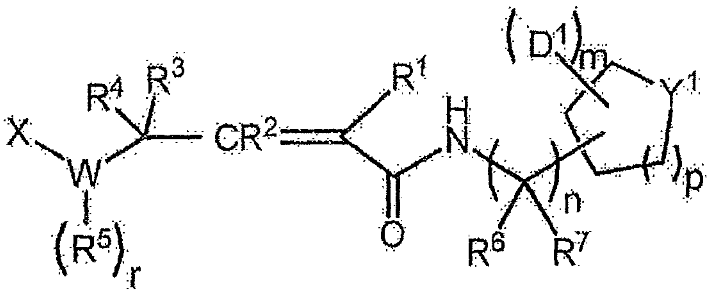

( I -A)

In the formula (I-A), compounds wherein $Y^1$ is an oxygen atom or a sulfur atom, m is 0 or 1, p is 0, 1 or 2, and $D^1$ is an ethyl group or a methyl group;
In the formula (I-A), compounds wherein $Y^1$ is an oxygen atom or a sulfur atom, m is 0 or 1, p is 0, 1 or 2, $D^1$ is an ethyl group or a methyl group, and n is 1;
In the formula (IV), compounds wherein Y is a group represented by formula (II-a), $Y^1$ is an oxygen atom or a sulfur atom, m is 0 or 1, and p is 0, 1 or 2, more specifically, compounds represented by formula (IV-A);

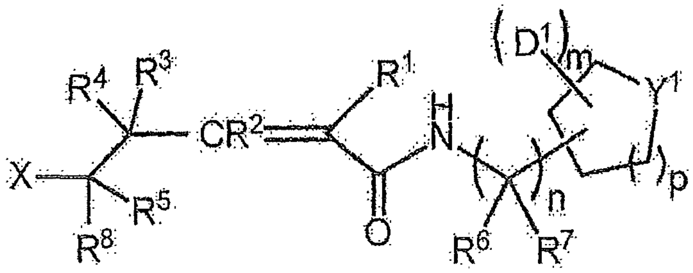

(IV-A)

In the formula (IV-A), compounds wherein $D^1$ is an ethyl group or a methyl group;
In the formula (IV-A), compounds wherein $D^1$ is an ethyl group or a methyl group, and n is 1;
In the formula (IV-A), compounds wherein $D^1$ is an ethyl group or a methyl group, n is l, and $R^6$ and $R^7$ are the same or different and are a methyl group or a hydrogen atom;
In the formula (IV-A), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, and $R^3$ and $R^4$ are a hydrogen atom;
In the formula (IV-A), compounds wherein $D^1$ is an ethyl group or a methyl group, n is l, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, and $R^2$ is a methyl group or a hydrogen atom;
In the formula (IV-A), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom, a methoxy group or a methylthio group (hereinafter represented as compound (IV-A-1));
In the formula (IV-A), compounds wherein $D^1$ is an ethyl group or a methyl group, n is 1, $R^6$ and $R^1$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a halogen atom, a methoxy group or a methylthio group (hereinafter represented as compound (IV-A-2));
In the formula (IV-A), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or

a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkynyl group optionally having one or more atoms or groups selected from group A, and the one or more atoms or groups selected from group A is a halogen atom, a methoxy group or a methylthio group (hereinafter represented as compound (IV-A-3));

In the formula (IV-R), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a chlorine atom (hereinafter represented as compound (IV-A-4));

In the formula (IV-A), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom (hereinafter represented as compound (IV-A-5));

In the formula (IV-A), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a methoxy group (hereinafter represented as compound (IV-A-6));

In the formula (IV-A-1), compounds wherein the relative configuration of $R^1$ and $R^2$ is a trans configuration;

In the formula (IV-A-1), compounds that are rich in compounds wherein the relative configuration of $R^1$ and $R^2$ is a trans configuration;

In the formula (IV-A-1), compounds that contain 80% or more of compounds wherein the relative configuration of $R^1$ and $R^2$ is a trans configuration;

In the formula (IV-A-1), compounds that contain 90% or more of compounds wherein the relative configuration of $R^1$ and $R^2$ is a trans configuration;

In the formula (IV-A-2), compounds wherein the relative configuration of $R^1$ and $R^2$ is a trans configuration;

In the formula (IV-A-2), compounds that are rich in compounds wherein the relative configuration of $R^1$ and $R^2$ is a trans configuration;

In the formula (IV-A-2), compounds that contain 80% or more of compounds wherein the relative configuration of $R^1$ and $R^2$ is a trans configuration;

In the formula (IV-A-2), compounds that contain 90% or more of compounds wherein the relative configuration of $R^1$ and $R^2$ is a trans configuration;

In the formula (IV-A-3), compounds wherein the relative configuration of $R^1$ and $R^2$ is a trans configuration;

In the formula (IV-A-3), compounds that are rich in compounds wherein the relative configuration of $R^1$ and $R^2$ is a trans configuration;

In the formula (IV-A-3), compounds that contain 80% or more of compounds wherein the relative configuration of $R^1$ and $R^2$ is a trans configuration;

In the formula (IV-A-3), compounds that contain 90% or more of compounds wherein the relative configuration of $R^1$ and $R^2$ is a trans configuration;

In the formula (IV-A-4), compounds wherein the relative configuration of $R^1$ and $R^2$ is a trans configuration;

In the formula (IV-A-4), compounds that are rich in compounds wherein the relative configuration of $R^1$ and $R^2$ is a trans configuration;

In the formula (IV-A-4), compounds that contain 80% or more of compounds wherein the relative configuration of $R^1$ and $R^2$ is a trans configuration;

In the formula (IV-A-4), compounds that contain 90% or more of compounds wherein the relative configuration of $R^1$ and $R^2$ is a trans configuration;

In the formula (IV-A-5), compounds wherein the relative configuration of $R^1$ and $R^2$ is a trans configuration;

In the formula (IV-A-5), compounds that are rich in compounds wherein the relative configuration of $R^1$ and $R^2$ is a trans configuration;

In the formula (IV-A-5), compounds that contain 80% or more of compounds wherein the relative configuration of $R^1$ and $R^2$ is a trans configuration;

In the formula (IV-A-5), compounds that contain 90% or more of compounds wherein the relative configuration of $R^1$ and $R^2$ is a trans configuration;

In the formula (IV-A-6), compounds wherein the relative configuration of $R^1$ and $R^2$ is a trans configuration;
In the formula (IV-A-6), compounds that are rich in compounds wherein the relative configuration of $R^1$ and $R^2$ is a trans configuration;
In the formula (IV-A-6), compounds that contain 80% or more of compounds wherein the relative configuration of $R^1$ and $R^2$ is a trans configuration;
In the formula (IV-A-6), compounds that contain 90% or more of compounds wherein the relative configuration of $R^1$ and $R^2$ is a trans configuration;
In the formula (V), compounds wherein Y is a group represented by formula (II-a), $Y^1$ is an oxygen atom or a sulfur atom, m is 0 or 1, and p is 0, 1 or 2, more specifically, compounds represented by formula (V-A);

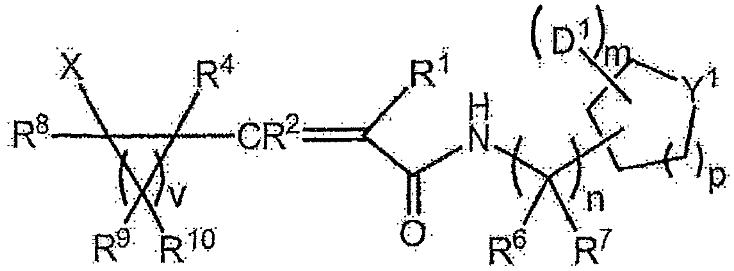

(V-A)

In the formula (V-A), compounds wherein $D^1$ is an ethyl group or a methyl group;
In the formula (V-A), compounds wherein $D^1$ is an ethyl group or a methyl group, and n is 1;
In the formula (V-A), compounds wherein $D^1$ is an ethyl group or a methyl group, n is 1, and $R^6$ and $R^7$ are the same or different and are a methyl group or a hydrogen atom;
In the formula (V-A), compounds wherein $D^1$ is an ethyl group or a methyl group, n is 1, $R^6$ and $R^7$ are the same or different and are a methyl group or a hydrogen atom, $R^4$ is a C1 to C4 alkyl group optionally having one or more halogen atoms, a hydrogen atom or a halogen atom, $R^8$ is a C1 to C4 alkyl group optionally having one or more halogen atoms, a hydrogen atom or a halogen atom, and $R^9$ and $R^{10}$ are the same or different and are a C1 to C4 alkyl group optionally having one or more halogen atoms, a hydrogen atom or a halogen atom;
In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, and $R^9$ and $R^{10}$ are the same or different and are a methyl group or a hydrogen atom;
In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom;
In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^9$ and $R^8$ is trans, $R^9$ and $R^{10}$ are the same or different and are a methyl group or a hydrogen atom, $R^1$ is a hydrogen atom or a fluorine atom, and $R^2$ is a methyl group or a hydrogen atom;
In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom, a methoxy group or a methylthio group (hereinafter represented as compound (V-A-1));
In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a halogen atom, a methoxy group or a methylthio group (hereinafter represented as compound (V-A-2));

In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^9$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkynyl group optionally having one or more atoms or groups selected from group A, and the one or more atoms or groups selected from group A is a halogen atom, a methoxy group or a methylthio group (hereinafter represented as compound (V-A-3));

In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a chlorine atom (hereinafter represented as compound (V-A-4));

In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom (hereinafter represented as compound (V-A-5)); In the formula (V-A), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^9$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a methoxy atom (hereinafter represented as compound (V-A-6));

In the formula (V-A-1), compounds wherein the relative configuration of $R^1$ and $R^2$ is a trans configuration;

In the formula (V-A-1), compounds that are rich in compounds wherein the relative configuration of $R^1$ and $R^2$ is a trans configuration;

In the formula (V-R-1), compounds that contain 80% or more of compounds wherein the relative configuration of $R^1$ and $R^2$ is a trans configuration;

In the formula (V-A-1), compounds that contain 90% or more of compounds wherein the relative configuration of $R^1$ and $R^2$ is a trans configuration;

In the formula (V-A-2), compounds wherein the relative configuration of $R^1$ and $R^2$ is a trans configuration;

In the formula (V-A-2), compounds that are rich in compounds wherein the relative configuration of $R^1$ and $R^2$ is a trans configuration;

In the formula (V-A-2), compounds that contain 80% or more of compounds wherein the relative configuration of $R^1$ and $R^2$ is a trans configuration;

In the formula (V-A-2), compounds that contain 90% or more of compounds wherein the relative configuration of $R^1$ and $R^2$ is a trans configuration;

In the formula (V-A-3), compounds wherein the relative configuration of $R^1$ and $R^2$ is a trans configuration;

In the formula (V-A-3), compounds that are rich in compounds wherein the relative configuration of $R^1$ and $R^2$ is a trans configuration;

In the formula (V-A-3), compounds that contain 80% or more of compounds wherein the relative configuration of $R^1$ and $R^2$ is a trans configuration;

In the formula (V-A-3), compounds that contain 90% or more of compounds wherein the relative configuration of $R^1$ and $R^2$ is a trans configuration;

In the formula (V-A-4), compounds wherein the relative configuration of $R^1$ and $R^2$ is a trans configuration;

In the formula (V-A-4), compounds that are rich in compounds wherein the relative configuration of $R^1$ and $R^2$ is a trans configuration;

In the formula (V-A-4), compounds that contain 80% or more of compounds wherein the relative configuration of $R^1$ and $R^2$ is a trans configuration;

In the formula (V-A-4), compounds that contain 90% or more of compounds wherein the relative configuration of $R^1$ and $R^2$ is a trans configuration;

In the formula (V-A-5), compounds wherein the relative configuration of $R^1$ and $R^2$ is a trans configuration;

In the formula (V-A-5), compounds that are rich in compounds wherein the relative configuration of $R^1$ and $R^2$ is a trans configuration;
In the formula (V-A-5), compounds that contain 80% or more of compounds wherein the relative configuration of $R^1$ and $R^2$ is a trans configuration;
In the formula (V-A-5), compounds that contain 90% or more of compounds wherein the relative configuration of $R^1$ and $R^2$ is a trans configuration;
In the formula (V-A-6), compounds wherein the relative configuration of $R^1$ and $R^2$ is a trans configuration;
In the formula (V-A-6), compounds that are rich in compounds wherein the relative configuration of $R^1$ and $R^2$ is a trans configuration;
In the formula (V-A-6), compounds that contain 80% or more of compounds wherein the relative configuration of $R^1$ and $R^2$ is a trans configuration;
In the formula (V-A-6), compounds that contain 90% or more of compounds wherein the relative configuration of $R^1$ and $R^2$ is a trans configuration;
In the formula (IV-A), compounds wherein $Y^1$ is an oxygen atom, and p is 1 or 2, more specifically, compounds represented by formula (IV-B);

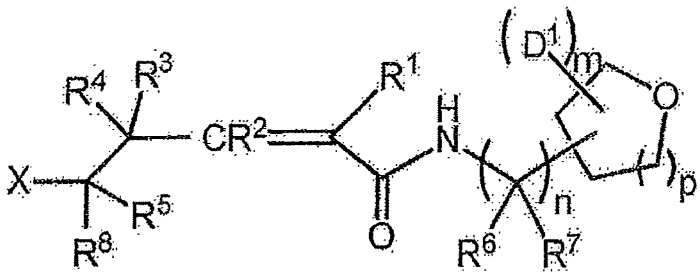

(IV-B)

In the formula (IV-B), compounds wherein $D^1$ is an ethyl group or a methyl group;
In the formula (IV-B), compounds wherein $D^1$ is an ethyl group or a methyl group, and n is 1;
In the formula (IV-B), compounds wherein $D^1$ is an ethyl group or a methyl group, n is 1, and $R^6$ and $R^7$ are the same or different and are a methyl group or a hydrogen atom;
In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, and $R^3$ and $R^4$ are a hydrogen atom;
In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, and $R^2$ is a methyl group or a hydrogen atom;
In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom, a methoxy group or a methylthio group (hereinafter represented as compound (IV-B-1));
In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a halogen atom, a methoxy group or a methylthio group (hereinafter represented as compound (IV-B-2));
In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkynyl group optionally having one or more atoms or groups selected from group A, and the one or more atoms or groups selected from group A is a halogen atom, a methoxy group or a methylthio group (hereinafter represented as compound (IV-B-3));

In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^9$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a chlorine atom (hereinafter represented as compound (IV-B-4)); In the formula (IV-B), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom (hereinafter represented as compound (IV-B-5));

In the formula (IV-B), compounds wherein $D^1$ is an ethyl group or a methyl group, n is I, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a methoxy group (hereinafter represented as compound (IV-B-6));

In the formula (IV-B-1), compounds wherein the relative configuration of $R^1$ and $R^2$ is a trans configuration;

In the formula (IV-B-1), compounds that are rich in compounds wherein the relative configuration of $R^1$ and $R^2$ is a trans configuration;

In the formula (IV-B-1), compounds that contain 80% or more of compounds wherein the relative configuration of $R^1$ and $R^2$ is a trans configuration;

In the formula (IV-B-1), compounds that contain 90% or more of compounds wherein the relative configuration of $R^1$ and $R^2$ is a trans configuration;

In the formula (IV-B-2), compounds wherein the relative configuration of $R^1$ and $R^2$ is a trans configuration;

In the formula (IV-B-2), compounds that are rich in compounds wherein the relative configuration of $R^1$ and $R^2$ is a trans configuration;

In the formula (IV-B-2), compounds that contain 80% or more of compounds wherein the relative configuration of $R^1$ and $R^2$ is a trans configuration;

In the formula (IV-B-2), compounds that contain 90% or more of compounds wherein the relative configuration of $R^1$ and $R^2$ is a trans configuration;

In the formula (IV-B-3), compounds wherein the relative configuration of $R^1$ and $R^2$ is a trans configuration;

In the formula (IV-B-3), compounds that are rich in compounds wherein the relative configuration of $R^1$ and $R^2$ is a trans configuration;

In the formula (IV-B-3), compounds that contain 80% or more of compounds wherein the relative configuration of $R^1$ and $R^2$ is a trans configuration;

In the formula (IV-B-3), compounds that contain 90% or more of compounds wherein the relative configuration of $R^1$ and $R^2$ is a trans configuration;

In the formula (IV-B-4), compounds wherein the relative configuration of $R^1$ and $R^2$ is a trans configuration;

In the formula (IV-B-4), compounds that are rich in compounds wherein the relative configuration of $R^1$ and $R^2$ is a trans configuration;

In the formula (IV-B-4), compounds that contain 80% or more of compounds wherein the relative configuration of $R^1$ and $R^2$ is a trans configuration;

In the formula (IV-B-4), compounds that contain 90% or more of compounds wherein the relative configuration of $R^1$ and $R^2$ is a trans configuration;

In the formula (IV-B-5), compounds wherein the relative configuration of $R^1$ and $R^2$ is a trans configuration;

In the formula (IV-B-5), compounds that are rich in compounds wherein the relative configuration of $R^1$ and $R^2$ is a trans configuration;

In the formula (IV-B-5), compounds that contain 80% or more of compounds wherein the relative configuration of $R^1$ and $R^2$ is a trans configuration;

In the formula (IV-B-5), compounds that contain 90% or more of compounds wherein the relative configuration of $R^1$ and $R^2$ is a trans configuration;

In the formula (IV-B-6), compounds wherein the relative configuration of $R^1$ and $R^2$ is a trans configuration;

In the formula (IV-B-6), compounds that are rich in compounds wherein the relative configuration of $R^1$ and $R^2$ is a trans configuration;

In the formula (IV-B-6), compounds that contain 80% or more of compounds wherein the relative configuration of $R^1$ and $R^2$ is a trans configuration;

In the formula (IV-B-6), compounds that contain 90% or more of compounds wherein the relative configuration of $R^1$ and $R^2$ is a trans configuration;

In the formula (V-A), compounds wherein $Y^1$ is an oxygen atom, m is 0 or 1, and p is 1 or 2, more specifically, compounds represented by formula (V-B);

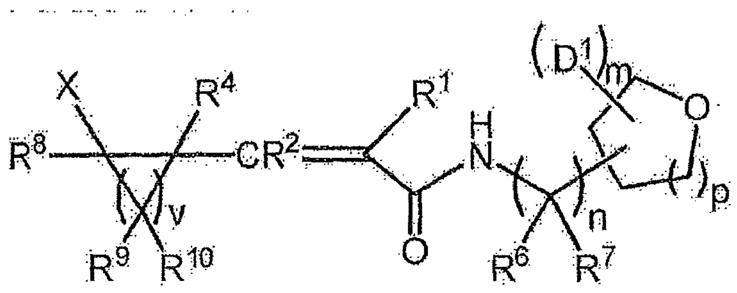

(V-B)

In the formula (V-B), compounds wherein $D^1$ is an ethyl group or a methyl group;

In the formula (V-B), compounds wherein $D^1$ is an ethyl group or a methyl group, and n is 1;

In the formula (V-B), compounds wherein $D^1$ is an ethyl group or a methyl group, n is 1, and $R^6$ and $R^7$ are the same or different and are a methyl group or a hydrogen atom;

In the formula (V-B), compounds wherein $D^1$ is an ethyl group or a methyl group, n is 1, $R^6$ and $R^7$ are the same or different and are a methyl group or a hydrogen atom, $R^4$ is a C1 to C4 alkyl group optionally having one or more halogen atoms, a hydrogen atom or a halogen atom, $R^8$ is a C1 to C4 alkyl group optionally having one or more halogen atoms, a hydrogen atom or a halogen atom, and $R^9$ and $R^{10}$ are the same or different and are a C1 to C4 alkyl group optionally having one or more halogen atoms, a hydrogen atom or a halogen atom;

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, and $R^9$ and $R^{10}$ are the same or different and are a methyl group or a hydrogen atom;

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom;

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, $R^9$ and $R^{10}$ are the same or different and are a methyl group or a hydrogen atom, $R^1$ is a hydrogen atom or a fluorine atom, and $R^2$ is a methyl group or a hydrogen atom;

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom, a methoxy group or a methylthio group (hereinafter represented as compound (V-B-1));

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a halogen atom, a methoxy group or a methylthio group (hereinafter represented as compound (V-B-2));

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group

or a hydrogen atom, X is a C1 to C5 alkyl group optionally having one or more atoms or groups selected from group A or a C2 to C5 alkynyl group optionally having one or more atoms or groups selected from group A, and the one or more atoms or groups selected from group A is a halogen atom, a methoxy group or a methylthio group (hereinafter represented as compound (V-B-3));

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a chlorine atom (hereinafter represented as compound (V-B-4));

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom (hereinafter represented as compound (V-B-5));

In the formula (V-B), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a methoxy group (hereinafter represented as compound (V-B-6));

In the formula (V-B-1), compounds wherein the relative configuration of $R^1$ and $R^2$ is a trans configuration;

In the formula (V-B-1), compounds that are rich in compounds wherein the relative configuration of $R^1$ and $R^2$ is a trans configuration;

In the formula (V-B-1), compounds that contain 80% or more of compounds wherein the relative configuration of $R^1$ and $R^2$ is a trans configuration;

In the formula (V-B-1), compounds that contain 90% or more of compounds wherein the relative configuration of $R^1$ and $R^2$ is a trans configuration;

In the formula (V-B-2), compounds wherein the relative configuration of $R^1$ and $R^2$ is a trans configuration;

In the formula (V-B-2), compounds that are rich in compounds wherein the relative configuration of $R^1$ and $R^2$ is a trans configuration;

In the formula (V-B-2), compounds that contain 80% or more of compounds wherein the relative configuration of $R^1$ and $R^2$ is a trans configuration;

In the formula (V-B-2), compounds that contain 90% or more of compounds wherein the relative configuration of $R^1$ and $R^2$ is a trans configuration;

In the formula (V-B-3), compounds wherein the relative configuration of $R^1$ and $R^2$ is a trans configuration;

In the formula (V-B-3), compounds that are rich in compounds wherein the relative configuration of $R^1$ and $R^2$ is a trans configuration;

In the formula (V-B-3), compounds that contain 80% or more of compounds wherein the relative configuration of $R^1$ and $R^2$ is a trans configuration;

In the formula (V-B-3), compounds that contain 90% or more of compounds wherein the relative configuration of $R^1$ and $R^2$ is a trans configuration;

In the formula (V-B-4), compounds wherein the relative configuration of $R^1$ and $R^2$ is a trans configuration;

In the formula (V-B-4), compounds that are rich in compounds wherein the relative configuration of $R^1$ and $R^2$ is a trans configuration;

In the formula (V-B-4), compounds that contain 80% or more of compounds wherein the relative configuration of $R^1$ and $R^2$ is a trans configuration;

In the formula (V-B-4), compounds that contain 90% or more of compounds wherein the relative configuration of $R^1$ and $R^2$ is a trans configuration;

In the formula (V-B-5), compounds wherein the relative configuration of $R^1$ and $R^2$ is a trans configuration;

In the formula (V-B-5), compounds that are rich in compounds wherein the relative configuration of $R^1$ and $R^2$ is a trans configuration;

In the formula (V-B-5), compounds that contain 80% or more of compounds wherein the relative configuration of $R^1$ and $R^2$ is a trans configuration;

In the formula (V-B-5), compounds that contain 90% or more of compounds wherein the relative configuration of $R^1$ and $R^2$ is a trans configuration;

In the formula (V-B-6), compounds wherein the relative configuration of $R^1$ and $R^2$ is a trans configuration;

In the formula (V-B-6), compounds that are rich in compounds wherein the relative configuration of $R^1$ and $R^2$ is a trans configuration;

In the formula (V-B-6), compounds that contain 80% or more of compounds wherein the relative configuration of $R^1$ and $R^2$ is a trans configuration;

In the formula (V-B-6), compounds that contain 90% or more of compounds wherein the relative configuration of $R^1$ and $R^2$ is a trans configuration;

In the formula (I), compounds wherein Y is a group represented by formula (II-b),

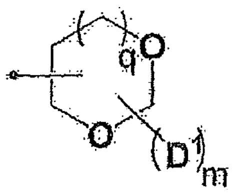
(II-b)

m is 0 or 1, and q is 0 or 1, more specifically, compounds represented by formula (I-B);

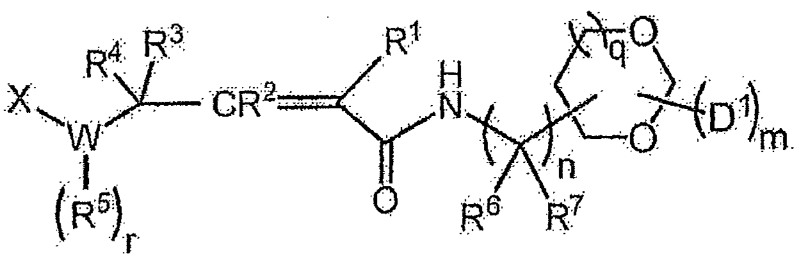
(I-B)

In the formula (I-B), compounds wherein m is 0 or 1, q is 0 or 1, and $D^1$ is an ethyl group or a methyl group;

In the formula (I-B), compounds wherein m is 0 or 1, q is 0 or 1, $D^1$ is an ethyl group or a methyl group, and n is 1;

In the formula (IV), compounds wherein Y is a group represented by the formula (II-b), m is 0 or 1, and q is 0 or 1, more specifically, compounds represented by formula (IV-C);

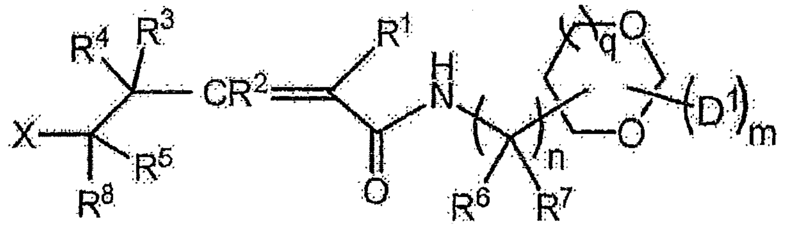
(IV-C)

In the formula (IV-C), compounds wherein $D^1$ is an ethyl group or a methyl group;

In the formula (IV-C), compounds wherein $D^1$ is an ethyl group or a methyl group, and n is 1;

In the formula (IV-C), compounds wherein $D^1$ is an ethyl group or a methyl group, n is 1, and $R^6$ and $R^7$ are the same or different and are a methyl group or a hydrogen atom;

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, and $R^3$ and $R^4$ are a hydrogen atom;

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, and $R^2$ is a methyl group or a hydrogen atom;

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or

a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom, a methoxy group or a methylthio group (hereinafter represented as compound (IV-C-1));

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a halogen atom, a methoxy group or a methylthio group (hereinafter represented as compound (IV-C-2));

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkynyl group optionally having one or more atoms or groups selected from group A, and the one or more atoms or groups selected from group A is a halogen atom, a methoxy group or a methylthio group (hereinafter represented as compound (IV-C-3));

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a chlorine atom (hereinafter represented as compound (IV-C-4));

In the formula (IV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom (hereinafter represented as compound (IV-C-5));

In the formula (TV-C), compounds wherein $D^1$ is an ethyl group or a methyl group, n is 1, $R^6$ and $R^7$ are the same or different and are a methyl group or a hydrogen atom, $R^8$ is a methyl group or a hydrogen atom, $R^5$ is a methyl group or a hydrogen atom, $R^3$ and $R^4$ are a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a methoxy group (hereinafter represented as compound (IV-C-6));

In the formula (IV-C-1), compounds wherein the relative configuration of $R^1$ and $R^2$ is a trans configuration;

In the formula (IV-C-1), compounds that are rich in compounds wherein the relative configuration of $R^1$ and $R^2$ is a trans configuration;

In the formula (IV-C-1), compounds that contain 80% or more of compounds wherein the relative configuration of $R^1$ and $R^2$ is a trans configuration;

In the formula (IV-C-1), compounds that contain 90% or more of compounds wherein the relative configuration of $R^1$ and $R^2$ is a trans configuration;

In the formula (IV-C-2), compounds wherein the relative configuration of $R^1$ and $R^2$ is a trans configuration;

In the formula (IV-C-2), compounds that are rich in compounds wherein the relative configuration of $R^1$ and $R^2$ is a trans configuration;

In the formula (IV-C-2), compounds that contain 80% or more of compounds wherein the relative configuration of $R^1$ and $R^2$ is a trans configuration;

In the formula (IV-C-2), compounds that contain 90% or more of compounds wherein the relative configuration of $R^1$ and $R^2$ is a trans configuration;

In the formula (IV-C-3), compounds wherein the relative configuration of $R^1$ and $R^2$ is a trans configuration;

In the formula (IV-C-3), compounds that are rich in compounds wherein the relative configuration of $R^1$ and $R^2$ is a trans configuration;

In the formula (IV-C-3), compounds that contain 80% or more of compounds wherein the relative configuration of $R^1$ and $R^2$ is a trans configuration;

In the formula (IV-C-3), compounds that contain 90% or more of compounds wherein the relative configuration of $R^1$ and $R^2$ is a trans configuration;

In the formula (IV-C-4), compounds wherein the relative configuration of $R^1$ and $R^2$ is a trans configuration;
In the formula (IV-C-4), compounds that are rich in compounds wherein the relative configuration of $R^1$ and $R^2$ is a trans configuration;
In the formula (IV-C-4), compounds that contain 80% or more of compounds wherein the relative configuration of $R^1$ and $R^2$ is a trans configuration;
In the formula (IV-C-4), compounds that contain 90% or more of compounds wherein the relative configuration of $R^1$ and $R^2$ is a trans configuration;
In the formula (IV-C-5), compounds wherein the relative configuration of $R^1$ and $R^2$ is a trans configuration;
In the formula (IV-C-5), compounds that are rich in compounds wherein the relative configuration of $R^1$ and $R^2$ is a trans configuration;
In the formula (IV-C-5), compounds that contain 80% or more of compounds wherein the relative configuration of $R^1$ and $R^2$ is a trans configuration;
In the formula (IV-C-5), compounds that contain 90% or more of compounds wherein the relative configuration of $R^1$ and $R^2$ is a trans configuration;
In the formula (IV-C-6), compounds wherein the relative configuration of $R^1$ and $R^2$ is a trans configuration;
In the formula (IV-C-6), compounds that are rich in compounds wherein the relative configuration of $R^1$ and $R^2$ is a trans configuration;
In the formula (IV-C-6), compounds that contain 80% or more of compounds wherein the relative configuration of $R^1$ and $R^2$ is a trans configuration;
In the formula (IV-C-6), compounds that contain 90% or more of compounds wherein the relative configuration of $R^1$ and $R^2$ is a trans configuration;
In the formula (V), compounds wherein Y is a group represented by the formula (II-b), m is 0 or 1, and q is 0 or 1, more specifically, compounds represented by formula (V-C);

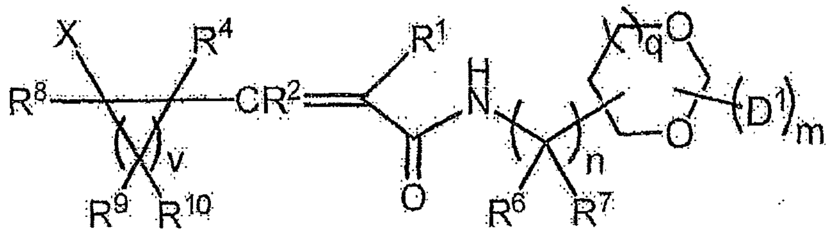
(V-C)

In the formula (V-C), compounds wherein $D^1$ is an ethyl group or a methyl group;
In the formula (V-C), compounds wherein $D^1$ is an ethyl group or a methyl group, and n is 1;
In the formula (V-C), compounds wherein $D^1$ is an ethyl group or a methyl group, n is 1, and $R^6$ and $R^7$ are the same or different and are a methyl group or a hydrogen atom;
In the formula (V-C), compounds wherein $D^1$ is an ethyl group or a methyl group, n is 1, $R^6$ and $R^7$ are the same or different and are a methyl group or a hydrogen atom, $R^4$ is a C1 to C4 alkyl group optionally having one or more halogen atoms, a hydrogen atom or a halogen atom, $R^8$ is a C1 to C4 alkyl group optionally having one or more halogen atoms, a hydrogen atom or a halogen atom, and $R^9$ and $R^{10}$ are the same or different and are a C1 to C4 alkyl group optionally having one or more halogen atoms, a hydrogen atom or a halogen atom;
In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, and $R^9$ and $R^{10}$ are the same or different and are a methyl group or a hydrogen atom;
In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom;
In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, $R^9$ and $R^{10}$ are the same or different and are a methyl group or a hydrogen atom, $R^1$ is a hydrogen atom or a fluorine atom, and $R^2$ is a methyl group or a hydrogen atom;
In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same

or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom, a methoxy group or a methylthio group (hereinafter represented as compound (V-C-1));

In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a halogen atom, a methoxy group or a methylthio group (hereinafter represented as compound (V-C-2));

In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkynyl group optionally having one or more atoms or groups selected from group A, and the one or more atoms or groups selected from group A is a halogen atom, a methoxy group or a methylthio group (hereinafter represented as compound (V-C-3));

In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a chlorine atom (hereinafter represented as compound (V-C-4)); In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a fluorine atom (hereinafter represented as compound (V-C-5));

In the formula (V-C), compounds wherein $D^1$ is an ethyl group or a methyl group, n is 1, $R^6$ and $R^7$ are the same or different and are a methyl group or a hydrogen atom, v is 1, $R^4$ is a methyl group, a hydrogen atom or a fluorine atom, $R^8$ is a methyl group or a hydrogen atom, the relative configuration of $R^4$ and $R^8$ is trans, and $R^9$ and $R^{10}$ are the same or different and are a methyl group or a hydrogen atom, $R^1$ is a hydrogen atom or a fluorine atom, $R^2$ is a methyl group or a hydrogen atom, X is a C1 to C5 alkyl group optionally having one or more atoms or groups selected from group A or a C2 to C5 alkenyl group optionally having one or more atoms or groups selected from group A, and the one or more atoms or groups selected from group A is a methoxy group (hereinafter represented as compound (V-C-6));

In the formula (V-C-1), compounds wherein the relative configuration of $R^1$ and $R^2$ is a trans configuration;

In the formula (V-C-1), compounds that are rich in compounds wherein the relative configuration of $R^1$ and $R^2$ is a trans configuration;

In the formula (V-C-1), compounds that contain 80% or more of compounds wherein the relative configuration of $R^1$ and $R^2$ is a trans configuration;

In the formula (V-C-1), compounds that contain 90% or more of compounds wherein the relative configuration of $R^1$ and $R^2$ is a trans configuration;

In the formula (V-C-2), compounds wherein the relative configuration of $R^1$ and $R^2$ is a trans configuration;

In the formula (V-C-2), compounds that are rich in compounds wherein the relative configuration of $R^1$ and $R^2$ is a trans configuration;

In the formula (V-C-2), compounds that contain 80% or more of compounds wherein the relative configuration of $R^1$ and $R^2$ is a trans configuration;

In the formula (V-C-2), compounds that contain 90% or more of compounds wherein the relative configuration of $R^1$ and $R^2$ is a trans configuration;

In the formula (V-C-3), compounds wherein the relative configuration of $R^1$ and $R^2$ is a trans configuration;

In the formula (V-C-3), compounds that are rich in compounds wherein the relative configuration of $R^1$ and $R^2$ is a trans

configuration;
In the formula (V-C-3), compounds that contain 80% or more of compounds wherein the relative configuration of $R^1$ and $R^2$ is a trans configuration;
In the formula (V-C-3), compounds that contain 90% or more of compounds wherein the relative configuration of $R^1$ and $R^2$ is a trans configuration;
In the formula (V-C-4), compounds wherein the relative configuration of $R^1$ and $R^2$ is a trans configuration;
In the formula (V-C-4), compounds that are rich in compounds wherein the relative configuration of $R^1$ and $R^2$ is a trans configuration;
In the formula (V-C-4), compounds that contain 80% or more of compounds wherein the relative configuration of $R^1$ and $R^2$ is a trans configuration;
In the formula (V-C-4), compounds that contain 90% or more of compounds wherein the relative configuration of $R^1$ and $R^2$ is a trans configuration;
In the formula (V-C-5), compounds wherein the relative configuration of $R^1$ and $R^2$ is a trans configuration;
In the formula (V-C-5), compounds that are rich in compounds wherein the relative configuration of $R^1$ and $R^2$ is a trans configuration;
In the formula (V-C-5), compounds that contain 80% or more of compounds wherein the relative configuration of $R^1$ and $R^2$ is a trans configuration;
In the formula (V-C-5), compounds that contain 90% or more of compounds wherein the relative configuration of $R^1$ and $R^2$ is a trans configuration;
In the formula (V-C-6), compounds wherein the relative configuration of $R^1$ and $R^2$ is a trans configuration;
In the formula (V-C-6), compounds that are rich in compounds wherein the relative configuration of $R^1$ and $R^2$ is a trans configuration;
In the formula (V-C-6), compounds that contain 80% or more of compounds wherein the relative configuration of $R^1$ and $R^2$ is a trans configuration;
In the formula (V-C-6), compounds that contain 90% or more of compounds wherein the relative configuration of $R^1$ and $R^2$ is a trans configuration;
In the formula (I), compounds wherein Y is a 5 to 6-membered saturated heterocyclic ring which contains, as ring-forming component (s), one or more atoms that are selected from the group consisting of an oxygen atom and a sulfur atom, the saturated heterocyclic ring may have one to three C1 to C4 alkyl groups optionally having one or more halogen atoms, X is a C1 to C6 chain hydrocarbon atom, or a cyclopropyl group optionally having one or more C1 to C6 alkyl groups, W is $-CR^8-$, r is 1, $R^8$ is a hydrogen atom, $R^1$ is a halogen atom or a hydrogen atom, $R^2$ is a C1 to C4 alkyl group optionally having one or more halogen atoms or a hydrogen atom, $R^4$ is a hydrogen atom, $R^3$ and $R^5$ are a hydrogen atom, or $R^3$ and $R^5$ are taken together to be - $(CR^9R^{10})_v$-, $R^9$ and $R^{10}$ are the same or different and are a C1 to C4 alkyl group optionally having one or more halogen atoms, or a hydrogen atom, v is 1, $R^6$ and $R^7$ are a hydrogen atom, and n is 1.

Next, the method for producing the compound of the present invention will be described.

The compound of the present invention can be produced, for example, according to the following (Production Method 1) to (Production Method 5).

(Production Method 1)

The compound of the present invention can be produced by reacting a compound represented by formula (4) with a compound represented by formula (6), in the presence of a condensing agent.

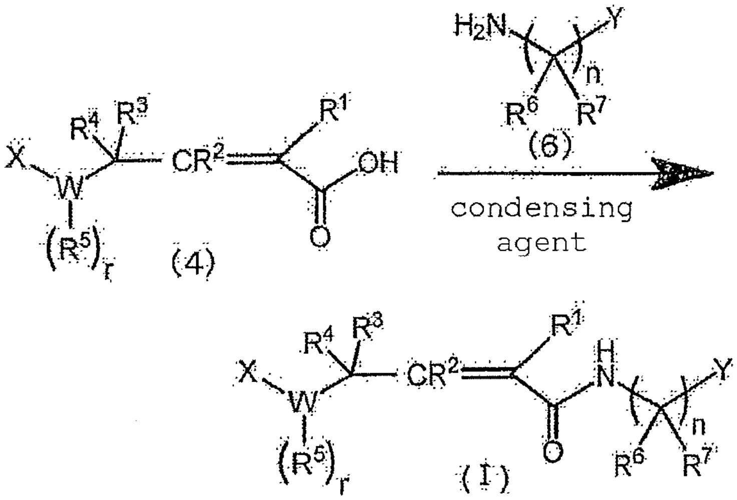

(4) (6) (I)

wherein W, X, Y, $R^1$, $R^2$, $R^3$, $R^4$, $R^5$, $R^6$, $R^7$, n and r represent the same meaning as above.

The reaction is usually carried out in a solvent, in the presence of a condensing agent, and in the presence of a base as necessary.

Examples of the condensing agent include dicyclohexylcarbodiimide, 1-ethyl-3-(3-dimethylaminopropyl) carbodiimide hydrochloride, and benzotriazol-1-yloxy-tris(dimethylamino)phosphonium salt.

Examples of the base include carbonates such as sodium carbonate and potassium carbonate, tertiary amines such as triethylamine, diisopropylethylamine, 1,8-diazabicyclo[5.4.0]undec-7-ene and 1,5-diazabicyclo[4.3.0]non-5-ene, and nitrogen-containing aromatic compounds such as pyridine and 4-dimethylaminopyridine.

Examples of the solvent include aromatic hydrocarbons such as benzene and toluene, aliphatic hydrocarbons such as hexane, ethers such as diethyl ether, tetrahydrofuran, 1,4-dioxane and tert-butyl methyl ether, halogenated hydrocarbons such as dichloromethane, chloroform, 1,2-dichloroethane and chlorobenzene and acid amides such as N,N-dimethylformamide, and mixtures thereof.

The reaction time is usually within the range of 5 minutes to 72 hours.

The reaction temperature is usually within the range of -20 to 100°C (with the proviso that, when the boiling point of the used solvent is less than 100°C, it is within the range of -20°C to the boiling point of the solvent).

The molar ratio of the compound represented by the formula (4) and the compound represented by the formula (6) used can be arbitrarily set, and is preferably an equimolar ratio or a ratio close thereto, for example, the ratio of the compound represented by the formula (6) is 0.5 to 3 mol, based on 1 mol of the compound represented by the formula (4).

Also, the condensing agent is used usually in an arbitrary ratio from 1 mol to an excess amount, and preferably in a ratio of 1 to 3 mol, based on 1 mol of the compound represented by the formula (4).

The base is used usually in an arbitrary ratio from 1 mol to an excess amount, and preferably in a ratio of 1 to 3 mol, based on 1 mol of the compound represented by the formula (4).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

(Production Method 2)

The compound of the present invention can be produced by reacting a compound represented by formula (5) with a compound represented by formula (6), in the presence of a base.

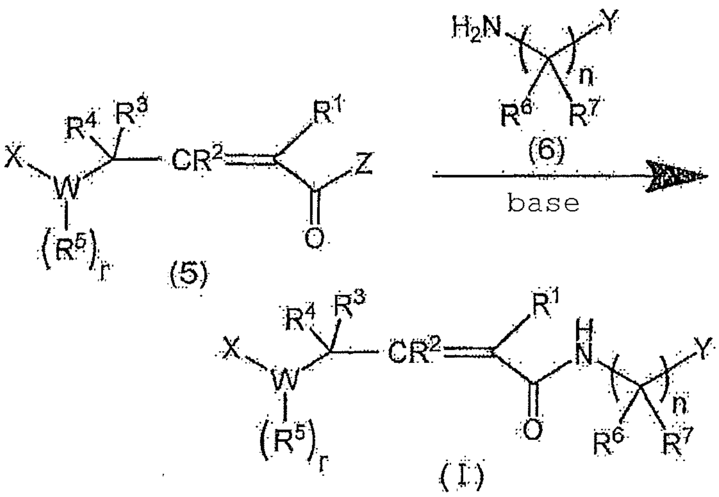


wherein Z represents a chlorine atom, a bromine atom or an iodine atom, and W, X, Y, $R^1$, $R^2$, $R^3$, $R^4$, $R^5$, $R^6$, $R^7$, r and n represent the same meaning as above.

The reaction is usually carried out in a solvent, in the presence of a base.

Examples of the base include carbonates such as sodium carbonate and potassium carbonate, tertiary amines such as triethylamine, diisopropylethylamine, 1,8-diazabicyclo[5.4.0]undec-7-ene and 1,5-diazabicyclo[4.3.0]non-5-ene, and nitrogen-containing aromatic compounds such as pyridine and 4-dimethylaminopyridine.

Examples of the solvent include ethers such as 1,4-dioxane, tetrahydrofuran, ethylene glycol dimethyl ether and tert-butyl methyl ether, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile and butyronitrile, acid amides such as N,N-dimethylformamide, sulfoxides such as dimethyl sulfoxide, and mixtures thereof.

The reaction time is usually within the range of 5 minutes to 72 hours.

The reaction temperature is usually within the range of -20 to 100°C (with the proviso that, when the boiling point of the used solvent is less than 100°C, it is within the range of -20°C to the boiling point of the solvent).

The molar ratio of the compound represented by the formula (5) and the compound represented by the formula (6) used can be arbitrarily set, and is preferably an equimolar ratio or a ratio close thereto, specifically, the ratio of the compound represented by the formula (6) is 0.5 to 3 mol, based on 1 mol of the compound represented by the formula (5).

The base is used usually in an arbitrary ratio from 1 mol to an excess amount, and preferably in a ratio of 1 to 3 mol, based on 1 mol of the compound represented by the formula (5).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

(Production Method 3)

The compound of the present invention can be produced by reacting a compound represented by formula (1) with a compound represented by formula (38-a) or a compound represented by formula (38-b), in the presence of a base.

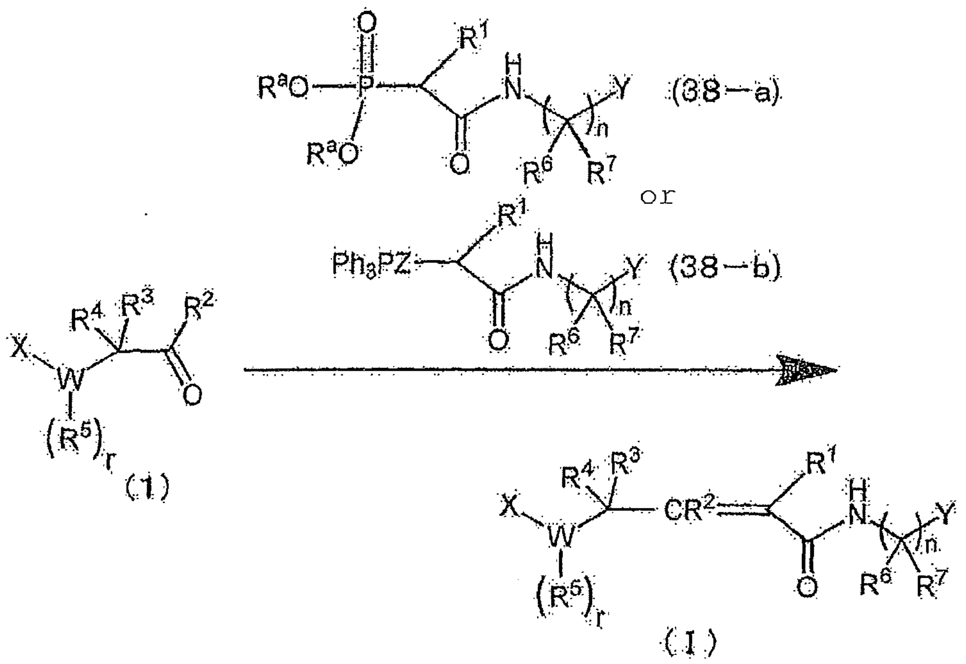

(38-a)

or

(38-b)

(1)

(I)

wherein $R^a$ represents a methyl group or an ethyl group, and W, X, Y, Z, $R^1$, $R^2$, $R^3$, $R^4$, $R^5$, $R^6$, $R^7$, r and n represent the same meaning as above.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as benzene, toluene and xylene, halogenated hydrocarbons such as chlorobenzene, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (1) with the compound represented by the formula (38-a) or the compound represented by the formula (38-b) used can be arbitrarily set, and is preferably an equimolar ratio or a ratio close thereto. Specifically, the ratio of the compound represented by the formula (38-a) or the compound represented by the formula (38-b) is preferably 0.5 to 3 mol, based on 1 mol of the compound represented by the formula (1).

The base is used usually in an arbitrary ratio from 1 mol to an excess amount, and preferably in a ratio of 1 to 5 mol, based on 1 mol of the compound represented by the formula (38-a) or the compound represented by the formula (38-b).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

(Production Method 4)

Among the compounds of the present invention, a compound represented by formula (I-a) wherein X is a C1 to C10 chain hydrocarbon group having one or more atoms or groups selected from group A can be produced by reacting a compound represented by formula (39) with a compound represented by formula (40), in the presence of a base.

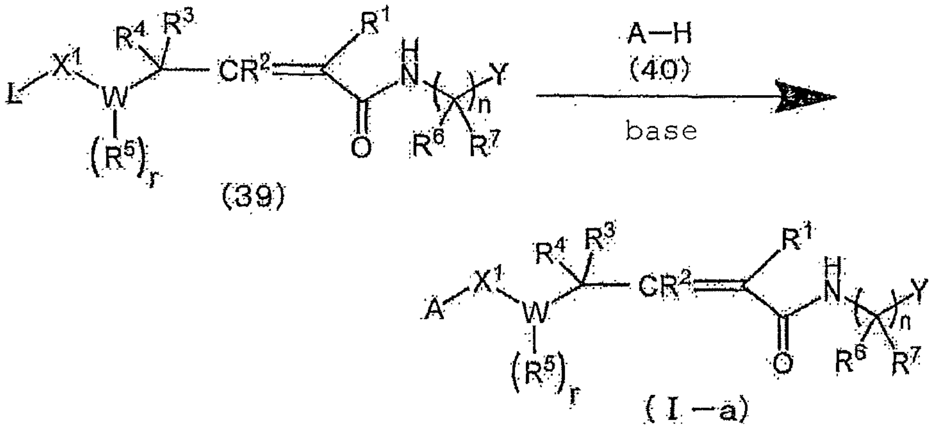


(39)

(I-a)

wherein L represents a leaving group such as a chlorine atom, a bromine atom, an iodine atom or a methanesulfonyl group, A represents one group that is selected from group A, $X^1$ represents a group in which L or A is removed in X, and W, Y, $R^1$, $R^2$, $R^3$, $R^4$, $R^5$, $R^6$, $R^7$, r and n represent the same meaning as above.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 72 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (39) and the compound represented by the formula (40) used can be arbitrarily set, and is preferably an equimolar ratio or a ratio close thereto, for example, the ratio of the compound represented by the formula (40) is 1 to 3 mol, based on 1 mol of the compound represented by the formula (39).

The base is used usually in an arbitrary ratio from 1 mol to an excess amount, and preferably in a ratio of 1 to 3 mol, based on 1 mol of the compound represented by the formula (40).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

(Production Method 5)

Among the compounds of the present invention, a compound represented by formula (I-a) wherein X is a C1 to C10 chain hydrocarbon group having one or more atoms or groups selected from group A can be produced by reacting a compound represented by formula (39) with a compound represented by formula (41).

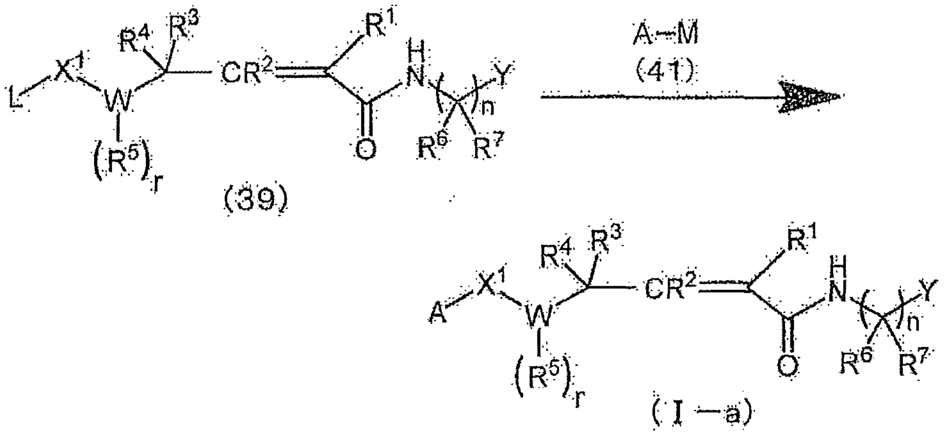


(39)

(I−a)

wherein M represents an alkali metal atom such as a lithium atom, a sodium atom or a potassium atom, and L, A, $X^1$, W, Y, $R^1$, $R^2$, $R^3$, $R^4$, $R^5$, $R^6$, $R^7$, r and n represent the same meaning as above.

The reaction is usually carried out in a solvent.

Examples of the solvent to be used include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 72 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (39) and the compound represented by the formula (41) used can be arbitrarily set, and is preferably an equimolar ratio or a ratio close thereto, for example, the ratio of the compound represented by the formula (41) is 1 to 3 mol, based on 1 mol of the compound represented by the formula (39).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

Next, the method for producing a production intermediate of the compound of the present invention will be described.

(Reference Production Method 1)

The compound represented by the formula (5) can be produced, for example, by reacting a compound represented by formula (4) with a halogenating agent.

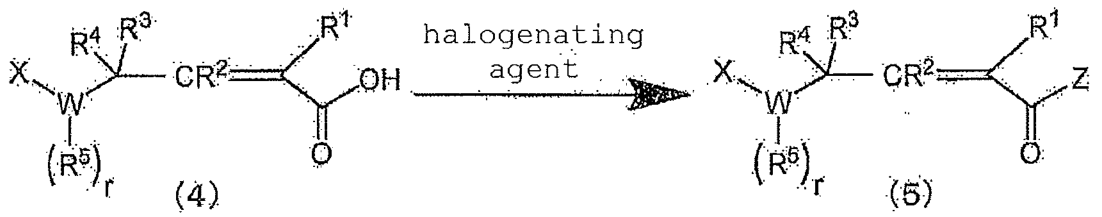


(4)

(5)

wherein W, X, Z, $R^1$, $R^2$, $R^3$, $R^4$, $R^5$ and r represent the same meaning as above.

The reaction is usually carried out in a solvent.

Examples of the halogenating agent include thionyl chloride, oxalyl chloride and phosphorus oxychloride.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as toluene and xylene, halogenated hydrocarbons such as chlorobenzene, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The halogenating agent is used usually in an arbitrary ratio from 1 mol to an excess amount, and preferably in

a ratio of 1 to 5 mol, based on 1 mol of the compound represented by the formula (4).

After completion of the reaction, the compound represented by formula (5) can be isolated by a post-treatment operation such as concentrating the reaction mixture as it is. The isolated compound represented by formula (5) is usually used in the following step without purification, but can be also purified by distillation or the like, as necessary.

(Reference Production Method 2)

The compound represented by the formula (4) can be produced, for example, by subjecting a compound represented by formula (3) to a hydrolysis reaction, in the presence of a base.

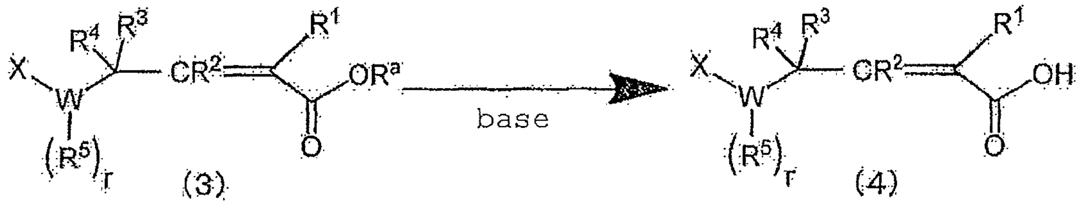


wherein $R^a$, W, X, $R^1$, $R^2$, $R^3$, $R^4$, $R^5$ and r represent the same meaning as above.

The reaction is carried out in the presence of a base, and in the presence of water and an organic solvent.

Examples of the base include alkali metal hydroxides such as lithium hydroxide, sodium hydroxide and potassium hydroxide.

Examples of the solvent include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, nitriles such as acetonitrile and butyronitrile, alcohols such as methanol, ethanol and propanol, and mixtures thereof.

The reaction time is usually within the range of 5 minutes to 72 hours.

The reaction temperature is usually within the range of 0 to 100°C (with the proviso that, when the boiling point of the used solvent is less than 100°C, it is within the range of 0°C to the boiling point of the solvent).

The base is used usually in an arbitrary ratio from 1 mol to an excess amount, and preferably in a ratio of 1 to 5 mol, based on 1 mol of the compound represented by the formula (3).

After completion of the reaction, the compound represented by formula (4) can be isolated by post-treatment operations such as adding an acidic aqueous solution (hydrochloric acid, etc.) to the reaction mixture to extract with an organic solvent, and drying and concentrating the organic layer. The isolated compound represented by formula (4) can be also further purified by chromatography, recrystallization and distillation, and can be used in the following step as it is.

(Reference Production Method 3)

The compound represented by the formula (3) can be produced, for example, by reacting a compound represented by formula (1) with a compound represented by formula (2-a) or a compound represented by formula (2-b), in the presence of a base.

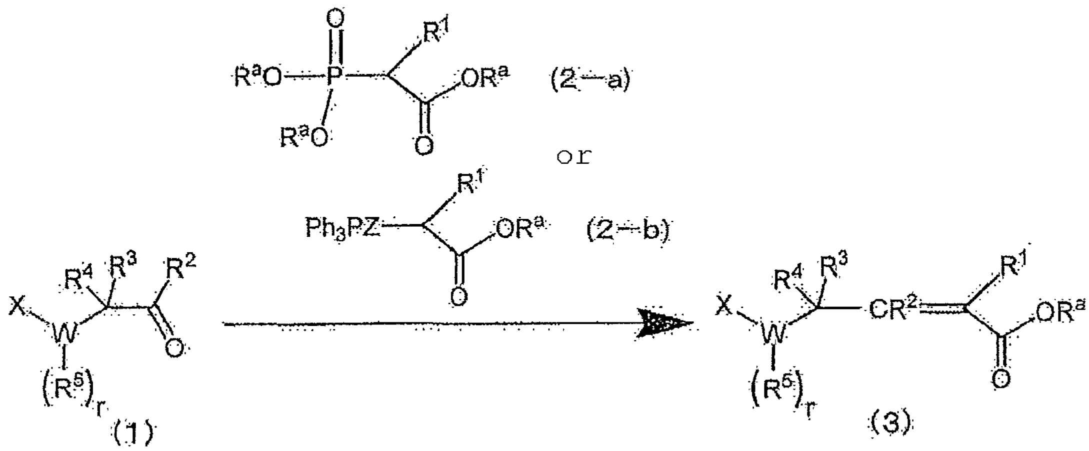

wherein W, X, Z, $R^a$, $R^1$, $R^2$, $R^3$, $R^4$, $R^5$ and r represent the same meaning as above.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as benzene, toluene and xylene, halogenated hydrocarbons such as chlorobenzene, and mixtures thereof.

The reaction time is usuallywithin the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (1) and the compound represented by the formula (2-a) or the compound represented by the formula (2-b) used can be arbitrarily set, and the reaction is preferably carried out at an equimolar ratio or a ratio close thereto. Specifically, the ratio of the compound represented by the formula (2-a) or the compound represented by the formula (2-b) is preferably 0.5 to 3 mol, based on 1 mol of the compound represented by the formula (1).

The base is used usually in an arbitrary ratio from 1 mol to an excess amount, and preferably in a ratio of 1 to 5 mol, based on 1 mol of the compound represented by the formula (2-a) or the compound represented by the formula (2-b).

After completion of the reaction, the compound represented by the formula (3) can be obtained by adding the reaction mixture to water and then subjecting it to usual post-treatment operations such as an extraction with an organic solvent and concentration. In addition, the obtained compound represented by the formula (3) can be further purified by procedures such as chromatography, recrystallization and distillation.

(Reference Production Method 4)

The compound represented by the formula (1) can be produced, for example, by subjecting a compound represented by formula (7) to an oxidation reaction.

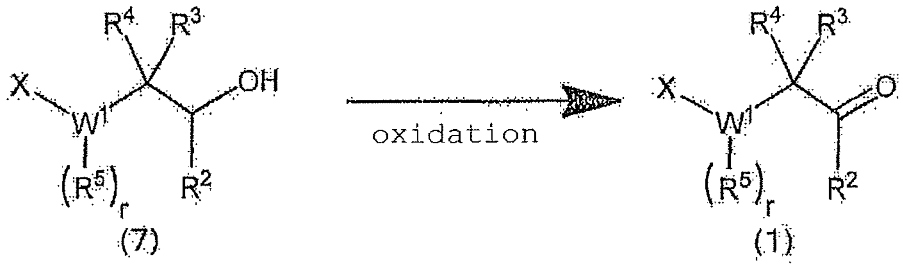


wherein $W^1$ represents $-CR^8-$ or an oxygen atom, and X, $R^2$, $R^3$, $R^4$, $R^5$ and r represent the same meaning as above.

Examples of the oxidation reaction include;

(1) a method of using a chromate (for example, pyridinium chlorochromate or pyridinium dichromate),
(2) a method of using a chromic acid (for example, Jones oxidation),
(3) a method of using DMSO oxidation (for example, Swern oxidation),
(4) a method of using a Dess-Martin reagent,
(5) a method of using TEMPO (2,2,6,6-tetramethylpiperidine 1-oxyl),

and the like.

(1) The method of using a chromate is specifically exemplified as an example.

The reaction is usually carried out in a solvent.

Examples of the oxidizing agent include chromates such as pyridinium chlorochromate and pyridinium dichromate.

Examples of the solvent include halogenated hydrocarbons such as dichloromethane, chloroform, 1,2-dichloroethane and chlorobenzene, aliphatic hydrocarbons such as hexane and heptane, aromatic hydrocarbons such as toluene and xylene, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).
The chromate is used usually in an arbitrary ratio from 1 mol to an excess amount, and preferably in a ratio of 1 to 3 mol, based on 1 mol of the compound represented by the formula (7).
After completion of the reaction, the compound represented by the formula (1) can be isolated by filtering the reaction mixture and then subjecting it to an operation such as concentration. The isolated compound represented by the formula (1) can be further also purified by chromatography, recrystallization, distillation and the like, as necessary.

(Reference Production Method 5)

The compound represented by the formula (1) can be also produced, for example, by reacting a compound represented by formula (8) with water, in the presence of an acid.

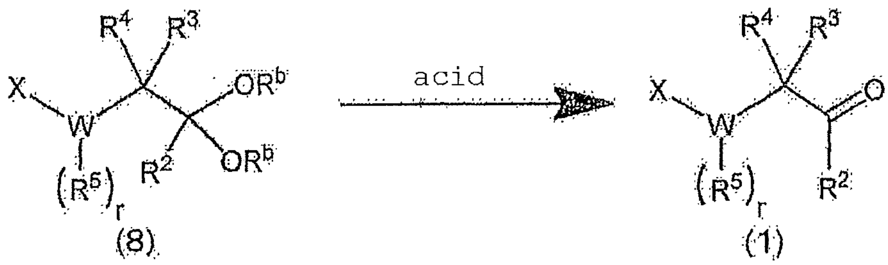


wherein $R^b$ represents a methyl group or an ethyl group, and W, X, $R^2$, $R^3$, $R^4$, $R^5$ and r represent the same meaning as above.
The reaction is carried out in the presence of an acid, and in the presence of water and an organic solvent.
Examples of the acid include inorganic acids such as hydrochloric acid and sulfuric acid and organic acids such as methanesulfonic acid and p-toluenesulfonic acid.
Examples of the organic solvent include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, nitriles such as acetonitrile and butyronitrile, alcohols such as methanol, ethanol and propanol, ketones such as acetone and methyl isobutyl ketone, and mixtures thereof.
The reaction time is usually within the range of 5 minutes to 24 hours.
The reaction temperature is usually within the range of 0 to 100°C (with the proviso that, when the boiling point of the used solvent is less than 100°C, it is within the range of 0°C to the boiling point of the solvent).
The acid is used usually in an arbitrary ratio from 1 mol to an excess amount, based on 1 mol of the compound represented by the formula (8).
After completion of the reaction, the compound represented by the formula (1) can be isolated by adding the reaction mixture to water and then subjecting it to usual post-treatment operations such as an extraction with an organic solvent and concentration. In addition, the isolated compound represented by the formula (1) can be also purified by procedures such as chromatography, recrystallization and distillation.

(Reference Production Method 6)

Among the compounds represented by the formula (8), a compound represented by formula (8-1) wherein W is an oxygen atom or a sulfur atom and r is 0 can be also produced, for example, by reacting a compound represented by formula (9) with a compound represented by formula (10), in the presence of a base.

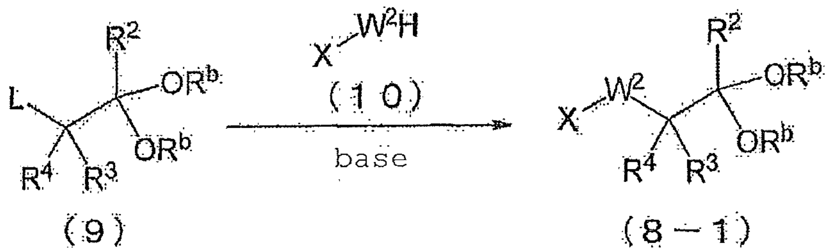


wherein $W^2$ represents an oxygen atom or a sulfur atom, and L, X, $R^b$, $R^2$, $R^3$ and $R^9$ represent the same meaning as above.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 72 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (9) and the compound represented by the formula (10) used can be arbitrarily set, and is preferably an equimolar ratio or a ratio close thereto, for example, the ratio of the compound represented by the formula (10) is 1 to 3 mol, based on 1 mol of the compound represented by the formula (9).

The base is used usually in an arbitrary ratio from 1 mol to an excess amount, and preferably in a ratio of 1 to 3 mol, based on 1 mol of the compound represented by the formula (10).

After completion of the reaction, the compound represented by the formula (8-1) can be isolated by adding the reaction mixture to water and then subjecting it to usual post-treatment operations such as an extraction with an organic solvent and concentration. In addition, the isolated compound represented by the formula (8-1) can be also purified by procedures such as chromatography, recrystallization and distillation.

(Reference Production Method 7)

Among the compounds represented by the formula (8), a compound represented by formula (8-so) wherein W is $-S(O)_u-$, r is 0, and u is 1 or 2 can be produced, for example, by subj ecting to an oxidation reaction a compound represented by formula (8-s) wherein $W^1$ is a sulfur atom in the formula (8-1).

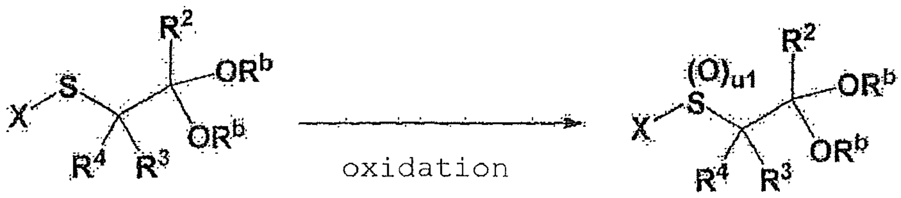


(8-s) (8-so)

wherein ul represents 1 or 2, and X, $R^b$, $R^2$, $R^3$ and $R^4$ represent the same meaning as above.

The reaction is usually carried out in a solvent.

Examples of the oxidizing agent include organic peroxides such as peracetic acid, trifluoroperacetic acid and m-chloroperbenzoic acid, halogen molecules of chlorine and bromine, halogen-containing imides such as N-chlorosuccinimide, halides such as perchloric acid (or salts thereof) and periodic acid (or salts thereof), permanganates such as potassium permanganate, chromates such as potassium chromates, peroxysulfates such as peroxy-potassium sulfate, and hydrogen peroxide.

Examples of the solvent include alcohols such as methanol and ethanol, halogenated hydrocarbons such as dichloromethane and chloroform, aromatic hydrocarbons such as toluene and xylene, aliphatic carbonates such as acetic acid and trifluoroacetic acid, water, and mixtures thereof.

The reaction time is usually within the range of 5 minutes to 24 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (8-s) and the oxidizing agent used can be arbitrarily set, and is preferably an equimolar ratio or a ratio close thereto, for example, the ratio of the oxidizing agent is 0.5 to 3 mol, based on 1 mol of the compound represented by the formula (8-s).

After completion of the reaction, the compound represented by the formula (8-so) can be isolated by adding the reaction mixture to water and then subjecting it to usual post-treatment operations such as an extraction with an organic solvent and concentration. In addition, the isolated compound represented by the formula (8-so) can be also purified by procedures such as chromatography, recrystallization and distillation.

(Reference Production Method 8)

Among the compounds represented by the formula (7), a compound represented by formula (7-1) wherein W is an oxygen atom or a sulfur atom and r is 0, can be produced, for example, by reacting a compound represented by formula (11) with a compound represented by formula (10), in the presence of a base.

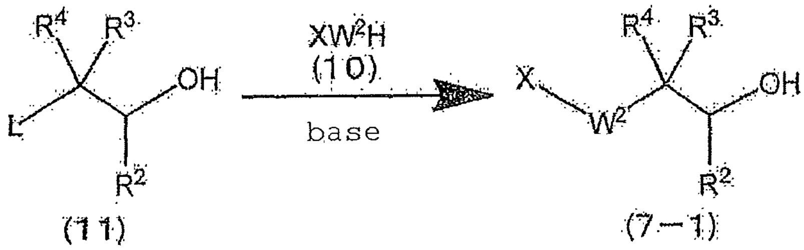


wherein $W^2$, L, X, $R^2$, $R^3$ and $R^4$ represent the same meaning as above.
The reaction is usually carried out in a solvent, in the presence of a base.
Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.
Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.
The reaction time is usually within the range of 5 minutes to 24 hours.
The reaction temperature is usually within the range of 0 to 100°C (with the proviso that, when the boiling point of the used solvent is less than 100°C, it is within the range of 0°C to the boiling point of the solvent).
The molar ratio of the compound represented by the formula (11) and the compound represented by the formula (10) used can be arbitrarily set, and is preferably an equimolar ratio or a ratio close thereto, for example, the ratio of the compound represented by the formula (10) is 1 to 3 mol, based on 1 mol of the compound represented by the formula (11).
The base is used usually in an arbitrary ratio from 1 mol to an excess amount, and preferably in a ratio of 1 to 3 mol, based on 1 mol of the compound represented by the formula (10).
After completion of the reaction, the compound represented by the formula (7-1) can be isolated by adding the reaction mixture to water and then subjecting it to usual post-treatment operations such as an extraction with an organic solvent and concentration. In addition, the isolated compound represented by the formula (7-1) can be also purified by procedures such as chromatography, recrystallization and distillation.

(Reference Production Method 9)

Among the compounds represented by the formula (7), a compound represented by formula (7-1) wherein W is an oxygen atom or a sulfur atom and r is 0, can be produced, for example, by reacting a compound represented by formula (12) with a compound represented by formula (13), in the presence of a base.

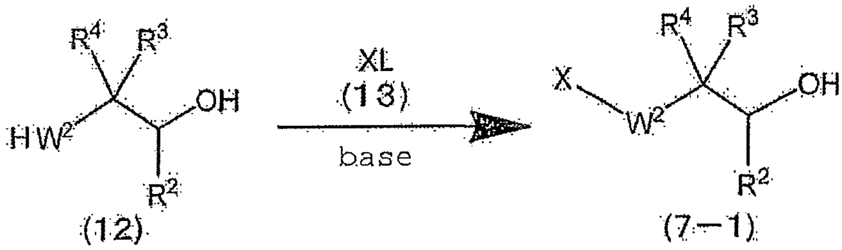


wherein $W^2$, L, X, $R^2$, $R^3$ and $R^4$ represent the same meaning as above.
The reaction is usually carried out in a solvent, in the presence of a base.
Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.
Examples of the solvent include ethers such as diethyl ether, tetrahydrofuran and dimethoxyethane, acid amides

such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (12) and the compound represented by the formula (13) used can be arbitrarily set, and is preferably a ratio that the compound represented by the formula (13) is excess, for example, the ratio of the compound represented by the formula (13) is 3 to 5 mol, based on 1 mol of the compound represented by the formula (12).

The base is used usually in an arbitrary ratio from 1 mol to an excess amount, and preferably in a ratio of 1 to 3 mol, based on 1 mol of the compound represented by the formula (12).

After completion of the reaction, the compound represented by the formula (7-1) can be isolated by adding the reaction mixture to water and then subjecting it to usual post-treatment operations such as an extraction with an organic solvent and concentration. In addition, the isolated compound represented by the formula (7-1) can be also purified by procedures such as chromatography, recrystallization and distillation.

(Reference Production Method 10)

Among the compounds represented by the formula (7), a compound represented by formula (7-2) wherein $R^3$ and $R^5$ are taken together to be $-(CR^9R^{10})_v-$, $R^9$ and $R^{10}$ are the same or different and are a C1 to C4 alkyl group optionally having one or more halogen atoms or a hydrogen atom, and v is 1, can be produced, for example, by reacting a compound represented by formula (14) with a compound represented by formula (15).

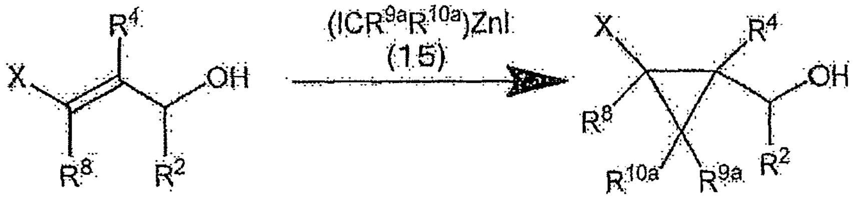


(14) (7−2)

wherein $R^{9a}$ and $R^{10a}$ represent a C1 to C4 alkyl group optionally having one or more halogen atoms or a hydrogen atom, and X, $R^2$, $R^4$ and $R^8$ represent the same meaning as above.

The reaction is usually carried out in a solvent.

Examples of the solvent include halogenated hydrocarbons such as dichloromethane, chloroform, 1,2-dichloroethane and chlorobenzene, ethers such as diethyl ether and tetrahydrofuran, aliphatic hydrocarbons such as hexane and heptane, aromatic hydrocarbons such as toluene and xylene, and mixtures thereof.

The reaction time is usually within the range of 1 hour to 72 hours.

The reaction temperature is usually within the range of -80 to 100°C (with the proviso that, when the boiling point of the used solvent is less than 100°C, it is within the range of -80°C to the boiling point of the solvent).

Regarding the amount of the compound represented by the formula (14) used, the compound represented by the formula (15) is usually an arbitrary ratio from 1 mol to an excess amount, and preferably a ratio of 1 to 5 mol, based on 1 mol of the compound represented by the formula (14).

After completion of the reaction, the compound represented by the formula (7-2) can be obtained by adding the reaction mixture to an acidic aqueous solution (hydrochloric acid, etc.) and then subjecting it to usual post-treatment operations such as an extraction with an organic solvent and concentration. In addition, the obtained compound represented by the formula (7-2) can be further purified by procedures such as chromatography, recrystallization and distillation.

(Reference Production Method 11)

Among the compounds represented by the formula (7), a compound represented by formula (7-2-1) wherein $R^2$ is a hydrogen atom, $R^3$ and $R^5$ are taken together to be $-(CR^9R^{10})_v-$, $R^9$ and $R^{10}$ are the same or different and are a C1 to C4 alkyl group optionally having one or more halogen atoms or a hydrogen atom, and v is 1, can be also produced, for example, by the following method.

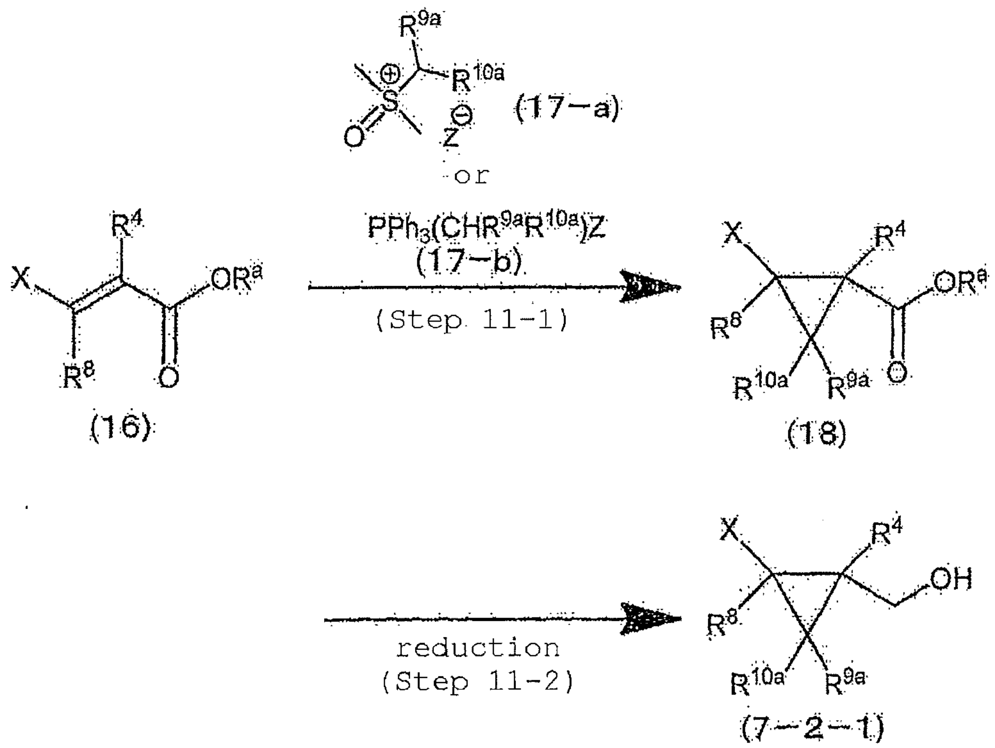


wherein X, Z, $R^a$, $R^4$, $R^8$, $R^{9a}$ and $R^{10a}$ represent the same meaning as above.

(Step 11-1)

A compound represented by formula (18) can be produced by reacting a compound represented by formula (16) with a compound represented by formula (17-a) or a compound represented by formula (17-b), in the presence of a base.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as diethyl ether, tetrahydrofuran and dimethoxyethane, organic sulfurs such as dimethylsulfoxide and sulfolane, aliphatic hydrocarbons such as hexane and heptane, aromatic hydrocarbons such as toluene and xylene, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (16) and the compound represented by the formula (17-a) or the compound represented by the formula (17-b) used can be arbitrarily set, and is preferably an equimolar ratio or a ratio close thereto. Specifically, the compound represented by the formula (17-a) or the compound represented by the formula (17-b) is preferably 1 to 3 mol, based on 1 mol of the compound represented by the formula (16).

The base is used usually in an arbitrary ratio from 1 mol to an excess amount, and preferably in a ratio of 1 to 3 mol, based on 1 mol of the compound represented by the formula (17-a) or the compound represented by the formula (17-b).

After completion of the reaction, the compound represented by the formula (18) can be obtained by adding the reaction mixture to an acidic aqueous solution and then subjecting it to usual post-treatment operations such as an extraction with an organic solvent and concentration. In addition, the obtained compound represented by the formula (18) can be further purified by procedures such as chromatography, recrystallization and distillation.

(Step 11-2)

A compound represented by formula (7-2-1) can be produced by subjecting the compound represented by the formula (18) to a reduction reaction.

Examples of the reduction reaction include;

(1) A method using a borane complex,
(2) A method using lithium borohydride ($LiBH_4$),
(3) A method using diisobutylaluminum hydride (DIBAL-H),
(4) A method using lithium aluminum hydride (LAH) ($LiAlH_4$), and the like.

(1) The method using a borane complex is specifically exemplified as an example.

The reaction is usually carried out in a solvent.
Examples of the borane complex to be used include a borane/tetrahydrofuran complex, a borane/dimethyl sulfide complex, and diborane.
Examples of the solvent to be used include ethers such as diethyl ether, tetrahydrofuran and dimethoxyethane, aliphatic hydrocarbons such as hexane and heptane, aromatic hydrocarbons such as toluene and xylene, and mixtures thereof.
The reaction time is usually within the range of 5 minutes to 24 hours.
The reaction temperature is usually within the range of -80 to 50°C (with the proviso that, when the boiling point of the used solvent is less than 50°C, it is within the range of -80°C to the boiling point of the solvent).
The borane complex is used usually in an arbitrary ratio from 1 mol to an excess amount, and preferably in a ratio of 1 to 2 mol, based on 1 mol of the compound represented by the formula (18).
After completion of the reaction, the compound represented by the formula (7-2-1) can be obtained by adding the reaction mixture to water and then subjecting it to usual post-treatment operations such as an extraction with an organic solvent and concentration. In addition, the obtained compound represented by the formula (7-2-1) can be further purified by procedures such as chromatography, recrystallization and distillation.

(Reference Production Method 12)

Among the compounds represented by the formula (7), a compound represented by formula (7-3) wherein $R^3$ and $R^5$ are taken together to be $-(CR^9R^{10})_v-$, $R^9$ and $R^{10}$ are the same or different and are a hydrogen atom or a halogen atom, and v is 1, can be also produced, for example, by reacting a compound represented by formula (14) with a compound represented by formula (19), in the presence of a base.

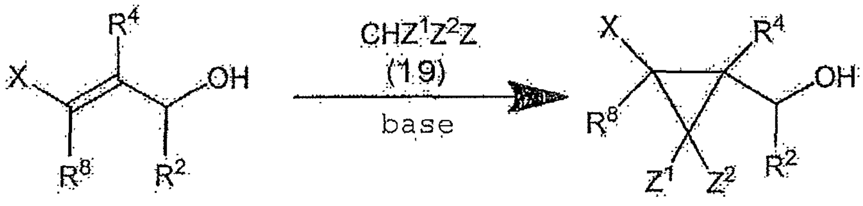

(14) (7-3)

wherein $Z^1$ and $Z^2$ represent a hydrogen atom or a halogen atom, and X, Z, $R^2$, $R^4$ and $R^8$ represent the same meaning as above.
The reaction is usually carried out in a solvent, in the presence of a base.
Examples of the base include alkali metal alkoxides such as sodium hydride, sodium hydroxide and potassium hydroxide.
Still another base is used for the reaction as necessary, and examples of the base include tertiary amines such as triethylamine, diisopropylethylamine, 1,8-diazabicyclo[5.4.0]undec-7-ene and 1,5-diazabicyclo[4.3.0]non-5-ene, and nitrogen-containing aromatic compounds such as pyridine and 4-dimethylaminopyridine.
Examples of the solvent include ethers such as diethyl ether and tetrahydrofuran, aliphatic hydrocarbons such as hexane and heptane, aromatic hydrocarbons such as toluene and xylene, and mixtures thereof.
The reaction time is usually within the range of 1 hour to 72 hours.
The reaction temperature is usually within the range of -20 to 100°C (with the proviso that, when the boiling point of the used solvent is less than 100°C, it is within the range of -20°C to the boiling point of the solvent).
The molar ratio of the compound represented by the formula (14) and the compound represented by the formula (19) used is an arbitrary ratio from 1 mol to an excess amount, and the compound represented by the formula (19) is preferably a ratio of 1 to 5 mol, based on 1 mol of the compound represented by the formula (14).
The base is used usually in an arbitrary ratio from 1 mol to an excess amount, and preferably in a ratio of 1 to

5 mol, based on 1 mol of the compound represented by the formula (19).

After completion of the reaction, the compound represented by the formula (7-3) can be obtained by adding the reaction mixture to an acidic aqueous solution and then subjecting it to usual post-treatment operations such as an extraction with an organic solvent and concentration. In addition, the obtained compound represented by the formula (7-3) can be further purified by procedures such as chromatography, recrystallization and distillation.

(Reference Production Method 13)

Among the compounds represented by the formula (7), a compound represented by formula (7-4) wherein $R^3$ and $R^5$ are taken together to be - $(CR^9R^{10})_v$-, v is 1, 2, 3 or 4, and $R^8$ is a hydrogen atom can be also produced, for example, by the following method.

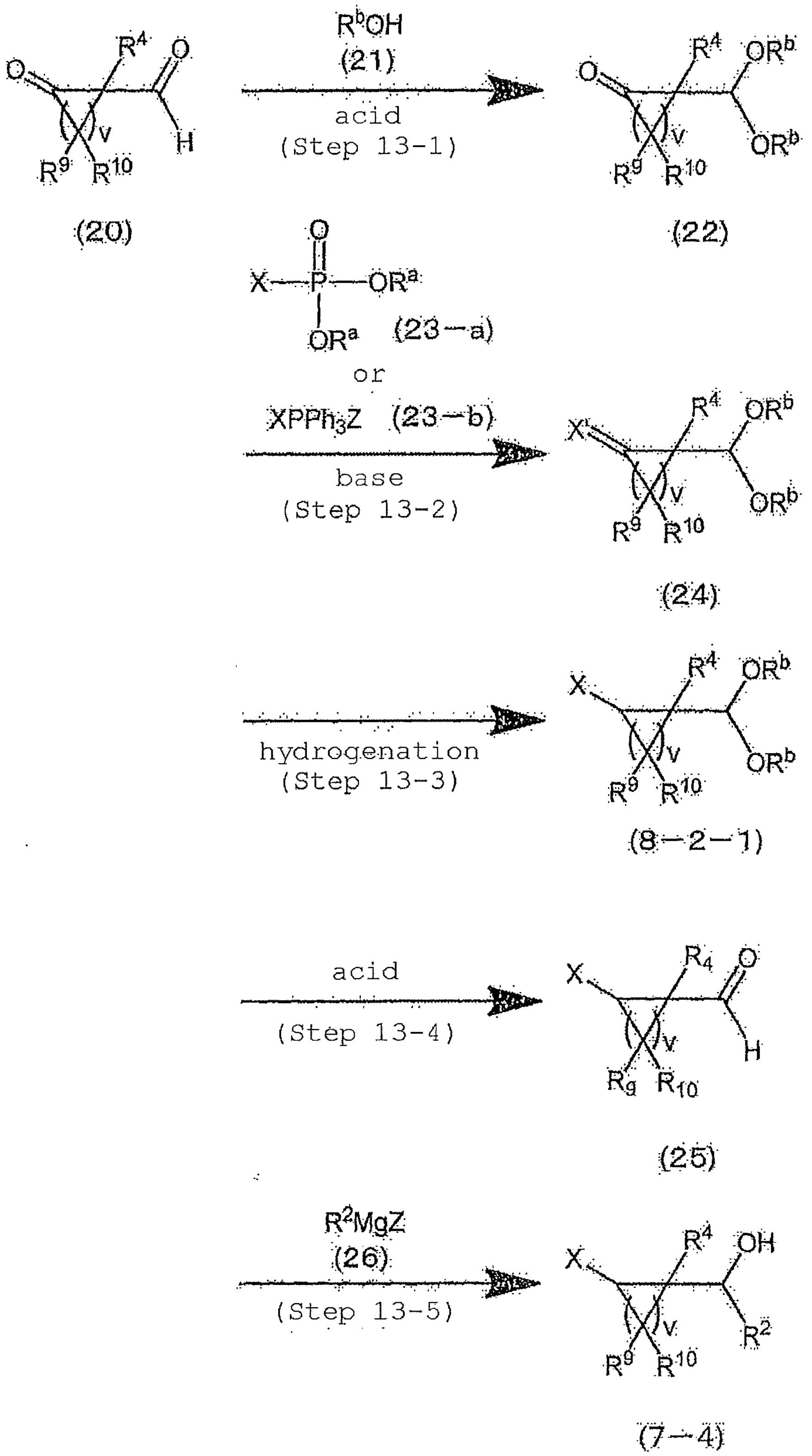


(20) (22) (24) (8-2-1) (25) (7-4)

wherein $X^1$ represents a divalent group from which a hydrogen atom bonding to a carbon atom at position 1 in X is removed, and X, Z, $R^a$, $R^b$, $R^2$, $R^4$, $R^9$ and $R^{10}$ represent the same meaning as above.

(Step 13-1)

A compound represented by formula (22) can be produced by reacting a compound represented by formula

(20) with a compound represented by formula (21), in the presence of an acid.

The reaction is carried out, in the presence of an acid, in a solvent as necessary.

Examples of the acid to be used include inorganic acids such as hydrochloric acid and sulfuric acid and organic acids such as methanesulfonic acid and p-toluenesulfonic acid.

Examples of the solvent to be used include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, and mixtures thereof.

The reaction time is usuallywithin the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The acid is used usually in an arbitrary ratio from 0.001 mol to an excess amount, based on 1 mol of the compound represented by the formula (20).

The compound represented by the formula (21) is usually used in an arbitrary ratio from 2 mol to the amount of the solvent, based on 1 mol of the compound represented by the formula (20).

After completion of the reaction, the compound represented by the formula (22) can be isolated by making the reaction mixture with a basic aqueous solution and then subjecting it to usual post-treatment operations such as an extraction with an organic solvent and concentration. In addition, the isolated compound represented by the formula (22) can be also purified by procedures such as chromatography, recrystallization and distillation.

(Step 13-2)

A compound represented by formula (24) can be produced by reacting the compound represented by the formula (22) with a compound represented by formula (23-a) or a compound represented by formula (23-b), in the presence of a base.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as benzene, toluene and xylene, halogenated hydrocarbons such as chlorobenzene, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (22) and the compound represented by the formula (23-a) or the compound represented by the formula (23-b) used can be arbitrarily set, and is preferably an equimolar ratio or a ratio close thereto. Specifically, the compound represented by the formula (23-a) or the compound represented by the formula (23-b) is preferably 0.5 to 3 mol, based on 1 mol of the compound represented by the formula (22).

The base is used usually in an arbitrary ratio from 1 mol to an excess amount, and preferably in a ratio of 1 to 5 mol, based on 1 mol of the compound represented by the formula (23-a) or the compound represented by the formula (23-b).

After completion of the reaction, the compound represented by the formula (24) can be isolated by adding the reaction mixture to water and then subjecting it to usual post-treatment operations such as an extraction with an organic solvent and concentration. In addition, the isolated compound represented by the formula (24) can be also purified by procedures such as chromatography, recrystallization and distillation.

(Step 13-3)

A compound represented by formula (8-2-1) can be produced by subjecting the compound represented by the formula (24) to a reduction reaction under a hydrogen atmosphere, in the presence of a hydrogenation catalyst.

The reaction is usually carried out in a solvent, under a hydrogen atmosphere.

Examples of the hydrogenation catalyst to be used include transition metal compounds such as a palladium carbon, palladium hydroxide, Raney nickel and platinum oxide.

Examples of the solvent to be used include alcohols such as methanol, ethanol and propanol, esters such as ethyl acetate and butyl acetate, ethers such as tetrahydrofuran and 1,4-dioxane, and mixtures thereof.

The reaction is usually carried out under a hydrogen atmosphere at 1 to 100 atm.

The reaction time is usuallywithin the range of 5 minutes to 72 hours.

The reaction temperature is usually within the range of -20 to 100°C (with the proviso that, when the boiling

point of the used solvent is less than 100°C, it is within the range of -20°C to the boiling point of the solvent).

The amount of the hydrogenation catalyst to be used is usually a ratio of 0.001 to 0. 5 mol, based on 1 mol of the compound represented by the formula (24).

After completion of the reaction, the compound represented by the formula (8-2-1) can be isolated by filtering the reaction mixture and then subjecting it to an operation such as concentration. In addition, the isolated compound represented by the formula (8-2-1) can be further also purified by procedures such as chromatography, recrystallization and distillation.

(Step 13-4)

A compound represented by formula (25) can be produced by reacting the compound represented by the formula (8-2-1) and water, in the presence of an acid.

The reaction is carried out in the presence of an acid, and in the presence of water and an organic solvent.

Examples of the acid to be used include inorganic acids such as hydrochloric acid and sulfuric acid and organic acids such as methanesulfonic acid and p-toluenesulfonic acid.

Examples of the organic solvent to be used include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, nitriles such as acetonitrile and butyronitrile, alcohols such as methanol, ethanol and propanol, ketones such as acetone and methyl isobutyl ketone, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The acid is used in an arbitrary amount, usually used in a ratio from 1 mol to an excess amount, based on 1 mol of the compound represented by the formula (8-2-1).

After completion of the reaction, the compound represented by the formula (25) can be isolated by adding the reaction mixture to water and then subjecting it to usual post-treatment operations such as an extraction with an organic solvent and concentration. In addition, the isolated compound represented by the formula (25) can be also purified by procedures such as chromatography, recrystallization and distillation.

(Step 13-5)

A compound represented by formula (7-4) can be produced by reacting the compound represented by the formula (25) with the compound represented by the formula (26).

The reaction is usually carried out in a solvent.

Examples of the organic solvent include ethers such as diethyl ether, 1,4-dioxane, tetrahydrofuran, ethylene glycol dimethyl ether and tert-butyl methyl ether, aromatic hydrocarbons such as toluene and xylene, and mixtures thereof.

The reaction time is usually within the range of 5 minutes to 24 hours.

The reaction temperature is usually within the range of -50 to 100°C (with the proviso that, when the boiling point of the used solvent is less than 100°C, it is within the range of -50°C to the boiling point of the solvent).

The molar ratio of the compound represented by the formula (25) and the compound represented by the formula (26) used can be arbitrarily set, and is preferably an equimolar ratio or a ratio close thereto. Specifically, the compound represented by the formula (26) is preferably 0.5 to 3 mol, based on 1 mol of the compound represented by the formula (25).

After completion of the reaction, the compound represented by the formula (7-4) can be isolated by adding the reaction mixture to an acidic aqueous solution (hydrochloric acid, etc.) and then subjecting it to usual post-treatment operations such as an extraction with an organic solvent and concentration. In addition, the isolated compound represented by the formula (7-4) can be also purified by procedures such as chromatography, recrystallization and distillation.

(Reference Production Method 14)

Among the compounds represented by the formula (7), a compound represented by formula (7-5) wherein $R^3$ and $R^5$ are taken together to be an oxygen atom can be produced, for example, by subjecting a compound represented by formula (14) to an oxidation reaction.

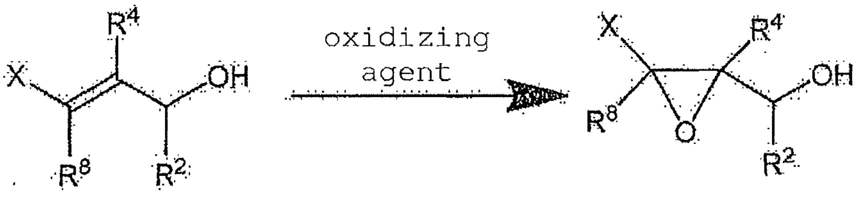


(14) (7-5)

wherein X, $R^2$, $R^4$ and $R^8$ represent the same meaning as above.

The reaction is usually carried out in a solvent.

Examples of the oxidizing agent include peracids such as perbenzoic acid, mCPBA (meta-chloroperbenzoic acid), monoperoxyphthalic acid, trifluoroperacetic acid and peracetic acid, peroxides such as dioxirane, and the like.

Examples of the solvent include halogenated hydrocarbons such as dichloromethane, chloroform, 1,2-dichloroethane and chlorobenzene, aliphatic hydrocarbons such as hexane and heptane, aromatic hydrocarbons such as toluene and xylene, and mixtures thereof.

The reaction time is usually within the range of 5 minutes to 72 hours.

The reaction temperature is usually within the range of -20 to 80°C (with the proviso that, when the boiling point of the used solvent is less than 80°C, it is within the range of -20°C to the boiling point of the solvent).

The molar ratio of the compound represented by the formula (14) and the oxidizing agent used can be arbitrarily set, and is preferably an equimolar ratio or a ratio close thereto. Specifically, the oxidizing agent is preferably 0.5 to 1 mol, based on 1 mol of the compound represented by the formula (14).

After completion of the reaction, the compound represented by the formula (7-5) can be obtained by filtering the reaction mixture then sequentially washing the filtrate with an aqueous solution of sodium sulfite, an aqueous solution of sodium bicarbonate and water, and subjecting it to usual post-treatment operations such as concentration. In addition, the obtained compound represented by the formula (7-5) can be further purified by procedures such as chromatography, recrystallization and distillation.

(Reference Production Method 15)

Among the compounds represented by the formula (7), a compound represented by formula (7-6) wherein $R^3$ and $R^5$ are taken together to be a sulfur atom can be produced, for example, by reacting a compound represented by formula (7-5) with a compound represented by formula (27), in the presence of an acid.

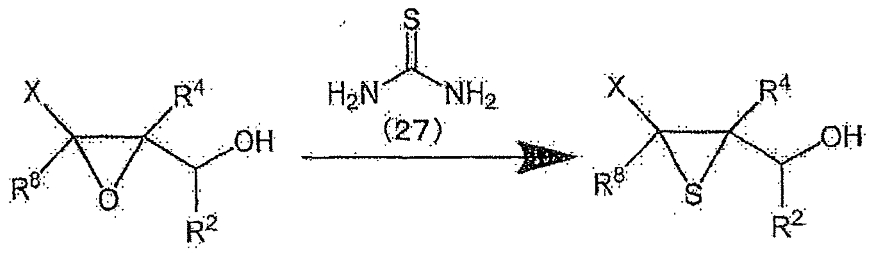


(7-5) (7-6)

wherein X, $R^2$, $R^9$ and $R^8$ represent the same meaning as above.

The reaction is usually carried out in a solvent as necessary.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The acid includes inorganic acids such as sulfuric acid and hydrochloric acid.

The reaction time is usually within the range of 5 minutes to 24 hours.

The reaction temperature is usually within the range of -20 to 80°C (with the proviso that, when the boiling point of the used solvent is less than 80°C, it is within the range of -20°C to the boiling point of the solvent).

The molar ratio of the compound represented by the formula (7-5) and the compound represented by the formula (27) used can be arbitrarily set, and is preferably an equimolar ratio or a ratio close thereto. Specifically, the ratio of the compound represented by the formula (27) is preferably 1 to 3 mol, based on 1 mol of the compound

represented by the formula (7-5).

The acid is used in an arbitrary amount, and usually in a ratio of an excess amount, based on 1 mol of the compound represented by the formula (7-5).

After completion of the reaction, the compound represented by the formula (7-6) can be obtained by filtering the reaction mixture then sequentially washing the filtrate with an aqueous solution of sodium sulfite, an aqueous solution of sodium bicarbonate and water, and subjecting it to usual post-treatment operations such as concentration. In addition, the obtained compound represented by the formula (7-6) can be further purified by procedures such as chromatography, recrystallization and distillation.

(Reference Production Method 16)

Among the compounds represented by the formula (8), a compound represented by formula (8-3) wherein W is $-CR^8-$, r is 1, and $R^3$ and $R^5$ are taken together to be $-NR^{11}-$, can be also produced, for example, by the following method.

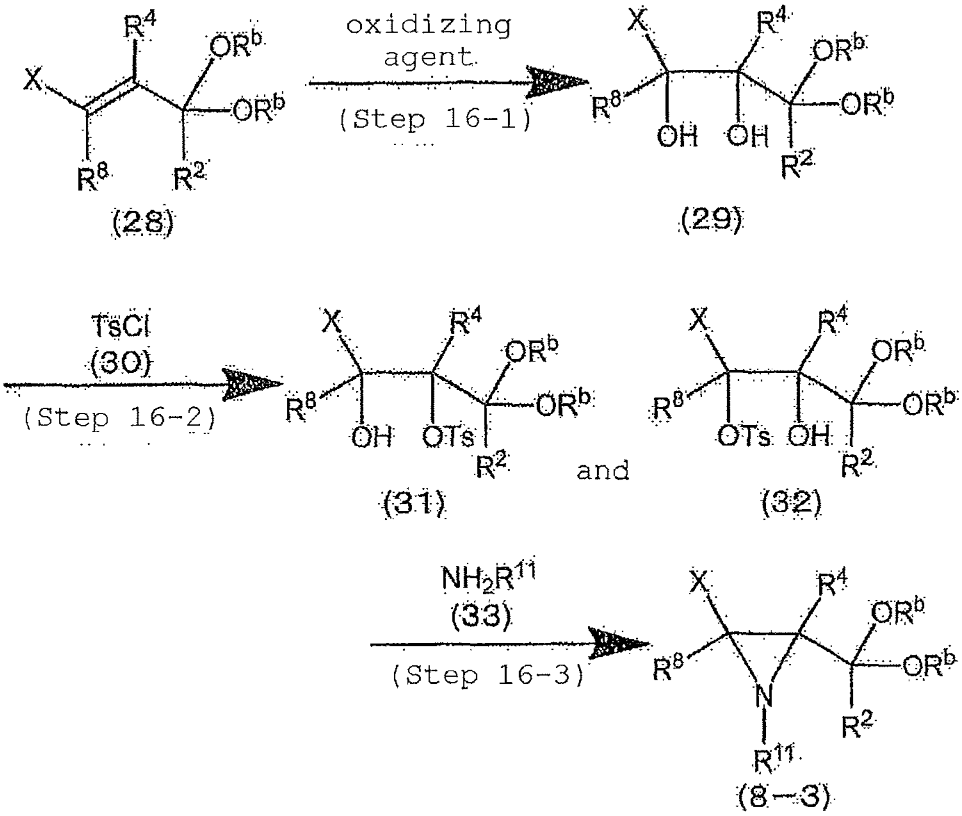


wherein Ts represents a 4-toluenesulfonyl group, and X, $R^b$, $R^2$, $R^4$, $R^8$ and $R^{11}$ represent the same meaning as above.

(Step 16-1)

A compound represented by formula (29) can be produced by subjecting a compound represented by formula (28) to an oxidation reaction.

The reaction is usually carried out in a solvent.

Examples of the oxidizing agent include osmium tetroxide and osmium tetroxide salts.

A co-oxidizing agent is used for the reaction together with the oxidizing agent as necessary, and examples of the co-oxidizing agent include aqueous hydrogen peroxide, sodium chlorate, potassium chlorate, tert-butyl hydroperoxide, N-methylmorpholine N-oxide, potassium hexacyanoferrate(III), and the like.

Examples of the solvent include ethers such as diethyl ether, tetrahydrofuran and dimethoxyethane, ketones such as acetone, aliphatic hydrocarbons such as hexane and heptane, aromatic hydrocarbons such as benzene, toluene and xylene, alcohols such as isopropyl alcohol and tert-butyl alcohol, water, and mixtures thereof.

The reaction time is usually within the range of 5 minutes to 72 hours.

The reaction temperature is usually within the range of -80 to 100°C (with the proviso that, when the boiling

point of the used solvent is less than 100°C, it is within the range of -80°C to the boiling point of the solvent).

The molar ratio of the compound represented by the formula (28) and the oxidizing agent used can be arbitrarily set, and is preferably an equimolar ratio or a ratio close thereto. Specifically, the ratio of the oxidizing agent is preferably 1 to 2 mol, based on 1 mol of the compound represented by the formula (28). Also, when a co-oxidizing agent is used together with the oxidizing agent, the amount of the oxidizing agent used is the catalyst amount, specifically, the oxidizing agent is preferably a ratio of 0.01 to 0.5 mol, based on 1 mol of the compound represented by the formula (28).

After completion of the reaction, the compound represented by the formula (29) can be obtained by adding the reaction mixture to an aqueous solution of sodium bisulfite and then subjecting it to usual post-treatment operations such as an extraction with an organic solvent and concentration. In addition, the obtained compound represented by the formula (29) can be further purified by procedures such as chromatography, recrystallization and distillation.

(Step 16-2)

A mixture of a compound represented by formula (31) and a compound represented by formula (32) can be produced by reacting the compound represented by the formula (29) with the compound represented by the formula (30), in the presence of a base.

The reaction is carried out in a solvent as necessary.

Examples of the base include tertiary amines such as triethylamine, diisopropylethylamine, 1,8-diazabicyclo[5.4.0]undec-7-ene and 1,5-diazabicyclo[4.3.0]non-5-ene, and pyridine.

Examples of the solvent include ethers such as 1,4-dioxane, tetrahydrofuran, ethylene glycol dimethyl ether and tert-butyl methyl ether, aliphatic hydrocarbons such as hexane, heptane and octane, aromatic hydrocarbons such as toluene and xylene, halogenated hydrocarbons such as chlorobenzene, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (29) and the compound represented by the formula (30) used can be arbitrarily set, and is preferably an equimolar ratio or a ratio close thereto. Specifically, the ratio of the compound represented by the formula (30) is preferably 1 to 2 mol, based on 1 mol of the compound represented by the formula (29).

The base is used usually in an arbitrary ratio from 1 mol to an excess amount, and preferably in a ratio of 1 to 3 mol, based on the compound represented by the formula (30).

After completion of the reaction, the mixture of the compound represented by the formula (31) and the compound represented by the formula (32) can be obtained by adding the reaction mixture to water and then subjecting it to usual post-treatment operations such as an extraction with an organic solvent and concentration. In addition, the obtained mixture of the compound represented by the formula (31) and the compound represented by the formula (32) can be further purified by procedures such as chromatography, recrystallization and distillation.

(Step 16-3)

A compound represented by formula (8-3) can be produced by reacting the mixture of the compound represented by the formula (31) and the compound represented by the formula (32) with a compound represented by formula (33), in the presence of a base.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (33) and the mixture of the compound represented by the formula (31) and the compound represented by the formula (32) used can be arbitrarily set, and is preferably an equimolar ratio or a ratio close thereto, for example, the ratio of the compound represented by the formula (33) is 1 to 3 mol, based on 1 mol of the mixture of the compound represented by the formula (31) and the compound represented by the formula (32).

The base is used usually in an arbitrary ratio from 1 mol to an excess amount, and preferably in a ratio of 1 to 3 mol, based on 1 mol of the compound represented by the formula (33).

After completion of the reaction, the compound represented by the formula (8-3) can be isolated by adding the reaction mixture to water and then subjecting it to usual post-treatment operations such as an extraction with an organic solvent and concentration. In addition, the isolated compound represented by the formula (8-3) can be also purified by procedures such as chromatography, recrystallization and distillation.

(Reference Production Method 17)

A compound represented by formula (6) can be produced, for example, by the following method.

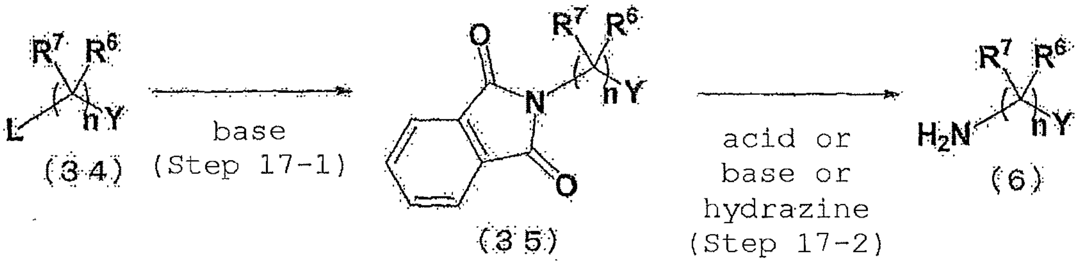


wherein L, Y, $R^6$, $R^7$ and n represent the same meaning as above.

(Reference Production Method 18)

A compound represented by formula (6) can be also produced, for example, by the following method.

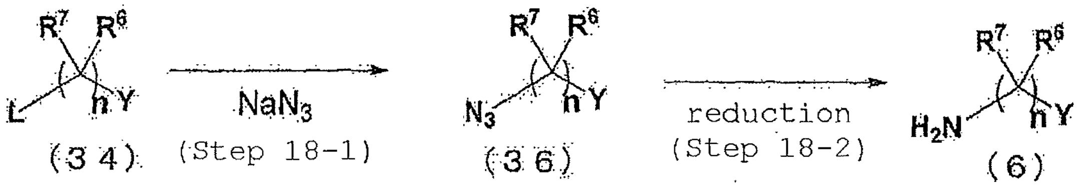


wherein L, Y, $R^6$, $R^7$ and n represent the same meaning as above.

(Step 18-1)

A compound represented by formula (36) can be produced, for example, by reacting a compound represented by formula (34) with sodium azide.

The reaction is usually carried out in a solvent.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 72 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (34) and the sodium azide used can be arbitrarily set, and is preferably an equimolar ratio or a ratio close thereto, for example, the ratio of the sodium azide is 1 to 3 mol, based on 1 mol of the compound represented by the formula (34).

After completion of the reaction, the compound represented by the formula (36) can be isolated by adding the reaction mixture to water and then subjecting it to usual post-treatment operations such as an extraction with an organic solvent and concentration. In addition, the isolated compound represented by the formula (36) can be also purified by procedures such as chromatography, recrystallization and distillation.

(Step 18-2)

A compound represented by formula (6) can be produced by subjecting the compound represented by the formula (36) to a reduction reaction.
Examples of the reduction reaction include;

(1) A method using a hydrogenation catalyst, and
(2) A method using a metal hydride such as sodium borohydride or lithium aluminum hydride.

(1) The method of using a hydrogenation catalyst is specifically exemplified as an example.

The reaction is usually carried out in a solvent, under a hydrogen atmosphere.
Examples of the hydrogenation catalyst include transition metal compounds such as a palladium carbon, palladium hydroxide, Raney nickel and platinum oxide.
Examples of the solvent include alcohols such as methanol, ethanol and propanol, esters such as ethyl acetate and butyl acetate, ethers such as tetrahydrofuran and 1,4-dioxane, and mixtures thereof.
The reaction is usually carried out under a hydrogen atmosphere at 1 to 100 atm.
The reaction time is usually within the range of 5 minutes to 24 hours.
The reaction temperature is usually within the range of -20 to 100°C (with the proviso that, when the boiling point of the used solvent is less than 100°C, it is within the range of -20°C to the boiling point of the solvent).
The amount of the hydrogenation catalyst is usually a ratio of 0.001 to 0.5 mol, based on 1 mol of the compound represented by the formula (36).
After completion of the reaction, the compound represented by the formula (6) can be isolated by filtering the reaction mixture and then subjecting it to an operation such as concentration. The isolated compound represented by the formula (6) can be also further purified by procedures such as chromatography, recrystallization and distillation.

(Reference Production Method 19)

Among the compounds represented by the formula (6), a compound represented by formula (6-1) wherein $R^6$ and $R^7$ are a hydrogen atom can be also produced, for example, by the following method.

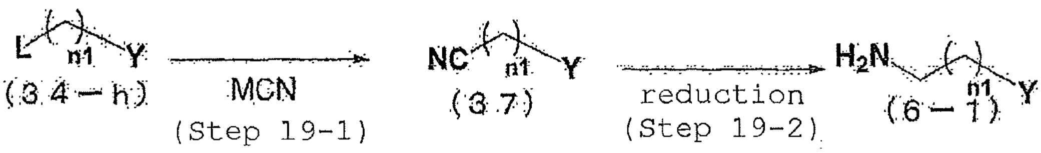


wherein n1 represents 0 or 1, and M, L and Y represent the same meaning as above.

(Step 19-1)

A compound represented by formula (37) can be produced by reacting a compound represented by formula (34-h) with metal cyanide (MCN).
The reaction is usually carried out in a solvent.
The metal cyanide to be used includes sodium cyanide and potassium cyanide.
Examples of the solvent to be used include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.
The reaction time is usually within the range of 5 minutes to 24 hours.
The reaction temperature is usually within the range of 0 to 100°C (with the proviso that, when the boiling point of the used solvent is less than 100°C, it is within the range of 0°C to the boiling point of the solvent).
The molar ratio of the compound represented by the formula (34-h) and the metal cyanide used can be arbitrarily set, and is preferably an equimolar ratio or a ratio close thereto, for example, the ratio of the metal cyanide is 1 to 3 mol based on 1 mol of the compound represented by the formula (34-h).
After completion of the reaction, the compound represented by the formula (37) can be isolated by adding the reaction mixture to water and then subjecting it to usual post-treatment operations such as an extraction with an organic solvent and concentration. In addition, the isolated compound represented by the formula (37) can be also purified by

procedures such as chromatography, recrystallization and distillation.

(Step 19-2)

A compound represented by formula (6-1) can be produced by subjecting the compound represented by the formula (37) to a reduction reaction.
Examples of the reduction reaction include;

(1) A method using a hydrogenation catalyst,
(2) A method using a metal hydride such as sodium borohydride or lithium aluminum hydride,

and the like.
The method of using a hydrogenation catalyst is specifically exemplified as an example.
The reaction is usually carried out in the presence of a solvent, under a hydrogen atmosphere.
Examples of the hydrogenation catalyst include transition metal compounds such as a palladium carbon, palladium hydroxide, Raney nickel and platinum oxide.
Examples of the solvent include alcohols such as methanol, ethanol and propanol, esters such as ethyl acetate and butyl acetate, ethers such as tetrahydrofuran and 1,4-dioxane, and mixtures thereof.
The reaction is usually carried out under a hydrogen atmosphere at 1 to 100 atm, and the reaction can be also carried out by further adding ammonia as necessary.
The reaction time is usually within the range of 5 minutes to 24 hours.
The reaction temperature is usually within the range of -20 to 100°C (with the proviso that, when the boiling point of the used solvent is less than 100°C, it is within the range of -20°C to the boiling point of the solvent).
The amount of the hydrogenation catalyst is usually a ratio of 0.001 to 0.5 mol, based on 1 mol of the compound represented by the formula (37).
After completion of the reaction, the compound represented by the formula (6-1) can be isolated by filtering the reaction mixture and then subjecting it to an operation such as concentration. The isolated compound represented by the formula (6-1) can be also further purified by procedures such as chromatography, recrystallization and distillation.

(Reference Production Method 20)

The compound represented by the formula (38-a) can be produced, for example, by reacting a compound represented by formula (42) with a compound represented by formula (43).

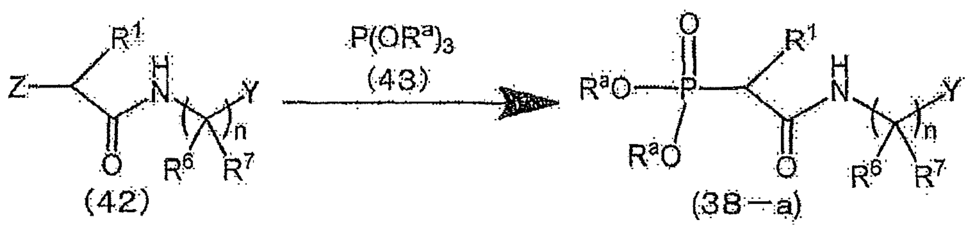


(42) (38-a)

wherein Y, Z, $R^a$, $R^1$, $R^6$, $R^7$ and n represent the same meaning as above.
The reaction is carried out in a solvent, as necessary.
Examples of the solvent include ethers such as 1,4-dioxane, tetrahydrofuran, ethylene glycol dimethyl ether and tert-butyl methyl ether, aromatic hydrocarbons such as benzene, toluene and xylene, acid amides such as N,N-dimethylformamide and N,N-dimethylacetoamide, nitriles such as acetonitrile and butyronitrile, and mixtures thereof.
The reaction time is usually within the range of 5 minutes to 72 hours.
The reaction temperature is usually within the range of 0 to 150°C (with the proviso that, when the boiling point of the used solvent is less than 150°C, it is within the range of 0°C to the boiling point of the solvent).
The molar ratio of the compound represented by the formula (42) and the compound represented by the formula (43) used can be arbitrarily set, and is preferably an excess amount. Specifically, the compound represented by the formula (43) is preferably 1 to 10 mol, based on 1 mol of the compound represented by the formula (42).
After completion of the reaction, the compound represented by the formula (38-a) can be obtained by a post-treatment operation such as concentrating the organic layer as it is. In addition, the obtained compound represented by the formula (38-a) can be further purified by procedures such as chromatography, recrystallization and distillation.

(Reference Production Method 21)

The compound represented by the formula (38-b) can be produced, for example, by reacting a compound represented by formula (42) with triphenylphosphine.

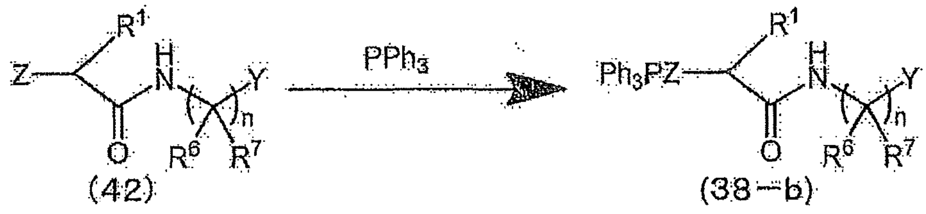


wherein Y, Z, $R^1$, $R^6$, $R^7$ and n represent the same meaning as above.
The reaction is carried out in a solvent, as necessary.
Examples of the solvent include ethers such as 1,4-dioxane, tetrahydrofuran, ethylene glycol dimethyl ether and tert-butyl methyl ether, aromatic hydrocarbons such as benzene, toluene and xylene, acid amides such as N,N-dimethylformamide and N,N-dimethylacetoamide, nitriles such as acetonitrile and butyronitrile, and mixtures thereof.
The reaction time is usually within the range of 5 minutes to 72 hours.
The reaction temperature is usually within the range of 0 to 150°C (with the proviso that, when the boiling point of the used solvent is less than 150°C, it is within the range of 0°C to the boiling point of the solvent).
The molar ratio of the compound represented by the formula (42) and triphenylphosphine used can be arbitrarily set, and is preferably an equimolar ratio or a ratio close thereto. Specifically, triphenylphosphine is preferably in a ratio of 0.5 to 3 mol, based on 1 mol of the compound represented by the formula (42).
After completion of the reaction, the compound represented by the formula (38-b) can be obtained by filtering the precipitated solid. In addition, the obtained compound represented by the formula (38-b) can be further purified by procedures such as recrystallization.

(Reference Production Method 22)

The compound represented by the formula (42) can be produced, for example, by reacting a compound represented by formula (44) with a compound represented by formula (6), in the presence of a base.

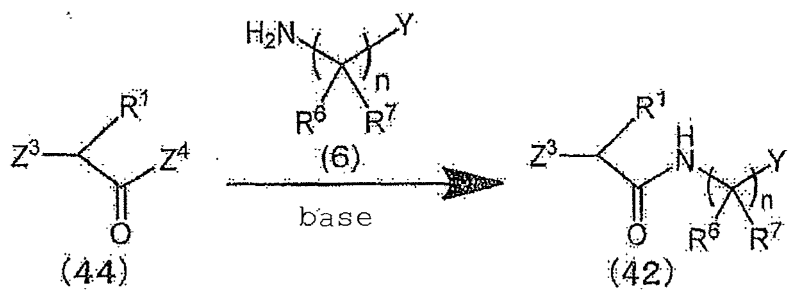


wherein $Z^3$ and $Z^4$ are the same or different and represent a chlorine atom, a bromine atom or an iodine atom, and $R^1$, $R^6$, $R^7$ and n represent the same meaning as above.
The reaction is usually carried out in a solvent, in the presence of a base.
Examples of the base include hydroxides such as sodium hydroxide and potassium hydroxide, carbonates such as sodium carbonate and potassium carbonate, tertiary amines such as triethylamine, diisopropylethylamine, 1,8-diazabicyclo[5.4.0]undec-7-ene and 1,5-diazabicyclo[4.3.0]non-5-ene, and nitrogen-containing aromatic compounds such as pyridine and 4-dimethylaminopyridine.
Examples of the solvent include ethers such as 1,4-dioxane, tetrahydrofuran, ethylene glycol dimethyl ether and tert-butyl methyl ether, aliphatic hydrocarbons such as hexane, heptane and octane, aromatic hydrocarbons such as toluene and xylene, halogenated hydrocarbons such as dichloromethane, chloroform, 1,2-dichloroethane and chlorobenzene, esters such as ethyl acetate and butyl acetate, nitriles such as acetonitrile and butyronitrile, acid amides such as N,N-dimethylformamide, sulfoxides such as dimethyl sulfoxide, water, and mixtures thereof.
The reaction time is usually within the range of 5 minutes to 72 hours.
The reaction temperature is usually within the range of -20 to 100°C (with the proviso that, when the boiling

point of the used solvent is less than 100°C, it is within the range of -20°C to the boiling point of the solvent).

The molar ratio of the compound represented by the formula (44) and the compound represented by the formula (6) used can be arbitrarily set, and is preferably an equimolar ratio or a ratio close thereto. Specifically, the ratio of the compound represented by the formula (6) is 0.5 to 3 mol, based on 1 mol of the compound represented by the formula (40).

The base is used usually in an arbitrary ratio from 1 mol to an excess amount, and preferably in a ratio of 1 to 3 mol, based on 1 mol of the compound represented by the formula (6).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

The pest on which the composition of the present invention has a control effect includes arthropod pests such as pest insects and pest mites. More specifically, examples are as described below.

Hemiptera: Delphacidae such as Laodelphax striatellus, Nilaparvatalugens and Sogatella furcifera, Deltocephalidae such as Nephotettix cincticeps and Nephotettix virescens, Aphididae such as Aphis gossypii and Myzus persicae, Pentatomidae such as Nezara antennata, Riptortus clavetus, Eysarcoris lewisi, Bemisia argentifolii, Eysarcoris parvus, Plautia stali, Halyomorpha mista, Stenotus rubrovittatus and Trigonotylus ruficornis, Aleyrodidae such as Trialeurodes vaporariorum and Bemisia argentifolii, Coccidae such as Aonidiella aurantii, Comstockaspis perniciosa, Unaspis citri, Ceroplastes rubens and Icerya purchasi, Tingidae, Cimicoidea such as Cimex lectularius, Psyliidae, etc.;

Lepidoptera: Pyralidae such as Chilo suppressalis, Cnaphalocrocis medinalis, Notarcha derogata and Plodia interpunctella, Noctuidae such as Spodoptera litura, Pseudaletia separata, Trichoplusia spp., Heliothis spp., and Helicoverpa spp., Pieridae such as Pieris rapae, Tortricidae such as Adoxophyes spp. , Grapholita molesta and Cydia pomonella, Carposinidae such as Carposina niponensis, Lyonetiidae such as Lyonetia spp., Lymantriidae such as Lymantria spp. and Euproctis spp., Yponomeutidae such as Plutella xylostella, Gelechiidae such as Pectinophora gossypiella, Arctiidae such as Hyphantria cunea, Tineidae such as Tinea translucens and Tineolabisselliella, etc.;

Diptera: Culex such as Culex pipiens pallens, Culex tritaeniorhynchus and Culex quinquefasciatus, Aedes spp. such as Aedes aegypti and Aedes albopictus, Anopheles spp. such as Anopheles sinensis, Chironomidae, Muscidae such as Musca domestica and Muscina stabulans, Calliphoridae, Sarcophagidae, Fanniidae, Anthomyiidae such as Delia platura and Delia antiqua, Agromyzidae such as Liriomyza trifolii, Tephritidae, Drosophilidae, Phoridae such as Megaselia spiracularis, Psychodidae such as Clogmia albipunctata, Simuliidae, Tabanidae, Stomoxys, etc.;

Coleoptera: Diabrotica such as Diabrotica virgifera virgifera and Diabrotica undecimpunctata howardi, Scarabaeidae such as Anomala cuprea and Anomala rufocuprea, Curculionidae such as Sitophilus zeamais, Lissorhoptrus oryzophilus and Callosobruchuys chienensis, Tenebrionidae such as Tenebrio molitor and Tribolium castaneum, Chrysomelidae such as Oulema oryzae, Aulacophora femoralis, Phyllotreta striolata and Leptinotarsa decemlineata, Dermestidae such as Dermestes maculates, Anobiidae, Epilachna such as Epilachna vigintioctopunctata, Lyctidae, Bostrychidae, Ptinidae, Cerambycidae, Paederus fuscipes, etc.;

Blattodea: Blattella germanica, Periplaneta fuliginosa, Periplaneta americana, Periplaneta brunnea, Blatta orientalis, etc.;

Thysanoptera: Thrips palmi, Thrips tabaci, Frankliniella occidentalis, Frankliniella intonsa, etc.;

Hymenoptera: Formicidae such as Monomorium pharaosis, Formica fusca japonica, Ochetellus glaber, Pristomyrmex pungens and Pheidole noda, Vespidae, Bethylidae, Tenthredinidae such as Athalia japonica, etc.;

Orthoptera: Gryllotalpidae, Acrididae, Grylloidea, etc.;

Siphonaptera: Ctenocephalides felis, Ctenocephalides canis, Pulex irritans, Xenopsylla cheopis, etc.;

Anoplura: Pediculus humanus corporis, Phthirus pubis, Haematopinus eurysternus, Dalmalinia ovis, Haematopinus suis, etc.;

Isoptera: Subterranean termites such as Reticulitermes speratus, Coptotermes formosanus, Reticulitermes flavipes, Reticulitermes hesperus, Reticulitermes virginicus, Reticulitermes tibialis and Heterotermes aureus, Drywood termites such as Incisitermes minor, Dampwood termites such as Zootermopsis nevadensis, etc.;

Acarina: Tetranychidae such as Tetranychus urticae, Tetranychus kanzawai, Panonychus citri, Panonychus ulmi and Oligonychus spp. , Eriophyidae such as Aculops lycopers, Aculops pelekassi and Aculus schlechtendali, Tarsonemidae such as Polyphagotarsonemus latu, Tenuipalpidae, Tuckerellidae, Metastigmata such as Haemaphysalis longicornis, Haemaphysalis flava, Dermacentor variabilis, Haemaphysalis flava, Dermacentor taiwanicus, Ixodes ovatus, Ixodes persulcatus, Ixodes scapularis, Boophilus microplus, Amblyomma americanum and Rhipicephalus sanguineus, Acaridae such as Tyrophagus putrescentiae, Pyroglyphidae such as Dermatophagoides farinae and Dermatophagoides pteronyssinus, Cheyletidae such as Cheyletus eruditus, Cheyletus malaccensis and Cheyletus moorei, Dermanyssidae such as Ornithonyssus bacoti, Ornithonyssus sylvairum and Dermanyssus gallinae, Trombiculidae such as Leptotrombidium akamushi, etc.;

Arachnida: Chiracanthium japonicum, Latrodectus hasseltii, etc.;

Chilopoda: Thereuonema hilgendorfi, Scolopendra subspinipes, etc.;

Diplopoda: Oxidus gracilis, Nedyopus tambanus, etc.;

Isopoda: Armadillidium vulgare, etc.;
Gastropoda: Limax marginatus, Limax flavus, etc.

The pest control agent of the present invention contains the compound of the present invention and an inert carrier. The pest control agent of the present invention is usually obtained by mixing the compound of the present invention and an inert carrier such as a solid carrier, a liquid carrier or a gaseous carrier, and adding a surfactant or other auxiliaries for formulation as necessary, to be formulated into emulsifiable concentrates, oil formulations, dust formulations, granules, wettable powders, flowables, microcapsule formulations, aerosols, fumigants, poisonous baits, resin formulations and the like. These formulations usually contain the compound of the present invention in an amount of 0.01 to 95% by weight.

Examples of the solid carrier which is used in the formulation include fine powder and granules of clay materials (kaolin clay, diatomaceous earth, bentonite, Fubasami clay, acid clay, etc.), synthetic hydrated silicon oxide, talc, ceramics, other inorganic minerals (sericite, quartz, sulfur, activated carbon, calcium carbonate, hydrated silica, etc.), chemical fertilizers (ammonium sulfate, ammonium phosphate, ammonium nitrate, urea, ammonium chloride, etc.) and the like.

Examples of the liquid carrier include water, alcohols (methanol, ethanol, isopropyl alcohol, butanol, hexanol, benzyl alcohol, ethylene glycol, propylene glycol, phenoxyethanol, etc.), ketones (acetone, methyl ethyl ketone, cyclohexanone, etc.), aromatic hydrocarbons (toluene, xylene, ethylbenzene, dodecylbenzene, phenylxylylethane, methylnaphthalene, etc.), aliphatic hydrocarbons (hexane, cyclohexane, kerosene, light oil, etc.), esters (ethyl acetate, butyl acetate, isopropyl myristate, ethyl oleate, diisopyl adipate, diisobutyl adipate, propylene glycol monomethyl ether acetate, etc.), nitriles (acetonitrile, isobutyronitrile, etc.), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol, etc.), acid amides (N,N-dimethylformamide, N,N-dimethylacetamide, etc.), halogenated hydrocarbons (dichloromethane, trichloroethane, carbon tetrachloride, etc.), sulfoxides (dimethyl sulfoxide, etc.), and propylene carbonate and vegetable oils (soybean oil, cottonseed oil, etc).

Examples of the gaseous carrier include fluorocarbon, butane gas, LPG (liquefied petroleum gas), dimethyl ether, and carbon dioxide.

Examples of the surfactant include nonionic surfactants such as polyoxyethylene alkyl ether, polyoxyethylene alkylaryl ether and polyethylene glycol fatty acid ester, and anionic surfactants such as alkylsulfates, alkylbenzene sulfonates and alkylsulfates.

The other auxiliaries for formulation include such as fixing agents, dispersants, colorants and stabilizers, specifically, for example, casein, gelatin, polysaccharides (starch, arabic gum, cellulose derivatives, alginic acid, etc.), lignin derivatives, bentonite, synthetic water-soluble polymers (polyvinyl alcohol, polyvinyl pyrrolidone, polyacrylic acid, etc.), PAP (isopropyl acid phosphate), BHT (2,6-di-tert-butyl-4-methylphenol) and BHA (mixtures of 2-tert-butyl-4-methoxyphenol and 3-tert-butyl-4-methoxyphenol).

The method for controlling pests of the present invention is carried out by applying an effective amount of the composition of the present invention to a pest or a place where a pest inhabits. In the method for controlling pests of the present invention, the compound of the present invention is usually used in the form of the pest control agent of the present invention.

The place where a pest inhabits includes paddy fields, fields, orchards, non-agricultural lands, houses and the like.

The application can be carried out by the application method similar to the conventional one, as long as the compound of the present invention can be brought into contact with or ingested by a pest.

Examples of the application method include spraying treatment, soil treatment, seed treatment and water culture medium treatment.

When the pest control agent of the present invention is used in pest controlling, the application amount is usually 1 to 10000 g per the amount of the compound of the present invention per 10000 $m^2$. When the pest control agent of the present invention is formulated into an emulsifiable concentrate, a wettable powder, a flowable or the like, the pest control agent is usually diluted with water so as to have a concentration of the active ingredient of 0.01 to 10000 ppm, and dust formulations, granules and the like are usually applied as they are.

These formulations and formulation solutions diluted with water may be directly treated by being sprayed on a pest or a plant such as crops which should be protected from pests, and also may be treated on a soil in order to control a pest that inhabits in the soil of cultivated land.

Also, the resin formulation processed into a sheet or string can be also treated by a method such as winding it around crops, spreading it in the vicinity of crops, or spreading it to the soil around crop roots.

When the pest control agent of the present invention is used in controlling the pest that inhabits in the house, the application amount is usually 0.01 to 1000 mg in an amount of the compound of the present invention per 1 $m^2$ of an area to be treated, in the case of using it on a planar area, and is usually 0.01 to 500 mg in an amount of the compound of the present invention per 1 $m^2$ of a space to be treated, in the case of using it in a space. When the pest control agent

of the present invention is formulated into an emulsifiable concentrate, a wettable powder, a flowable or the like, the pest control agent is usually diluted with water so as to have a concentration of the active ingredient of 0.1 to 1000 ppm and applied, and oil formulations, aerosols, fumigants, poisonous baits and the like are applied as they are.

The compound of the present invention can be used in the farmland where the following "crops" are grown.

Crops; corn, rice, wheat, barley, rye, oat, sorghum, cotton, soybean, peanut, sarrazin, sugar beet, rapeseed, sunflower, sugar cane, tobacco, etc.

Vegetables; Solanaceae vegetables (eggplant, tomato, green pepper, hot pepper, potato, etc.), Cucurbitaceae vegetables (cucumber, pumpkin, zucchini, watermelon, melon, etc.), Cruciferae vegetables (Japanese radish, turnip, horseradish, kohlrabi, Chinese cabbage, cabbage, brown mustard, broccoli, cauliflower, etc.), Compositae vegetables (burdock, garland chrysanthemum, artichoke, lettuce, etc.), Liliaceae vegetables (Welsh onion, onion, garlic, asparagus, etc.), Umbelliferae vegetables (carrot, parsley, celery, parsnip, etc.), Chenopodiaceae vegetables (spinach, Swiss chard, etc.), Labiatae vegetables (Japanese mint, mint, basil, etc.), strawberry, sweat potato, yam, aroid, etc.

Flowers;

Foliage plants;

Fruit trees; pomaceous fruits (apple, common pear, Japanese pear, Chinese quince, quince, etc.), stone fleshy fruits (peach, plum, nectarine, Japanese plum, cherry, apricot, prune, etc.), citrus plants (Satsuma mandarin, orange, lemon, lime, grapefruits, etc.), nuts (chestnut, walnut, hazel nut, almond, pistachio, cashew nut, macadamia nut, etc.), berry fruits (blueberry, cranberry, blackberry, raspberry, etc.), grape, persimmon, olive, loquat, banana, coffee, date, coconut, oil palm, etc.;

Trees other than fruit trees; tea, mulberry, flowering trees and shrubs, street trees (ash tree, birch, dogwood, eucalyptus, ginkgo, lilac, maple tree, oak, poplar, cercis, Chinese sweet gum, plane tree, zelkova, Japanese arborvitae, fir tree, Japanese hemlock, needle juniper, pine, spruce, yew), etc.

The "crops" also contains genetically modified crops.

The pest control agent of the present invention can be used as a mixture with or in combination with other insecticide, miticide, nematicide, fungicide, plant growth regulator, herbicide or synergist. Examples of the active ingredient of said insecticide, miticide, nematicide, fungicide, plant growth regulator, herbicide and synergist include are shown below.

Active Ingredients of Insecticide

(1) Organic Phosphorus Compounds

acephate, Aluminium phosphide, butathiofos, cadusafos, chlorethoxyfos, chlorfenvinphos, chlorpyrifos, chlorpyrifos-methyl, cyanophos: CYAP, diazinon, DCIP (dichlorodiisopropyl ether), dichlofenthion: ECP, dichlorvos: DDVP, dimethoate, dimethylvinphos, disulfoton, EPN, ethion, ethoprophos, etrimfos, fenthion: MPP, fenitrothion: MEP, fosthiazate, formothion, Hydrogen phosphide, isofenphos, isoxathion, malathion, mesulfenfos, methidathion: DMTP, monocrotophos, naled: BRP, oxydeprofos: ESP, parathion, phosalone, phosmet: PMP, pirimiphos-methyl, pyridafenthion, quinalphos, phenthoate: PAP, profenofos, propaphos, prothiofos, pyraclorfos, salithion, sulprofos, tebupirimfos, temephos, tetrachlorvinphos, terbufos, thiometon, trichlorphon: DEP, vamidothion, phorate, and cadusafos.

(2) Carbamate Compounds

alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb: MIPC, metolcarb, methomyl, methiocarb, NAC, oxamyl, pirimicarb, propoxur: PHC, XMC, thiodicarb, xylylcarb, and aldicarb.

(3) Pyrethroid Compounds

acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, profluthrin, dimefluthrin, 2,3,5,6-tetrafluoro-4-(methoxymethyl)benzyl (EZ)-(1RS, 3RS; 1RS, 3SR)-2,2-dimethyl-3-prop-1-enylcyclopropanecarboxylate, 2,3,5,6-tetrafluoro-4-methylbenzyl (EZ)-(1RS, 3RS; 1RS, 3SR)-2,2-dimethyl-3-prop-1-enylcyclopropanecarboxylate, and

2,3,5,6-tetrafluoro-4-(methoxymethyl)benzyl (1RS, 3RS; 1RS, 3SR)-2,2-dimethyl-3-(2-methyl-1-propenyl)cyclopropanecarbox ylate.

(4) Nereistoxin Compounds

cartap, bensultap, thiocyclam, monosultap, andbisultap.

(5) Neonicotinoid Compounds

imidacloprid, nitenpyram, acetamiprid, thiamethoxam, thiacloprid, dinotefuran, and clothianidin.

(6) Benzoyl Urea Compounds

chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron.

(7) Phenylpyrazole-Based Compounds

acetoprole, ethiprole, fipronil, vaniliprole, pyriprole, and pyrafluprole.

(8) Bt Toxins

Living spores derived from Bacillus thuringiensis and produced crystalline toxins and mixtures thereof.

(9) Hydrazine Compounds

chromafenozide, halofenozide, methoxyfenozide, and tebufenozide.

(10) Organic Chlorine Compounds

aldrin, dieldrin, dienochlor, endosulfan, and methoxychlor.

(11) Other Active Ingredients of Insecticide

machine oil and nicotine-sulfate; avermectin-B, bromopropylate, buprofezin, chlorphenapyr, cyantraniliprole, cyromazine, D-D (1,3-Dichloropropene), emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, Arsenic acid, benclothiaz, Calcium cyanamide, Calcium polysulfide, chlordane, DDT, DSP, flufenerim, flonicamid, flurimfen, formetanate, metam-ammonium, metam-sodium, Methyl bromide, Potassium oleate, protrifenbute, spiromesifen, sulfoxaflor, Sulfur, metaflumizone, spirotetramat, pyrifluquinazone, spinetoram, chlorantraniliprole, tralopyril, and cyantraniliprole. Active ingredients of Miticide

acequinocyl, amitraz, benzoximate, bifenaate, bromopropylate, chinomethionat, chlorobenzilate, CPCBS (chlorfenson), clofentezine, cyflumetofen, kelthane (dicofol), etoxazole, fenbutatin oxide, fenothiocarb, fenpyroximate, fluacrypyrim, fluproxyfen, hexythiazox, propargite: BPPS, polynactins, pyridaben, pyrimidifen, tebufenpyrad, tetradifon, spirodiclofen, spiromesifen, spirotetramat, amidoflumet, and cyenopyrafen.

Active ingredients of Nematicide

DCIP, fosthiazate, levamisol hydrochloride (levamisol), methylisothiocyanate, morantel tartarate, and imicyafos.

Active ingredients of Fungicide

Azole fungicidal compounds such as propiconazole, prothioconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, and flutriafol;
Cyclic amine fungicidal compounds such as fenpropimorph, tridemorph, and fenpropidin;
Benzimidazole fungicidal compounds such as carbendezim, benomyl, thiabendazole, and thiophanate-methyl;

procymidone; cyprodinil; pyrimethanil; diethofencarb; thiuram; fluazinam; mancozeb; iprodione; vinclozolin; chlorothalonil; captan; mepanipyrim; fenpiclonil; fludioxonil; dichlofluanid; folpet; kresoxim-methyl; azoxystrobin; trifloxystrobin; fluoxastrobin; picoxystrobin; pyraclostrobin; dimoxystrobin; pyribencarb; spiroxamine; quinoxyfen; fenhexamid; famoxadone; fenamidone; zoxamide; ethaboxam; amisulbrom; iprovalicarb; benthiavalicarb; cyazofamid; mandipropamid; boscalid; penthiopyrad; metrafenone; fluopiran; bixafen; cyflufenamid; proquinazid; isotianil, and tiadinil.

Active ingredients of Herbicide

(1) Phenoxy fatty acid herbicidal compounds
2,4-PA, MCP, MCPB, phenothiol, mecoprop, fluroxypyr, triclopyr, clorneprop, and naproanilide.
(2) Benzoate herbicidal compounds
2,3,6-TBA, dicamba, clopyralid, picloram, aminopyralid, quinclorac, and quinmerac.
(3) Urea herbicidal compounds
diuron, linuron, chlortoluron, isoproturon, fluometuron, isouron, tebuthiuron, methabenzthiazuron, cumyluron, daimuron, and methyl-daimuron.
(4) Triazine herbicidal compounds
atrazine, ametoryn, cyanazine, simazine, propazine, simetryn, dimethametryn, prometryn, metribuzin, triaziflam, and indaziflam.
(5) Bipyridinium herbicidal compounds
paraquat, and diquat.
(6) Hydroxybenzonitrile herbicidal compounds
bromoxynil, and ioxynil.
(7) Dinitroaniline herbicidal compounds
pendimethalin, prodiamine, and trifluralin.
(8) Organophosphorus herbicidal compounds
amiprofos-methyl, butamifos, bensulide, piperophos, anilofos, glyphosate, glufosinate, glufosinate-P, and bialaphos.
(9) Carbamate herbicidal compounds
di-allate, tri-allate, EPTC, butylate, benthiocarb, esprocarb, molinate, dimepiperate, swep, chlorpropham, phenmedipham, phenisopham, pyributicarb, and asulam.
(10) Acid amide herbicidal compounds
propanil, propyzamide, brornobutide, and etobenzanid.
(11) Chloroacetanilide herbicidal compounds
acetochlor, alachlor, butachlor, dimethenamid, propachlor, metazachlor, metolachlor, pretilachlor, thenylchlor, and pethoxamid.
(12) Diphenyl ether herbicidal compounds
acifluorfen-sodium, bifenox, oxyfluorfen, lactofen, fomesafen, chlomethoxynil, and aclonifen.
(13) Cyclic imide herbicidal compounds
oxadiazon, cinidon-ethyl, carfentrazone-ethyl, surfentrazone, flumiclorac-pentyl, flumioxazin, pyraflufen-ethyl, oxadiargyl, pentoxazone, fluthiacet-methyl, butafenacil, benzfendizone, bencarbazone, and saflufenacil.
(14) Pyrazole herbicidal compounds
benzofenap, pyrazolate, pyrazoxyfen, topramezone, and pyrasulfotole.
(15) Triketone herbicidal compounds
isoxaflutole, benzobicyclon, sulcotrione, mesotrione, tembotrione, and tefuryltrione.
(16) Aryloxyphenoxypropionate herbicidal compounds
clodinafop-propargyl, cyhalofop-butyl, diclofop-methyl, fenoxaprop-ethyl, fluazifop-butyl, haloxyfop-methyl, and quizalofop-ethyl, metamifop.
(17) Trione oxime herbicidal compounds
alloxydim-sodium, sethoxydim, butroxydim, clethodim, cloproxydim, cycloxydim, tepraloxydim, tralkoxydim, and profoxydim.
(18) Sulfonyl urea herbicidal compounds
chlorsulfuron, sulfometuron-methyl, metsulfuron-methyl, chlorimuron-ethyl, tribenuron-methyl, triasulfuron, bensulfuron-methyl, thifensulfuron-methyl, pyrazosulfuron-ethyl, primisulfuron-methyl, nicosulfuron, amidosulfuron, cinosulfuron, imazosulfuron, rimsulfuron, halosulfuron-methyl, prosulfuron, ethametsulfuron-methyl, triflusulfuron-methyl, flazasulfuron, cyclosulfamuron, flupyrsulfuron, sulfosulfuron, azimsulfuron, ethoxysulfuron, oxasulfuron, iodosulfuron-methyl-sodium, foramsulfuron, mesosulfuron-methyl, trifloxysulfuron, tritosulfuron, orthosulfamuron, flucetosulfuron, and propyrisulfuron.

(19) Imidazolinone herbicidal compounds

imazamethabenz-methyl, imazamethapyr, imazamox, imazapyr, imazaquin, and imazethapyr.

(20) Sulfonamide herbicidal compounds

flumetsulam, metosulam, diclosulam, florasulam, cloransulam-methyl, penoxsulam, and pyroxsulam.

(21) Pyrimidinyloxybenzoate herbicidal compounds

pyrithiobac-sodium, bispyribac-sodium, pyriminobac-methyl, pyribenzoxim, pyriftalid, and pyrimisulfan.

(22) Other herbicidal compounds

bentazon, bromacil, terbacil, chlorthiamid, isoxaben, dinoseb, amitrole, cinmethylin, tridiphane, dalapon, diflufenzopyr-sodium, dithiopyr, thiazopyr, flucarbazone-sodium, propoxycarbazone-sodium, mefenacet, flufenacet, fentrazamide, cafenstrole, indanofan, oxaziclomefone, benfuresate, ACN, pyridate, chloridazon, norflurazon, flurtamone, diflufenican, picolinafen, beflubutamid, clomazone, amicarbazone, pinoxaden, pyraclonil, pyroxasulfone, thiencarbazone-methyl, aminocyclopyrachlor, ipfencarbazone, and methiozolin.

Active ingredients of Synergist

piperonyl butoxide, sesamex, sulfoxide, N-(2-ethylhexyl)-8,9,10-trinorborn-5-ene-2,3-dicarboximide (MGK 264), N-declyimidazole, WARF-antiresistant, TBPT, TPP, IBP, PSCP, methyl iodide ($CH_3I$), t-phenylbutenone, diethylmaleate, DMC, FDMC, ETP, and ETN.

EXAMPLES

Hereinbelow, the present invention will be further illustrated by production examples, formulation examples, test examples, and the like. However, the present invention is not limited to these examples.

First, the production examples of the compounds of the present invention are shown below.

Production Example 1

Tetrahydrofuran-3-ylmethylamine hydrochloride (1.71 g, 12.4 mmol) and triethylamine (1.89 g, 18.7 mmol) were added to tetrahydrofuran (10 mL). To the mixture solution was added (E)-2-octenoic acid chloride (1.00 g, 6.22 mmol) under ice cooling, then the mixture was stirred at room temperature for 4 hours. Thereafter, water was added to the reaction mixture, and the mixture was extracted twice with ethyl acetate. The organic layer was washed with an aqueous solution of sodium bicarbonate, and dried over anhydrous sodium sulfate, then filtered, and the filtrate was concentrated under reduced pressure. The residue was applied to a silica gel column chromatography, to obtain 1.25 g of (E)-N-(tetrahydrofuran-3-ylmethyl)-2-octenamide (hereinafter, described as Compound (1) of the present invention) represented by the following formula.

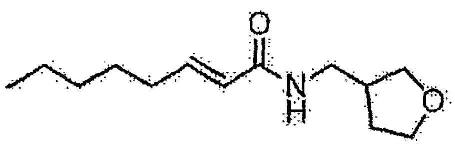 (1)

Compound (1) of the present invention

$^1$H-NMR ($CDCl_3$, TMS) $\delta$ (ppm): 0.88 (t, 3H), 1.22-1.38 (m, 4H), 1.40-1.48 (m, 2H), 1.58-1.68 (m, 1H), 2.00-2.09 (m, 1H), 2.17 (dq, 2H), 2.46-2.57 (m, 1H), 3.30-3.38 (m, 2H), 3.55 (dd, 1H), 3.74 (dd, 1H), 3.82 (dd, 1H), 3.89 (td, 1H), 5.65 (br s, 1H), 5.75 (dt, 1H), 6.84 (dt, 1H)

Production Example 2

(E/Z)-2-Nonenoic acid (547 mg, 3.50 mmol), triethylamine (708 mg, 7.00 mmol) and tetrahydrofuran-3-ylmethylamine hydrochloride (963mg, 7.00 mmol) were added to tetrahydrofuran (15 mL). To the mixture solution was added 1-ethyl-3-(3-dimethylaminopropyl)carbodiimide hydrochloride (1.33 g, 7.00 mmol) at room temperature, then the mixture

was stirred at room temperature for 4 hours. Thereafter, water was added to the reaction mixture, and the mixture was extracted twice with ethyl acetate. The organic layer was washed with a saturated salt solution, and dried over anhydrous sodium sulfate, then filtered, and the filtrate was concentrated under reduced pressure. The residue was applied to a silica gel column chromatography, to obtain 0.48 g of (E)-N-(tetrahydrofuran-3-ylmethyl)-2-nonenamide (hereinafter, described as Compound (2) of the present invention) represented by the following formula.

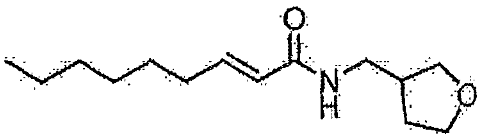 (2)

Compound (2) of the present invention

$^1$H-NMR ($CDCl_3$, TMS) δ (ppm) : 0.88 (t, 3H), 1.24-1.35 (m, 6H), 1.39-1.48 (m, 2H), 1.59-1.68 (m, 1H), 2.00-2.09 (m, 1H), 2.17 (dq, 2H), 2.48-2.56 (m, 1H), 3.30-3.38 (m, 2H), 3.55 (dd, 1H), 3.74 (dd, 1H), 3.81 (dd, 1H), 3.89 (td, 1H), 5.63 (br s, 1H), 5.75 (dt, 1H), 6.84 (dt, 1H)

Production Example 3

The reaction was carried out in the same manner, to obtain 0.13 g of (Z)-N-(tetrahydrofuran-3-ylmethyl)-2-nonenamide (hereinafter, described as Compound (3) of the present invention) represented by the following formula.

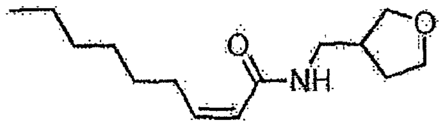 (3)

Compound (3) of the present invention

$^1$H-NMR ($CDCl_3$, TMS) δ (ppm): 0.88 (t, 3H), 1.24-1.37 (m, 5H), 1.37-1.47 (m, 2H), 1.61-1.67 (m, 2H), 2.00-2.09 (m, 1H), 2.46-2.55 (m, 1H), 2.64 (dq, 2H), 3.32 (dd, 2H), 3.54 (dd, 1H), 3.74 (dd, 1H), 3.82 (dd, 1H), 3.89 (td, 1H), 5.60 (br s, 1H), 5.66 (dt, 1H), 6.00 (dt, 1H)

Production Example 4

The reaction was carried out in the same manner using 0. 51 g of (E)-2-decenoic acid in place of (E/Z)-2-nonenoic acid in Production Example 2, to obtain 0.41 g of (E)-N-(tetrahydrofuran-3-ylmethyl)-2-decenamide (hereinafter, described as Compound (4) of the present invention) represented by the following formula.

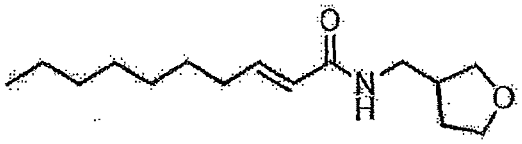 (4)

Compound (4) of the present invention

$^1$H-NMR ($CDCl_3$, TMS) δ (ppm): 0.88 (t, 3H), 1.22-1.35 (m, 8H), 1.55-1.68 (m, 2H), 2.00-2.20 (m, 2H), 2.17 (q, 2H), 2.47-2.55 (m, 1H), 3.35 (d, 2H), 3.55 (dd, 1H), 3.74 (dq, 1H), 3.82 (dt, 1H), 3.89 (dq, 1H), 5.58 (br s, 1H), 5.75 (dt, 1H), 6.84 (dt, 1H)

Production Example 5

(E)-2-Undecenoic acid (369 mg, 2.00 mmol), triethylamine (304 mg, 3.00 mmol) and tetrahydrofuran-3-ylmethylamine hydrochloride (413mg, 3.00 mmol) were added to tetrahydrofuran (4 mL). To the mixture solution was added 1-ethyl-3-(3-dimethylaminopropyl)carbodiimide hydrochloride (458 mg, 2.40 mmol) at room temperature, then the mixture was stirred at room temperature for 4 hours. Thereafter, water was added to the reaction mixture, and the mixture was

extracted twice with ethyl acetate. The organic layer was washed with a saturated salt solution, and dried over anhydrous sodium sulfate, then filtered, and the filtrate was concentrated under reduced pressure. The residue was applied to a silica gel column chromatography, to obtain 0.37 g of (E)-N-(tetrahydrofuran-3-ylmethyl)-2-undecenamide (hereinafter, described as Compound (5) of the present invention) represented by the following formula.

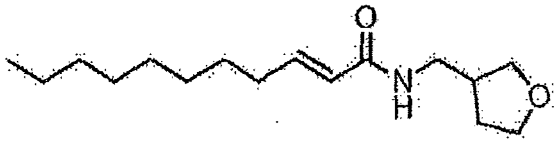

(5)

Compound (5) of the present invention

$^1$H-NMR ($CDCl_3$, TMS) $\delta$ (ppm): 0.88 (t, 3H), 1.19-1.38 (m, 10H), 1.38-1.50 (m, 2H), 1.62-1.68 (m, 1H), 2.01-2.10 (m, 1H), 2.16 (q, 2H), 2.46-2.56 (m, 1H), 3.22-3.38 (m, 2H), 3.55 (dd, 1H), 3.72 (q, 1H), 3.83 (dt, 2H), 5.92 (d, 1H), 6.81 (dt, 1H), 7.43 (br s, 1H)

Production Example 6

The reaction was carried out in the same manner using 0. 85 g of (E)-3-methyl-2-nonenoic acid chloride in place of (E)-2-octenoic acid chloride in Production Example 1, to obtain 0.34 g of

(E)-N-(tetrahydrofuran-3-ylmethyl)-3-methyl-2-nonenamide (hereinafter, described as Compound (6) of the present invention) represented by the following formula.

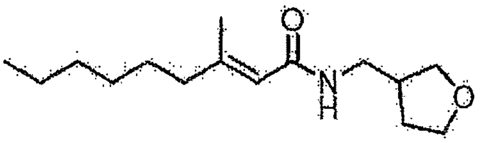

(6)

Compound (6) of the present invention

$^1$H-NMR ($CDCl_3$, TMS) $\delta$ (ppm): 0.89 (t, 3H), 1.23-1.35 (m, 6H), 1.35-1.50 (m, 2H), 1.59-1.67 (m, 1H), 2.00-2.09 (m, 3H), 2.14 (s, 3H), 2.45-2.56 (m, 1H), 3.25-3.37 (m, 2H), 3.54 (dd, 1H), 3.74 (q, 1H), 3.82 (dd, 1H), 3.88 (td, 1H), 5.50 (br s, 1H), 5.52 (s, 1H)

Production Example 7

(Z)-2-Fluoro-2-nonoic acid (0.97 g, 5.5 mmol) and tetrahydrofuran-3-ylmethylamine hydrochloride (0.92 g, 6.7 mmol) were added to N,N-dimethylformamide (15 mL). To the mixture solution was added triethylamine (1.45 g, 14.3 mmol) under ice cooling, then, benzotriazol-1-yloxy-tris(dimethylamino)phosphonium salt (3.20 g, 7.2 mmol) was added. The mixture was stirred under ice cooling for 1 hour, then stirred at room temperature for 12 hours, and thereafter, the reaction solution was added to a mixture of 20 mL of 5% of hydrochloric acid and 50 mL of ice water, and the mixture was extracted twice with 100 mL of ethyl acetate. The ethyl acetate layers were combined, and washed with 50 mL of a saturated sodium bicarbonate solution, followed by 50 mL of a saturated salt solution. Thereafter, the organic layer was dried over magnesium sulfate and was concentrated under reduced pressure conditions, and the residue was applied to a silica gel column chromatography, to obtain 0.73 g of (Z)-N-(tetrahydrofuran-3-ylmethyl)-2-fluoro-2-nonenamide (hereinafter, described as Compound (7) of the present invention) represented by the following formula.

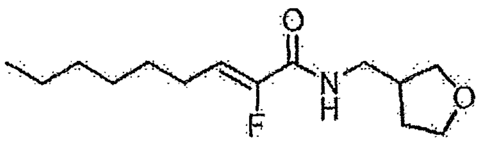

(7)

Compound (7) of the present invention

$^1$H-NMR ($CDCl_3$, TMS) $\delta$ (ppm) : 0.88 (t, 3H), 1.24-1.36 (m, 6H), 1.40-1.47 (m, 2H), 1.61-1.68 (m, 1H), 2.02-2.10

(m, 1H), 2.20 (ddd, 2H), 2.48-2.58 (m, 1H), 3.32-3.42 (m, 2H), 3.56 (dd, 1H), 3.75 (dd, 1H), 3.83 (dd, 1H), 3.90 (td, 1H), 6.10 (dt, 1H), 6.35 (br s, 1H)

Production Example 8

The reaction was carried out in the same manner using 0.20 g of (E/Z)-2-fluoro-3-methyl-2-nonenoic acid in place of (E)-2-undecenoic acid in Production Example 5, to obtain 0.13 g of (Z)-N-(tetrahydrofuran-3-ylmethyl)-2-fluoro-3-methyl-2-none namide (hereinafter, described as Compound (8) of the present invention) represented by the following formula.

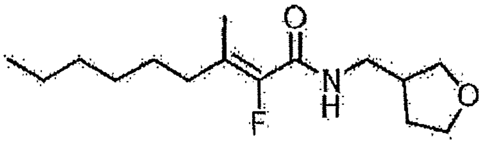 (8)

Compound (8) of the present invention

$^1$H-NMR ($CDCl_3$, TMS) δ (ppm): 0.89 (t, 3H), 1.30 (t, 6H), 1.38-1.49 (m, 2H), 1.58-1.69 (m, 1H), 2.01-2.10 (m, 1H), 2.15-2.21 (m, 2H), 2.16 (d, 3H), 2.47-2.57 (m, 1H), 3.28-3.37 (m,2H), 3.55 (dd, 1H), 3.75 (dd, 1H), 3.83 (dd, 1H), 3.89 (td, 1H), 6.39 (br s, 1H)

Production Example 9

The reaction was carried out in the same manner as in Production Example 8, to obtain 0.18 g of (E)-N-(tetrahydrofuran-3-ylmethyl)-2-fluoro-3-methyl-2-none namide (hereinafter, described as Compound (9) of the present invention) represented by the following formula.

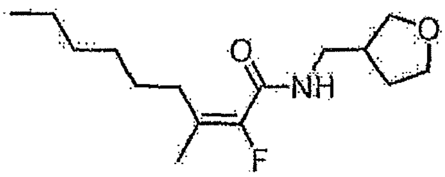 (9)

Compound (9) of the present invention

$^1$H-NMR ($CDCl_3$, TMS) δ (ppm): 0.88 (t, 3H), 1.26-1.36 (m, 6H), 1.45 (dd, 2H), 1.59-1.67 (m, 1H), 1.81 (d, 3H), 2.01-2.09 (m, 1H), 2.48-2.56 (m, 1H), 2.64 (t, 2H), 3.31-3.36 (m, 2H), 3.54 (dd, 1H), 3. 75 (dd, 1H), 3. 82 (dd, 1H), 3. 89 (td, 1H), 6. 37 (br s, 1H)

Production Example 10

The reaction was carried out in the same manner using 0. 09 g of (E)-octa-2,7-dienoic acid in place of (E)-2-undecenoic acid in Production Example 5, to obtain 0.11 g of (E)-N-(tetrahydrofuran-3-ylmethyl)-octa-2,7-dienamide (hereinafter, described as Compound (10) of the present invention) represented by the following formula.

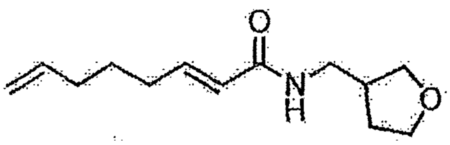 (10)

Compound (10) of the present invention

$^1$H-NMR ($CDCl_3$, TMS) δ (ppm) : 1.52-1.80 (m, 3H), 1.61-1.68 (m, 1H), 1.75 (m, 1H), 2.01-2.23 (m, 4H), 2.48-2.55 (m, 1H), 3.35 (td, 1H), 3.51-3.57 (m, 1H), 3.74 (dd, 1H), 3.79-3.84 (m, 1H), 3.89 (td, 1H), 4.94-5.06 (m, 2H), 5.64 (brs, 1H), 5.74-5.84 (m, 2H), 6.85 (dt, 1H)

Production Example 11

The reaction was carried out in the same manner using 0.30 g of (E)-nona-2,8-dienoic acid in place of (E)-2-undecenoic acid in Production Example 5, to obtain 0.38 g of (E)-N-(tetrahydrofuran-3-ylmethyl)-nona-2,8-dienamide (hereinafter, described as Compound (11) of the present invention) represented by the following formula.

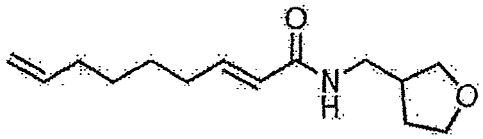 (11)

Compound (11) of the present invention

$^{1}H$-NMR ($CDCl_3$, TMS) $\delta$ (ppm) : 1.38-1.51 (m, 3H), 1.59-1.67 (m, 2H), 2.01-2.10 (m, 3H), 2.19 (q, 2H), 2.48-2.55 (m, 1H), 3.30-3.38 (m, 2H), 3.55 (dd, 1H), 3.74 (dd, 1H), 3.81 (dd, 1H), 3.89 (td, 1H), 4.94-5.03 (m, 2H), 5.61 (br s, 1H), 5.73-5.84 (m, 2H), 6.84 (dt, 1H)

Production Example 12

The reaction was carried out in the same manner using 0.34 g of (E)-deca-2,9-dienoic acid in place of (E)-2-undecenoic acid in Production Example 5, to obtain 0.26 g of (E)-N-(tetrahydrofuran-3-ylmethyl)-deca-2,9-dienamide (hereinafter, described as Compound (12) of the present invention) represented by the following formula.

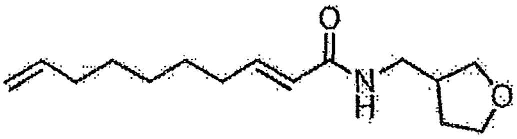 (12)

Compound (12) of the present invention

$^{1}H$-NMR ($CDCl_3$, TMS) $\delta$ (ppm): 1.30-1.50 (m, 5H), 1.59-1.67 (m, 2H), 2.00-2.09 (m, 3H), 2.15-2.21 (m, 2H), 2.48-2.55 (m, 1H), 3.29-3.40 (m, 2H), 3.55 (dd, 1H), 3.74 (dd, 1H), 3.82 (dd, 1H), 3.89 (td, 1H), 4.92-5.02 (m, 2H), 5. 59 (br s, 1H), 5.73-5.85 (m, 2H), 6.84 (dt, 1H)

Production Example 13

The reaction was carried out in the same manner using 0.70 g of (2E, 6Z)-nona-2,6-dienoic acid in place of (Z)-2-fluoro-2-nonenoic acid in Production Example 7 , to obtain 0.68 g of (2E, 6Z)-N-(tetrahydrofuran-3-ylmethyl)-nona-2,6-dienamide (hereinafter, described as Compound (13) of the present invention) represented by the following formula.

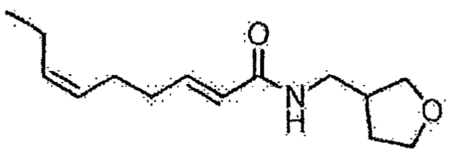 (13)

Compound (13) of the present invention

$^{1}H$-NMR ($CDCl_3$, TMS) $\delta$ (ppm): 0.96 (t, 3H), 1.56-1.68 (m, 1H), 1.99-2.09 (m, 3H), 2.16-2.27 (m, 4H), 2.47-2.56 (m, 1H), 3.29-3.40 (m, 2H), 3.55 (dd, 1H), 3.74 (dd, 1H), 3.81 (dd, 1H), 3.89 (td, 1H), 5.28-5.34 (m, 1H), 5.38-5.45 (m, H), 5.59 (br s, 1H), 5.77 (d, 1H), 6.84 (dt, 1H)

Production Example 14

The reaction was carried out in the same manner using 0.84 g of (E)-3,7-dimethylocta-2,6-dienoic acid in place of (E/Z)-2-nonenoic acid in Production Example 2, to obtain 0.08 g of (E)-N-(tetrahydrofuran-3-ylmethyl)-3,7-dimethyl-

octa-2,6-die namide (hereinafter, described as Compound (14) of the present invention) represented by the following formula.

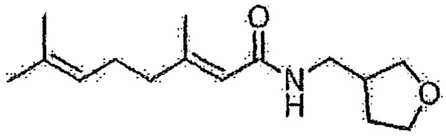
(14)

Compound (14) of the present invention

$^1$H-NMR ($CDCl_3$, TMS) δ (ppm): 1.50-1.67 (m, 1H), 1.56 (s, 3H), 1.61 (s, 3H), 1.69 (s, 3H), 2.06-2.11 (m, 5H), 2.46-2.55 (m, 1H), 3.25-3.38 (m, 2H), 3.54 (dd, 1H), 3.74 (q, 1H), 3.82 (t, 1H), 3.85-3.92 (m, 1H), 5.08 (t, 1H), 5.49 (br s, 1H), 5.53 (s, 1H)

Production Example 15

The reaction was carried out in the same manner using 0.25 g of (E)-oct-2-en-7-ynoic acid in place of (E)-2-undecenoic acid in Production Example 5, to obtain 0.31 g of (E)-N-(tetrahydrofuran-3-ylmethyl)-oct-2-en-7-ynamide (hereinafter, described as Compound (15) of the present invention) represented by the following formula.

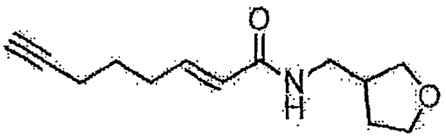
(15)

Compound (15) of the present invention

$^1$H-NMR ($CDCl_3$, TMS) δ (ppm): 1.59-1.72 (m, 3H), 1.99-2.08 (m, 2H), 2.22 (td, 2H), 2.31 (q, 2H), 2.50 (td, 1H), 3.32 (tt, 2H), 3.55 (dd, 1H), 3.73 (q, 1H), 3.81 (dd, 1H), 3.85-3.90 (m, 1H), 5.87 (dt, 1H), 6.51(br s, 1H), 6.77-6.84 (m, 1H) Production Example 16

The reaction was carried out in the same manner using 0.46 g of (E)-non-2-en-8-ynoic acid in place of (E)-2-undecenoic acid in Production Example 5, to obtain 0.47 g of (E)-N-(tetrahydrofuran-3-ylmethyl)-non-2-en-8-ynamide (hereinafter, described as Compound (16) of the present invention) represented by the following formula.

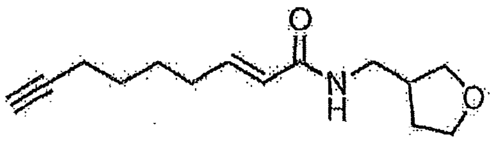
(16)

Compound (16) of the present invention

$^1$H-NMR ($CDCl_3$, TMS) δ (ppm): 1.52-1.68 (m, 5H), 1.97 (t, 1H), 1.99-2.08 (m, 1H), 2.17-2.25 (m, 4H), 2.51 (s, 1H), 3.25-3.40 (m, 2H), 3.53-3.57 (m, 1H), 3.73 (q, 1H), 3.79-3.84 (m, 1H), 3.85-3.91 (m, 1H), 5.83 (dt, 1H), 6.31 (br s, 1H), 6.78-6.87 (m, 1H)

Production Example 17

The reaction was carried out in the same manner using 0.28 g of (E)-3-(trans-2'-ethylcyclopropyl)-2-propenoic acid in place of (E)-2-undecenoic acid in Production Example 5, to obtain 0.24 g of (E)-N-(tetrahydrofuran-3-ylmethyl)-3-(trans-2'-ethylcyclopr opyl)-2-propenamide (hereinafter, described as Compound (17) of the present invention) represented by the following formula.

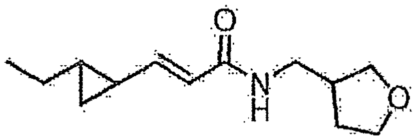
(17)

Compound (17) of the present invention

$^1$H-NMR ($CDCl_3$, TMS) δ (ppm): 0.68-0.79 (m, 2H), 0.96 (t, 4H), 1.23-1.36 (m, 3H), 1.63 (td, 1H), 1.99-2.08 (m, 1H), 2.46-2.55 (m, 1H), 3.27-3.39 (m, 2H), 3.54 (dd, 1H), 3.73 (dd, 1H), 3.81 (dd, 1H), 3.88 (td, 1H), 5.62 (br s, 1H), 5.79 (d, 1H), 6.38 (dd, 1H)

Production Example 18

The reaction was carried out in the same manner using 0.46 g of (E)-3-(trans-2'-propylcyclopropyl)-2-propenoic acid in place of (E)-2-undecenoic acid in Production Example 5, to obtain 0.30 g of (E)-N-(tetrahydrofuran-3-ylmethyl)-3-(trans-2'-propylcyclop ropyl)-2-propenamide (hereinafter, described as Compound (18) of the present invention) represented by the following formula.

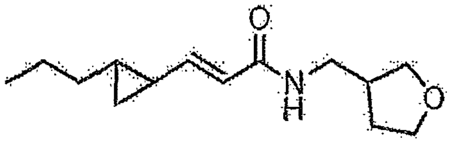
(18)

Compound (18) of the present invention

$^1$H-NMR ($CDCl_3$, TMS) δ (ppm): 0.66-0.73 (m, 1H), 0.74-0.79 (m, 1H), 0.91 (t, 3H), 0.94-1.01 (m, 1H), 1.19-1.33 (m, 3H), 1.35-1.45 (m, 2H), 1.58-1.67 (m, 1H), 1.98-2.07 (m, 1H), 2.45-2.55 (m, 1H), 3.25-3.38 (m, 2H), 3.54 (dd, 1H), 3.73 (dd, 1H), 3.81 (dd, 1H), 3.87 (td, 1H), 5.82 (d, 1H), 6.06 (br s, 1H), 6.37 (dd, 1H)

Production Example 19

The reaction was carried out in the same manner using 0.14 g of (E)-3-(trans-2'-butylcyclopropyl)-2-propenoic acid in place of (E)-2-undecenoic acid in Production Example 5, to obtain 0.09 g of (E)-N-(tetrahydrofuran-3-ylmethyl)-3-(trans-2'-butylcyclopr opyl)-2-propenamide (hereinafter, described as Compound (19) of the present invention) represented by the following formula. Compound (19) of the present invention

$^1$H-NMR ($CDCl_3$, TMS) δ (ppm) : 0.67-0.73 (m, 1H), 0.75-0.80 (m, 1H), 0.88 (t, 3H), 0.93-1.00 (m, 1H), 1.22-1.37 (m, 5H), 1.58-1.67 (m, 3H), 2.00-2.08 (m, 1H), 2.47-2.54 (m, 1H), 3.33 (ddd, 2H), 3.55 (dd, 1H), 3.73 (dd, 1H), 3.81 (dd, 1H), 3.89 (td, 1H), 5.53 (br s, 1H), 5.78 (d, 1H), 6.38 (dd, 1H)

Production Example 20

The reaction was carried out in the same manner using 0.17 g of (E)-5-(trans-2'-ethylcyclopropyl)-2-propenoic acid in place of (E)-2-undecenoic acid in Production Example 5, to obtain 0.17 g of (E)-N-(tetrahydrofuran-3-ylmethyl)-5-(trans-2'-ethylcyclopr opyl)-2-pentenamide (hereinafter, described as Compound (20) of the present invention) represented by the following formula.

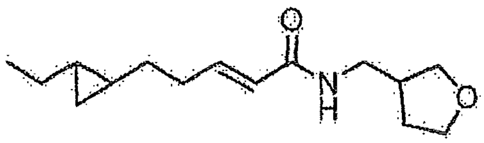
(20)

Compound (20) of the present invention

$^1$H-NMR ($CDCl_3$, TMS) δ (ppm): 0.15-0.22 (m, 1H), 0.36-0.45 (m, 1H), 0.93 (t, 3H), 1.12-1.43 (m, 3H), 1.63 (td, 1H), 1.98-2.08 (m, 2H), 2.21-2.29 (m, 2H), 2.45-2.56 (m, 1H), 3.23-3.40 (m, 2H), 3.55 (dd, 1H), 3.73 (dd, 2H), 3.81 (dd,

2H), 3.88 (td, 1H), 5.79 (dt, 1H), 6.05 (br s, 1H), 6.80-6.90 (m, 1H)

Production Example 21

Tetrahydrofuran-2-ylmethylamine (1.18 g, 8.58 mmol) and triethylamine (868 mg, 8.58 mmol) were added to tetrahydrofuran (10 mL). To the mixture solution was added (E/Z)-2-nonenoic acid chloride (1.00 g, 5.73 mmol) under ice cooling, then the mixture was stirred at room temperature for 4 hours. Thereafter, water was added to the reaction mixture, and the mixture was extracted twice with ethyl acetate. The organic layer was washed with an aqueous solution of sodium bicarbonate, and dried over anhydrous sodium sulfate, then filtered, and the filtrate was concentrated under reduced pressure. The residue was applied to a silica gel column chromatography, to obtain 0.73 g of (E)-N-(tetrahydrofuran-2-ylmethyl)-2-nonenamide (hereinafter, described as Compound (21) of the present invention) represented by the following formula.

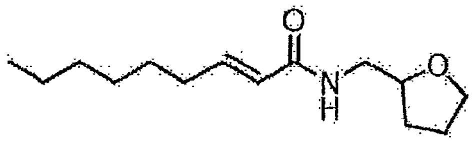
(21)

Compound (21) of the present invention

$^1$H-NMR ($CDCl_3$, TMS) $\delta$ (ppm): 0.88 (t, 3H), 1.23-1.35 (m, 6H), 1.40-1.48 (m, 2H), 1.52-1.64 (m, 1H), 1.86-1.94 (m, 2H), 1.95-2.01 (m, 1H), 2.17 (q, 2H), 3.19 (ddd, 1H), 3.66 (ddd, 1H), 3.73-3.78 (m, 1H), 3.83-3.89 (m, 1H), 3.95-4.02 (m, 1H), 5.76 (dt, 1H), 5.79 (br s, 1H), 6.87-6.80 (m, 1H)

Production Example 22

The reaction was carried out in the same manner as in Production Example 21 to obtain 0.17 g of (Z)-N-(tetrahydrofuran-2-ylmethyl)-2-nonenamide (hereinafter, described as Compound (22) of the present invention) represented by the following formula.

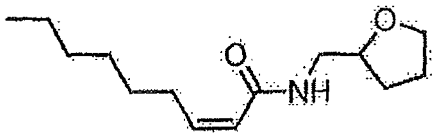
(22)

Compound (22) of the present invention

$^1$H-NMR ($CDCl_3$, TMS) $\delta$ (ppm): 0.87 (t, 3H), 1.20-1.45 (m, 8H), 1.52-1.60 (m, 1H), 2.03-1.87 (m, 3H), 2.64 (dq, 2H), 3.14-3.21 (m, 1H), 3.63 (ddd, 1H), 3.72-3.78 (m, 1H), 3.83-3.89 (m, 1H), 3.98 (dq, 1H), 5.68 (dt, 1H), 5.83 (br s, 1H), 5.99 (dt, 1H)

Production Example 23

(E)-2-nonenoic acid (156 mg, 1.00 mmol) and 3-methyltetrahydrofuran-3-ylmethylamine (173 mg, 1.50 mmol) were added to tetrahydrofuran (2 mL). To the mixture solution was added 229 mg of 1-ethyl-3-(3-dimethylaminopropyl)carbodiimide hydrochloride at room temperature, then the mixture was stirred at room temperature for 4 hours. Thereafter, water was added to the reaction mixture, and the mixture was extracted twice with ethyl acetate. The organic layer was washed with a saturated saline solution, and dried over anhydrous sodium sulfate, then filtered, and the filtrate was concentrated under reduced pressure. The residue was applied to a silica gel column chromatography, to obtain 0.29 g of (E)-N-(3-methyltetrahydrofuran-3-ylmethyl)-2-nonenamide (hereinafter, described as Compound (23) of the present invention) represented by the following formula.

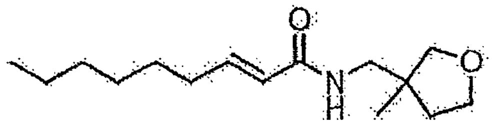
(23)

Compound (23) of the present invention

$^1$H-NMR ($CDCl_3$, TMS) $\delta$ (ppm): 0.88 (t, 3H), 1.12 (s, 3H), 1.24-1.35 (m, 5H), 1.41-1.48 (m, 2H), 1.63-1.70 (m, 2H), 1.82-1.90 (m, 1H), 2.18 (ddd, 2H), 3.30-3.42 (m, 3H), 3.66 (d, 1H), 3.84 (td, 1H), 3.93 (td, 1H), 5.70 (br s, 1H), 5.77 (dt, 1H), 6.85 (dt, 1H)

Production Example 24

Tetrahydropyran-4-ylrnethylamine (484 mg, 4.20 mmol) and triethylamine (455 mg, 4.50 mmol were added to tetrahydrofuran (6 mL). To the mixture solution was added (E)-2-octenoic acid chloride (482 mg, 3.00 mmol) under ice cooling, then the mixture was stirred at room temperature for 4 hours. Thereafter, water was added to the reaction mixture, and the mixture was extracted twice with ethyl acetate. The organic layer was washed with an aqueous solution of sodium bicarbonate, and dried over anhydrous sodium sulfate, then filtered, and the filtrate was concentrated under reduced pressure. The residue was applied to a silica gel column chromatography, to obtain 0.51 g of (E)-N-(tetrahydropyran-4-ylmethyl)-2-octenamide (hereinafter, described as Compound (24) of the present invention) represented by the following formula.

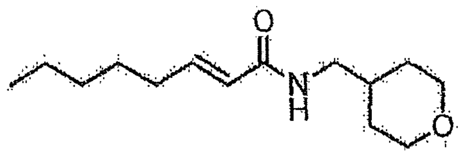

(24)

Compound (24) of the present invention

$^1$H-NMR ($CDCl_3$, TMS) $\delta$ (ppm): 0.89 (t, 3H), 1.33 (ddd, 6H), 1.41-1.48 (m, 2H), 1.59 (dd, 2H), 1.73-1.87 (m, 1H), 2.17 (q, 2H), 3.23 (t, 2H), 3.36 (td, 2H), 3.97 (dd, 2H), 5.48 (br s, 1H), 5.75 (d, 1H), 6.81-6.88 (m, 1H)

Production Example 25

(E)-2-Nonenoic acid (1.00 g, 6.40 mmol) and tetrahydropyran-4-ylmethylamine (1.11 g, 9.60 mmol) were added to tetrahydrofuran (15 mL). To the mixture solution was added 1-ethyl-3-(3-dimethylaminopropyl)carbodiimide hydrochloride (1.83 g, 9.60 mmol) at room temperature, then the mixture was stirred at room temperature for 4 hours. Thereafter, water was added to the reaction mixture, and the mixture was extracted twice with ethyl acetate. The organic layer was washed with a saturated saline solution, and dried over anhydrous sodium sulfate, then filtered, and the filtrate was concentrated under reduced pressure. The residue was applied to a silica gel column chromatography, to obtain 0.53 g of (E)-N-(tetrahydropyran-4-ylmethyl)-2-nonenamide (hereinafter, described as Compound (25) of the present invention) represented by the following formula.

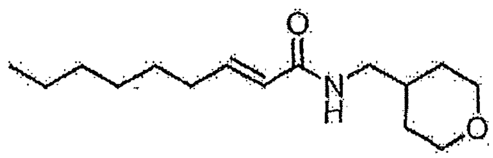

(25)

Compound (25) of the present invention

$^1$H-NMR ($CDCl_3$, TMS) $\delta$ (ppm): 0.88 (t, 3H), 1.30-1.36 (m, 9H), 1.41-1.44 (m, 2H), 1.60-1.67 (m, 1H), 1.77-1.83 (m, 1H), 2.14-2.21 (m, 2H), 3.22 (t, 2H), 3.36 (td, 2H), 3.97 (dd, 2H), 5.51 (br s, 1H), 5.75 (d, 1H), 6.81-6.88 (m, 1H)

Production Example 26

The reaction was carried out in the same manner using 0.51 g of (E)-2-decenoic acid in place of (E)-2-nonenoic acid in Production Example 25, to obtain 0.51 g of (E)-N-(tetrahydropyran-4-ylmethyl)-2-decenamide (hereinafter, described as Compound (26) of the present invention) represented by the following formula.

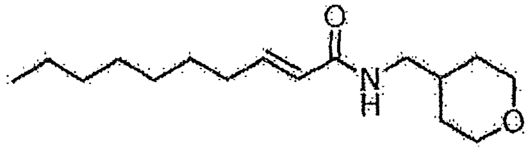

(26)

Compound (26) of the present invention

$^1$H-NMR ($CDCl_3$, TMS) δ (ppm): 0.88 (3H, t), 1.27-1.46 (m, 12H), 1.56-1.64 (m, 2H), 1.76-1.85 (m, 1H), 2.17 (q, 2H), 3.23 (t, 2H), 3.36 (td, 2H), 3.97 (dd, 2H), 5.49 (br s, 1H), 5.74 (d, 1H), 6.81-6.88 (m, 1H)

Production Example 27

The reaction was carried out in the same manner using 0.37 g of (E)-2-undecenoic acid in place of (E)-2-nonenoic acid in Production Example 25, to obtain 0.42 g of (E)-N-(tetrahydropyran-4-ylmethyl)-2-undecenamide (hereinafter, described as Compound (27) of the present invention) represented by the following formula.

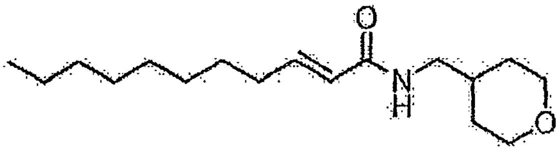

(27)

Compound (27) of the present invention

$^1$H-NMR ($CDCl_3$, TMS) δ (ppm): 0.88 (t, 3H), 1.18-1.38 (m, 12H), 1.38-1.48 (m, 2H), 1.62 (d, 2H), 1.73-1.85 (m, 1H), 2.16 (q, 2H), 3.21 (t, 2H), 3.36 (t, 2H), 3.96 (dd, 2H), 5.79 (d, 1H), 5.94 (br s, 1H), 6.83 (dt, 1H)

Production Example 28

The reaction was carried out in the same manner using 0. 27 g of (E)-3-methyl-2-nonenoic acid in place of (E)-2-nonenoic acid in Production Example 25, to obtain 0.15 g of (E)-N-(tetrahydropyran-4-ylmethyl)-3-methyl-2-nonenamide (hereinafter, described as Compound (28) of the present invention) represented by the following formula.

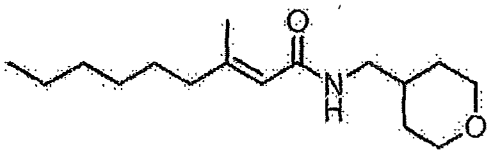

(28)

Compound (28) of the present invention

$^1$H-NMR ($CDCl_3$, TMS) δ (ppm): 0.89 (t, 3H), 1.23-1.38 (m, 8H), 1.39-1.50 (m, 2H), 1.58-1.66 (m, 2H), 1.73-1.84 (m, 1H), 2.07 (t, 2H), 2.14 (d, 3H), 3.18 (t, 2H), 3.37 (dt, 2H), 3.97 (dd, 2H), 5.47 (br s, 1H), 5.53 (d, 1H)

Production Example 29

The reaction was carried out in the same manner using 0.20 g of (E/Z)-2-fluoro-3-methyl-2-nonenoic acid in place of (E)-2-nonenoic acid in Production Example 25, to obtain 0.12 g of (Z)-N-(tetrahydropyran-4-ylmethyl)-2-fluoro-3-methyl-2-none namide (hereinafter, described as Compound (29) of the present invention) represented by the following formula.

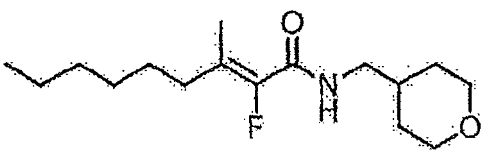

(29)

Compound (29) of the present invention

$^1$H-NMR ($CDCl_3$, TMS) $\delta$ (ppm): 0.89 (t, 3H), 1.20-1.40 (m, 8H), 1.40-1.50 (m, 2H), 1.54-1.68 (m, 2H), 1.73-1.78 (m, 1H), 2.15-2.22 (m, 2H), 2.16 (d, 3H), 3.22 (t, 2H), 3.37 (td, 2H), 3.98 (dd, 2H), 6.33 (br s, 1H)

Production Example 30

The reaction was carried out in the same manner as in Production Example 29, to obtain 0.16 g of (E)-N-(tetrahydropyran-4-ylmethyl)-2-fluoro-3-methyl-2-none namide (hereinafter, described as Compound (30) of the present invention) represented by the following formula.

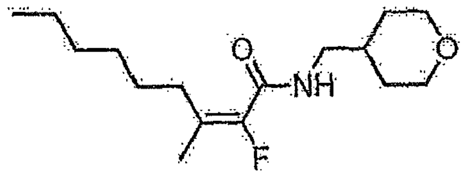

(30)

Compound (30) of the present invention

$^1$H-NMR ($CDCl_3$, TMS) $\delta$ (ppm): 0.87 (t, 3H), 1.25-1.50 (m, 10H), 1.58-1.66 (m, 2H), 1.74-1.85 (m, 1H), 1.81 (d, 3H), 2.64 (td, 2H), 3.21 (t, 2H), 3.37 (td, 2H), 3.98 (dd, 2H), 6.32 (br s, 1H)

Production Example 31

The reaction was carried out in the same manner using 0. 38 g of (E) -hepta-2, 6-dienoic acid in place of (E)-2-nonenoic acid in Production Example 25, to obtain 0.47 g of (E)-N-(tetrahydropyran-4-ylmethyl)-hepta-2,6-dienamide (hereinafter, described as Compound (31) of the present invention) represented by the following formula.

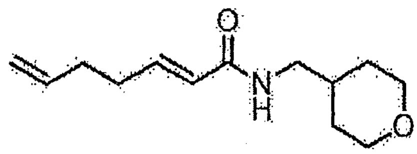

(31)

Compound (31) of the present invention

$^1$H-NMR ($CDCl_3$, TMS) $\delta$ (ppm): 1.32 (ddd, 2H), 1.63 (d,2H), 1.73-1.84 (m, 1H), 2.19-2.31 (m, 4H), 3.21 (t, 2H), 3.36 (t, 2H), 3.96 (dd, 2H), 4.98-5.07 (m, 2H), 5.75-5.85 (m, 2H), 6.08 (br s, 1H), 6.83 (dt, 1H)

Production Example 32

The reaction was carried out in the same manner using 0.30 g of (E)-nona-2,8-dienoic acid in place of (E) -2-nonenoic acid in Production Example 25, to obtain 0.41 g of (E)-N-(tetrahydropyran-4-ylmethyl)-nona-2,8-dienamide (hereinafter, described as Compound (32) of the present invention) represented by the following formula.

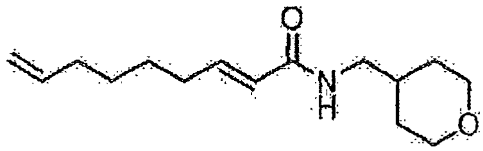

(32)

Compound (32) of the present invention

$^1$H-NMR ($CDCl_3$, TMS) $\delta$ (ppm): 1.32 (ddd, 2H), 1.39-1.50 (m, 4H), 1.62 (dd, 2H), 1.73-1.85 (m, 1H), 2.05 (q, 2H), 2.18 (q, 2H), 3.22 (t, 2H), 3.36 (td, 2H), 3.97 (dd, 2H), 4.93-5.03 (m, 2H), 5.74-5.84 (m, 2H), 5.78 (br s, 1H), 6.83 (dt, 1H) Production Example 33

The reaction was carried out in the same manner using 0. 34 g of (E)-deca-2,9-dienoic acid in place of (E)-2-nonenoic acid in Production Example 25, to obtain 0.40 g of (E)-N-(tetrahydropyran-4-ylmethyl)-deca-2,9-dienamide (hereinafter, described as Compound (33) of the present invention) represented by the following formula.

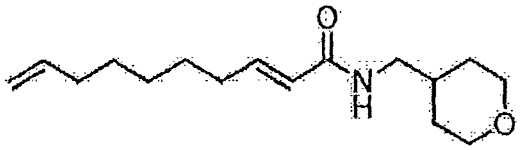
(33)

Compound (33) of the present invention

$^1$H-NMR ($CDCl_3$, TMS) δ (ppm) : 1.27-1.49 (m, 8H), 1.62 (dq, 2H), 1.78 (dd, 1H), 2.04 (q, 2H), 2.17 (ddd, 2H), 3.22 (t, 2H), 3.36 (td, 2H), 3.97 (dd, 2H), 4.92-5.03 (m, 2H), 5.68-5.85 (m, 2H), 5.74 (br s, 1H), 6.84 (dt, 1H)

Production Example 34

The reaction was carried out in the same manner using 0.19 g of (2E, 6E)-nona-2,6-dienoic acid in place of (E)-2-nonenoic acid in Production Example 25, to obtain 0.10 g of (2E, 6E)-N-(tetrahydropyran-4-ylmethyl)-nona-2,6-dienamide (hereinafter, described as Compound (34) of the present invention) represented by the following formula.

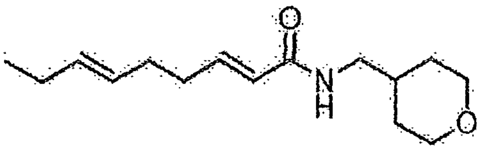
(34)

Compound (34) of the present invention

$^1$H-NMR ($CDCl_3$, TMS) δ (ppm): 0.96 (t, 3H), 1.32 (ddd, 2H), 1.62 (d, 2H), 1.73-1.85 (m, 1H), 1.96-2.03 (m, 2H), 2.13 (dd, 2H), 2.24 (ddd, 2H), 3.22 (t, 2H), 3.36 (td, 2H), 3.97 (dd, 2H), 5.38 (ddd, 1H), 5.49 (dt, 1H), 5.58 (br s, 1H), 5.77 (dt, 1H), 6.84 (dt, 1H)

Production Example 35

The reaction was carried out in the same manner using 0.50 g of (E/Z)-3,7-dimethyl-octa-2,6-dienoic acid in place of (E)-2-nonenoic acid in Production Example 25, to obtain 0.26 g of (E)-N-(tetrahydropyran-4-ylmethyl)-3,7-dimethyl-octa-2,6-di enamide (hereinafter, described as Compound (35) of the present invention) represented by the following formula.

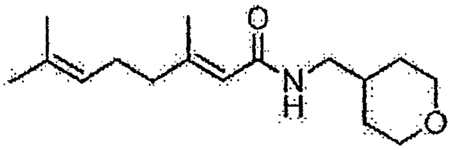
(35)

Compound (35) of the present invention

$^1$H-NMR ($CDCl_3$, TMS) δ (ppm): 1.24-1.37 (m, 2H), 1.55-1.67 (m, 3H), 1.61 (s, 6H), 1.69 (s, 3H), 1.71-1.83 (m, 1H), 2.08-2.17 (m, 3H), 3.19 (t, 2H), 3.37 (td, 2H), 3.97 (dd, 2H), 5.07 (t, 1H), 5.46 (br s, 1H), 5.53 (s, 1H)

Production Example 36

The reaction was carried out in the same manner as in Production Example 35, to obtain 0.18 g of (Z)-N-(tetrahydropyran-4-ylmethyl)-3,7-dimethyl-octa-2,6-di enamide (hereinafter, described as Compound (36) of the present invention) represented by the following formula.

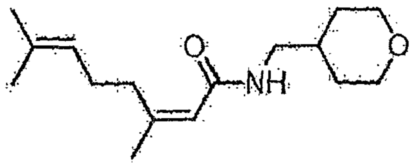

(36)

Compound (36) of the present invention

$^1$H-NMR ($CDCl_3$, TMS) $\delta$ (ppm) : 1.25-1.36 (m, 2H), 1.47-1.65 (m, 2H), 1.62 (s, 3H), 1.68 (s, 3H), 1.72-1.81 (m, 1H), 1.83 (s, 3H), 2.17 (q, 2H), 2.62 (t, 2H), 3.17 (t, 2H), 3.36 (td, 2H), 3.97 (dd, 2H), 5.16 (t, 1H), 5.49 (br s, 1H), 5.55 (s, 1H) Production Example 37

The reaction was carried out in the same manner using 0.25 g of (E)-oct-2-en-7-ynoic acid in place of (E)-2-nonenoic acid in Production Example 25, to obtain 0.35 g of (E)-N-(tetrahydropyran-4-ylmethyl)-oct-2-en-7-ynamide (hereinafter, described as Compound (37) of the present invention) represented by the following formula.

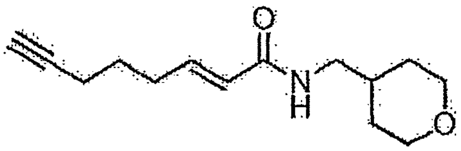

(37)

Compound (37) of the present invention

$^1$H-NMR ($CDCl_3$, TMS) $\delta$ (ppm): 1.33 (ddd, 2H), 1.59-1.65 (m, 2H), 1.70 (q, 2H), 1.74-1.85 (m, 1H), 1.97 (t, 1H), 2.23 (td, 2H), 2.32 (ddd, 2H), 3.23 (t, 2H), 3.37 (td, 2H), 3.97 (dd, 2H), 5.53 (br s, 1H), 5.80 (dt, 1H), 6.83 (dt, 1H)

Production Example 38

The reaction was carried out in the same manner using 0.46 g of (E)-non-2-en-8-ynoic acid in place of (E)-2-nonenoic acid in Production Example 25, to obtain 0.39 g of (E)-N-(tetrahydropyran-4-ylmethyl)-non-2-en-8-ynamide (hereinafter, described as Compound (38) of the present invention) represented by the following formula.

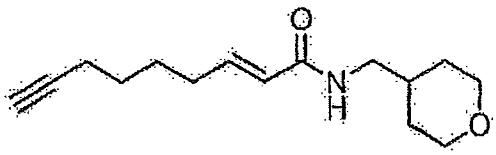

(38)

Compound (38) of the present invention

$^1$H-NMR ($CDCl_3$, TMS) $\delta$ (ppm): 1.24-1.38 (m, 2H), 1.52-1.68 (m, 6H), 1.74-1.85 (m, 1H), 1.95 (t, 1H), 2.18-2.24 (m, 4H), 3.23 (t, 2H), 3.37 (td, 2H), 3.97 (dd, 2H), 5.56 (br s, 1H), 5.77 (dt, 1H), 6.84 (dt, 1H)

Production Example 39

The reaction was carried out in the same manner using 0.51 g of (E)-3-(trans-2'-methylcyclopropyl)-2-propenoic acid in place of (E)-2-nonenoic acid in Production Example 25, to obtain 0.74 g of (E)-N-(tetrahydropyran-4-ylmethyl)-3-(trans-2'-methylcyclop ropyl)-2-propenamide (hereinafter, described as Compound (39) of the present invention) represented by the following formula.

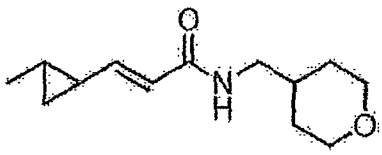

(39)

Compound (39) of the present invention

$^1$H-NMR ($CDCl_3$, TMS) δ (ppm) : 0.63-0.80 (m, 2H), 0.94-1.03 (m, 1H), 1.08-1.11 (m, 3H), 1.18-1.36 (m, 3H), 1.62 (dd, 2H), 1.72-1.85 (m, 1H), 3.19 (t, 2H), 3.35 (td, 2H), 3.96 (dd, 2H), 5.84 (d, 1H), 6.08 (br s, 1H), 6.34 (dd, 1H)

Production Example 40

The reaction was carried out in the same manner using 0.28 g of (E)-3-(trans-2'-ethylcyclopropyl)-2-propenoic acid in place of (E)-2-nonenoic acid in Production Example 25, to obtain 0.29 g of (E)-N-(tetrahydropyran-4-ylmethyl)-3-(trans-2'-ethylcyclopr opyl)-2-propenamide (hereinafter, described as Compound (40) of the present invention) represented by the following formula.

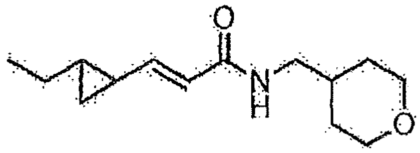

(40)

Compound (40) of the present invention

$^1$H-NMR ($CDCl_3$, TMS) δ (ppm): 0.68-0.79 (m, 2H), 0.96 (t, 4H), 1.22-1.37 (m, 5H), 1.60-1.69 (m, 2H), 1.71-1.84 (m, 1H), 3.21 (t, 2H), 3.36 (td, 2H), 3.97 (dd, 2H), 5.49 (br s, 1H), 5.78 (d, 1H), 6.38 (dd, 1H)

Production Example 41

The reaction was carried out in the same manner using 0.46 g of (E)-3-(trans-2'-propylcyclopropyl)-2-propenoic acid in place of (E)-2-nonenoic acid in Production Example 25, to obtain 0.33 g of (E)-N-(tetrahydropyran-4-ylmethyl)-3-(trans-2'-propylcyclop ropyl)-2-propenamide (hereinafter, described as Compound (41) of the present invention) represented by the following formula.

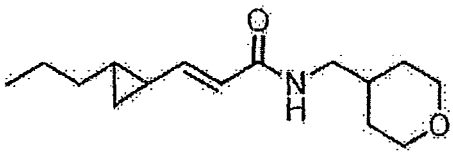

(41)

Compound (41) of the present invention

$^1$H-NMR ($CDCl_3$, TMS) δ (ppm) : 0.67-0.72 (m, 1H), 0.74-0.79 (m, 1H), 0.91 (t, 3H), 0.94-1.01 (m, 1H), 1.19-1.45 (m, 7H), 1.62 (dq, 2H), 1.72-1.84 (m, 1H), 3.20 (t, 2H), 3.35 (td, 2H), 3.96 (dd, 2H), 5.81 (d, 1H), 5.82 (br s, 1H), 6.37 (dd, 1H) Production Example 42

The reaction was carried out in the same manner using 0.14 g of (E)-3-(trans-2'-butylcyclopropyl)-2-propenoic acid in place of (E)-2-nonenoic acid in Production Example 25, to obtain 0.18 g of (E)-N-(tetrahydropyran-4-ylmethyl)-3-(trans-2'-butylcyclopr opyl)-2-propenamide (hereinafter, described as Compound (42) of the present invention) represented by the following formula.

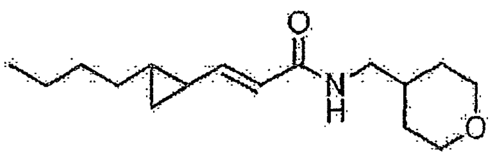

(42)

Compound (42) of the present invention

$^1$H-NMR ($CDCl_3$, TMS) δ (ppm) : 0.67-0.73 (m, 1H), 0.75-0.79 (m, 1H), 0.88 (t, 3H), 0.94-1.00 (m, 1H), 1.22-1.38 (m, 7H), 1.62 (dq, 2H), 1.68 (d, 1H), 1.78 (d, 1H), 3.21 (t, 2H), 3.36 (td, 2H), 3.97 (dd, 2H), 5.51 (br s, 1H), 5.78 (d, 1H), 6.37 (dd, 1H)

Production Example 43

The reaction was carried out in the same manner using 0.65 g of (E)-3-(trans-2'-butyl-3',3'-dimethylcyclopropyl)-2-propenoi c acid in place of (E)-2-nonenoic acid in Production Example 25, to obtain 0.73 g of (E)-N-(tetrahydropyran-4-ylmethyl)-3-(trans-2'-butyl-3',3'-dimethylcyclopropyl)-2-propenamide (hereinafter, described as Compound (43) of the present invention) represented by the following formula.

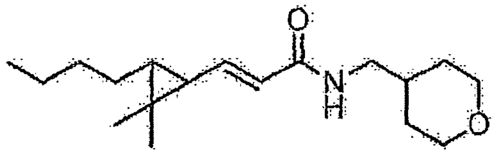
(43)

Compound (43) of the present invention

$^1$H-NMR ($CDCl_3$, TMS) $\delta$ (ppm): 0.82-0.86 (m, 1H), 0.88 (t, 3H), 1.01 (dd, 1H), 1.09 (s, 3H), 1.14 (s, 3H), 1.29-1.46 (m, 8H), 1.62 (d, 2H), 1.74-1.84 (m, 1H), 3.21 (t, 2H), 3.36 (td, 2H), 3.97 (dd, 2H), 5.45 (br s, 1H), 5.78 (d, 1H), 6.62 (dd, 1H)

Production Example 44

The reaction was carried out in the same manner using 0.17 g of (E)-5-(trans-2'-ethylcyclopropyl)-2-pentenoic acid in place of (E)-2-nonenoic acid in Production Example 25, to obtain 0.18 g of (E)-N-(tetrahydropyran-4-ylmethyl)-5-(trans-2'-ethylcyclopr opyl)-2-pentenamide (hereinafter, described as Compound (44) of the present invention) represented by the following formula.

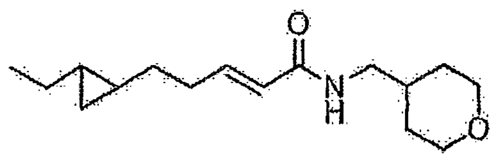
(44)

Compound (44) of the present invention

$^1$H-NMR ($CDCl_3$, TMS) $\delta$ (ppm): 0.19 (td, 1H), 0.41 (dd, 1H), 0.93 (t, 3H), 1.10-1.45 (m, 7H), 1.62 (d, 2H), 1.78 (t, 1H), 2.00 (t, 1H), 2.21-2.28 (m, 2H), 3.22 (t, 2H), 3.36 (t, 2H), 3.97 (dd, 2H), 5.79 (d, 1H), 5.80 (br s, 1H), 6.85 (dq, 1H)

Production Example 45

(E)-2-Nonenoic acid (187 mg, 1.20 mmol) and tetrahydropyran-3-ylmethylamine (115 mg, 1.00 mmol) were added to tetrahydrofuran (2 mL). To the mixture solution was added 1-ethyl-3-(3-dimethylaminopropyl)carbodiimide hydrochloride (286 mg, 1.50 mmol) at room temperature, then the mixture was stirred at room temperature for 4 hours. Thereafter, water was added to the reaction mixture, and the mixture was extracted twice with ethyl acetate. The organic layer was washed with a saturated saline solution, and dried over anhydrous sodium sulfate, then filtered, and the filtrate was concentrated under reduced pressure. The residue was applied to a silica gel column chromatography, to obtain 0.18 g of (E)-N-(tetrahydrofuran-3-ylmethyl)-2-nonenamide (hereinafter, described as Compound (45) of the present invention) represented by the following formula.

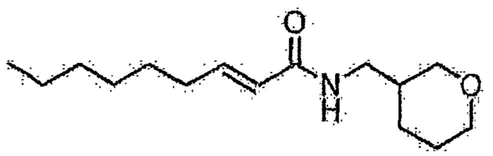
(45)

Compound (45) of the present invention

$^1$H-NMR ($CDCl_3$, TMS) $\delta$ (ppm) : 0.88 (t, 3H), 1.23-1.35 (m, 7H), 1.39-1.47 (m, 2H), 1.52-1.68 (m, 1H), 1.83-1.83 (m, 3H), 2.17 (q, 2H), 3.15-3.25 (m, 3H), 3.39-3.45 (m, 1H), 3.80-3.89 (m, 2H), 5.50 (br s, 1H), 5.74 (dt, 1H), 6.83 (dt, 1H)

Production Example 46

Tetrahydropyran-2-ylmethylamine (174 mg, 1.5 mmol) and triethylamine (182 mg, 1.8 mmol) were added to tetrahydrofuran (3 mL). To the mixture solution was added (E)-2-nonenoic acid chloride (174 mg, 1.0 mmol) under ice cooling, then the mixture was stirred at room temperature for 4 hours. Thereafter, water was added to the reaction mixture, and the mixture was extracted twice with ethyl acetate. The organic layer was washed with an aqueous solution of sodium bicarbonate, and dried over anhydrous sodium sulfate, then filtered, and the filtrate was concentrated under reduced pressure. The residue was applied to a silica gel column chromatography, to obtain 0.21 g of (E)-N-(tetrahydropyran-2-ylmethyl)-2-nonenamide (hereinafter, described as Compound (46) of the present invention) represented by the following formula.

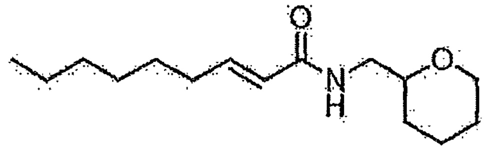

(46)

Compound (46) of the present invention

$^1$H-NMR ($CDCl_3$, TMS) δ (ppm): 0.88 (t, 3H), 1.23-1.35 (m, 7H), 1.33-1.63 (m, 6H), 1.84 (d, 1H), 2.17 (q, 2H), 3.03-3.12 (1H, m), 3.41 (dq, 2H), 3.60-3.67 (m, 1H), 3.97 (dd, 1H), 5.77 (d, 1H), 5.88 (br s, 1H), 6.80-6.87 (m, 1H)

Production Example 47

(E)-2-Nonenoic acid (0.31 g, 2.0 mmol) and 1,4-dioxan-2-ylmethylamine (0.28 g, 2.4 mmol) were added to tetrahydrofuran (4 mL). To the mixture solution was added 1-ethyl-3-(3-dimethylaminopropyl)carbodiimide hydrochloride (572 g, 3.0 mmol) at room temperature, then the mixture was stirred at room temperature for 4 hours. Thereafter, water was added to the reaction mixture, and the mixture was extracted twice with ethyl acetate. The organic layer was washed with a saturated saline solution, and dried over anhydrous sodium sulfate, then filtered, and the filtrate was concentrated under reduced pressure. The residue was applied to a silica gel column chromatography, to obtain 0.30 g of (E)-N-(1,4-dioxan-2-ylmethyl)-2-nonenamide (hereinafter, described as Compound (47) of the present invention) represented by the following formula.

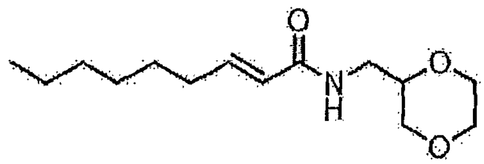

(47)

Compound (47) of the present invention

$^1$H-NMR ($CDCl_3$, TMS) δ (ppm) : 0.88 (t, 3H), 1.23-1.35 (m, 6H), 1.40-1.48 (m, 2H), 2.17 (q, 2H), 3.22-3.15 (m, 1H), 3.33 (t, 1H), 3.62-3.51 (m, 2H), 3.65-3.81 (m, 5H), 5.78 (d, 1H), 5.91 (br s, 1H), 6.85 (dt, 1H) Production Example 48

5-Ethyl-1,3-dioxan-5-ylmethylamine (232 mg, 1.6 mmol) and triethylamine (182 mg, 1.8 mmol) were added to tetrahydrofuran (3 mL). To the mixture solution was added (E)-2-nonenoic acid chloride (174 mg, 1.0 mmol) under ice cooling, then the mixture was stirred at room temperature for 4 hours. Thereafter, water was added to the reaction mixture, and the mixture was extracted twice with ethyl acetate. The organic layer was washed with an aqueous solution of sodium bicarbonate, and dried over anhydrous sodium sulfate, then filtered, and the filtrate was concentrated under reduced pressure. The residue was applied to a silica gel column chromatography, to obtain 0.14 g of (E)-N-(5-ethyl-1,3-dioxan-5-ylmethyl)-2-nonenamide (hereinafter, described as Compound (48) of the present invention) represented by the following formula.

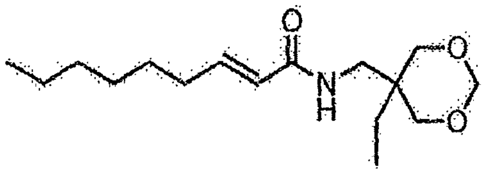

(48)

Compound (48) of the present invention

$^1$H-NMR ($CDCl_3$, TMS) δ (ppm) : 0.89 (t, 6H), 1.23-1.34 (m, 8H), 1.39-1.49 (m, 2H), 2.17 (dq, 2H), 3.51-3.57 (m, 4H), 3.79 (d, 2H), 4.67 (d, 1H), 4.97 (d, 1H), 5.77 (dt, 1H), 5.79 (br s, 1H), 6.84 (dt, 1H)

Production Example 49

(E)-2-Nonenoic acid (0.20 g, 1.3 mmol) and tetrahydrothiopyran-4-ylmethylamine hydrochloride (0.15 g, 0.90 mmol) were added to N,N-dimethylformamide (5mL). To the mixture solution was added triethylamine (0.25 g, 2.5 mmol) under ice cooling, then, benzotriazol-1-yloxy-tris(dimethylamino)phosphonium salt (0.52 g, 1.2 mmol) was added. The mixture was stirred under ice cooling for 1 hour, then stirred at room temperature for 12 hours, and thereafter, the reaction solution was added to a mixture of 20 mL of 5% of hydrochloric acid and 50 mL of ice

water, and the mixture was extracted twice with 100 mL of ethyl acetate. The ethyl acetate layers were combined, and washed with 50 mL of a saturated sodium bicarbonate solution, followed by 50 mL of a saturated salt solution, then the organic layer was dried over magnesium sulfate. The dried organic layer was concentrated under reduced pressure conditions, and the residue was applied to a silica gel column chromatography, to obtain 0.13 g of (E)-N-(tetrahydrothiopyran-4-ylmethyl)-2-nonenamide (hereinafter, described as Compound (49) of the present invention) represented by the following formula.

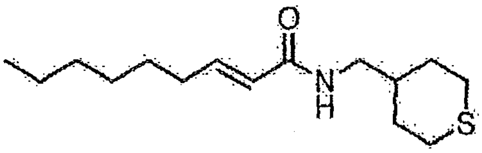

(49)

Compound (49) of the present invention

$^1$H-NMR ($CDCl_3$, TMS) δ (ppm): 0.88 (t, 3H), 1.25-1.46 (m, 11H), 2.00-2.06 (m, 2H), 2.17 (q, 2H), 2.58-2.70 (m, 4H), 3.19 (t, 2H), 5.46 (br s, 1H), 5.74 (d, 1H), 6.84 (dt, 1H) Production Example 50

The reaction was carried out in the same manner using 0.20 g of (E)-3-methyl-3-(trans-2'-propylcyclopropyl)-2-propenoic acid in place of (E)-2-undecenoic acid in Production Example 5, to obtain 0.17 g of (E)-N-(tetrahydrofuran-3-ylmethyl)-3-methyl-3-(trans-2'-pro pylcyclopropyl)-2-propenamide (hereinafter, described as Compound (50) of the present invention) represented by the following formula.

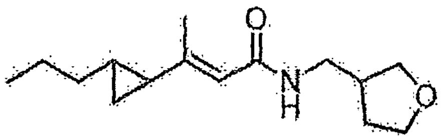

(50)

Compound (50) of the present invention

$^1$H-NMR ($CDCl_3$, TMS) δ (ppm): 0.53 (dt, 1H), 0.81-0.86 (m, 1H), 0.88-1.01 (m, 4H), 1.15-1.45 (m, 5H), 1.58-1.66 (m, 2H), 2.01 (s, 3H), 2.46-2.53 (m, 1H), 3.23-3.36 (m, 2H), 3.54 (dd, 1H), 3.73 (dd, 1H), 3.82 (dd, 1H), 3.88 (td, 1H), 5.47 (br s, 1H), 5.51 (s, 1H)

Production Example 51

The reaction was carried out in the same manner using 0.33 g of (E)-3-(trans-2'-(trans-2"-methylcyclopropyl)cyclopropyl)-2 -propenoic acid in place of (E) -2-undecenoic acid in Production Example 5, to obtain 0.36 g of (E)-N-(tetrahydrofuran-3-ylmethyl)-3-(trans-2'-(trans-2"-m ethylcyclopropyl)cyclopropyl)-2-propenamide (hereinafter, described

as Compound (51) of the present invention) represented by the following formula.

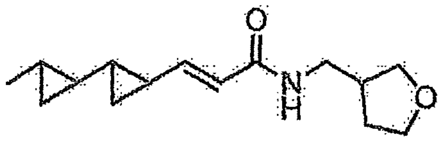 (51)

Compound (51) of the present invention

$^1$H-NMR ($CDCl_3$, TMS) $\delta$ (ppm): 0.07-0.78 (m, 6H), 0.88-1.00 (m, 2H), 1.04-1.09 (m, 3H), 1.58-1.67 (m, 1H), 1.98-2.07 (m, 1H), 2.45-2.55 (m, 1H), 3.26-3.39 (m, 2H), 3.55 (dd, 1H), 3.73 (dd, 1H), 3.81 (dd, 1H), 3.88 (td, 1H), 5.67 (br s, 1H), 5.80 (d, 1H), 6.38 (dd, 1H)

Production Example 52

The reaction was carried out in the same manner using 0.20 g of (E)-3-methyl-3-(trans-2'-propylcyclopropyl)-2-propenoic acid in place of (E)-2-undecenoic acid in Production Example 25, to obtain 0.20 g of (E)-N-(tetrahydropyran-4-ylmethyl)-3-methyl-3-(trans-2'-pro pylcyclopropyl)-2-propenamide (hereinafter, described as Compound (52) of the present invention) represented by the following formula.

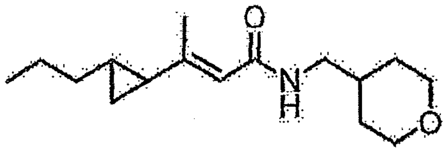 (52)

Compound (52) of the present invention

$^1$H-NMR ($CDCl_3$, TMS) $\delta$ (ppm): 0.50-0.55 (m, 1H), 0.81-0.85 (m, 1H), 0.88-1.00 (m, 4H), 1.15-1.20 (m, 1H), 1.20-1.45 (m, 6H), 1.55-1.72 (m, 2H), 1.72-1.83 (m, 1H), 2.02 (s, 3H), 3.17 (t, 2H), 3.36 (dt, 2H), 3.97 (dd, 2H), 5.46 (brs, 1H), 5.52 (s, 1H)

Production Example 53

The reaction was carried out in the same manner using 0.33 g of (E)-3-(trans-2'-(trans-2"-methylcyclopropyl)cyclopropyl)-2 -propenoic acid in place of (E)-2-undecenoic acid in Production Example 25, to obtain 0.27 g of (E)-N-(tetrahydropyran-4-ylmethyl)-3-(trans-2'-(trans-2"-m ethylcyclopropyl)cyclopropyl)-2-propenamide (hereinafter, described as Compound (53) of the present invention) represented by the following formula.

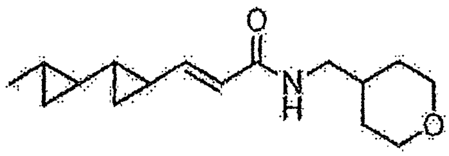 (53)

Compound (53) of the present invention

$^1$H-NMR ($CDCl_3$, TMS) $\delta$ (ppm) : 0.08-0.80 (m, 6H), 0.92-1.01 (m, 1H), 1.05-1.10 (m, 3H), 1.18-1.36 (m, 3H), 1.60-1.68 (m, 2H), 1.71-1.84 (m, 1H), 3.20 (t, 2H), 3.35 (td, 2H), 3.96 (dd, 2H), 5.84 (d, 1H), 6.01 (br s, 1H), 6.38 (dd, 1H)

Production Example 54

((Tetrahydropyran-4-ylmethyl)carbamoylmethyl)tripheny lphosphonium chloride (9.63 g, 21.2 mmol) and 5-chloropentanal (2.56 g, 21.2 mmol) were added to tetrahydrofuran (70 mL) and then the mixture was cooled to 0°C. To the mixture solution was added sodium hydride (55% oil-based) (0.97 g, 22.3 mmol), then the mixture was stirred at room temperature for 12 hours. Thereafter, water was added to the reaction mixture, and the mixture was extracted twice with ethyl acetate. The organic layer was washed with a saturated saline solution, and dried over anhydrous sodium

sulfate, then filtered, and the filtrate was concentrated under reduced pressure. The residue was applied to a silica gel column chromatography, to obtain 3.21 g of (E)-N-(tetrahydropyran-4-ylmethyl)-7-chloro-2-heptenamide (hereinafter, described as Compound (54) of the present invention) represented by the following formula.

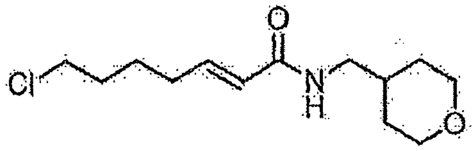 (54)

Compound (54) of the present invention

$^1$H-NMR ($CDCl_3$, TMS) δ (ppm): 1.24-1.38 (m, 2H), 1.57-1.66 (m, 4H), 1.75-1.90 (dt, 3H), 2.18-2.26 (m, 2H), 3.23 (t, 2H), 3.37 (td, 2H), 3.54 (t, 2H), 3.97 (dd, 2H), 5.53 (br s, 1H), 5.78 (dt, 1H), 6.84 (dt, 1H)

Production Example 55

(E)-N-(Tetrahydropyran-4-ylmethyl)-7-chloro-2-heptena mide (0.80 g, 3.1 mmol) was dissolved in N,N-dimethylformamide (5 mL). To the solution was added sodium methoxide (28% methanol solution) (0.89 g, 4.6 mmol), then the mixture was stirred at 80°C for 12 hours. Thereafter, water was added to the reaction mixture, and the mixture was extracted twice with ethyl acetate. The organic layer was washed with a saturated saline solution, and dried over anhydrous sodium sulfate, then filtered, and the filtrate was concentrated under reduced pressure. The residue was applied to a silica gel column chromatography, to obtain 0.13 g of (E)-N-(tetrahydropyran-4-ylmethyl)-7-methoxy-2-heptenamide (hereinafter, described as Compound (55) of the present invention) represented by the following formula.

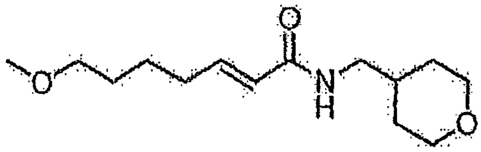 (55)

Compound (55) of the present invention

$^1$H-NMR ($CDCl_3$, TMS) δ (ppm): 1.23-1.37 (m, 3H), 1.49-1.63 (m, 5H), 1.70-1.85 (m, 1H), 2.20 (ddd, 2H), 3.22 (t, 2H), 3.33 (s, 3H), 3.36-3.39 (m, 4H), 3.97 (ddd, 2H), 5.59 (br s, 1H), 5.77 (dt, 1H), 6.84 (dt, 1H)

Next, reference production examples of intermediates used in the production of the compound of the present invention are shown.

Reference Production Example 1

Under a nitrogen atmosphere, to a mixture of 25 g of (tetrahydrofuran-3-yl)-methanol represented by the following formula

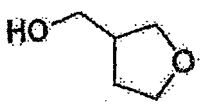

and 125 ml of pyridine was added 56 g of p-toluenesulfonyl chloride under ice cooling, and the mixture was stirred under ice cooling for 4 hours. Thereafter, water was added to the reaction mixture, and the mixture was extracted twice with tert-butyl methyl ether. The organic layer was washed with 1 mol/L hydrochloric acid, followed by a saturated salt solution. The organic layer was dried over anhydrous sodium sulfate, and then filtered. The filtrate was concentrated under reduced pressure to obtain 58 g of a crude product of tetrahydrofuran-3-ylmethyl p-toluenesulfonate represented by the following formula.

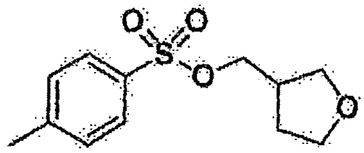

The product was used in Reference Production Example 2 without purification.

Reference Production Example 2

Under a nitrogen atmosphere, to a mixture of 29 g of tetrahydrofuran-3-ylmethyl p-toluenesulfonate, 23 g of potassium iodide and 300 ml of N,N-dimethylformamide was added 22 g of phthalimide potassium salt, and the mixture was stirred at 80°C for 3 hours. Thereafter, the reaction mixture was ice-cooled, water was added to the mixture, and the resulting mixture was stirred for 30 minutes. The reaction mixture was filtered to collect a solid. The solid was dried under reduced pressure to obtain 14 g of 2-(tetrahydrofuran-3-ylmethyl)-isoindole-1,3-dione represented by the following formula.

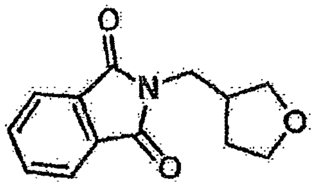

$^1$H-NMR ($CDCl_3$, TMS) $\delta$ (ppm) : 7.86 (2H, dd), 7.73 (2H, dd), 3.94 (1H, td), 3.85-3.67 (4H, m), 3.61 (1H, dd), 2.79-2.68 (1H, m), 2.06-1.97 (1H, m), 1.77-1.68 (1H, m)

Reference Production Example 3

Under a nitrogen atmosphere, to a mixture of 14 g of 2-(tetrahydrofuran-3-ylmethyl)-isoindole-1,3-dione and 150 ml of ethanol was added 4.6 g of hydrazine monohydrate, and the mixture was stirred at 70°C for 6 hours. Thereafter, the reaction mixture was filtered, and the residue was washed with ethanol. To the ethanol solution was added 1 ml of a suspension of Raney nickel, and the mixture was stirred at 60°C for 4 hours. The reaction solution was cooled to room temperature, and then filtered. To the filtrate was added 90 ml of a 1 mol/L solution of hydrogen chloride in diethyl ether. The mixture solution was concentrated under reduced pressure to obtain 10 g of a crude product of (tetrahydrofuran-3-yl)-methylamine hydrochloride represented by the following formula.

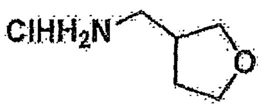

The product was used in the next reaction without purification.

Reference Production Example 4

At room temperature, tetrahydropyran-4-ylmethylamine (14.4 g, 125 mmol) was dissolved in dichloromethane (300 mL), and an aqueous solution (400 mL) of sodium hydroxide (15.0 g, 375 mmol) was added, then the mixture was cooled to 0°C. A dichloromethane solution (100 mL) of chloroacetylchloride (42.4 g, 375 mmol) was added dropwise thereto, and the mixture was stirred at room temperature for 2 hours. Thereafter, the organic layer was separated and washed with a saturated aqueous solution of sodium bicarbonate and a saturated saline solution, and dried over anhydrous sodium sulfate, then filtered. The filtrate was concentrated under reduced pressure, to obtain 23.7 g of a crude product of N-(tetrahydropyran-4-ylmethyl)-chloroacetoamide represented by the following formula.

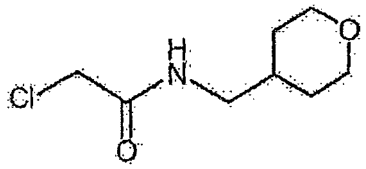

The product was used in the next reaction without purification. $^1$H-NMR ($CDCl_3$, TMS) δ (ppm): 1.28-1.40 (m, 2H), 1.58-1.67 (m, 2H), 1.74-1.86 (m, 1H), 3.22 (t, 2H), 3.38 (td, 2H), 3.98 (dd, 2H), 4.07 (s, 2H), 6.73 (br s, 1H)

Reference Production Example 5

At room temperature, N-(tetrahydropyran-4-ylmethyl)-chloroacetoamide (22.6 g, 118 mmol) was dissolved in toluene (400 mL), and triphenylphosphine (31.0 g, 118 mmol) was added, then the mixture was stirred under heating and reflux for 2 days. Thereafter, the organic layer was separated and washed with a saturated aqueous solution of sodium bicarbonate and a saturated saline solution, and dried over anhydrous sodium sulfate, then filtered. The filtrate was concentrated under reduced pressure, to obtain 53. 6 g of a crude product of ((tetrahydropyran-4-ylmethyl)carbamoylmethyl)triphenylphosp honium chloride represented by the following formula.

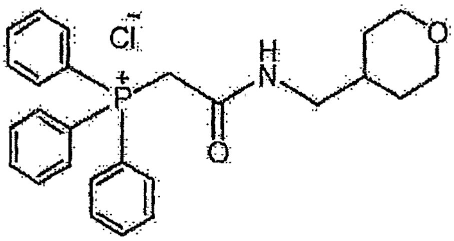

The product was used in the next reaction without purification.

Next, formulation examples are shown. The term "part(s)" means part(s) by weight.

Formulation Example 1

20 parts of any one of Compounds (1) to (55) of the present invention is dissolved in 65 parts of xylene, and 15 parts of SORPOL 3005X (registered trade name of TOHO Chemical Industry Co., Ltd.) is added thereto. The mixture is well stirred and mixed to obtain an emulsifiable concentrate. Formulation Example 2

4 0 parts of any one of Compounds (1) to (55) of the present invention and 5 parts of SORPOL 3005X are well mixed, and then 32 parts of Carplex #80 (synthetic hydrous silicon oxide, registered trade name of Shionogi & Co., Ltd.), and 23 parts of 300-mesh diatomaceous earth are added thereto. The mixture is stirred and mixed with a juice mixer to obtain a wettable powder.

Formulation Example 3

1. 5 parts of any one of Compounds (1) to (55) of the present invention, 1 part of Tokuseal GUN (synthetic hydrous silicon oxide, manufactured by Tokuyama corporation), 2 parts of Reax 85A (sodium ligninsulfonate, manufactured by West vaco chemicals), 30 parts of Bentonite Fuji (bentonite, manufactured by HOJUN Co., Ltd.), and 65.5 parts of Shokozan A clay (kaolin clay, manufactured by SHOKOZAN MINING Co., Ltd.) are well pulverized and mixed, and water is added thereto. The mixture is then kneaded well, granulated with an extrusion granulator, and dried to obtain 1.5% granules.

Formulation Example 4

10 parts of any one of Compounds (1) to (55) of the present invention, 10 parts of phenylxylylethane, and 0.5 part of Surnijule L-75 (tolylene diisocyanate, manufactured by Sumitomo Bayer Urethane Co., Ltd.) are mixed, and then the mixture is added to 20 parts of a 10% aqueous solution of arabic gum, followed by stirring the resulting mixture with a homomixer to obtain an emulsion having an average particle diameter of 20 μm. Thereto is added 2 parts of ethylene glycol, and the mixture is further stirred in a warm bath at 60°C for 24 hours to obtain a microcapsule slurry. Separately, 0.2 parts of xanthan gum and 1.0 part of Veegum R (aluminum magnesium silicate, manufactured by Sanyo Chemical

Industries, Ltd.) are dispersed in 56.3 parts of ion-exchanged water to obtain a thickening agent solution. 42.5 parts of the microcapsule slurry and 57.5 parts of the thickening agent solution are mixed to obtain a microcapsule formulation.

Formulation Example 5

10 parts of any one of Compounds (1) to (55) of the present invention and 10 parts of phenylxylylethane are mixed, and then the mixture is added to 20 parts of 10% aqueous solution of polyethylene glycol, followed by stirring the resulting mixture with a homomixer to obtain an emulsion having an average particle diameter of 3 μm. Separately, 0.2 parts of xanthan gum and 1.0 part of Veegum R (aluminum magnesium silicate, manufactured by Sanyo Chemical Industries, Ltd.) are dispersed in 58.8 parts of ion-exchanged water to obtain a thickening agent solution. 40 parts of the emulsion solution and 60 parts of the thickening agent solution are mixed to obtain a flowable. Formulation Example 6

5 parts of any one of Compounds (1) to (55) of the present invention, 3 parts of Carplex #80 (a fine powder of synthetic hydrous silicon oxide, registered trade name of Shionogi & Co., Ltd.), 0.3 parts of PAP (a mixture of monoisopropyl phosphate and diisopropyl phosphate) and 91.7 parts of talc (300 mesh) are stirred and mixed with a juice mixer to obtain a dust formulation.

Formulation Example 7

0.1 part of any one of Compounds (1) to (55) of the present invention is dissolved in 10 parts of isopropyl alcohol, and the mixture is mixed with 89.9 parts of deodorized kerosene to obtain an oil solution.

Formulation Example 8

1 part of any one of Compounds (1) to (55) of the present invention, 5 parts of dichloromethane and 34 parts of deodorized kerosene are mixed and dissolved. The resulting solution is filled into an aerosol container, and a valve part is attached to the container, then 60 parts of a propellant (liquefied petroleum gas) is filled under pressure into the container through the valve part to obtain an oil-based aerosol. Formulation Example 9

0.6 parts of any one of Compounds (1) to (55) of the present invention, 5 parts of xylene, 3.4 parts of deodorized kerosene and 1 part of ATMOS 300 (emulsifier, registered trade name of Atlas Chemical Company) are mixed and dissolved, and the resulting solution and 50 parts of water are filled into an aerosol container, and 40 parts of a propellant (liquefied petroleum gas) is filled under pressure into the container through the valve part to obtain an aqueous aerosol.

Formulation Example 10

0.3 g of any one of Compounds (1) to (55) of the present invention is dissolved in 20 ml of acetone. The resulting solution is uniformly stirred and mixed with 99.7 g of a base material for an insecticidal coil (a mixture of Tabu powder, Pyrethrum marc and wood powder in a ratio of 4 : 3 : 3). Thereafter, 100 ml of water is added to the mixture, and the resulting mixture is sufficiently kneaded, and molded and dried to obtain an insecticidal coil.

Formulation Example 11

0.8 g of any one of Compounds (1) to (55) of the present invention and 0.4 g of piperonyl butoxide are dissolved in acetone to have a total amount of 10 ml. A base material for an insecticidal mat for electric heating with a size of 2.5 cm × 1.5 cm, 0.3 cm in thickness (a plate of compacted fibrils of a mixture of cotton linter and pulp) is uniformly impregnated with 0.5 ml of this solution to obtain an insecticidal mat for electric heating.

Formulation Example 12

3 parts of any one of Compounds (1) to (55) of the present invention is dissolved in 97 parts of deodorized kerosene to obtain a solution, and this solution is put in a container made of polyvinyl chloride. Into the container is inserted an absorptive wick (which is prepared by solidifying powders of an inorganic powder with a binder followed by sintering them) whose upper portion can be heated by a heater, to obtain a part to be used for an absorptive wick type electric heating vaporizer.

Formulation Example 13

100 mg of any one of Compounds (1) to (55) of the present invention is dissolved in an appropriate amount of acetone, and a porous ceramic plate with a size of 4.0 cm × 4.0 cm, 1.2 cm in thickness is impregnated with the solution

to obtain a fumigant for heating.

Formulation Example 14

100 μg of any one of Compounds (1) to (55) of the present invention is dissolved in an appropriate amount of acetone, and the solution is uniformly applied on a filter paper of 2 cm × 2 cm, 0.3 mm in thickness. Then, the filter paper is air-dried to remove acetone to obtain a formulation vaporizable at room temperature.

Formulation Example 15

10 parts of any one of Compounds (1) to (55) of the present invention, 35 parts of white carbon containing 50 parts of polyoxyethylene alkyl ether sulfate ammonium salt and 55 parts of water are mixed, and the mixture is finely pulverized by wet grinding method to obtain a formulation.

Next, the pest control effect of the compound of the present invention is shown as test examples. Test Example 1

The formulations of Compounds (7), (46), (48) and (54) of the present invention obtained in Formulation Example 7 were diluted with a mixture solution of isopropyl alcohol/deodorized kerosene of 1/9, so as to have a concentration of active ingredient of 2.0% w/v, to prepare a test drug solution.

Ten German cockroaches (Blattella germanica) (5 males and 5 females) were released in a test container (8.75 cm in diameter, 7.5 cm in height, and the bottom made of 16 mesh metallic wire) with the inner wall on which butter was applied, and the container was placed on the bottom of a test chamber (bottom: 46 cm × 46 cm, height: 70 cm). From a height of 60 cm above the upper surface of the container, 1.5 ml of the test drug solution was sprayed using a spray gun (a spraying pressure of 0.42 $kg/cm^2$). Thirty seconds after spraying, the container was taken out from the test chamber, and after 5 minutes, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (7), (46), (48) and (54) of the present invention, the knockdown rate of the tested insect was 80% or more.

Test Example 2

The formulations of Compounds (1), (2), (4), (6) to (8), (10) to (14), (17) to (20), (23) to (25), (28), (31) to (33), (39) to (42), (44) and (50) to (53) of the present invention obtained in Formulation Example 7 were diluted with a mixture solution with a ratio of isopropyl alcohol/deodorized kerosene of 1/9, so as to have a concentration of active ingredient of 0.5% w/v, to prepare a test drug solution.
The test was performed in the same manner as in Test Example 1.

As a result, in treatments with Compounds (1), (2), (4), (6) to (8), (10) to (14), (17) to (20), (23) to (25), (28), (31) to (33), (39) to (42), (44) and (50) to (53) of the present invention, the knockdown rate of the tested insect was 70% or more.

Test Example 3

The formulations of Compounds (7), (46) and (48) of the present invention obtained in Formulation Example 7 were diluted with a mixture solution with a ratio of isopropyl alcohol/deodorized kerosene of 1/9, so as to have a concentration of active ingredient of 2.0% w/v, to prepare a test drug solution.

Ten adult houseflies (5 males and 5 females) were released in a polyethylene cup (a bottom diameter of 10.6 cm) which was then capped with 16-mesh nylon gauze. The polyethylene cup was placed on the bottom of a test chamber (bottom: 46 cm × 46 cm, height: 70 cm). From a height of 30 cm above the upper surface of the polyethylene cup, 0.5 ml of the test drug solution was sprayed using a spray gun (a spraying pressure of 0.9 $kg/cm^2$). Immediately after spraying, the cup was taken out from the test container, and after 10 minutes, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (7), (46) and (48) of the present invention, the knockdown rate of the tested insect was 80% or more.

Test Example 4

The formulations of Compounds (4), (6), (8), (17) to (19), (28), (29), (33), (40) to (42) and (51) to (53) of the present invention obtained in Formulation Example 7 were diluted with a mixture solution with a ratio of isopropyl alcohol/deodorized kerosene of 1/9, so as to have a concentration of active ingredient of 0.5% w/v, to prepare a test drug solution. The test was performed in the same manner as in Test Example 3.

As a result, in treatments with Compounds (4), (6), (8), (17) to (19), (28), (29), (33), (40) to (42) and (51) to (53) of the present invention, the knockdown rate of the tested insect was 80% or more.

Test Example 5

The formulations of Compounds (2), (7), (48) and (49) of the present invention obtained in Formulation Example 7 were diluted with a mixture solution with a ratio of isopropyl alcohol/deodorized kerosene of 1/9, so as to have a concentration of active ingredient of 0.1% w/v, to prepare a test drug solution.

Ten adult common house mosquitos were released in a polyethylene cup (a bottom diameter of 10.6 cm) which was then capped with 16-mesh nylon gauze. The polyethylene cup was placed on the bottom of a test chamber (bottom: 46 cm × 46 cm, height: 70 cm). From a height of 30 cm above the upper surface of the polyethylene cup, 0.5 ml of the test drug solution was sprayed using a spray gun (a spraying pressure of 0.4 kg/cm$^2$). Immediately after spraying, the cup was taken out from the test container, and after 15 minutes, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (2), (7), (48) and (49) of the present invention, the knockdown rate of the tested insect was 80% or more.

Test Example 6

Each of the formulations of Compounds (19) and (40) to (42) of the present invention obtained in Formulation Example 7 was diluted with a mixture solution with a ratio of isopropyl alcohol/deodorized kerosene of 1/9, so as to have a concentration of active ingredient of 0.02% w/v, to prepare a test drug solution.
The test was performed in the same manner as in Test Example 5.

As a result, in treatments with Compounds (19) and (40) to (42) of the present invention, the knockdown rate of the tested insect was 90% or more.

Test Example 7

Each of the formulations of Compounds (2), (4) to (6), (8), (9), (18), (21) to (23), (27), (29), (32) to (36), (38), (41), (42) and (53) of the present invention obtained in Formulation Example 15 was diluted with water, so as to have a concentration of active ingredient of 500 ppm, to prepare a test drug solution.

On the bottom of a polyethylene cup with a diameter of 5.5 cm, a filter paper of the same size was laid down, and 0.7 ml of the test drug solution was dropped onto the filter paper, then 30 mg of sucrose was uniformly put as bait. Ten adult houseflies (Musca domestica) were released in the polyethylene cup which was then capped. After 2 hours, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (2), (4) to (6), (8), (9), (18), (21) to (23), (27), (29), (32) to (36), (38), (41), (42) and (53) of the present invention, the knockdown rate of the tested insect was 100%.

Test Example 8

Each of the formulations of Compounds (2) to (6), (8), (12), (15) to (18), (27), (29), (33), (37) to (39), (41), (42) and (45) of the present invention obtained in Formulation Example 15 was diluted with water, so as to have a concentration of active ingredient of 500 ppm, to prepare a test drug solution.
On the bottom of a polyethylene cup with a diameter of 5.5 cm, a filter paper of the same size was laid down, and 0.7 ml of the test drug solution was dropped onto the filter paper, then 30 mg of sucrose was uniformly put as bait. Ten adult houseflies (Musca domestica) were released in the polyethylene cup which was then capped. After 24 hours, the houseflies were observed for life or death to obtain the mortality.
As a result, in treatments with Compounds (2) to (6), (8), (12), (15) to (18), (27), (29), (33), (37) to (39), (41), (42) and (45) of the present invention, the mortality showed 90% or more.

Test Example 9

Each of the formulations of Compounds (26) and (45) of the present invention obtained in Formulation Example 15 was diluted with water, so as to have a concentration of active ingredient of 500 ppm, to prepare a test drug solution.
0.7 ml of the test drug solution was added to 100 ml of ion-exchanged water (a concentration of active ingredient of 3.5 ppm). 20 last instar larvae of common house mosquitoes (Culex pipiens pallens) were released in the solution, and after a day, the insects were observed for life or death to obtain the mortality.
As a result, in treatments with Compounds (26) and (45) of the present invention, the mortality showed 95% or more.

Test Example 10

Each of the formulations of Compounds (5), (6), (24), (27), (29), (30), (33), (38) and (47) of the present invention obtained in Formulation Example 15 was diluted with water, so as to have a concentration of active ingredient of 500 ppm, to prepare a test drug solution.
0.7 ml of the test drug solution was added to 100 ml of ion-exchanged water (a concentration of active ingredient of 3.5 ppm). 20 last instar larvae of common house mosquitoes (Culex pipiens pallens) were released in the solution, and after 8 days, the insects were observed for life or death to obtain the mortality.
As a result, in treatments with Compounds (5), (6), (24), (27), (29), (30), (33), (38) and (47) of the present invention, the mortality showed 95% or more.

Test Example 11

Each of the formulations of Compounds (43) and (45) of the present invention obtained in Formulation Example 15 was diluted with water, so as to have a concentration of active ingredient of 500 ppm, to prepare a test spray solution.
On the other hand, a cabbage was planted in a polyethylene cup and grown until the third true leaf or the fourth true leaf was developed. The test spray solution was sprayed in a ratio of 20 ml/cup on the cabbage.
After the drug solution sprayed on the cabbage was dried, 10 third-instar larvae of diamondback moth (Plutella xylostella) were put on the cabbage. After 5 days, the number of diamondback moths was counted, and a controlling value was calculated by the following equation.

$$\text{Controlling Value (\%)} = \{1 - (Cb \times Tai) / (Cai \times Tb)\} \times 100$$

Letters in the formula have the following meanings.

Cb: the number of insects in an untreated section before treatment
Cai: the number of insects in an untreated section on observation
Tb: the number of insects in a treated section before treatment Tai: the number of insects in a treated section on observation

As a result, in a section treated with the test spray solutions of Compounds (43) and (45) of the present invention, the controlling value showed 100%.

INDUSTRIAL APPLICABILITY

The compound of the present invention has a control effect on pests, and is useful as an active ingredient of a pest control agent.

## Claims

1. An amide compound represented by formula (I)

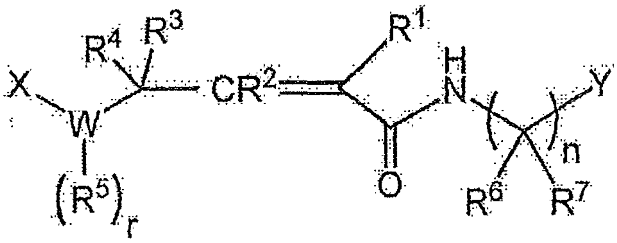

(I)

wherein

Y represents a 3 to 7-membered saturated heterocyclic ring which contains, as ring-forming component(s), one or more atoms or groups that are selected from the group consisting of an oxygen atom and $-S(O)_t-$, and the saturated heterocyclic ring may have one to three atoms or groups selected from group D,
t represents 0, 1 or 2,
X represents a C1 to C10 chain hydrocarbon group optionally having one or more atoms or groups selected from group A or a cyclopropyl group optionally having one or more groups selected from group B,
W represents $-CR^8-$, an oxygen atom or $-S(O)_u-$,
when W is $-CR^8-$, r represents 1,
when W is an oxygen atom or $-S(O)_u-$, r represents 0,
u represents 0, 1 or 2,
$R^8$ represents a C1 to C4 alkyl group optionally having one or more halogen atoms, a halogen atom or a hydrogen atom,
$R^1$ and $R^2$ are the same or different and represent a C1 to C4 alkyl group optionally having one or more halogen atoms, a C1 to C4 alkoxy group optionally having one or more halogen atoms, a halogen atom or a hydrogen atom,
$R^4$ represents a C1 to C4 alkyl group optionally having one or more halogen atoms, a halogen atom or a hydrogen atom,
$R^3$ and $R^5$ are the same or different and represent a C1 to C4 alkyl group optionally having one or more halogen atoms, a halogen atom or a hydrogen atom, or $R^3$ and $R^5$ are taken together to represent - $(CR^9R^{10})_v-$, $-NR^{11}-$, an oxygen atom or a sul fur atom,
$R^9$ and $R^{10}$ are the same or different and represent a C1 to C4 alkyl group optionally having one or more halogen atoms, a halogen atom or a hydrogen atom,
$R^{11}$ represents a C1 to C4 alkyl group optionally having one or more halogen atoms or a hydrogen atom,
v represents 1, 2, 3 or 4,
$R^6$ and $R^7$ are the same or different and represent a C1 to C4 alkyl group optionally having one or more halogen atoms or a hydrogen atom,
n represents 0, 1 or 2,
Group D: A group consisting of C1 to C4 alkyl groups optionally having one or more halogen atoms, C1 to C4 alkoxy groups optionally having one or more halogen atoms and halogen atoms.
Group A: A group consisting of C1 to C4 alkoxy groups optionally having one or more halogen atoms, C1 to C4 alkylthio groups optionally having one or more halogen atoms, C1 to C4 alkylsulfinyl groups optionally having one or more halogen atoms, C1 to C4 alkylsulfonyl groups optionally having one or more halogen atoms, C1 to C4 alkoxycarbonyl groups optionally having one or more halogen atoms, C1 to C4 alkoxyimino groups optionally having one or more halogen atoms, $-CONR^{12}R^{13}$ group, a carboxyl group and halogen atoms. $R^{12}$ and $R^{13}$ are the same or different and represent a C1 to C4 alkyl group optionally having one or more halogen atoms or a hydrogen atom.

Group B: A group consisting of C1 to C8 chain hydrocarbon groups optionally having one or more atoms or groups selected from group A.

**2.** The amide compound according to claim 1, wherein Y is represented by the following formula (II-a) or (II-b)

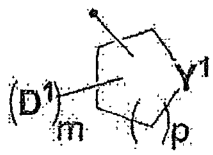 (II-a) 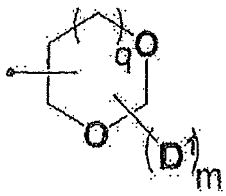 (II-b)

wherein $Y^1$ represents an oxygen atom or a sulfur atom, $D^1$ represents an atom or a group selected from group D, m represents 0 or 1, p represents 0, 1 or 2, and q is 0 or 1.

**3.** The amide compound according to claim 2, wherein p is 1, or q is 0.

**4.** The amide compound according to claim 2, wherein p is 2, or q is 1.

**5.** The amide compound according to any of claims 2 to 4, wherein $Y^1$ is an oxygen atom.

**6.** The amide compound according to claim 2, wherein Y is a group represented by the formula (II-a), and $Y^1$ is an oxygen atom.

**7.** The amide compound according to claim 2, wherein Y is a group represented by the formula (II-b).

**8.** The amide compound according to any of claims 1 to 7, wherein
W is $-CR^8-$, r is 1, $R^1$ is a halogen atom or a hydrogen atom,
$R^2$ is a C1 to C4 alkyl group optionally having one or more halogen atoms or a hydrogen atom,
$R^9$ is a C1 to C4 alkyl group optionally having one or more halogen atoms, a halogen atom or a hydrogen atom,
$R^3$ and $R^5$ are the same or different and are a C1 to C4 alkyl group optionally having one or more halogen atoms, a halogen atom or a hydrogen atom, or $R^3$ and $R^5$ are taken together to represent $-(CR^9R^{10})_v-$, v is 1, $R^9$ and $R^{10}$ are the same or different and are a C1 to C4 alkyl group optionally having one or more halogen atoms, a halogen atom or a hydrogen atom,
$R^8$ is a hydrogen atom, and
$R^6$ and $R^7$ are the same or different and are a C1 to C4 alkyl group optionally having one or more halogen atoms or a hydrogen atom.

**9.** The amide compound according to any of claims 1 to 7, wherein
W is $-CR^8-$, r is 1, $R^1$ is a halogen atom or a hydrogen atom,
$R^2$ is a C1 to C4 alkyl group optionally having one or more halogen atoms or a hydrogen atom,
$R^4$ is a C1 to C4 alkyl group optionally having one or more halogen atoms, a halogen atom or a hydrogen atom,
$R^3$ and $R^5$ are the same or different and are a C1 to C4 alkyl group optionally having one or more halogen atoms, a halogen atom or a hydrogen atom,
$R^8$ is a hydrogen atom, and
$R^6$ and $R^7$ are the same or different and are a C1 to C4 alkyl group optionally having one or more halogen atoms or a hydrogen atom.

**10.** The amide compound according to any of claims 1 to 7, wherein
W is $-CR^8-$, r is 1, $R^1$ is a halogen atom or a hydrogen atom,
$R^2$ is a C1 to C4 alkyl group optionally having one or more halogen atoms or a hydrogen atom,
$R^4$ is a C1 to C4 alkyl group optionally having one or more halogen atoms, a halogen atom or a hydrogen atom,
$R^3$ and $R^5$ are taken together to be $-(CR^9R^{10})_v-$, v is 1, $R^9$ and $R^{10}$ are the same or different and are a C1 to C4 alkyl group optionally having one or more halogen atoms, a halogen atom or a hydrogen atom,
$R^8$ is a hydrogen atom, and
$R^6$ and $R^7$ are the same or different and are a C1 to C4 alkyl group optionally having one or more halogen atoms

or a hydrogen atom.

**11.** The amide compound according to any of claims 1 to 10, wherein X is a C1 to C5 alkyl group optionally having one or more atoms or groups selected from group A, a C2 to C5 alkenyl group optionally having one or more atoms or groups selected from group A, or a C2 to C5 alkynyl group optionally having one or more atoms or groups selected from group A.

**12.** The amide compound according to any of claims 1 to 10, wherein X is a C1 to C5 alkyl group, a C2 to C5 alkenyl group or a C2 to C5 alkynyl group.

**13.** A pest control agent comprising the amide compound as defined in any of claims 1 to 12 and an inert carrier.

**14.** A method for controlling pests comprising the step of applying an effective amount of the amide compound as defined in any of claims 1 to 12 to a pest or a place where a pest inhabits.

## INTERNATIONAL SEARCH REPORT

International application No.
PCT/JP2013/053167

A. CLASSIFICATION OF SUBJECT MATTER
*C07D307/14*(2006.01)i, *A01N43/16*(2006.01)i, *A01N43/32*(2006.01)i, *A01P7/04*(2006.01)i, *C07D309/04*(2006.01)i, *C07D319/06*(2006.01)i, *C07D319/12*(2006.01)i, *C07D335/02*(2006.01)i

According to International Patent Classification (IPC) or to both national classification and IPC

B. FIELDS SEARCHED

Minimum documentation searched (classification system followed by classification symbols)
C07D307/14, A01N43/16, A01N43/32, A01P7/04, C07D309/04, C07D319/06, C07D319/12, C07D335/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2011/011415 A2 (MARRONE BIO INNOVATIONS, INC.), 27 January 2011 (27.01.2011), & US 2011/0021358 A1 & EP 2456305 A & KR 10-2012-0052333 A & JP 2013-500252 A | 1-14 |
| A | JP 59-93035 A (The Wellcome Foundation Ltd.), 29 May 1984 (29.05.1984), & US 4855086 A & US 4931469 A & GB 8315566 A & EP 111105 A2 & DE 3382048 C | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 March, 2013 (01.03.13) | 12 March, 2013 (12.03.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *The Journal of Organic Chemistry,* 1945, vol. 10, 236-242